(19) Europäisches Patentamt / European Patent Office / Office européen des brevets

(11) EP 4 628 499 A1

(12) **EUROPEAN PATENT APPLICATION**
published in accordance with Art. 153(4) EPC

(43) Date of publication:
**08.10.2025 Bulletin 2025/41**

(21) Application number: **23896904.2**

(22) Date of filing: **30.11.2023**

(51) International Patent Classification (IPC):
***C07K 7/64*** (2006.01) ***C07K 1/00*** (2006.01)
***A61P 1/00*** (2006.01) ***A61P 11/00*** (2006.01)
***A61P 17/06*** (2006.01) ***A61P 19/02*** (2006.01)
***A61P 35/00*** (2006.01) ***A61P 37/00*** (2006.01)
***A61K 38/00*** (2006.01) ***A61K 38/12*** (2006.01)

(52) Cooperative Patent Classification (CPC):
**A61K 38/00; A61K 38/12; A61P 1/00; A61P 11/00; A61P 17/06; A61P 19/02; A61P 35/00; A61P 37/00; C07K 1/00; C07K 7/64**

(86) International application number:
**PCT/CN2023/135637**

(87) International publication number:
**WO 2024/114762 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**30.11.2022 CN 202211530438**
**03.03.2023 CN 202310206299**
**15.03.2023 CN 202310261818**
**31.03.2023 CN 202310349519**
**05.09.2023 CN 202311144489**

(71) Applicants:
- **Shanghai Hansoh Biomedical Co., Ltd.**
  **Shanghai 201203 (CN)**
- **Jiangsu Hansoh Pharmaceutical Group Co., Ltd.**
  **Lianyungang, Jiangsu 222069 (CN)**

(72) Inventors:
- **DONG, Jiaqiang**
  **Shanghai 201203 (CN)**
- **YU, Wensheng**
  **Shanghai 201203 (CN)**
- **CHEN, Yun**
  **Shanghai 201203 (CN)**
- **CHENG, Bao**
  **Shanghai 201203 (CN)**
- **GONG, Zhen**
  **Shanghai 201203 (CN)**

(74) Representative: **Murgitroyd & Company**
**165-169 Scotland Street**
**Glasgow G5 8PL (GB)**

(54) **POLYPEPTIDE INHIBITOR, AND PREPARATION METHOD THEREFOR AND USE THEREOF**

(57) The present invention relates to a polypeptide inhibitor, and a preparation method therefor and the use thereof. In particular, the present invention relates to a compound represented by general formula (I), a preparation method therefor, a pharmaceutical composition containing the compound, and the use of the compound as an inhibitor in treating various inflammatory and autoimmune diseases and cancers. The definitions of substituents in general formula (I) are the same as those in the description.

EP 4 628 499 A1

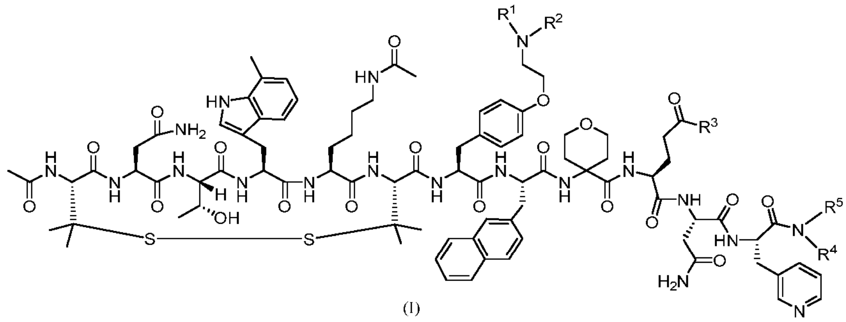

(I)

## Description

The present application claims the priority of Chinese patent application 2022115304381 filed on November 30, 2022. The above-mentioned Chinese patent application is incorporated in the present application by reference in its entirety; and

the present application claims the priority of Chinese patent application 2023102062995 filed on March 03, 2023. The above-mentioned Chinese patent application is incorporated in the present application by reference in its entirety; and the present application claims the priority of Chinese patent application 2023102618188 filed on March 15, 2023. The above-mentioned Chinese patent application is incorporated in the present application by reference in its entirety; and the present application claims the priority of Chinese patent application 202310349519X filed on March 31, 2023. The above-mentioned Chinese patent application is incorporated in the present application by reference in its entirety; and the present application claims the priority of Chinese patent application 2023111444895 filed on September 05, 2023. The above-mentioned Chinese patent application is incorporated in the present application by reference in its entirety.

## Technical Field

The present invention belongs to the field of biomedicine and particularly relates to a polypeptide inhibitor, a preparation method therefor, and the use thereof.

## Background Art

Cytokine IL23 plays an important role in human innate immunity and adaptive immunity. IL-23 induces lymphocytes to express inflammatory cytokines, and among the lymphocytes, helper T cells (TH17), innate lymphoid cells (ILCs), and $\gamma\delta$ T cells are the most significant. IL-23 heterodimeric receptor consists of two subunits, IL-23R and IL-12R$\beta$1, wherein IL-23R is a subunit unique to the IL-23 pathway. IL-12R$\beta$1 is shared by the above receptor and IL-12 receptor. Similarly, IL-23 cytokine consists of two subunits, p19 and p40, wherein p19 subunit is unique to IL-23 and p40 is shared by IL-23 and IL-12.

IL-23 provides necessary conditions for the generation and survival of Th17 cells. In addition, a large number of evidences from preclinical models and clinical practice have shown that Th17 cells play a vital role in the pathology of many autoimmune diseases, including inflammatory bowel disease, psoriasis, rheumatoid arthritis, systemic lupus erythematosus (SLE), and multiple sclerosis (MS). The evidences from preclinical models and clinical practice have shown that blocking IL23 signal transduction is effective in the treatment of autoimmune diseases. However, the nature of the shared ligand and receptor subunit between the IL-23 and IL-12 pathways means more complicated biological processes, and the data of tumorigenesis, infection susceptibility, autoimmune disorder, etc., have shown that IL-23 blocking seems to have therapeutic advantages in effect and safety over IL-12 blocking.

The limitations of antibody therapies include a high production cost, a relatively poor stability, and higher requirements for transportation, storage, use and production conditions, as well as generally the need for infusion or injection during treatment, which is challenging for patient compliance. Antibody immunosuppressive therapies are usually systemic, and tuberculosis patients who have received such therapies may face the risk of recurrence and other serious infections. Therefore, a relatively high proportion of patients with latent tuberculosis or hepatitis B (HBV) have to be excluded when anti-TNF or anti-IL23 therapies are carried out in many developing countries. Systemic antibody therapies have such a long half-life that anti-drug antibodies (ADAs) are produced to neutralize antibody drugs and cause reduced curative effects. Intermittent doses of anti-TNF antibodies greatly increase the possibility of the development of ADAs, which may increase risks in patients during treatment.

Therefore, the objects of the present invention are as follows: molecules that specifically block IL-23 heterodimeric receptor by blocking IL-23R, a composition comprising these molecules, a method for screening these molecules, and a method of using these molecules to treat various inflammatory and autoimmune diseases and cancers. These molecules should have good affinity and specificity and be distributed in inflammatory diseased tissues, such that the systemic immunity cannot be significantly affected.

## Summary of the Invention

An object of the present invention is to provide a compound as represented by general formula (I-A) or a stereoisomer or pharmaceutically acceptable salt thereof, wherein the compound as represented by general formula (I-A) has the following structure:

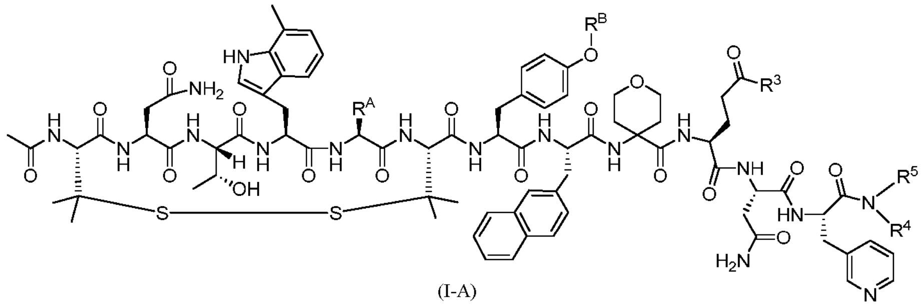

(I-A)

wherein:

$R^A$ is $-(CH_2)_{mA}-R^{AA}$;
$R^{AA}$ is selected from $-NH_2$, $-N^+H_3$, $-N^+(CH_3)_3$, or $-NH-C(O)-CH_3$;
$m^A$ is selected from integers of 1-6;
preferably, $R^A$ is selected from

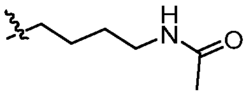 or 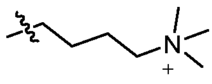 ;

$R^B$ is $-(CH_2)_{mB}-R^{BB}$;
$R^{BB}$ is selected from $-NR^1R^2$, $-N^+(R^1)_2R^2$, or $-NH-C(O)-R^1$;
$R^1$ is selected from H, alkyl,

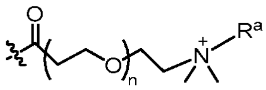

or an amino acid, preferably, $R^1$ is selected from H and alkyl;
$R^2$ is selected from H, alkyl,

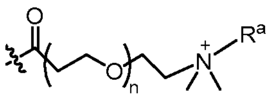

or an amino acid;
$R^a$ is selected from H, alkyl,

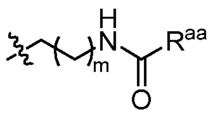

or an amino acid;
$R^{aa}$ is selected from alkyl,

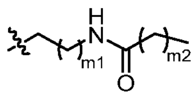

,

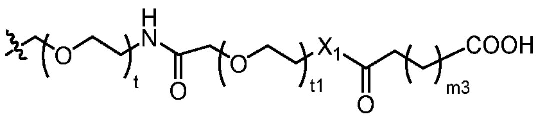

,

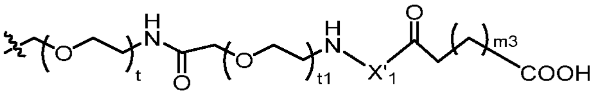

or an amino acid;

$X_1$ is an amino acid;

$X'_1$ is an amino acid;

preferably, $R^{BB}$ is selected from $-NH_2$, $-N^+H_3$, $-N^+(CH_3)_3$, or $-NH-C(O)-CH_3$;

$m^B$ is selected from integers of 1-6;

preferably, $R^B$ is selected from

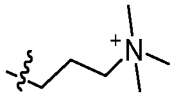
,
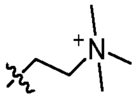
or
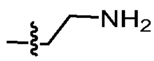
;

$R^3$ is selected from hydroxyl, alkyl, $-NR^6R^7$ or an amino acid;

$R^6$ is selected from H, alkyl, $-CH_2C(O)NH_2$,

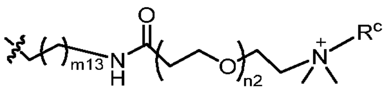
,

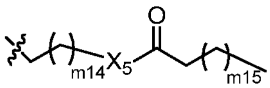
,
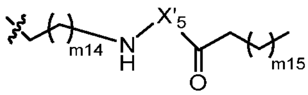
,

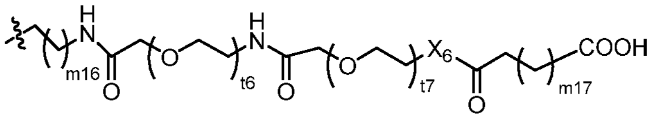
,

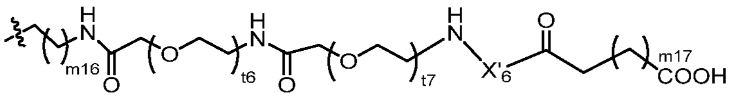

or an amino acid;

$R^7$ is selected from H, alkyl, $-CH_2C(O)NH_2$,

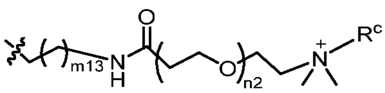
,

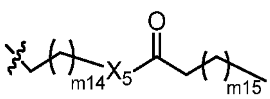
,
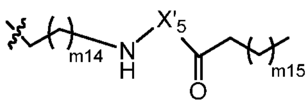
,

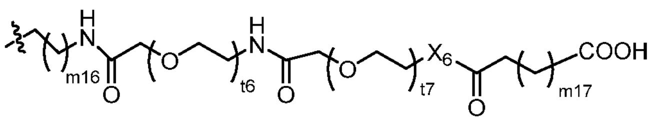
,

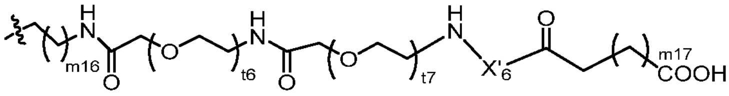

or an amino acid;
$R^c$ is selected from H, alkyl,

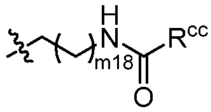

or an amino acid;
$R^{cc}$ is selected from alkyl,

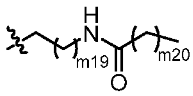
,

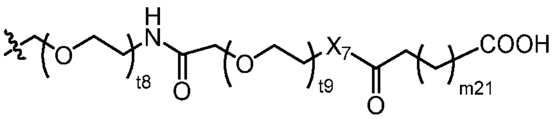
,

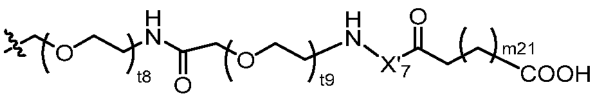

or an amino acid;
$X_5$ is an amino acid;
$X'_5$ is an amino acid;
$X_6$ is an amino acid;
$X'_6$ is an amino acid;
$X_7$ is an amino acid;
$X'_7$ is an amino acid;
$R^4$ is selected from H, alkyl, $-CH_2C(O)NH_2$,

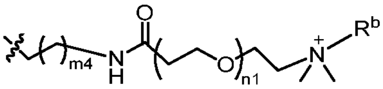
,

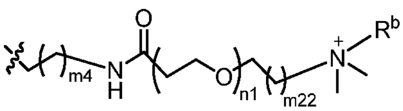
,

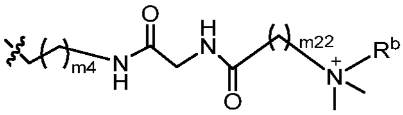
,

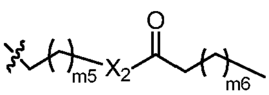
,

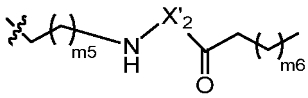
,

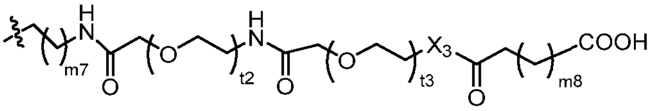

,

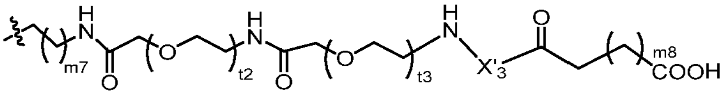

or an amino acid;

$R^5$ is selected from H, alkyl, $-CH_2C(O)NH_2$,

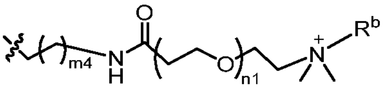

,

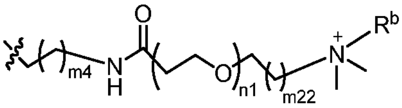

,

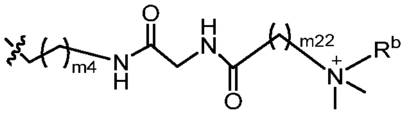

,

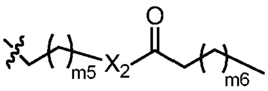

,

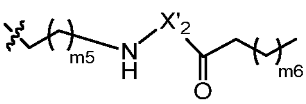

,

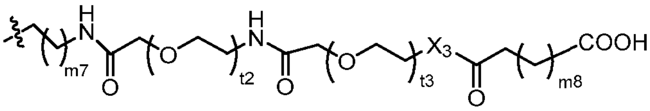

,

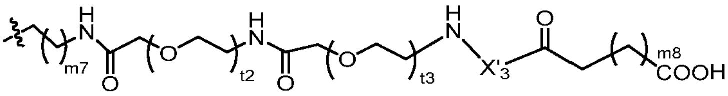

or an amino acid; preferably from

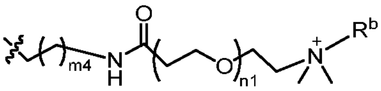

,

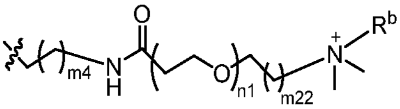

,

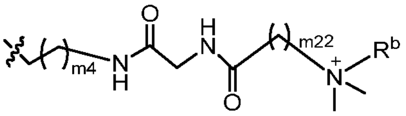

,

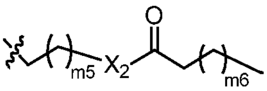

,

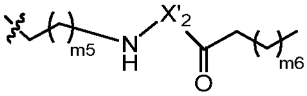

,

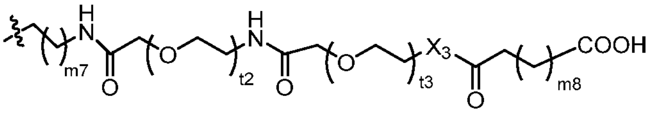

or

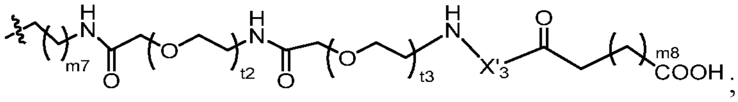

$R^b$ is selected from H, alkyl,

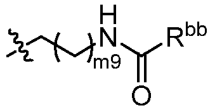

or an amino acid, wherein the alkyl can be further substituted with hydroxyl or alkoxy;
$R^{bb}$ is selected from alkyl,

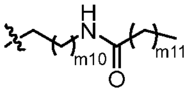

,

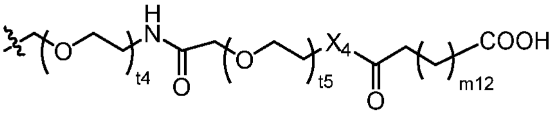

,

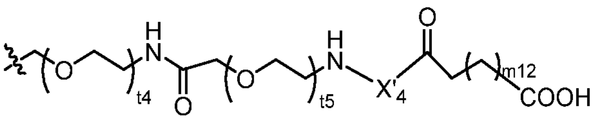

or an amino acid;
$X_2$ is an amino acid;
$X'_2$ is an amino acid;
$X_3$ is an amino acid;
$X'_3$ is an amino acid;
$X_4$ is an amino acid;
$X'_4$ is an amino acid;
n1-n2 are each independently selected from integers of 0-12;
t2-t9 are each independently selected from integers of 0-12;
m4-m22 are each independently selected from integers of 0-24.

An object of the present invention is to provide a compound as represented by general formula (I) or a stereoisomer or pharmaceutically acceptable salt thereof, wherein the compound as represented by general formula (I) has the following structure:

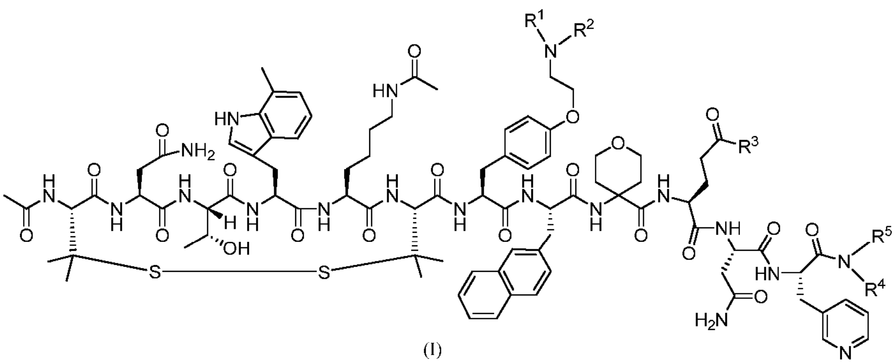

(I)

wherein:

$R^1$ and $R^2$ are each independently selected from H, alkyl,

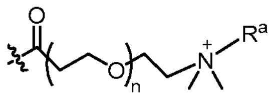

or an amino acid;
$R^a$ is selected from H, alkyl,

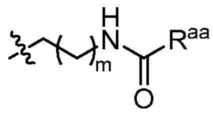

or an amino acid;
$R^{aa}$ is selected from alkyl,

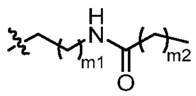

,

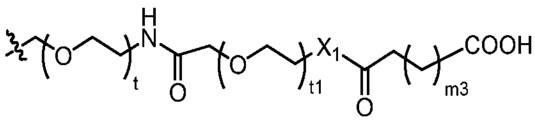

or an amino acid;
$X_1$ is an amino acid;
preferably, $R^{aa}$ is selected from alkyl,

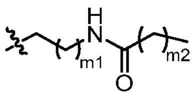

,

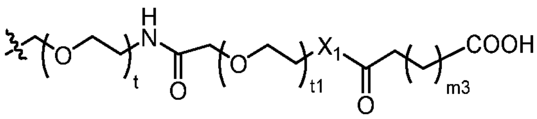

,

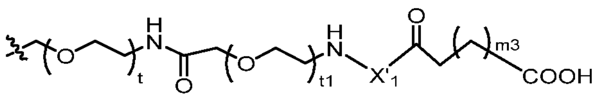

or an amino acid;
$X_1$ is an amino acid;
$X'_1$ is an amino acid;
$R^3$ is selected from hydroxyl, alkyl, $-NR^6R^7$ or an amino acid;
$R^6$ and $R^7$ are each independently selected from H, alkyl, $-CH_2C(O)NH_2$,

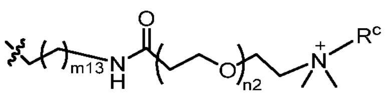

,

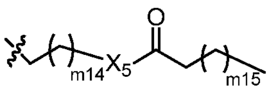

,

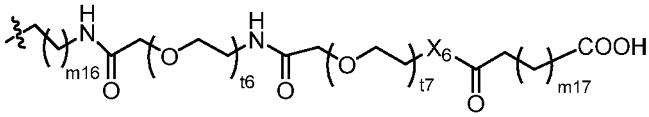

or an amino acid;
$R^c$ is selected from H, alkyl,

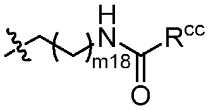

or an amino acid;
$R^{cc}$ is selected from alkyl,

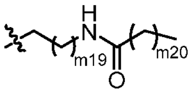

,

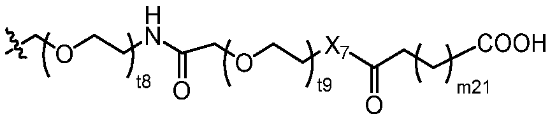

or an amino acid;
$X_5$ is an amino acid;
$X_6$ is an amino acid;
$X_7$ is an amino acid;
preferably, $R^3$ is selected from hydroxyl, alkyl, $-NR^6R^7$ or an amino acid;
$R^6$ is selected from H, alkyl, $-CH_2C(O)NH_2$,

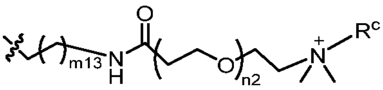

,

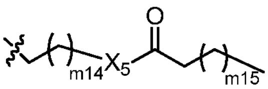

,

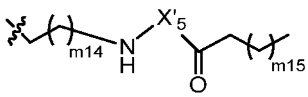

,

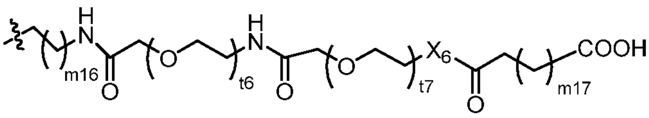

,

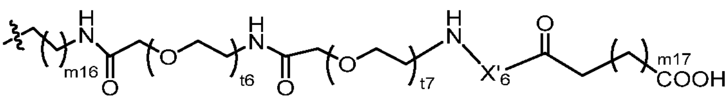

or an amino acid;
$R^7$ is selected from H, alkyl, $-CH_2C(O)NH_2$,

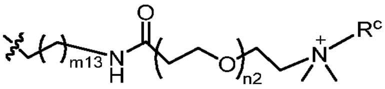

,

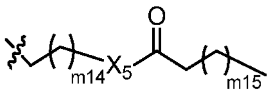 , 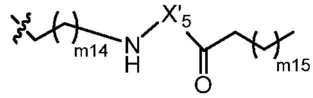 ,

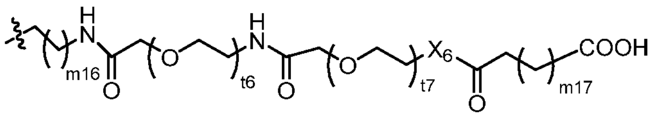 ,

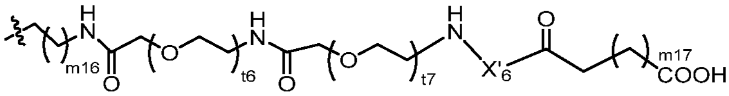

or an amino acid;
$R^c$ is selected from H, alkyl,

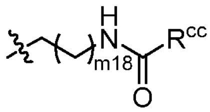

or an amino acid;
$R^{cc}$ is selected from alkyl,

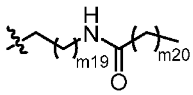 ,

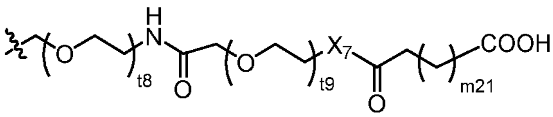 ,

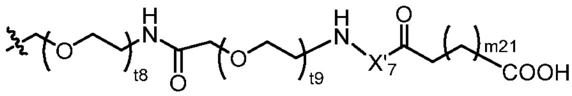

or an amino acid;
$X_5$ is an amino acid;
$X'_5$ is an amino acid;
$X_6$ is an amino acid;
$X'_6$ is an amino acid;
$X_7$ is an amino acid;
$X'_7$ is an amino acid;
$R^4$ and $R^5$ are each independently selected from H, alkyl, $-CH_2C(O)NH_2$,

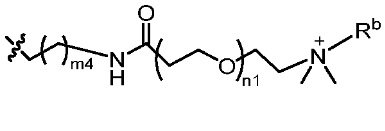 , 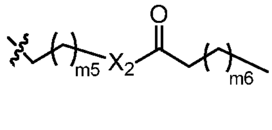 ,

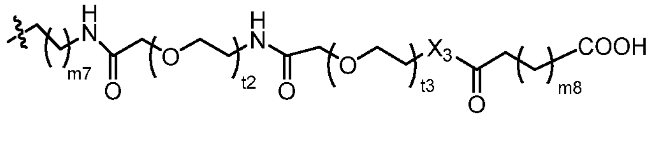

or an amino acid;

$R^b$ is selected from H, alkyl,

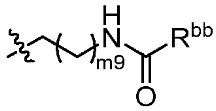

or an amino acid;

$R^{bb}$ is selected from alkyl,

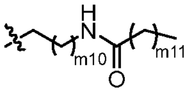

,

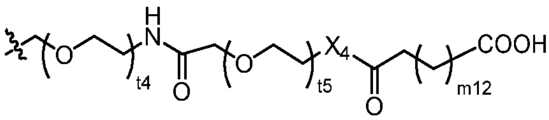

or an amino acid;

$X_2$ is an amino acid;

$X_3$ is an amino acid;

$X_4$ is an amino acid;

preferably, $R^4$ is selected from H, alkyl, $-CH_2C(O)NH_2$,

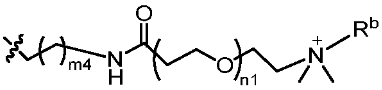

,

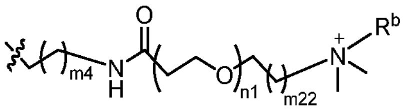

,

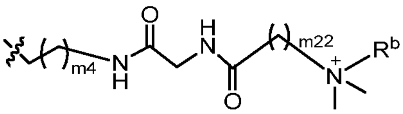

,

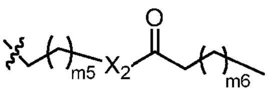

,

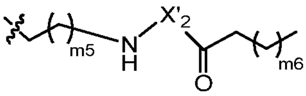

,

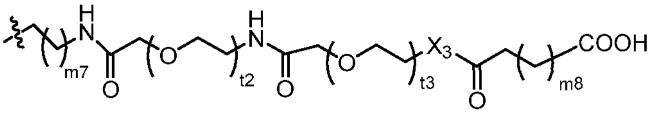

,

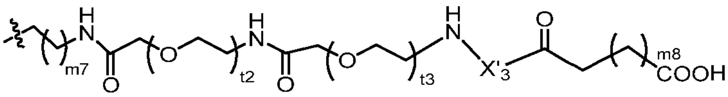

or an amino acid;

$R^5$ is selected from H, alkyl, $-CH_2C(O)NH_2$,

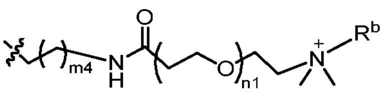

,

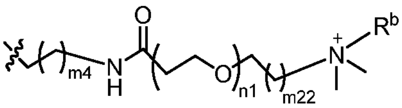 , 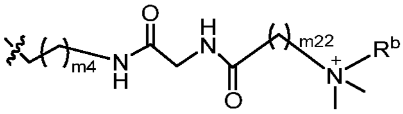 ,

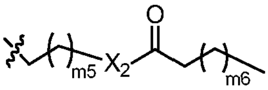 , 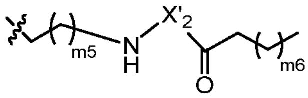 ,

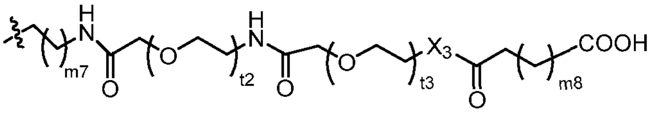 ,

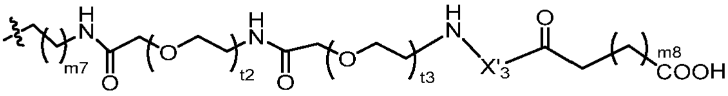

or an amino acid; preferably from

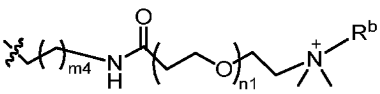 , 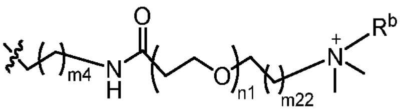 ,

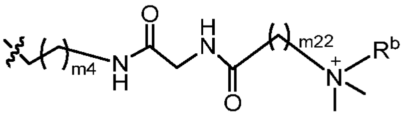 , 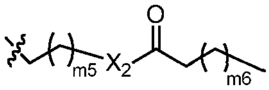 , 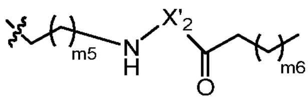 ,

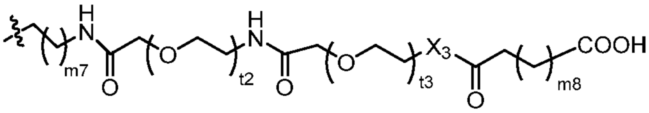 or

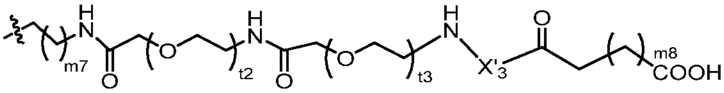 ,

$R^b$ is selected from H, alkyl,

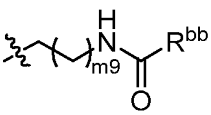

or an amino acid, wherein the alkyl can be further substituted with hydroxyl or alkoxy;
$R^{bb}$ is selected from alkyl,

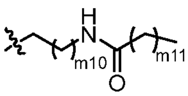 ,

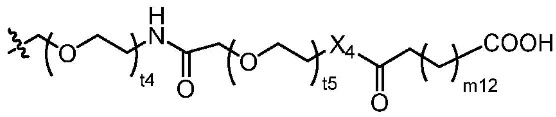

,

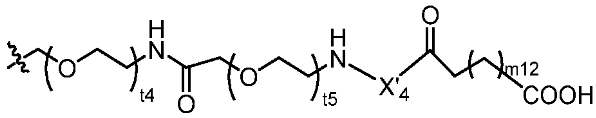

or an amino acid;

$X_2$ is an amino acid;

$X'_2$ is an amino acid;

$X_3$ is an amino acid;

$X'_3$ is an amino acid;

$X_4$ is an amino acid;

$X'_4$ is an amino acid;

n-n2 are each independently selected from integers of 0-12;

t-t9 are each independently selected from integers of 0-12;

m-m21 are each independently selected from integers of 0-24;

m22 is selected from integers of 0-24; and

the compound is not

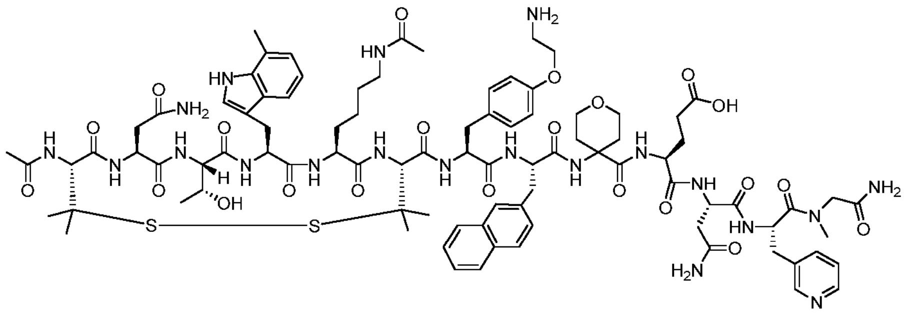

.

Another object of the present invention is to provide a compound as represented by general formula (I-V) or a stereoisomer or pharmaceutically acceptable salt thereof, wherein the compound as represented by general formula (I-V) has the following structure:

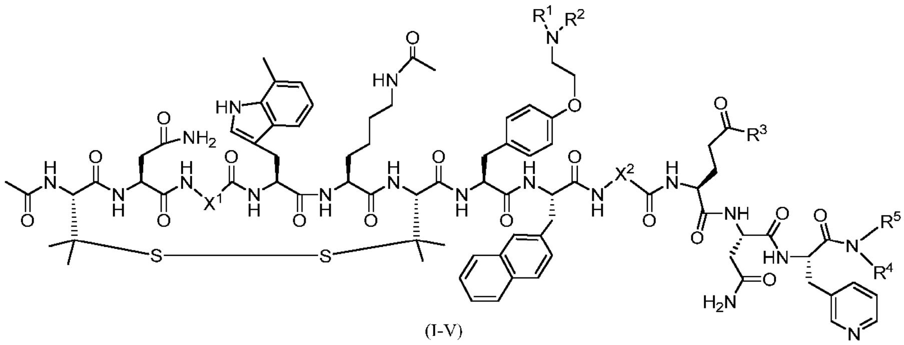

(I-V)

wherein:

$X^1$ is an amino acid;

$X^2$ is an amino acid;

$R^1$ and $R^2$ are each independently selected from H, alkyl,

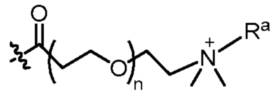

or an amino acid;

$R^a$ is selected from H, alkyl,

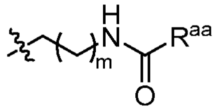

or an amino acid;

$R^{aa}$ is selected from alkyl,

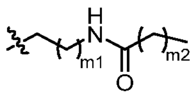

,

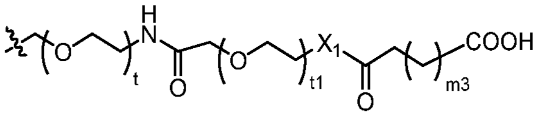

,

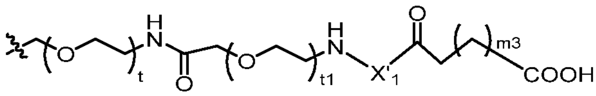

or an amino acid;

$X_1$ is an amino acid;

$X'_1$ is an amino acid;

$R^3$ is selected from hydroxyl, alkyl, $-NR^6R^7$ or an amino acid;

$R^6$ is selected from H, alkyl, $-CH_2C(O)NH_2$,

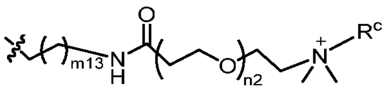

,

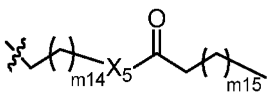

,

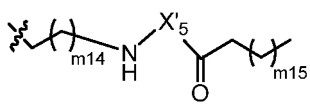

,

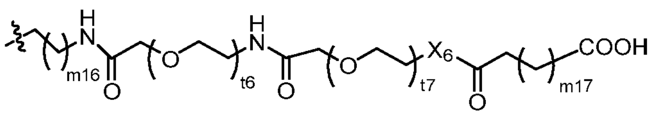

,

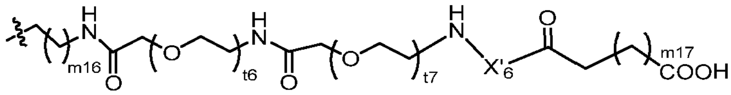

or an amino acid;

$R^7$ is selected from H, alkyl, $-CH_2C(O)NH_2$,

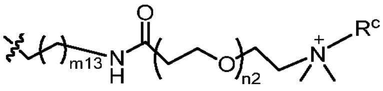

,

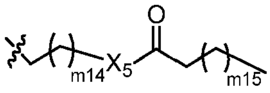

,

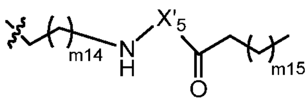

,

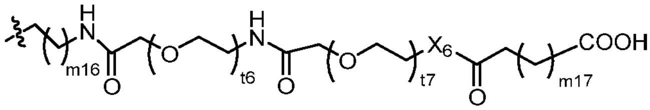

,

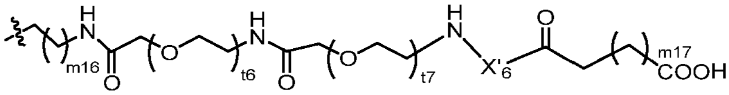

or an amino acid;
$R^c$ is selected from H, alkyl,

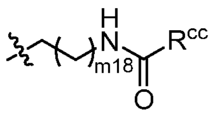

or an amino acid;
$R^{cc}$ is selected from alkyl,

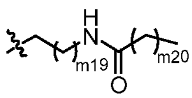

,

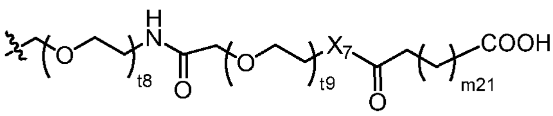

,

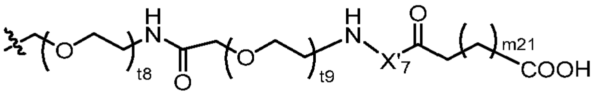

or an amino acid;
$X_5$ is an amino acid;
$X'_5$ is an amino acid;
$X_6$ is an amino acid;
$X'_6$ is an amino acid;
$X_7$ is an amino acid;
$X'_7$ is an amino acid;
$R^4$ is selected from H, alkyl, $-CH_2C(O)NH_2$,

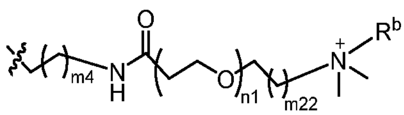

,

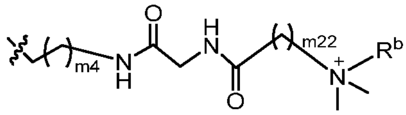 , 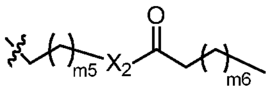 , 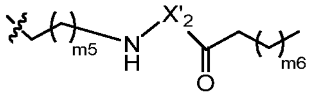 ,

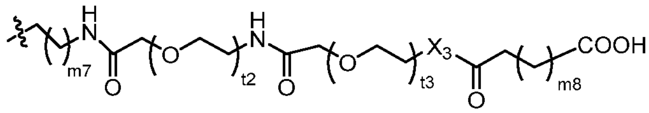

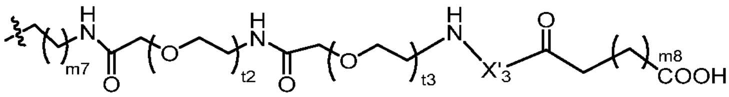

, or an amino acid;

$R^5$ is selected from H, alkyl, $-CH_2C(O)NH_2$,

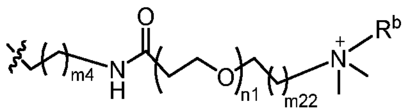 ,

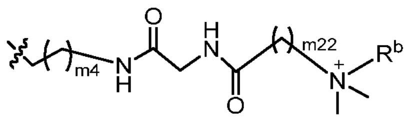 , 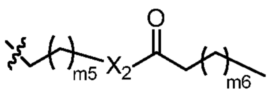 , 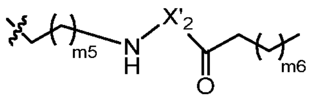 ,

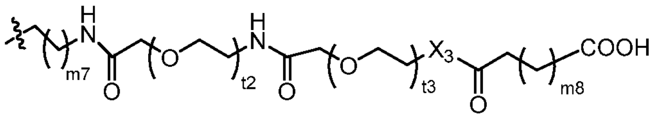 ,

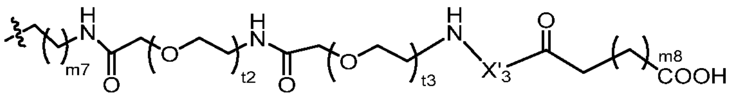

or an amino acid;

$R^b$ is selected from H, alkyl,

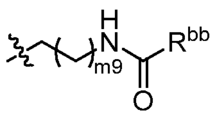

or an amino acid, wherein the alkyl can be further substituted with hydroxyl or alkoxy;

$R^{bb}$ is selected from alkyl,

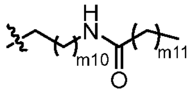 ,

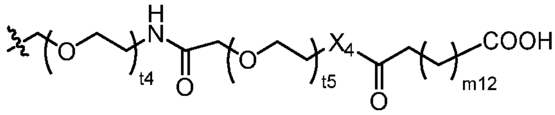 ,

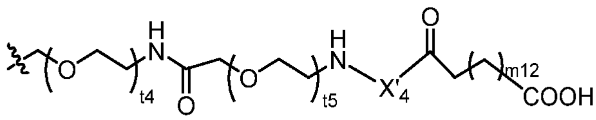

or an amino acid;

$X_2$ is an amino acid;

$X'_2$ is an amino acid;

$X_3$ is an amino acid;

$X'_3$ is an amino acid;

$X_4$ is an amino acid;

$X'_4$ is an amino acid;

n-n2 are each independently selected from integers of 0-12;

t-t9 are each independently selected from integers of 0-12;

m-m21 are each independently selected from integers of 0-24;

m22 is selected from integers of 0-24; and

the compound is not

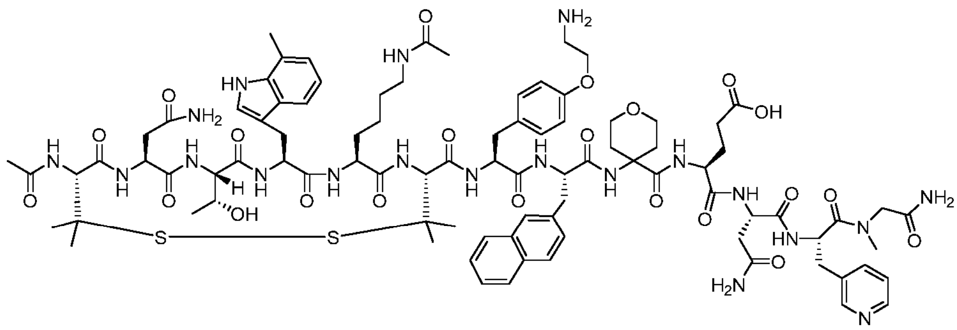

.

In a preferred embodiment of the present invention, in the compound or the stereoisomer or pharmaceutically acceptable salt thereof, $R^1$ and $R^2$ are each independently selected from H, alkyl,

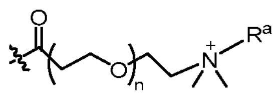

or an amino acid;

$R^a$ is selected from H, alkyl,

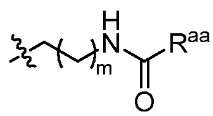

or an amino acid;

$R^{aa}$ is selected from alkyl,

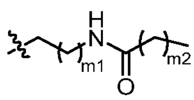

,

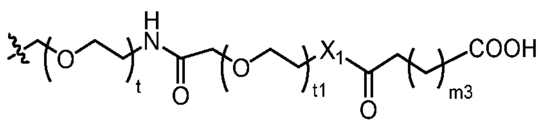

or an amino acid;
$X_1$ is an amino acid;
preferably, $R^{aa}$ is selected from alkyl,

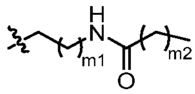

,

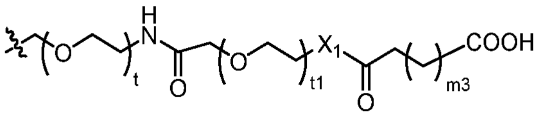

,

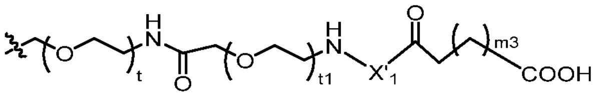

or an amino acid;
$X_1$ is an amino acid;
$X'_1$ is an amino acid;
$R^3$ is hydroxyl;
$R^4$ and $R^5$ are each independently selected from H, alkyl and $-CH_2C(O)NH_2$;
n is selected from integers of 0-12;
t-t1 are each independently selected from integers of 0-12; and
m-m3 are each independently selected from integers of 0-24.

In a preferred embodiment of the present invention, in the compound or the stereoisomer or pharmaceutically acceptable salt thereof, $R^1$ and $R^2$ are H;

$R^3$ is selected from hydroxyl, alkyl, $-NR^6R^7$ or an amino acid;
$R^6$ and $R^7$ are each independently selected from H, alkyl, $-CH_2C(O)NH_2$,

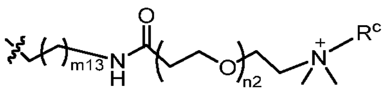

,

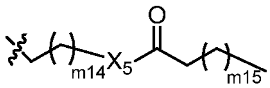

,

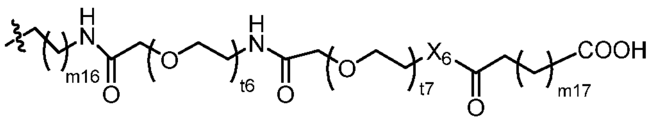

or an amino acid;
$R^c$ is selected from H, alkyl,

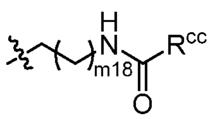

or an amino acid;
$R^{cc}$ is selected from alkyl,

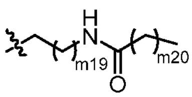

,

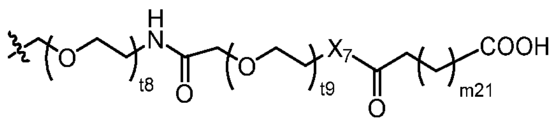

or an amino acid;
$X_5$ is an amino acid;
$X_6$ is an amino acid;
$X_7$ is an amino acid;
preferably, $R^3$ is selected from hydroxyl, alkyl, $-NR^6R^7$ or an amino acid;
$R^6$ is selected from H, alkyl, $-CH_2C(O)NH_2$,

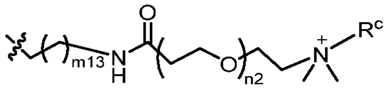
,
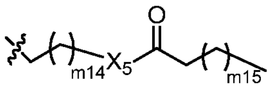
,
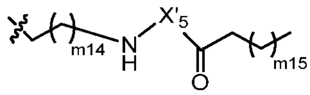
,
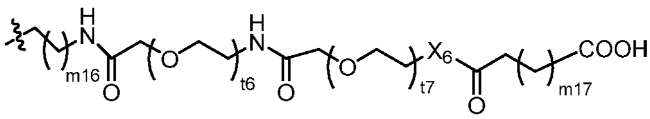
,
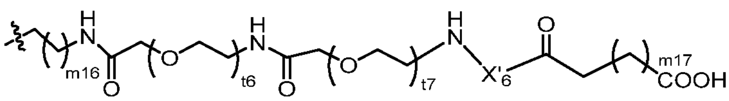

or an amino acid;
$R^7$ is selected from H, alkyl, $-CH_2C(O)NH_2$,

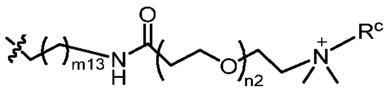
,
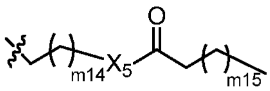
,
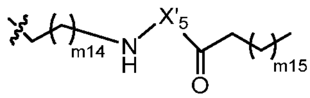
,
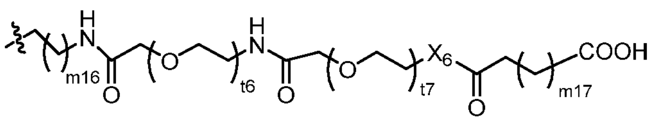
,
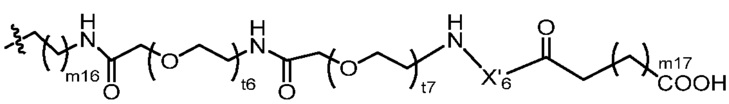

or an amino acid;
$R^c$ is selected from H, alkyl,

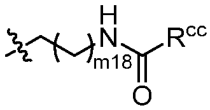

or an amino acid;
$R^{cc}$ is selected from alkyl,

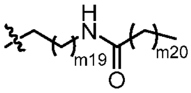

,

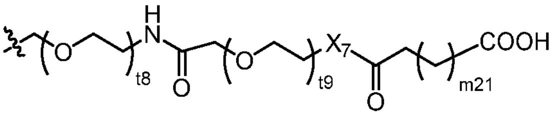

,

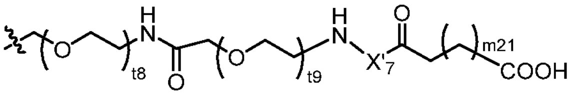

or an amino acid;
$X_5$ is an amino acid;
$X'_5$ is an amino acid;
$X_6$ is an amino acid;
$X'_6$ is an amino acid;
$X_7$ is an amino acid;
$X'_7$ is an amino acid;
$R^4$ and $R^5$ are each independently selected from H, alkyl and $-CH_2C(O)NH_2$;
n2 is selected from integers of 0-12;
t6-t9 are each independently selected from integers of 0-12; and
m13-m21 are each independently selected from integers of 0-24.

In a preferred embodiment of the present invention, in the compound or the stereoisomer or pharmaceutically acceptable salt thereof, $R^1$ and $R^2$ are H;

$R^3$ is hydroxyl;
$R^4$ and $R^5$ are each independently selected from H, alkyl, $-CH_2C(O)NH_2$,

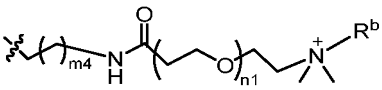

,

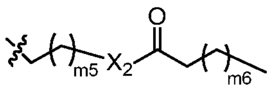

,

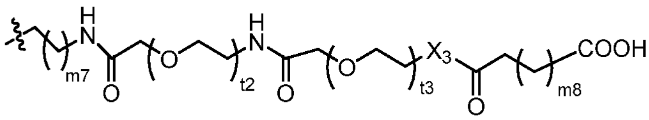

or an amino acid;
$R^b$ is selected from H, alkyl,

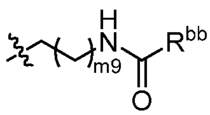

or an amino acid;

$R^{bb}$ is selected from alkyl,

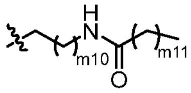,

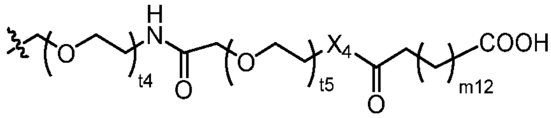

or an amino acid;
$X_2$ is an amino acid;
$X_3$ is an amino acid;
$X_4$ is an amino acid;
preferably, $R^4$ is selected from H, alkyl, $-CH_2C(O)NH_2$,

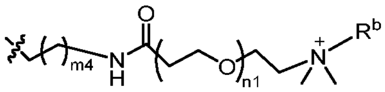, 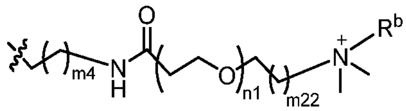,

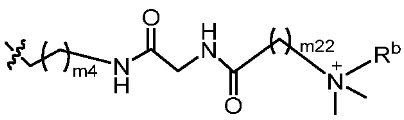, 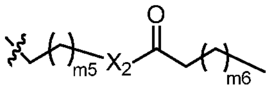, 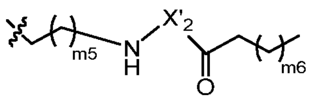,

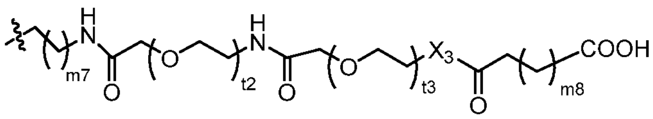,

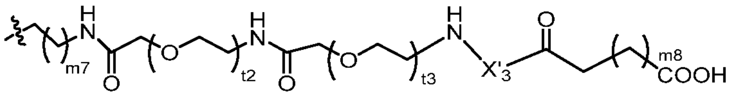

or an amino acid;
$R^5$ is selected from H, alkyl, $-CH_2C(O)NH_2$,

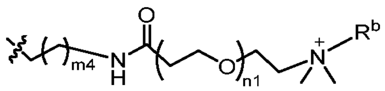,

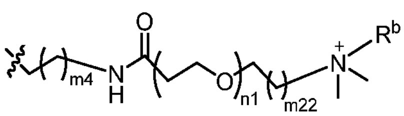, 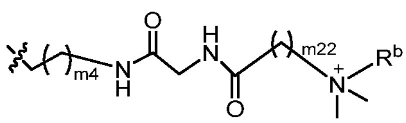,

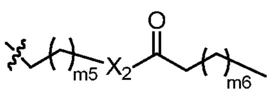, 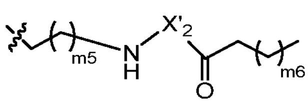,

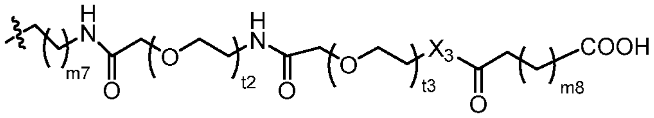

,

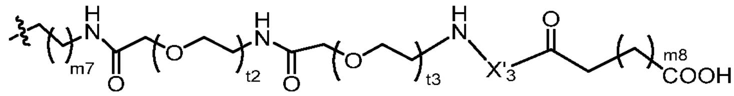

or an amino acid; preferably from

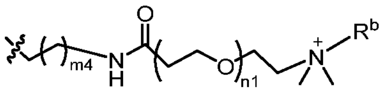

,

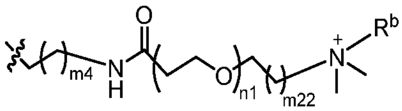

,

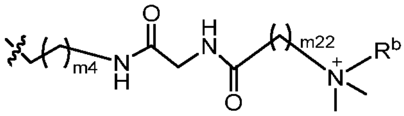

,

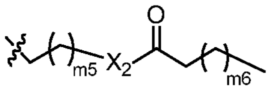

,

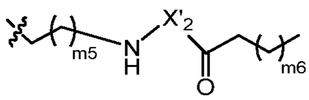

,

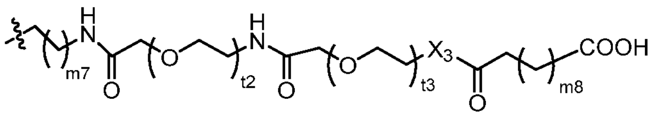

or

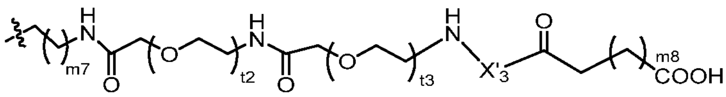

,

$R^b$ is selected from H, alkyl,

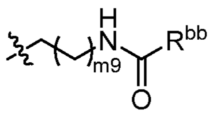

or an amino acid, wherein the alkyl can be further substituted with hydroxyl or alkoxy;
$R^{bb}$ is selected from alkyl,

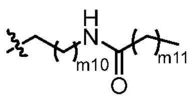

,

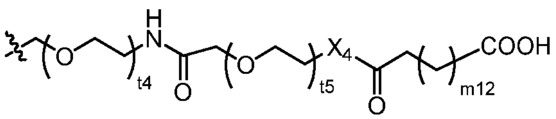

,

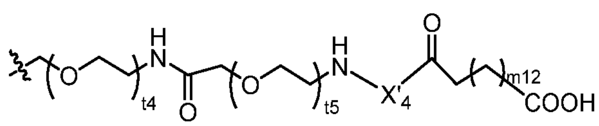

or an amino acid;
$X_2$ is an amino acid;

$X'_2$ is an amino acid;
$X_3$ is an amino acid;
$X'_3$ is an amino acid;
$X_4$ is an amino acid;
$X'_4$ is an amino acid;
n1 is selected from integers of 0-12;
t2-t5 are each independently selected from integers of 0-12;
m4-m12 are each independently selected from integers of 0-24; and
m22 is selected from integers of 0-24.

In a preferred embodiment of the present invention, in the compound or the stereoisomer or pharmaceutically acceptable salt thereof, the compound is further as represented by general formula (II):

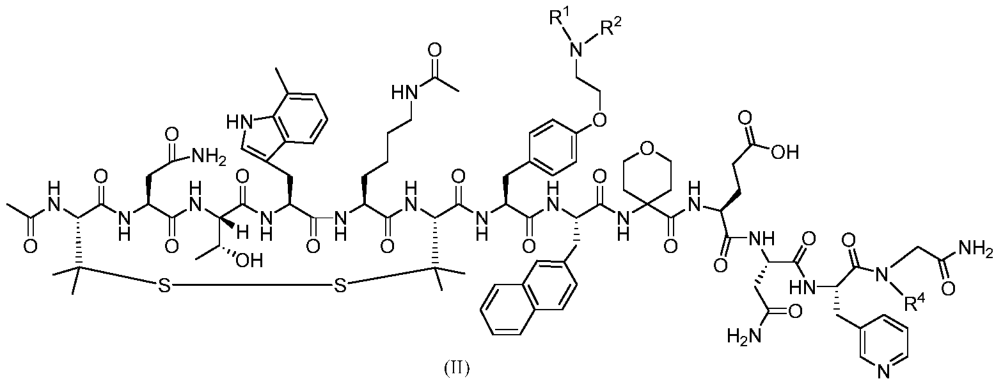

(II)

wherein:

$R^1$ and $R^2$ are each independently selected from H, alkyl,

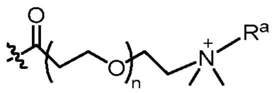

or an amino acid;
$R^a$ is selected from H, alkyl,

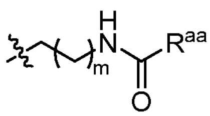

or an amino acid;
$R^{aa}$ is selected from alkyl,

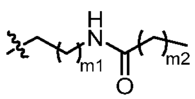

,

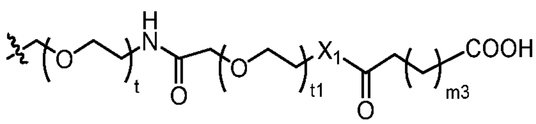

or an amino acid;
$X_1$ is an amino acid;
preferably, $R^{aa}$ is selected from alkyl,

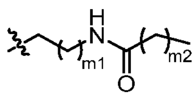

,

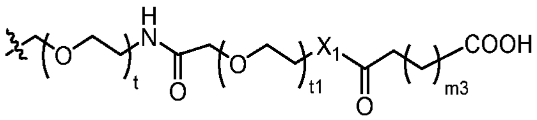

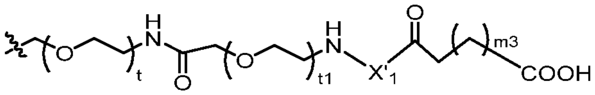

or an amino acid;

$X_1$ is an amino acid;

$X'_1$ is an amino acid;

$R^4$ is selected from H or alkyl;

n is selected from integers of 0-12;

t-t1 are each independently selected from integers of 0-12; and

m-m3 are each independently selected from integers of 0-24.

In a preferred embodiment of the present invention, in the compound or the stereoisomer or pharmaceutically acceptable salt thereof, the compound is further as represented by general formula (III):

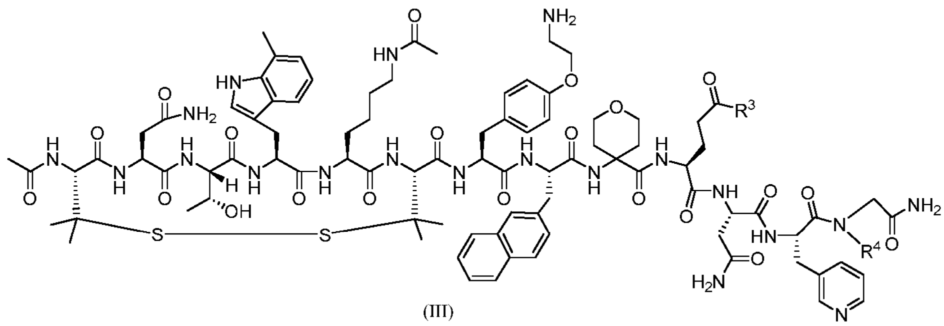

(III)

wherein:

$R^3$ is selected from hydroxyl, alkyl, $-NR^6R^7$ or an amino acid;

$R^6$ and $R^7$ are each independently selected from H, alkyl, $-CH_2C(O)NH_2$,

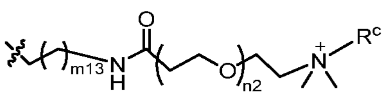

,

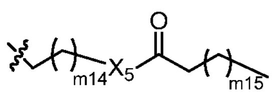

,

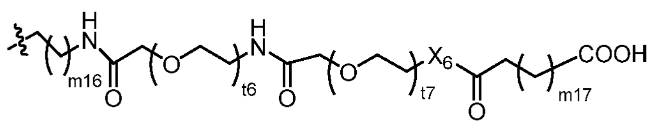

or an amino acid;

$R^c$ is selected from H, alkyl,

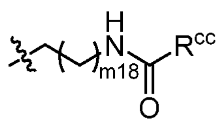

or an amino acid;
$R^{cc}$ is selected from alkyl,

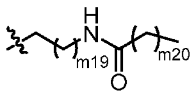

,

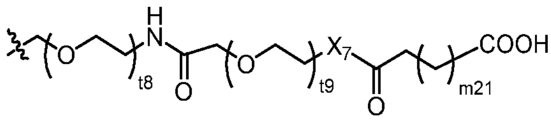

or an amino acid;
$X_5$ is an amino acid;
$X_6$ is an amino acid;
$X_7$ is an amino acid;
preferably, $R^3$ is selected from hydroxyl, alkyl, $-NR^6R^7$ or an amino acid;
$R^6$ is selected from H, alkyl, $-CH_2C(O)NH_2$,

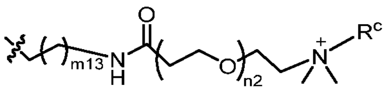

,

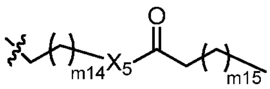

,

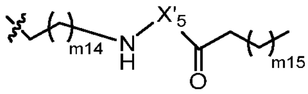

,

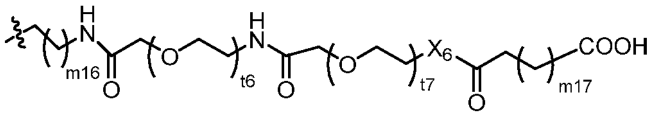

,

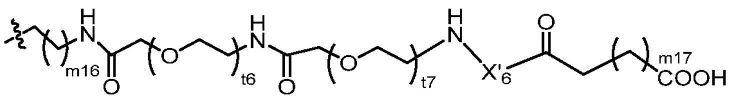

or an amino acid;
$R^7$ is selected from H, alkyl, $-CH_2C(O)NH_2$,

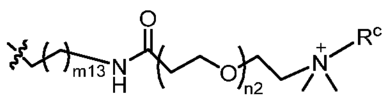

,

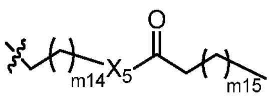

,

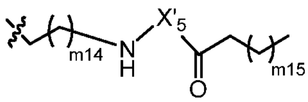

,

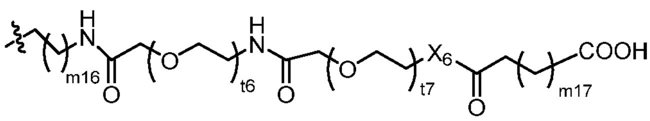

,

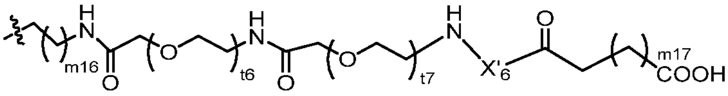

or an amino acid;

$R^c$ is selected from H, alkyl,

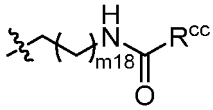

or an amino acid;

$R^{cc}$ is selected from alkyl,

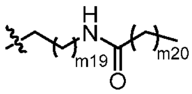

,

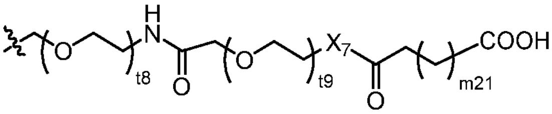

,

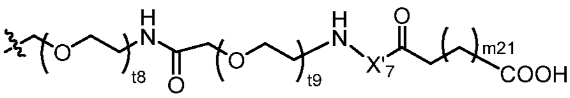

or an amino acid;

$X_5$ is an amino acid;

$X'_5$ is an amino acid;

$X_6$ is an amino acid;

$X'_6$ is an amino acid;

$X_7$ is an amino acid;

$X'_7$ is an amino acid;

$R^4$ is selected from H or alkyl;

n2 is selected from integers of 0-12;

t6-t9 are each independently selected from integers of 0-12; and

m13-m21 are each independently selected from integers of 0-24.

In a preferred embodiment of the present invention, in the compound or the stereoisomer or pharmaceutically acceptable salt thereof, the compound is further as represented by general formula (V):

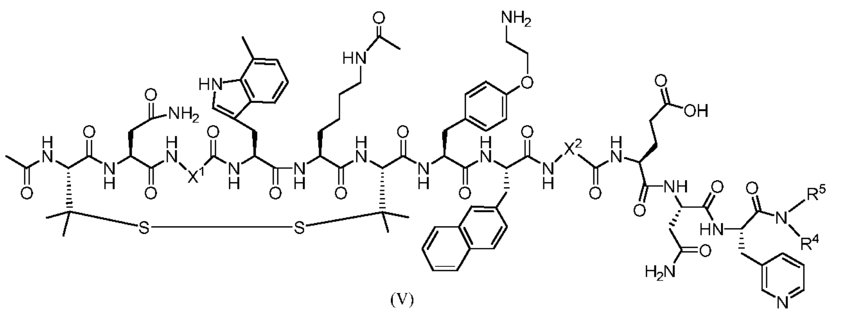

(V)

wherein:

$X^1$ is an amino acid;

$X^2$ is an amino acid;

$R^4$ is selected from H, alkyl, $-CH_2C(O)NH_2$,

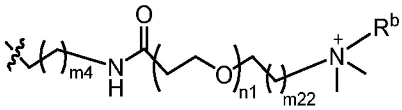

,

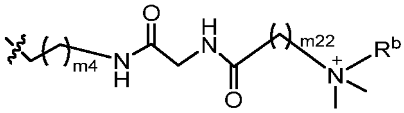

,

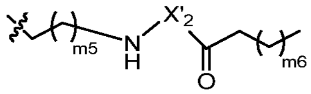

,

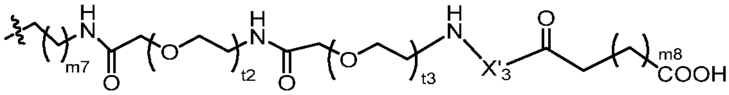

or an amino acid;

$R^5$ is selected from H, alkyl, $-CH_2C(O)NH_2$,

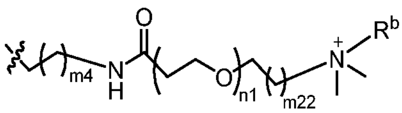

,

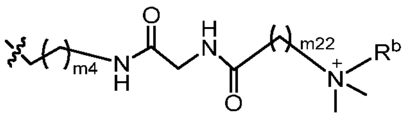

,

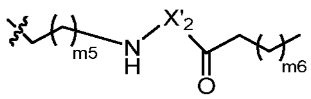

,

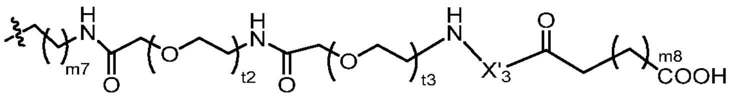

or an amino acid;

$R^b$ is selected from H, alkyl,

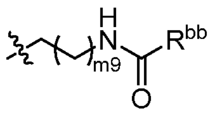

or an amino acid, wherein the alkyl can be further substituted with hydroxyl or alkoxy;

$R^{bb}$ is selected from alkyl,

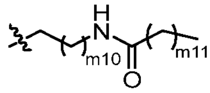

,

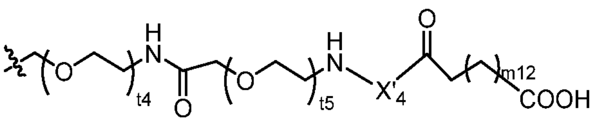

or an amino acid;

$X'_2$ is an amino acid;
$X'_3$ is an amino acid;
$X_4$ is an amino acid;
n1 is selected from integers of 0-12;
t2-t5 are each independently selected from integers of 0-12;
m4-m12 are each independently selected from integers of 0-24;
m22 is selected from integers of 0-12; and
the compound is not

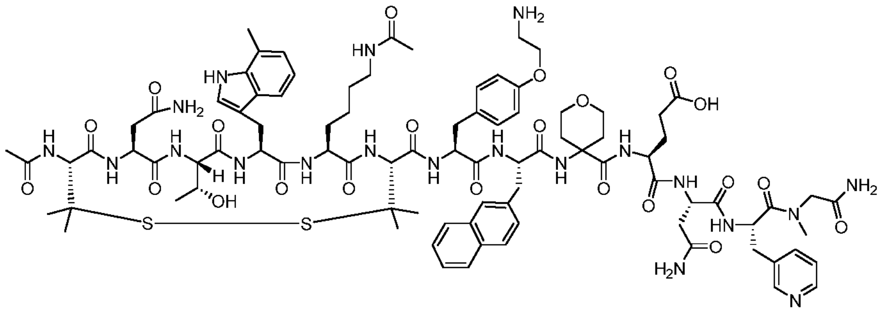.

In a preferred embodiment of the present invention, in the compound or the stereoisomer or pharmaceutically acceptable salt thereof, $X^1$ is selected from Thr,

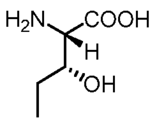, 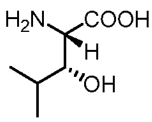 or 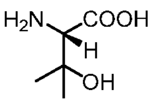;

preferably

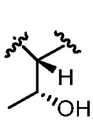, 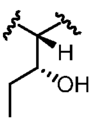, 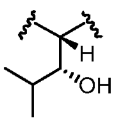 or

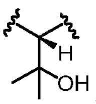.

In a preferred embodiment of the present invention, in the compound or the stereoisomer or pharmaceutically acceptable salt thereof, $X^2$ is selected from

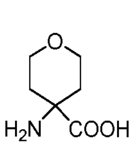, 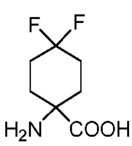, 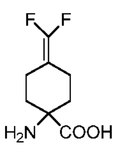, 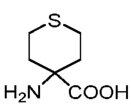, 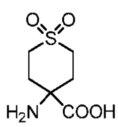 or 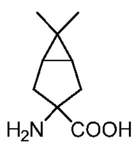 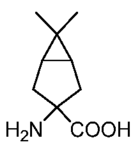;

preferably

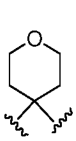, 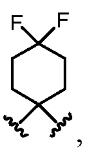, 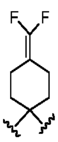, 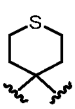, 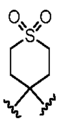 or 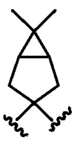.

In a preferred embodiment of the present invention, in the compound or the stereoisomer or pharmaceutically

acceptable salt thereof, the compound is further as represented by general formula (IV):

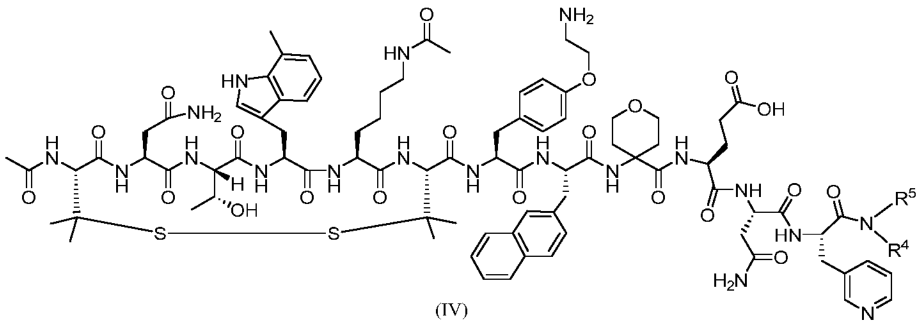

(IV)

wherein:

$R^4$ is selected from H or alkyl;
$R^5$ is selected from alkyl, $-CH_2C(O)NH_2$,

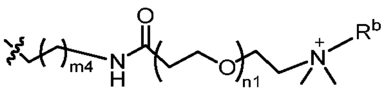

,

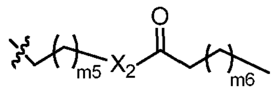

,

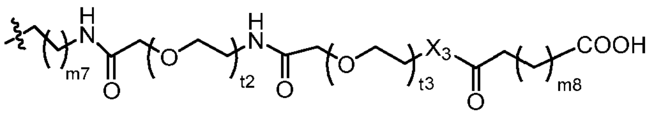

or an amino acid;
$R^b$ is selected from H, alkyl,

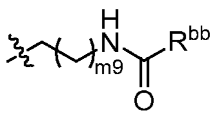

or an amino acid;
$R^{bb}$ is selected from alkyl,

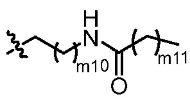

,

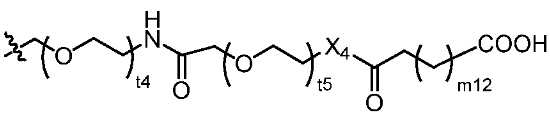

or an amino acid;
$X_2$ is an amino acid;
$X_3$ is an amino acid;
$X_4$ is an amino acid;
preferably, $R^4$ is selected from H, alkyl, $-CH_2C(O)NH_2$,

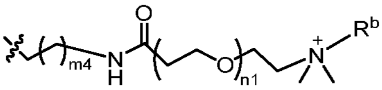
,
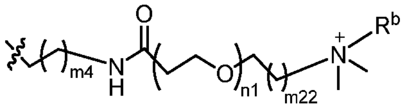
,

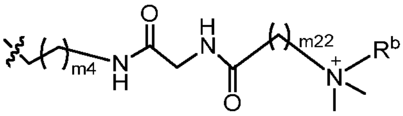
,
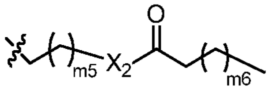
,
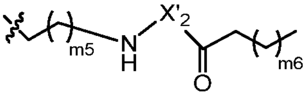
,

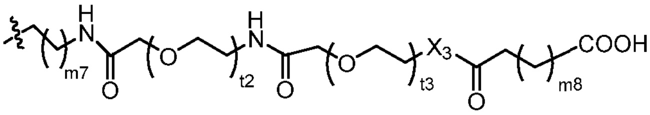
,

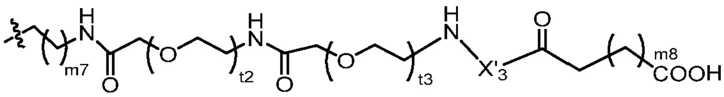

or an amino acid;
$R^5$ is selected from H, alkyl, $-CH_2C(O)NH_2$,

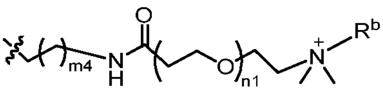
,

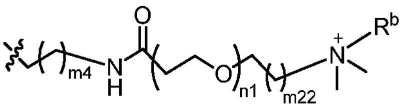
,
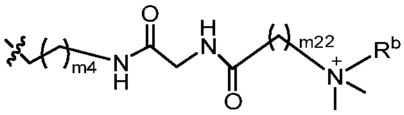
,

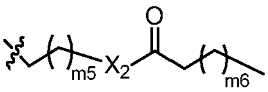
,
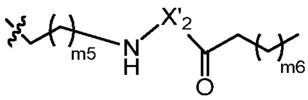
,

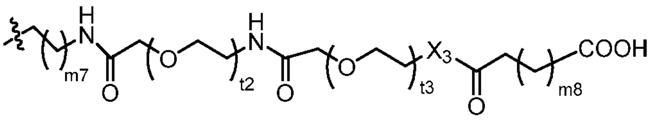
,

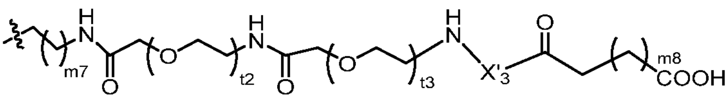

or an amino acid; preferably from

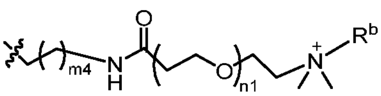
,
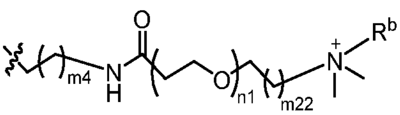
,

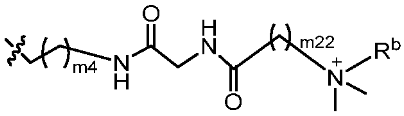, 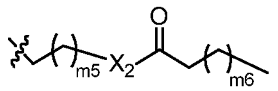, 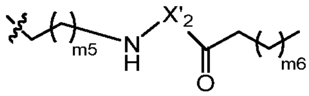,

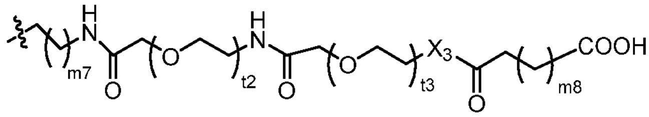

or

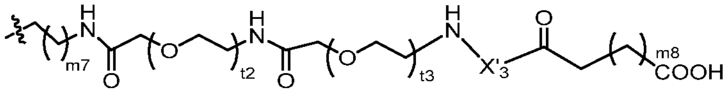,

$R^b$ is selected from H, alkyl,

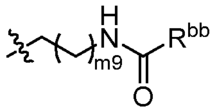

or an amino acid, wherein the alkyl can be further substituted with hydroxyl or alkoxy;
$R^{bb}$ is selected from alkyl,

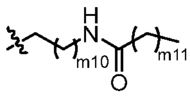,

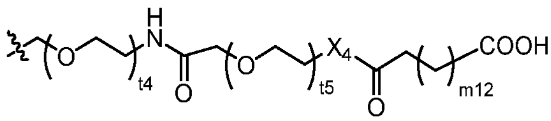

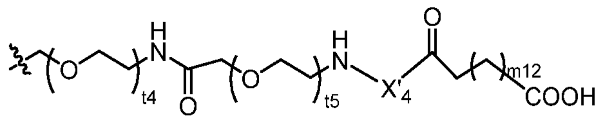

or an amino acid;
$X_2$ is an amino acid;
$X'_2$ is an amino acid;
$X_3$ is an amino acid;
$X'_3$ is an amino acid;
$X_4$ is an amino acid;
$X'_4$ is an amino acid;
n1 is selected from integers of 0-12;
t2-t5 are each independently selected from integers of 0-12;
m4-m12 are each independently selected from integers of 0-24; and
m22 is selected from integers of 0-24.

In a preferred embodiment of the present invention, in the compound or the stereoisomer or pharmaceutically acceptable salt thereof, $R^1$ is selected from H, $C_{1-6}$ alkyl,

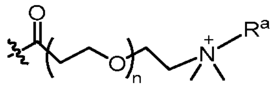

or an amino acid;

$R^a$ is selected from H, $C_{1-6}$ alkyl,

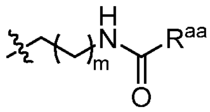

or an amino acid;
$R^{aa}$ is selected from $C_{1-6}$ alkyl,

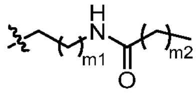

,

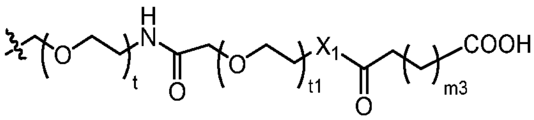

or an amino acid;
$X_1$ is an amino acid;
preferably, $R^{aa}$ is selected from $C_{1-6}$ alkyl,

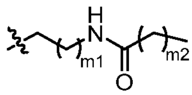

,

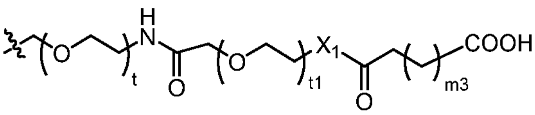

,

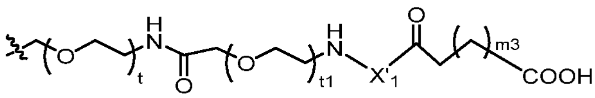

or an amino acid;
$X_1$ is an amino acid;
$X'_1$ is an amino acid;
n is selected from integers of 0-12;
t-t1 are each independently selected from integers of 0-12;
m-m3 are each independently selected from integers of 0-24.

In a preferred embodiment of the present invention, in the compound or the stereoisomer or pharmaceutically acceptable salt thereof, $R^2$ is selected from H, $C_{1-6}$ alkyl,

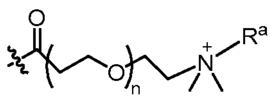

or an amino acid;

$R^a$ is selected from H, $C_{1-6}$ alkyl,

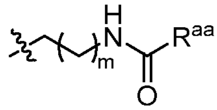

or an amino acid;

$R^{aa}$ is selected from $C_{1-6}$ alkyl,

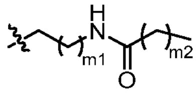

,

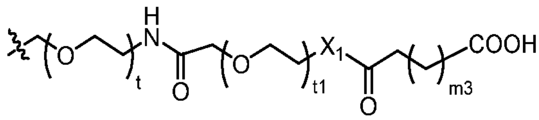

or an amino acid;

$X_1$ is an amino acid;

preferably, $R^{aa}$ is selected from $C_{1-6}$ alkyl,

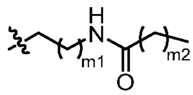

,

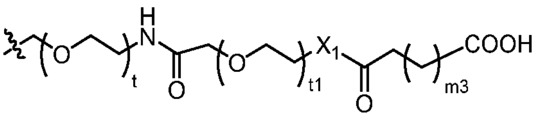

,

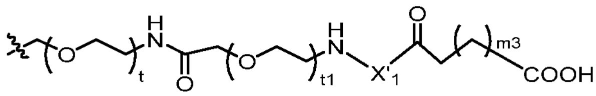

or an amino acid;

$X_1$ is an amino acid;

$X'_1$ is an amino acid;

n is selected from integers of 0-12;

t-t1 are each independently selected from integers of 0-12; and

m-m3 are each independently selected from integers of 0-24.

In a preferred embodiment of the present invention, in the compound or the stereoisomer or pharmaceutically acceptable salt thereof, $R^3$ is selected from hydroxyl, $C_{1-6}$ alkyl, $-NR^6R^7$ or an amino acid;

$R^6$ and $R^7$ are each independently selected from H, alkyl, $-CH_2C(O)NH_2$,

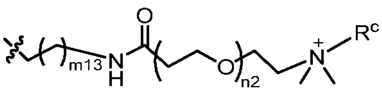

,

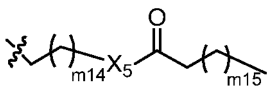

,

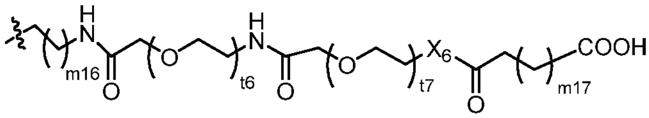

or an amino acid;

$R^c$ is selected from H, alkyl,

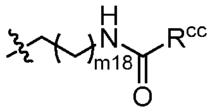

or an amino acid;

$R^{cc}$ is selected from alkyl,

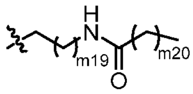

,

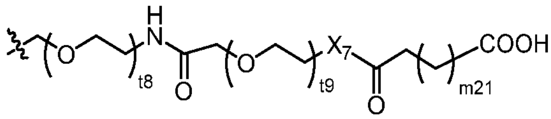

or an amino acid;

$X_5$ is an amino acid;

$X_6$ is an amino acid;

$X_7$ is an amino acid;

preferably, $R^3$ is selected from hydroxyl, $C_{1-6}$ alkyl, $-NR^6R^7$ or an amino acid;

$R^6$ is selected from H, $C_{1-6}$ alkyl, $-CH_2C(O)NH_2$,

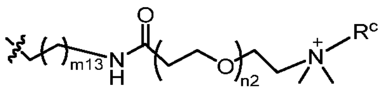

,

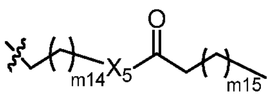

,

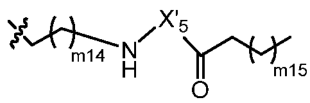

,

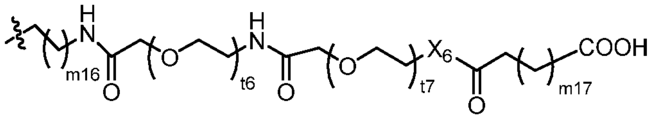

,

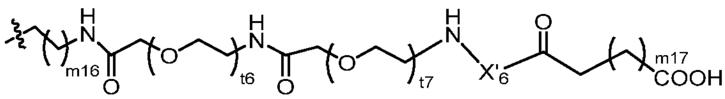

or an amino acid;

$R^7$ is selected from H, $C_{1-6}$ alkyl, $-CH_2C(O)NH_2$,

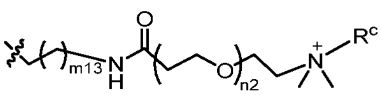

,

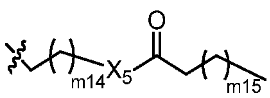

,

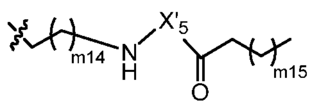

,

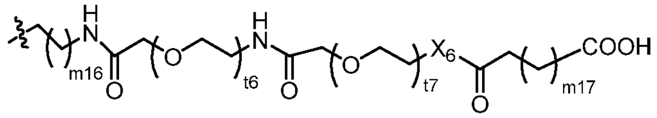

,

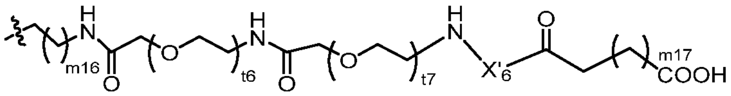

or an amino acid;

$R^c$ is selected from H, $C_{1-6}$ alkyl,

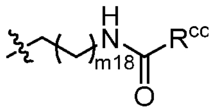

or an amino acid;

$R^{cc}$ is selected from $C_{1-6}$ alkyl,

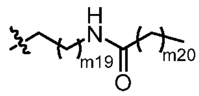

,

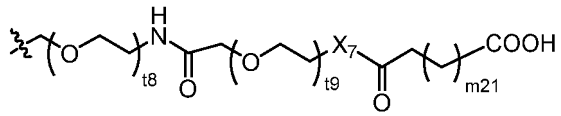

,

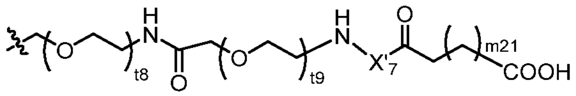

or an amino acid;

$X_5$ is an amino acid;

$X'_5$ is an amino acid;

$X_6$ is an amino acid;

$X'_6$ is an amino acid;

$X_7$ is an amino acid;

$X'_7$ is an amino acid;

n2 is selected from integers of 0-12;

t6-t9 are each independently selected from integers of 0-12; and

m13-m21 are each independently selected from integers of 0-24.

In a preferred embodiment of the present invention, in the compound or the stereoisomer or pharmaceutically acceptable salt thereof, $R^4$ is selected from H, $C_{1-6}$ alkyl, $-CH_2C(O)NH_2$,

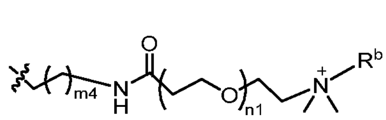

,

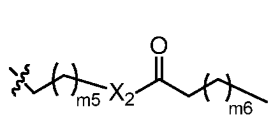

,

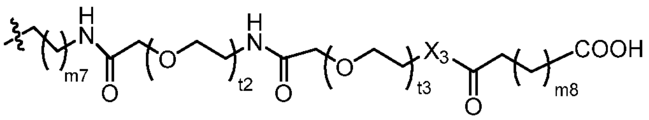

or an amino acid;

$R^b$ is selected from H, $C_{1-6}$ alkyl,

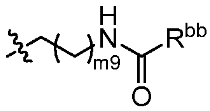

or an amino acid;

$R^{bb}$ is selected from $C_{1-6}$ alkyl,

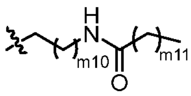

,

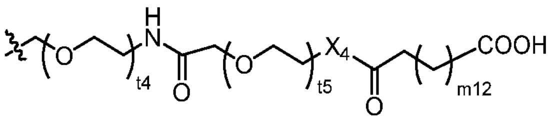

or an amino acid;

$X_2$ is an amino acid;

$X_3$ is an amino acid;

$X_4$ is an amino acid;

preferably, $R^4$ is selected from H, $C_{1-6}$ alkyl, $-CH_2C(O)NH_2$,

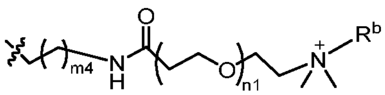

,

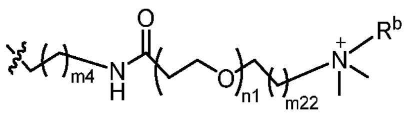

,

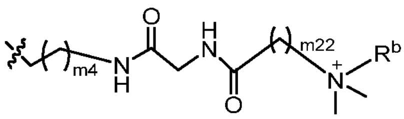

,

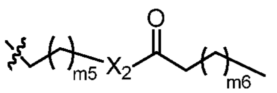

,

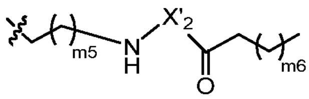

,

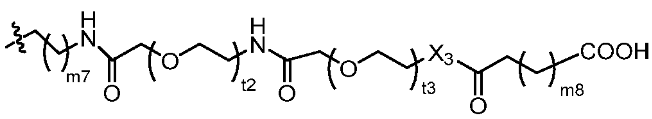

,

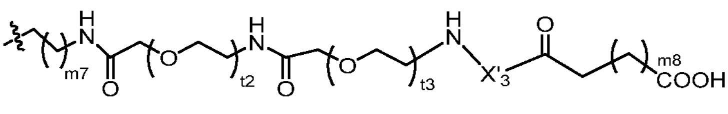

or an amino acid;

$R^b$ is selected from H, $C_{1-6}$ alkyl

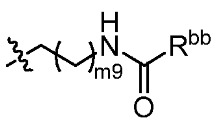

,

or an amino acid, wherein the alkyl can be further substituted with hydroxyl or alkoxy;

$R^{bb}$ is selected from $C_{1-6}$ alkyl,

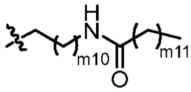

,

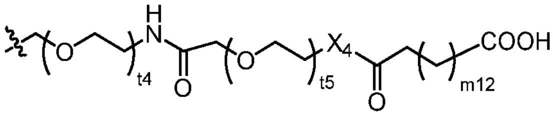

,

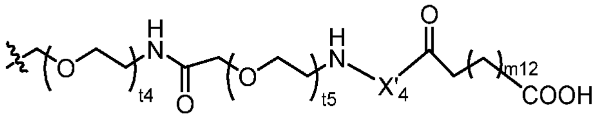

or an amino acid;

$X_2$ is an amino acid;

$X'_2$ is an amino acid;

$X_3$ is an amino acid;

$X'_3$ is an amino acid;

$X_4$ is an amino acid;

$X'_4$ is an amino acid;

n1 is selected from integers of 0-12;

t2-t5 are each independently selected from integers of 0-12;

m4-m12 are each independently selected from integers of 0-24; and

m22 is selected from integers of 0-24.

In a preferred embodiment of the present invention, in the compound or the stereoisomer or pharmaceutically acceptable salt thereof, $R^5$ is selected from H, $C_{1-6}$ alkyl, $-CH_2C(O)NH_2$,

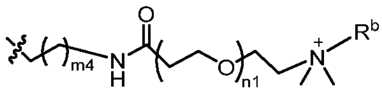

,

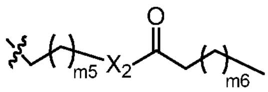

,

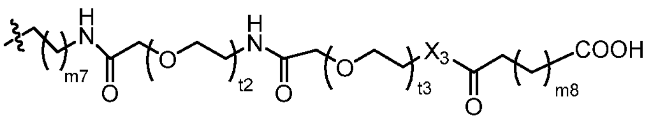

or an amino acid;

$R^b$ is selected from H, $C_{1-6}$ alkyl,

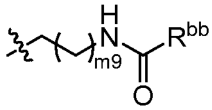

or an amino acid;

$R^{bb}$ is selected from $C_{1-6}$ alkyl,

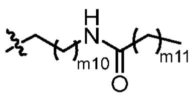

,

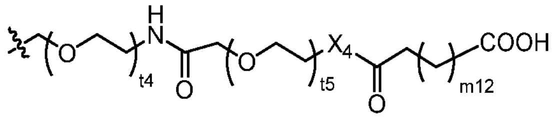

or an amino acid;

$X_2$ is an amino acid;

$X_3$ is an amino acid;

$X_4$ is an amino acid;

preferably, $R^5$ is selected from H, $C_{1-6}$ alkyl, $-CH_2C(O)NH_2$,

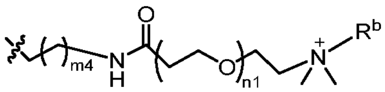,
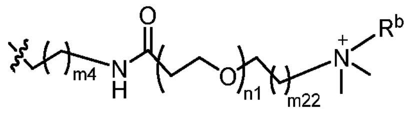,

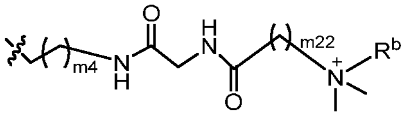, 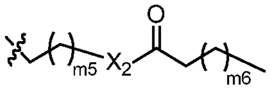, 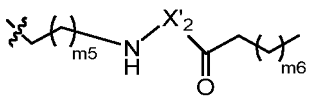,

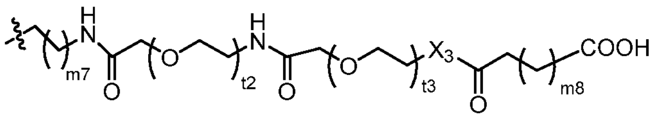,

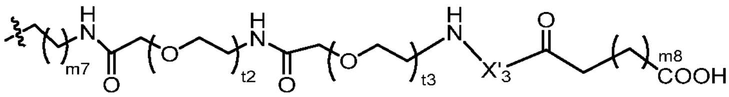

or an amino acid, further preferably from

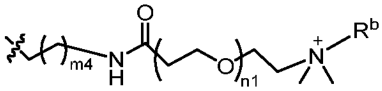, 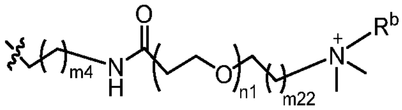,

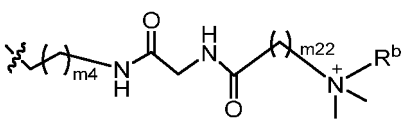, 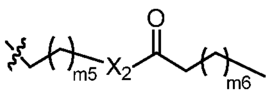, 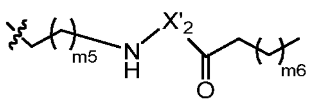,

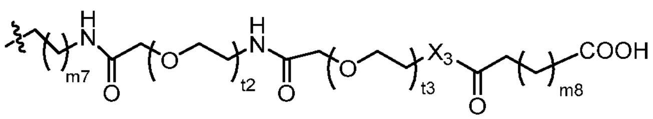

or

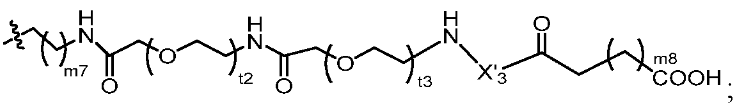,

$R^b$ is selected from H, $C_{1-6}$ alkyl,

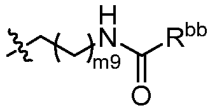

or an amino acid, wherein the alkyl can be further substituted with hydroxyl or alkoxy;

$R^{bb}$ is selected from $C_{1-6}$ alkyl,

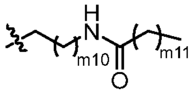

,

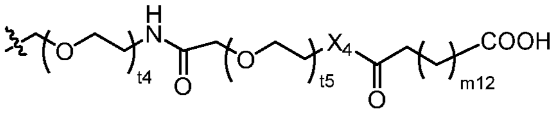

,

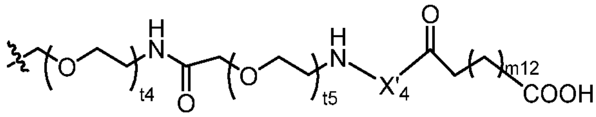

or an amino acid;

$X_2$ is an amino acid;

$X'_2$ is an amino acid;

$X_3$ is an amino acid;

$X'_3$ is an amino acid;

$X_4$ is an amino acid;

$X'_4$ is an amino acid;

n1 is selected from integers of 0-12;

t2-t5 are each independently selected from integers of 0-12;

m4-m12 are each independently selected from integers of 0-24; and

m22 is selected from integers of 0-24.

In a preferred embodiment of the present invention, in the compound or the stereoisomer or pharmaceutically acceptable salt thereof,

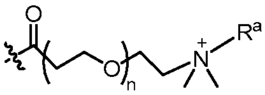

is selected from

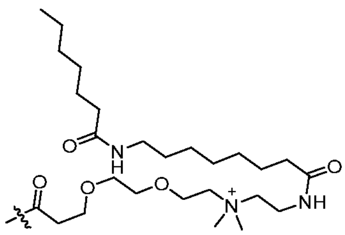

or

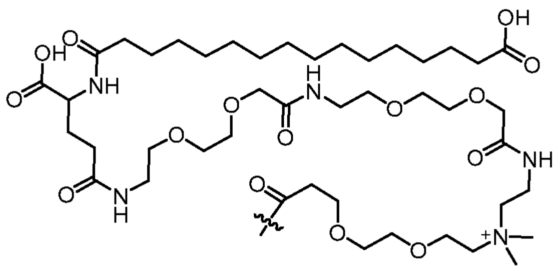.

In a preferred embodiment of the present invention, in the compound or the stereoisomer or pharmaceutically acceptable salt thereof,

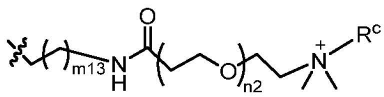

is selected from

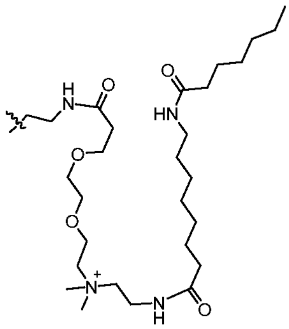,

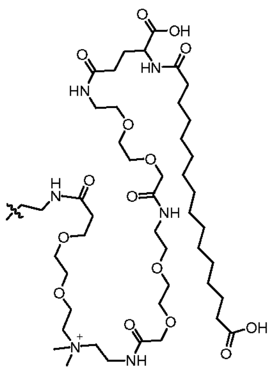, 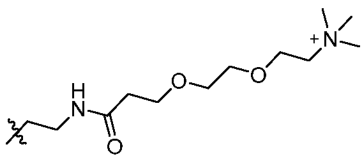 or 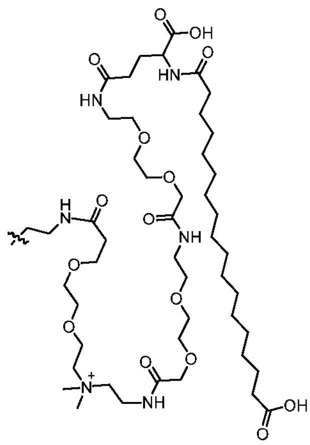.

In a preferred embodiment of the present invention, in the compound or the stereoisomer or pharmaceutically acceptable salt thereof,

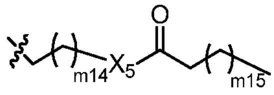 is

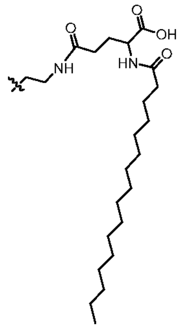

;

or

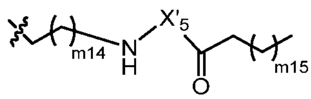 is 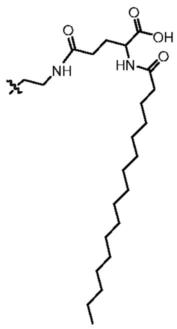.

In a preferred embodiment of the present invention, in the compound or the stereoisomer or pharmaceutically acceptable salt thereof,

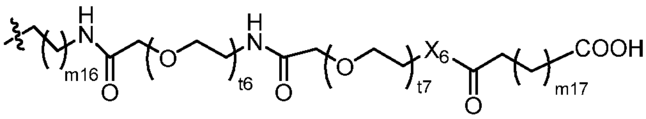

is

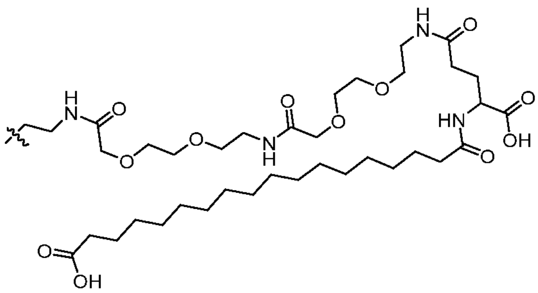

;

or

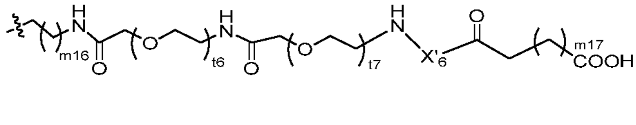

is

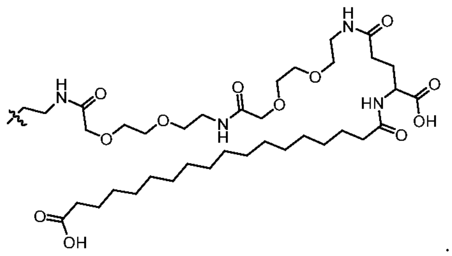

.

In a preferred embodiment of the present invention, in the compound or the stereoisomer or pharmaceutically acceptable salt thereof,

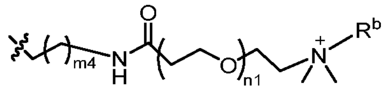

is selected from

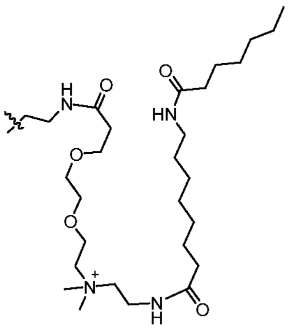

,

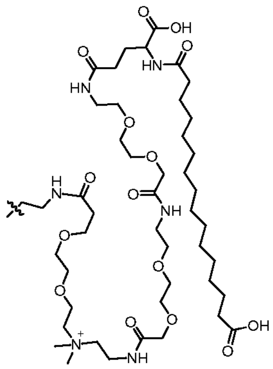

, 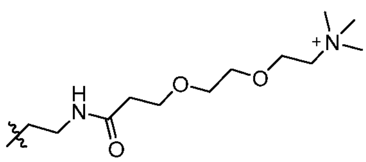 or 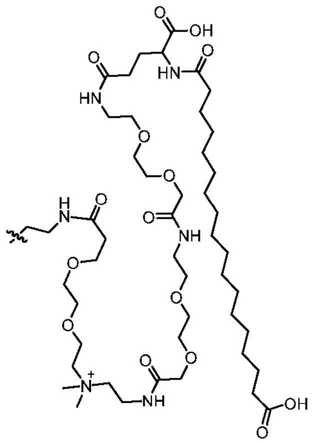 ;

preferably

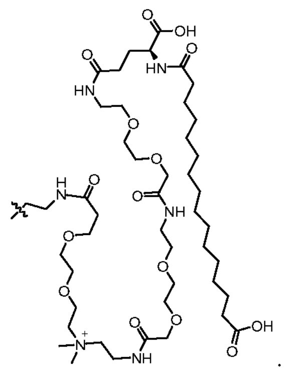

.

In a preferred embodiment of the present invention, in the compound or the stereoisomer or pharmaceutically acceptable salt thereof,

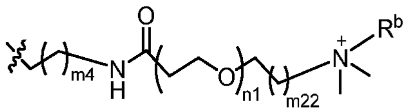

is selected from

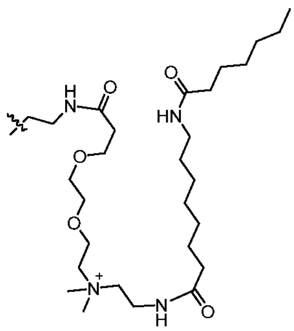

,

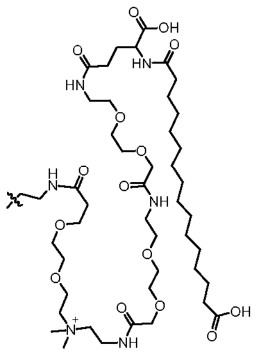

,

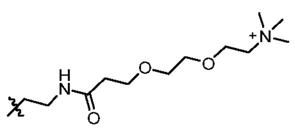

,

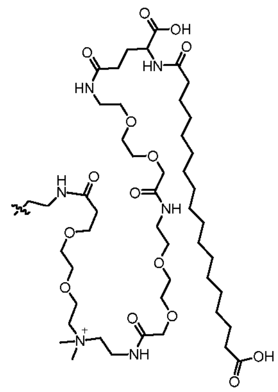

,

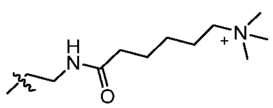

,

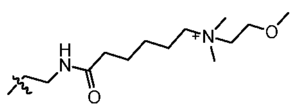

,

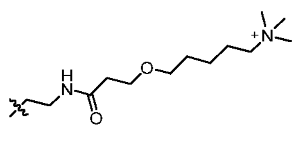

,

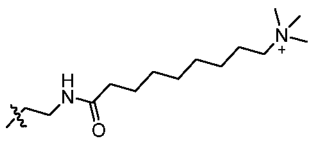

or

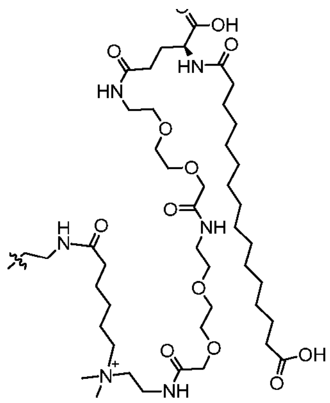

;

preferably

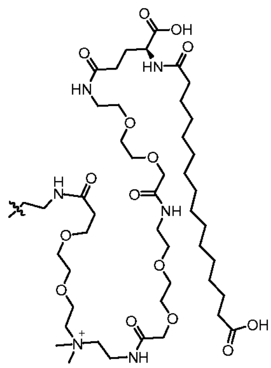

.

In a preferred embodiment of the present invention, in the compound or the stereoisomer or pharmaceutically acceptable salt thereof,

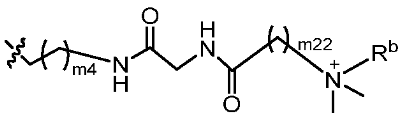

is

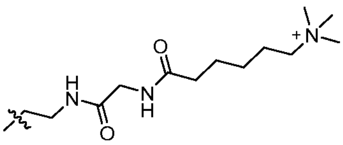

.

In a preferred embodiment of the present invention, in the compound or the stereoisomer or pharmaceutically acceptable salt thereof,

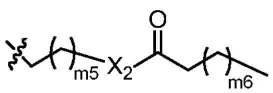

is

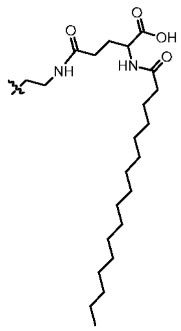

;

or

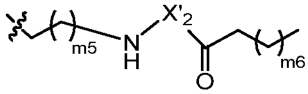

is

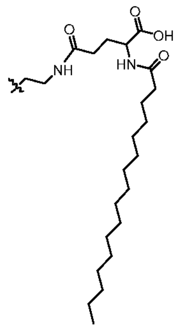

.

In a preferred embodiment of the present invention, in the compound or the stereoisomer or pharmaceutically acceptable salt thereof,

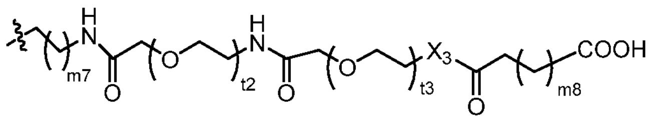

is

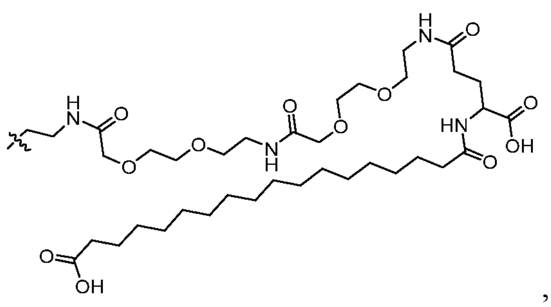

,

or

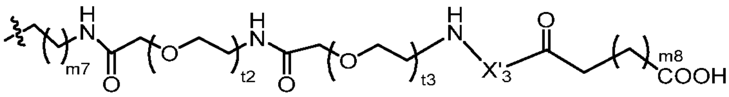

is

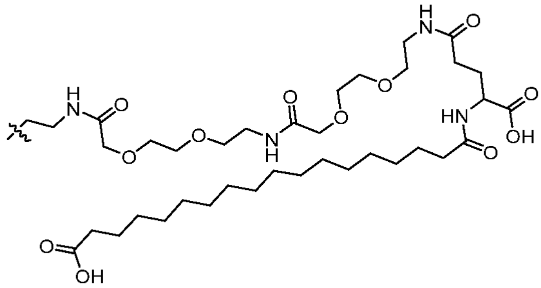

.

In a preferred embodiment of the present invention, the amino acid is Gly, Ala, Val, Leu, Ile, Phe, Trp, Tyr, Asp, His, Asn, Glu, Lys, Gln, Met, Arg, Ser, Thr, Cys, or Pro;

preferably, the amino acid is Gln;
more preferably, the amino acid is Gln or Glu.

In a further embodiment of the present invention, n is selected from 0, 1, 2, 3, 4, 5, 6, 7, 8, 9, 10, 11, or 12;

n1 is selected from 0, 1, 2, 3, 4, 5, 6, 7, 8, 9, 10, 11, or 12;
n2 is selected from 0, 1, 2, 3, 4, 5, 6, 7, 8, 9, 10, 11, or 12;
t is selected from 0, 1, 2, 3, 4, 5, 6, 7, 8, 9, 10, 11, or 12;
t1 is selected from 0, 1, 2, 3, 4, 5, 6, 7, 8, 9, 10, 11, or 12;
t2 is selected from 0, 1, 2, 3, 4, 5, 6, 7, 8, 9, 10, 11, or 12;
t3 is selected from 0, 1, 2, 3, 4, 5, 6, 7, 8, 9, 10, 11, or 12;
t4 is selected from 0, 1, 2, 3, 4, 5, 6, 7, 8, 9, 10, 11, or 12;
t5 is selected from 0, 1, 2, 3, 4, 5, 6, 7, 8, 9, 10, 11, or 12;
t6 is selected from 0, 1, 2, 3, 4, 5, 6, 7, 8, 9, 10, 11, or 12;
t7 is selected from 0, 1, 2, 3, 4, 5, 6, 7, 8, 9, 10, 11, or 12;
t8 is selected from 0, 1, 2, 3, 4, 5, 6, 7, 8, 9, 10, 11, or 12;
t9 is selected from 0, 1, 2, 3, 4, 5, 6, 7, 8, 9, 10, 11, or 12;
m is selected from 0, 1, 2, 3, 4, 5, 6, 7, 8, 9, 10, 11, 12, 13, 14, 15, 16, 17, 18, 19, 20, 21, 22, 23, or 24;
m1 is selected from 0, 1, 2, 3, 4, 5, 6, 7, 8, 9, 10, 11, 12, 13, 14, 15, 16, 17, 18, 19, 20, 21, 22, 23, or 24;
m2 is selected from 0, 1, 2, 3, 4, 5, 6, 7, 8, 9, 10, 11, 12, 13, 14, 15, 16, 17, 18, 19, 20, 21, 22, 23, or 24;
m3 is selected from 0, 1, 2, 3, 4, 5, 6, 7, 8, 9, 10, 11, 12, 13, 14, 15, 16, 17, 18, 19, 20, 21, 22, 23, or 24;
m4 is selected from 0, 1, 2, 3, 4, 5, 6, 7, 8, 9, 10, 11, 12, 13, 14, 15, 16, 17, 18, 19, 20, 21, 22, 23, or 24;
m5 is selected from 0, 1, 2, 3, 4, 5, 6, 7, 8, 9, 10, 11, 12, 13, 14, 15, 16, 17, 18, 19, 20, 21, 22, 23, or 24;
m6 is selected from 0, 1, 2, 3, 4, 5, 6, 7, 8, 9, 10, 11, 12, 13, 14, 15, 16, 17, 18, 19, 20, 21, 22, 23, or 24;
m7 is selected from 0, 1, 2, 3, 4, 5, 6, 7, 8, 9, 10, 11, 12, 13, 14, 15, 16, 17, 18, 19, 20, 21, 22, 23, or 24;
m8 is selected from 0, 1, 2, 3, 4, 5, 6, 7, 8, 9, 10, 11, 12, 13, 14, 15, 16, 17, 18, 19, 20, 21, 22, 23, or 24;
m9 is selected from 0, 1, 2, 3, 4, 5, 6, 7, 8, 9, 10, 11, 12, 13, 14, 15, 16, 17, 18, 19, 20, 21, 22, 23, or 24;
m10 is selected from 0, 1, 2, 3, 4, 5, 6, 7, 8, 9, 10, 11, 12, 13, 14, 15, 16, 17, 18, 19, 20, 21, 22, 23, or 24;
m11 is selected from 0, 1, 2, 3, 4, 5, 6, 7, 8, 9, 10, 11, 12, 13, 14, 15, 16, 17, 18, 19, 20, 21, 22, 23, or 24;
m12 is selected from 0, 1, 2, 3, 4, 5, 6, 7, 8, 9, 10, 11, 12, 13, 14, 15, 16, 17, 18, 19, 20, 21, 22, 23, or 24;
m13 is selected from 0, 1, 2, 3, 4, 5, 6, 7, 8, 9, 10, 11, 12, 13, 14, 15, 16, 17, 18, 19, 20, 21, 22, 23, or 24;
m14 is selected from 0, 1, 2, 3, 4, 5, 6, 7, 8, 9, 10, 11, 12, 13, 14, 15, 16, 17, 18, 19, 20, 21, 22, 23, or 24;
m15 is selected from 0, 1, 2, 3, 4, 5, 6, 7, 8, 9, 10, 11, 12, 13, 14, 15, 16, 17, 18, 19, 20, 21, 22, 23, or 24;
m16 is selected from 0, 1, 2, 3, 4, 5, 6, 7, 8, 9, 10, 11, 12, 13, 14, 15, 16, 17, 18, 19, 20, 21, 22, 23, or 24;
m17 is selected from 0, 1, 2, 3, 4, 5, 6, 7, 8, 9, 10, 11, 12, 13, 14, 15, 16, 17, 18, 19, 20, 21, 22, 23, or 24;
m18 is selected from 0, 1, 2, 3, 4, 5, 6, 7, 8, 9, 10, 11, 12, 13, 14, 15, 16, 17, 18, 19, 20, 21, 22, 23, or 24;
m19 is selected from 0, 1, 2, 3, 4, 5, 6, 7, 8, 9, 10, 11, 12, 13, 14, 15, 16, 17, 18, 19, 20, 21, 22, 23, or 24;
m20 is selected from 0, 1, 2, 3, 4, 5, 6, 7, 8, 9, 10, 11, 12, 13, 14, 15, 16, 17, 18, 19, 20, 21, 22, 23, or 24; and
m21 is selected from 0, 1, 2, 3, 4, 5, 6, 7, 8, 9, 10, 11, 12, 13, 14, 15, 16, 17, 18, 19, 20, 21, 22, 23, or 24.
m22 is selected from 0, 1, 2, 3, 4, 5, 6, 7, 8, 9, 10, 11, 12, 13, 14, 15, 16, 17, 18, 19, 20, 21, 22, 23, or 24.

In a preferred embodiment of the present invention, in the compound or the stereoisomer or pharmaceutically

acceptable salt thereof, the amino acid is Gly, Ala, Val, Leu, Ile, Phe, Trp, Tyr, Asp, His, Asn, Glu, Lys, Gln, Met, Arg, Ser, Thr, Cys, Pro,

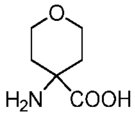 , 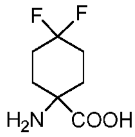 , 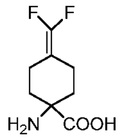 , 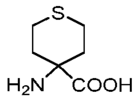 , 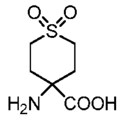 , 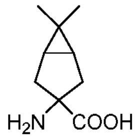 ,

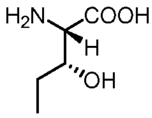 , 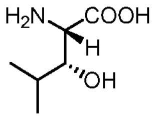 or 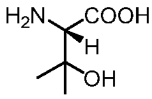 ;

preferably, the amino acid is Glu, Thr,

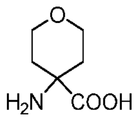 , 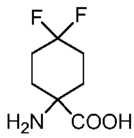 , 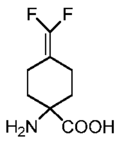 ,

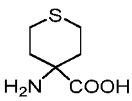 , 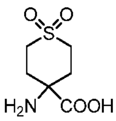 , 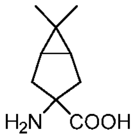 , 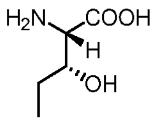 , 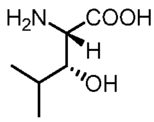 or 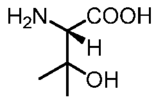 .

The present invention further provides a method for preparing the above compound or the stereoisomer or pharmaceutically acceptable salt thereof, wherein the method is based on solid-phase or liquid-phase synthesis;

preferably, the synthesis method comprises the following steps:

1) synthesizing a resin peptide based on a solid-phase synthesis method;
2) cleaving the resin peptide obtained in step 1) to obtain a polypeptide intermediate;
3) subjecting the polypeptide intermediate to a condensation reaction with a side chain; and
4) removing a protective group from a peptide fragment obtained in step 3) and then performing cyclization to obtain the final product;

further preferably, the synthesis method comprises the following steps:

1) based on a solid-phase synthesis method of an Fmoc method, synthesizing a resin peptide and capping the resin peptide with acetic anhydride;
2) cleaving the resin peptide obtained in step 1) to obtain a polypeptide intermediate;
3) subjecting the polypeptide intermediate to a condensation reaction with a side chain by using a coupling agent; and
4) removing a protective group from a peptide fragment obtained in step 3) and then performing cyclization by oxidizing a disulfide bond to obtain the final product.

The present invention further relates to a pharmaceutical composition, comprising a therapeutically effective dose of the above compound or the stereoisomer or pharmaceutically acceptable salt thereof, and one or more pharmaceutically acceptable carriers, diluents, or excipients.
In a preferred embodiment of the present invention, the pharmaceutical composition is selected from tablets, capsules, liquid preparations, or injections.
In a preferred embodiment of the present invention, the pharmaceutical composition is a fast-release preparation or a slow-release preparation.
In certain embodiments of the present invention, the compound or the stereoisomer or pharmaceutically acceptable salt thereof can be administered by any convenient method, for example, by oral, parenteral, oral cavity,

sublingual, nasal cavity, rectal, intrathecal, or transdermal administration, and as pharmaceutical compositions adjusted accordingly.

In certain embodiments of the present invention, the compound or the stereoisomer or pharmaceutically acceptable salt thereof can be prepared into a liquid or solid preparation, such as a syrup, a suspension, an emulsion, tablets, capsules, a powder, granules, or a lozenge.

The present invention further relates to the use of the compound or the stereoisomer or pharmaceutically acceptable salt thereof, or the pharmaceutical composition in the preparation of an IL-23R inhibitor drug. The present invention further relates to the use of the compound or the stereoisomer or pharmaceutically acceptable salt thereof or the pharmaceutical composition in the preparation of a drug for treating inflammatory and autoimmune diseases and cancers, e.g., inflammatory bowel disease (IBD), ulcerative colitis, Crohn's disease, celiac disease (nontropical sprue), enteropathy associated with seronegative arthropathy, microscopic colitis, collagenous colitis, eosinophilic gastroenteritis, colitis associated with radiotherapy or chemotherapy, colitis associated with congenital immune conditions such as leukocyte adhesion deficiency-1, chronic granulomatosis, hepatic glycogen storage disease type 1b, Hermansky-Pudlak syndrome, Chediak-Higashi syndrome and Wiskott-Aldrich syndrome, pouchitis following proctocolectomy and ileoanal anastomosis, gastrointestinal cancer, pancreatitis, insulin-dependent diabetes mellitus, mastitis, cholecystitis, cholangitis, pericholangitis, chronic bronchitis, chronic sinusitis, asthma, psoriasis, psoriatic arthritis, irritable bowel syndrome (IBS), multiple sclerosis (MS), psoriasis, psoriatic arthritis, rheumatoid arthritis, pemphigus vulgaris, organ transplant rejection, Crohn's disease, systemic lupus erythematosus (SLE), or diabetic disease.

The present invention further relates to the use of the compound or the stereoisomer or pharmaceutically acceptable salt thereof or the pharmaceutical composition in the preparation of a drug for treating inflammatory and autoimmune diseases and cancers, e.g., inflammatory bowel disease (IBD), ulcerative colitis, Crohn's disease, celiac disease (nontropical sprue), enteropathy associated with seronegative arthropathy, microscopic colitis, collagenous colitis, eosinophilic gastroenteritis, colitis associated with radiotherapy or chemotherapy, colitis associated with congenital immune conditions such as leukocyte adhesion deficiency-1, chronic granulomatosis, hepatic glycogen storage disease type 1b, Hermansky-Pudlak syndrome, Chediak-Higashi syndrome and Wiskott-Aldrich syndrome, pouchitis following proctocolectomy and ileoanal anastomosis, gastrointestinal cancer, pancreatitis, insulin-dependent diabetes mellitus, mastitis, cholecystitis, cholangitis, pericholangitis, chronic bronchitis, chronic sinusitis, asthma, psoriasis, psoriatic arthritis, irritable bowel syndrome (IBS), multiple sclerosis (MS), psoriasis, psoriatic arthritis, rheumatoid arthritis, pemphigus vulgaris, organ transplant rejection, Crohn's disease, systemic lupus erythematosus (SLE), or diabetic disease.

The present invention further relates to a method for treating and/or preventing inflammatory and autoimmune diseases and cancers, e.g., inflammatory bowel disease (IBD), ulcerative colitis, Crohn's disease, celiac disease (nontropical sprue), enteropathy associated with seronegative arthropathy, microscopic colitis, collagenous colitis, eosinophilic gastroenteritis, colitis associated with radiotherapy or chemotherapy, colitis associated with congenital immune conditions such as leukocyte adhesion deficiency-1, chronic granulomatosis, hepatic glycogen storage disease type 1b, Hermansky-Pudlak syndrome, Chediak-Higashi syndrome and Wiskott-Aldrich syndrome, pouchitis following proctocolectomy and ileoanal anastomosis, gastrointestinal cancer, pancreatitis, insulin-dependent diabetes mellitus, mastitis, cholecystitis, cholangitis, pericholangitis, chronic bronchitis, chronic sinusitis, asthma, psoriasis, psoriatic arthritis, irritable bowel syndrome (IBS), multiple sclerosis (MS), psoriasis, psoriatic arthritis, rheumatoid arthritis, pemphigus vulgaris, organ transplant rejection, Crohn's disease, systemic lupus erythematosus (SLE), or diabetic disease, which comprises administering to a patient a therapeutically effective dose of the compound or the stereoisomer or pharmaceutically acceptable salt thereof, or the pharmaceutical composition thereof.

The present invention further relates to a method for treating inflammatory and autoimmune diseases and cancers, e.g., inflammatory bowel disease (IBD), ulcerative colitis, Crohn's disease, celiac disease (nontropical sprue), enteropathy associated with seronegative arthropathy, microscopic colitis, collagenous colitis, eosinophilic gastroenteritis, colitis associated with radiotherapy or chemotherapy, colitis associated with congenital immune conditions such as leukocyte adhesion deficiency-1, chronic granulomatosis, hepatic glycogen storage disease type 1b, Hermansky-Pudlak syndrome, Chediak-Higashi syndrome and Wiskott-Aldrich syndrome, pouchitis following proctocolectomy and ileoanal anastomosis, gastrointestinal cancer, pancreatitis, insulin-dependent diabetes mellitus, mastitis, cholecystitis, cholangitis, pericholangitis, chronic bronchitis, chronic sinusitis, asthma, psoriasis, psoriatic arthritis, irritable bowel syndrome (IBS), multiple sclerosis (MS), psoriasis, psoriatic arthritis, rheumatoid arthritis, pemphigus vulgaris, organ transplant rejection, Crohn's disease, systemic lupus erythematosus (SLE), or diabetic disease, or other conditions in a mammal, which comprises administering to the mammal a therapeutically effective amount of the compound or a pharmaceutically acceptable salt, ester, prodrug, solvate, hydrate or derivative thereof according to the present invention.

In some embodiments, the present method involves inflammatory and autoimmune diseases and cancers, e.g., inflammatory bowel disease (IBD), ulcerative colitis, Crohn's disease, celiac disease (nontropical sprue), enteropathy associated with seronegative arthropathy, microscopic colitis, collagenous colitis, eosinophilic gastroenteritis, colitis associated with radiotherapy or chemotherapy, colitis associated with congenital immune conditions such as leukocyte

adhesion deficiency-1, chronic granulomatosis, hepatic glycogen storage disease type 1b, Hermansky-Pudlak syndrome, Chediak-Higashi syndrome and Wiskott-Aldrich syndrome, pouchitis following proctocolectomy and ileoanal anastomosis, gastrointestinal cancer, pancreatitis, insulin-dependent diabetes mellitus, mastitis, cholecystitis, cholangitis, pericholangitis, chronic bronchitis, chronic sinusitis, asthma, psoriasis, psoriatic arthritis, irritable bowel syndrome (IBS), multiple sclerosis (MS), psoriasis, psoriatic arthritis, rheumatoid arthritis, pemphigus vulgaris, organ transplant rejection, Crohn's disease, systemic lupus erythematosus (SLE), or diabetic disease
preferably inflammatory bowel disease, arthritis, or psoriasis.

## Detailed Description of the Invention

Unless otherwise defined herein, scientific and technical terms used in the present patent application shall have the meanings commonly understood by those of ordinary skill in the art. Generally, the nomenclatures used in conjunction with, and the techniques of, chemistry, molecular biology, cell and cancer biology, immunology, microbiology, pharmacology, and protein and nucleic acid chemistry described herein are those well-known and commonly used in the art.

The term "peptide" broadly refers to a sequence of two or more amino acids joined together by peptide bonds. It should be understood that this term does not imply the specific length of an amino acid polymer, nor is it intended to imply or distinguish between polypeptides produced by recombinant techniques and chemical or enzymatic synthesis, or naturally occurring polypeptides. The term peptide includes cyclic peptides.

The term "amino acid" refers to any and all amino acids and residues thereof, including naturally occurring amino acids (e.g., a-amino acids), unnatural amino acids, modified amino acids, synthetic amino acids or rare amino acids, including both D-amino acids and L-amino acids. Natural amino acids include amino acids found in nature, such as 23 amino acids incorporated into peptide chains to form a large number of building blocks of proteins. These stereoisomers are mainly L stereoisomers, but there are some D-amino acids present in bacterial envelopes and some antibiotics. 20 "standard" natural amino acids are alanine (Ala); arginine (Arg); asparagine (Asn); aspartic acid (Asp); cysteine (Cys); glutamine (Gln); glutamic acid (Glu); glycine (Gly); histidine (His); isoleucine (Ile); leucine (Leu); lysine (Lys); methionine (Met); phenylalanine (Phe); proline (Pro); serine (Ser); threonine (Thr); tryptophan (Trp); tyrosine (Tyr); and valine (Val). "Non-standard" natural amino acids are pyrrolysine (found in methanogens and other eukaryotes), selenocysteine (found in many non-eukaryotes and most eukaryotes), and N-formylmethionine (encoded by the start codon AUG in bacteria, mitochondria, and chloroplasts). "Unnatural amino acids" or "non-natural amino acids" are naturally occurring or chemically synthesized non-protein amino acids (i.e., amino acids that are not naturally encoded or found in genetic codes). More than 140 unnatural amino acids are known and there may be thousands of combinations. Examples of "unnatural" amino acids include β-amino acids ($\beta^3$ and $\beta^2$), homoamino acids, proline derivatives and pyruvic acid derivatives, 3-substituted alanine derivatives, glycine derivatives, cyclosubstituted phenylalanine and tyrosine derivatives, linear amino acids, diaminoacids, D-diaminoacids, α-methylaminoacids, and N-methylaminoacids. Unnatural or non-natural amino acids also include modified amino acids. "Modified" amino acids include amino acids (e.g., natural amino acids) that have been chemically modified to comprise groups or chemical moieties that are not naturally present on the amino acids. Preferably, the unnatural amino acids described in the present invention include, but are not limited to,

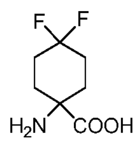, 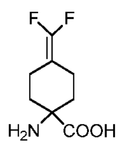, 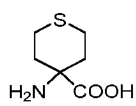,

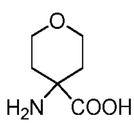, 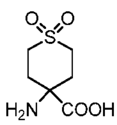, 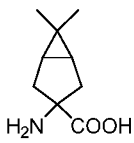, 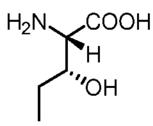, 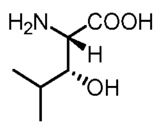 or 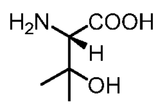.

The term "alkyl" refers to a saturated aliphatic hydrocarbon group, which is a linear or branched group containing 1 to 20 carbon atoms, preferably alkyl containing 1 to 8 carbon atoms, more preferably alkyl containing 1 to 6 carbon atoms, and most preferably alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. More preferably, the alkyl is a lower alkyl group containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc.

The hydrogen atoms of the present invention can be replaced with its isotope deuterium, and any hydrogen atom in the example compounds involved in the present invention can also be replaced with a deuterium atom.

"Hydroxyl" refers to an -OH group.

"THF" refers to tetrahydrofuran.

"MeOH" refers to methanol.

"DMF" refers to *N,N*-dimethylformamide.

"TFA" refers to trifluoroacetic acid.

"TEA" refers to triethylamine.

"MeI" refers to iodomethane.

"DMA" refers to *N,N*-dimethylacetamide.

"$Et_2O$" refers to diethyl ether.

"DCM" refers to dichloromethane.

"DMAP" refers to 4-dimethylaminopyridine.

"DCC" refers to dicyclohexylcarbodiimide.

"DCE" refers to 1,2-dichloroethane.

"DIEA" refers to *N,N*-diisopropylethylamine.

"NBS" refers to *N*-bromosuccinimide.

"NIS" refers to *N*-iodosuccinimide.

"Cbz-Cl" refers to benzyl chloroformate.

"$Pd_2(dba)_3$" refers to tris(dibenzylideneacetone)dipalladium.

"Dppf" refers to 1,1'-bisdiphenylphosphine ferrocene.

"HATU" refers to 2-(7-azabenzotriazolyl)-*N,N,N',N'*-tetramethyluronium hexafluorophosphate.

"KHMDS" refers to potassium hexamethyldisilamide.

"LiHMDS" lithium bistrimethylsilylamide.

"MeLi" refers to lithium methyl.

"n-BuLi" refers to n-butyl lithium.

"$NaBH(OAc)_3$" refers to sodium triacetoxyborohydride.

"Pharmaceutical composition" denotes a mixture containing one or more compounds described herein or physiologically/pharmaceutically acceptable salts or prodrug thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

"Pharmaceutically acceptable salts" refer to salts of the compounds of the present invention, which are safe and effective when used in mammals, and have appropriate biological activity.

**Detailed Description of Embodiments**

The present invention will be further described below in conjunction with examples, but these examples are not meant to limit the scope of the present invention.

Examples

The structure of the compound of the present invention is determined by nuclear magnetic resonance (NMR) or/and liquid chromatography-mass spectrometry (LC-MS). NMR chemical shift ($\delta$) is given in parts per million (ppm) unit. NMR is determined using Bruker AVANCE-400 nuclear magnetic instrument. The solvents for determination are deuterated dimethyl sulfoxide (DMSO-d6), deuterated methanol ($CD_3OD$), deuterated chloroform ($CDCl_3$), or deuterium water ($D_2O$), and the internal standard was tetramethylsilane (TMS).

For determination by liquid chromatography-mass spectrometry LC-MS, electrospray ion chromatography Thermo.Scientific-LTQ-XL is used, and for determination by HPLC, Agilent 1260 high pressure liquid chromatgraph (Phenomenex Gemini C18, 4.6 × 150 mm, 5 μm chromatographic column) is used. Yantai Huanghai HSGF254 or Qingdao GF254 silica gel plate is used as a thin layer chromatography silica plate, and TLC is of the specification of

0.15-0.20 mm, and the specification when separating and purifying a product by thin layer chromatography is 0.4-0.5 mm. For column chromatography, Yantai Huanghai silica gel of 200-300 mesh silica gel is generally used as a carrier.

The starting materials in the examples of the present invention are known and can be purchased on the market, or can be synthesized using or according to methods known in the art.

Unless otherwise specified, all reactions in the present invention are carried out under continuous magnetic stirring in a dry nitrogen or argon atmosphere, the solvent is a dry solvent, and the reaction temperature unit is degrees Celsius.

Eluent systems for silica gel column chromatography and developer systems for thin layer chromatography, which are used for the purification of compounds in the intermediates and examples, include: A: dichloromethane and methanol system, B: n-hexane and ethyl acetate system, and C: dichloromethane and acetone system, wherein the volume ratio between the solvents can be adjusted depending on the polarity of the compound, or can also be adjusted by adding a small amount of basic or acidic reagents such as triethylamine and acetic acid.

## Intermediate A

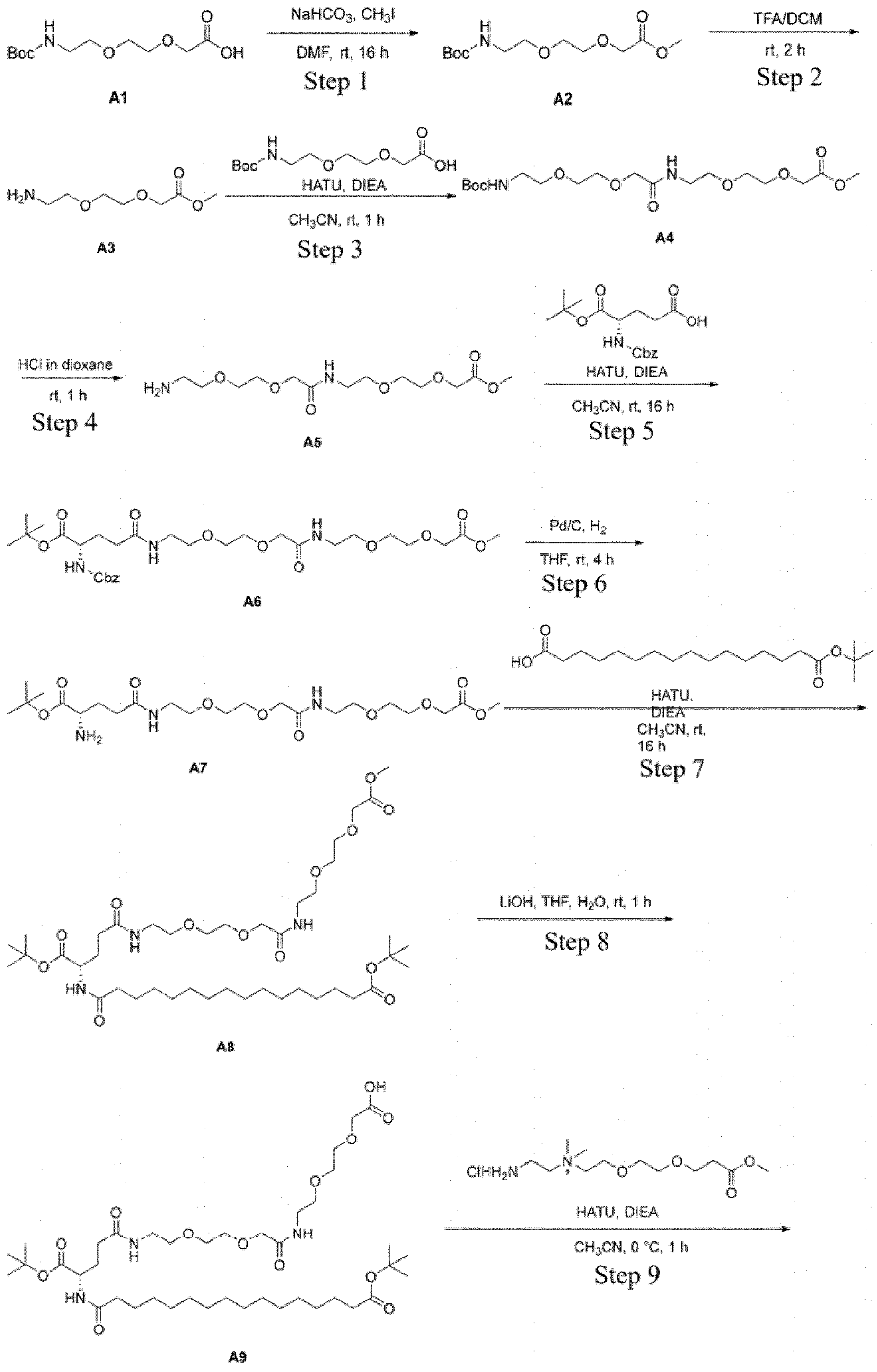

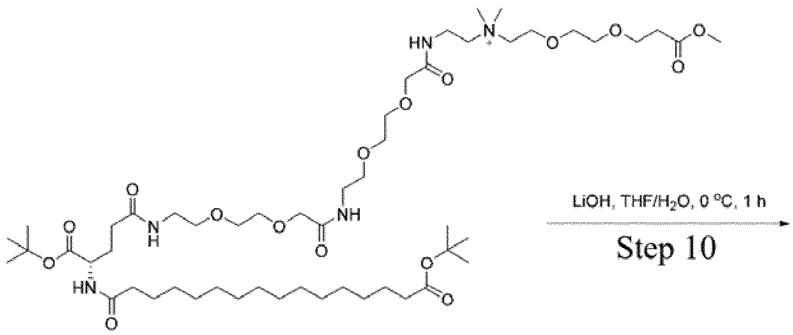


A10

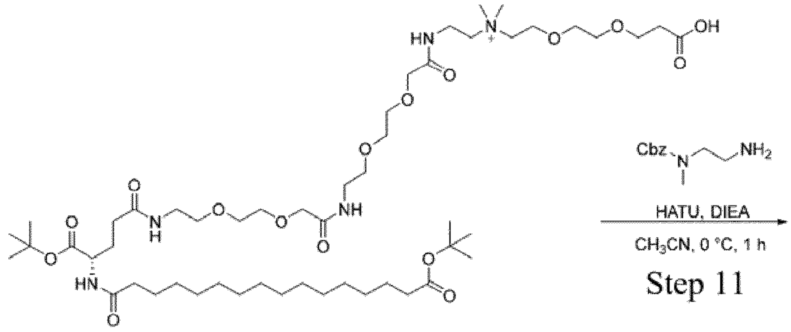


A11

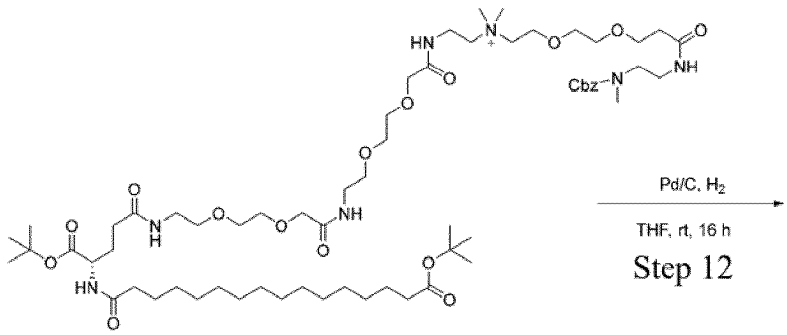


A12

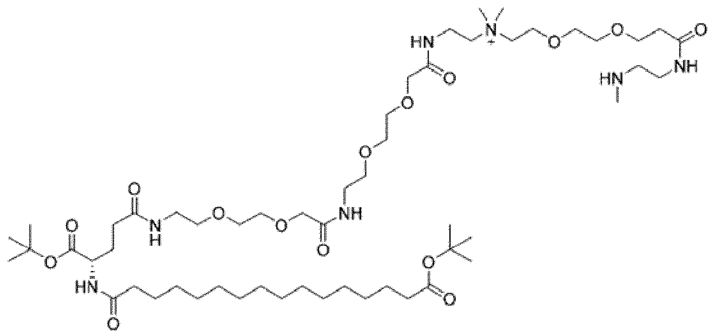

**Intermediate A**

Step 1

At room temperature, $NaHCO_3$ (79.8 g, 0.95 mol) and MeI (80.9 g, 0.57 mol) were added to a solution of compound **A1** (50 g, 0.19 mol) in DMF (350 mL). The mixture was stirred at 30°C for 16 hours, then diluted with water, and extracted with ethyl acetate. The combined organic layers were washed with a saturated aqueous sodium chloride solution, dried over $Na_2SO_4$, and then filtered, and the filtrate was concentrated to obtain compound **A2** (55 g). The crude product could be directly used in the next reaction.

LC/MS: $[M+H]^+$-100 = 178.0.

Step 2

Compound **A2** (10 g, 36.06 mmol) was dissolved in TFA/DCM (v:v = 1:2, 100 mL) and stirred for 2 hours at room temperature. The reaction liquid was concentrated to obtain compound **A3** (10 g). The crude product could be directly used in the next reaction.
LC/MS: $[M+H]^+$ = 178.1.

Step 3

At 0°C, compound **A3** (6.4 g, 36 mmol), DIEA (23.3 g, 180 mmol), and HATU (13.6 g, 36 mmol) were added to a solution of compound **A1** (9.5 g, 36 mmol) in $CH_3CN$ (250 mL), and the reaction liquid was stirred for 1 hour at room temperature, diluted with water, and then extracted three times with ethyl acetate. The combined organic layers were washed with a saturated aqueous sodium chloride solution, dried over $Na_2SO_4$, and filtered. The filtrate was concentrated, and the residue was purified by silica gel column chromatography (0-5%, MeOH/DCM) to obtain compound **A4** (15 g), with a yield of 98.7%.
LC/MS: $[M+H]^+$-100 = 323.2.

Step 4

Compound **A4** (9.1 g, 21.54 mmol) was dissolved in HCl/dioxane (4N, 100 mL) and stirred for 1 hour at room temperature. The reaction liquid was concentrated to obtain compound **A5** (7 g). The crude product could be directly used in the next reaction.
LC/MS: $[M+H]^+$ = 323.4.

Step 5

At 0°C, compound **A5** (6.88 g, 21.34 mmol), DIEA (13.79 g, 106.71 mmol), and HATU (8.05 g, 21.34 mmol) were added to a solution of (S)-4-(((benzyloxy)carbonyl)amino)-5-(tert-butoxy)-5-oxopentanoic acid (7.2 g, 21.34 mmol) in $CH_3CN$ (80 mL), and the reaction liquid was stirred for 16 hour at room temperature. The mixture was diluted with an aqueous sodium chloride solution and then extracted three times with ethyl acetate. The combined organic layers were washed with a saturated aqueous sodium chloride solution, dried over $Na_2SO_4$, and filtered. The filtrate was concentrated, and the residue was purified by silica gel column chromatography (0-5%, MeOH/DCM) to obtain compound **A6** (7 g), with a yield of 51.1%.
LC/MS: $[M+H]^+$ = 642.6.

Step 6

At room temperature, 10% Pd/C (700 mg) was added to a solution of compound **A6** (7 g, 10.91 mmol) in THF (150 mL), and the mixture was stirred for 16 hours in a hydrogen atmosphere (1.5 atmospheres) at room temperature. The reaction liquid was filtered through diatomite, and the filtrate was concentrated under reduced pressure to obtain compound **A7** (5.07 g), with a yield of 91.6%.
LC/MS: $[M+H]^+$ = 508.4.

Step 7

At 0°C, compound **A7** (5.07 g, 10 mmol), DIEA (6.45 g, 50 mmol), and HATU (4.45 g, 13 mmol) were added to a solution of mono-tert-butyl hexadecanedioate (4.45 g, 12.99 mmol) in $CH_3CN$ (80 mL), and the reaction liquid was stirred for 16 hours at room temperature, then diluted with an aqueous sodium chloride solution, and then extracted three times with EtOAc. The combined organic layers were washed with a saturated aqueous sodium chloride solution, dried over $Na_2SO_4$, and filtered. The filtrate was concentrated, and the residue was purified by silica gel column chromatography (0-5%, MeOH/DCM) to obtain compound **A8** (7.7 g), with a yield of 92.6%.

LC/MS: $[M+H]^+$ = 832.7;
$^1$H NMR (400 MHz, $CD_3OD$) δ 4.24 (dd, *J* = 9.1, 5.1 Hz, 1H), 4.16 (s, 2H), 4.00 (s, 2H), 3.73 (s, 3H), 3.69 (dt, *J* = 5.3, 2.7 Hz, 4H), 3.66 - 3.62 (m, 4H), 3.56 (dt, *J* = 8.4, 5.5 Hz, 4H), 3.44 (t, *J* = 5.4 Hz, 2H), 3.37 (t, *J* = 5.5 Hz, 2H), 2.29 (t, *J* = 7.7 Hz, 2H), 2.25 - 2.16 (m, 4H), 2.10 (qd, *J* = 7.6, 5.3 Hz, 1H), 1.89 (dt, *J* = 16.5, 7.5 Hz, 1H), 1.64 - 1.52 (m, 4H), 1.45 (d, J= 9.1 Hz, 18H), 1.29 (s, 20H).

Step 8

At 0°C, a solution of LiOH (0.23 g, 9.61 mmol) in water (10 mL) was added to a solution of compound **A8** (4 g, 4.81 mmol) in THF (40 mL) and reacted for 1 hour at this temperature. The reaction liquid was adjusted to pH = 4 by adding 1N HCl, and the aqueous phase was extracted three times with EtOAc. The combined organic layers were washed with a saturated aqueous sodium chloride solution, dried over $Na_2SO_4$, and filtered. The filtrate was concentrated under reduced pressure to obtain compound **A9** (4 g). The crude product could be directly used in the next reaction.
LC/MS: $[M+H]^+$ = 818.7.

Step 9

At 0°C, DIEA (3.32 g, 25.67 mmol), HATU (1.94 g, 5.13 mmol), and 2-amino-N-(2-(2-(3-methoxy-3-oxopropoxy) ethoxy)ethyl)-*N,N*-dimethylethane-1-ammonium hydrochloride (1.92 g, 6.42 mmol) were successively added to a solution of compound **A9** (3.5 g, 4.28 mmol) in $CH_3CN$ (40 mL), and the solution was then stirred for 1 hour at 0°C. The mixture was concentrated under reduced pressure, and the residue was purified by reverse column chromatography (5-40%, 40-70%, $CH_3CN$/0.03% TFA/water) to obtain compound **A10** (as a TFA salt, 2.16 g), with a yield of 43.7%.
LC/MS: $[M]^+$ = 1062.8.

Step 10

At 0°C, an aqueous LiOH solution (135 mg, 5.64 mmol, 10 mL) was added to a solution of compound **A10** (2 g, 1.88 mmol) in THF (20 mL) and reacted for 1 hour at this temperature. The reaction liquid was concentrated to remove THF, the residue was adjusted to pH = 7 by dropwise adding 1N HCl, and the mixture was purified by reverse column chromatography (5-40%, 40-70%, $CH_3CN$/0.03% TFA/water) to obtain compound **A11** (as a TFA salt, 0.7 g), with a yield of 35.5%.
LC/MS: $[M]^+$ = 1048.8.

Step 11

At 0°C, DIEA (517 mg, 4.0 mmol), HATU (302 mg, 0.8 mmol), and 2-amino-N-(2-(2-(3-methoxy-3-oxopropoxy) ethoxy)ethyl)-*N,N*-dimethylethane-1-ammonium hydrochloride (208 mg, 1.0 mmol) were successively added to a solution of compound **A11** (0.7 g, 0.67 mmol) in $CH_3CN$ (10 mL), and the solution was then stirred for 1 hour at 0°C. The mixture was concentrated under reduced pressure, and the residue was purified by reverse column chromatography (5-40%, 40-70%, $CH_3CN$/0.03% TFA/water) to obtain compound **A12** (as a TFA salt, 820 mg), with a yield of 99.2%.
LC/MS: $[(M+H)/2]^+$ = 620.4.

Step 12

At room temperature, 10% Pd/C (70 mg) was added to a solution of compound **A12** (700 mg, 0.56 mmol) in THF (20 mL), and the reaction was stirred for 16 hours in a hydrogen atmosphere (1.5 atmospheres) at room temperature. The reaction liquid was filtered through diatomite, the filtrate was concentrated under reduced pressure, and the residue was purified by reverse column chromatography (5-40%, 40-70%, $CH_3CN$/0.03% TFA/water) to obtain the compound **intermediate A** (as a TFA salt, 600 mg), with a yield of 95.1%.
LC/MS: $[M]^+$ = 1104.7.
$^1H$ NMR (400 MHz, $CD_3OD$) δ 4.24 (dd, *J* = 9.2, 5.2 Hz, 1H), 4.02 (d, *J* = 14.0 Hz, 4H), 3.94 (s, 2H), 3.74 (q, *J* = 6.1 Hz, 4H), 3.71 - 3.63 (m, 14H), 3.61 - 3.54 (m, 6H), 3.48 (dt, *J* = 10.6, 5.6 Hz, 4H), 3.40 - 3.35 (m, 2H), 3.22 (s, 6H), 3.13 (t, *J* = 5.8 Hz, 2H), 2.73 (s, 3H), 2.51 (t, *J* = 6.2 Hz, 2H), 2.29 (t, *J* = 7.6 Hz, 2H), 2.26 - 2.17 (m, 4H), 2.11 (ddd, *J* = 15.6, 10.3, 6.6 Hz, 1H), 1.95 - 1.82 (m, 1H), 1.59 (dt, *J* = 22.3, 7.1 Hz, 4H), 1.45 (d, *J* = 9.0 Hz, 18H), 1.29 (s, 20H).

**Intermediate A1**

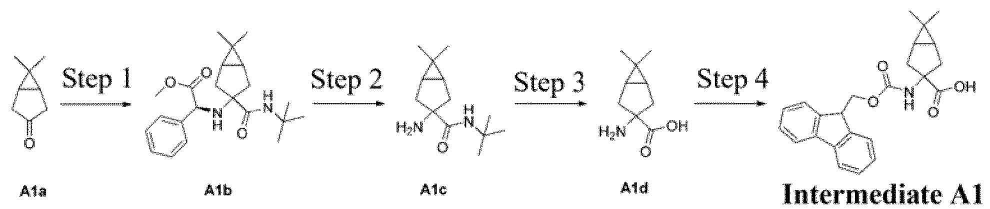


A1a A1b A1c A1d **Intermediate A1**

Step 1

L-phenylglycine (3.35 g, 40.26 mmol) was dissolved in methanol (150 mL) and cooled to -78°C, a solution of 6,6-dimethylbicyclo[3.1.0]hexan-3-one **A1a** (CAS: 13855-29-3, 5.0 g, 40.26 mmol) in methanol (20 mL) and a solution of tert-butyl isocyanate in methanol (20 mL) were successively dropwise added, and the mixture was gradually heated to room temperature and reacted for 24 hours. The reaction liquid was concentrated and then dissolved in 200 mL of diethyl ether for slurrying, and insoluble substances were filtered out. After the filtrate was concentrated, the residue was subjected to silica gel column chromatography (gradient elution with an EA/PE system) to obtain the compound methyl (2S)-2-((3-(tert-butylcarbamoyl)-6,6-dimethylbicyclo[3.1.0]hex-3-yl)amino)-2-phenylacetate **A1b** (10.5 g), with a yield of 70.0%.

LC/MS: $[M+H]^+$ = 373.3.

Step 2

Methyl (2*S*)-2-((3-(tert-butylcarbamoyl)-6,6-dimethylbicyclo[3.1.0]hex-3-yl)amino)-2-phenylacetate **A1b** (10.0 g, 26.84 mmol) and palladium hydroxide (1.13 g, 8.05 mmol) were dissolved in methanol (200 mL). After hydrogen displacement three times, the mixture was maintained in a hydrogen atmosphere and reacted for 16 hours at room temperature. The reaction liquid was filtered through diatomite, the filtrate was concentrated, then dissolved in diethyl ether (150 mL), and extracted twice with 2N dilute hydrochloric acid (50 mL), and the aqueous phases were combined, adjusted to pH 8 with potassium carbonate solid, extracted twice with diethyl ether (100 mL), and concentrated under reduced pressure to obtain 3-amino-N-(tert-butyl)-6,6-dimethylbicyclo[3.1.0]hexane-3-carboxamide **A1c** (4.9 g), with a yield of 81.4%.

LC/MS: $[M+H]^+$ = 225.2.

Step 3

3-Amino-N-(tert-butyl)-6,6-dimethylbicyclo[3.1.0]hexane-3-carboxamide **A1c** (4.9 g, 21.84 mmol) was dissolved in 6N hydrochloric acid (50 mL) and reacted under reflux for 16 hours. The reaction liquid was cooled to room temperature, extracted with diethyl ether, and washed, and the aqueous phase was collected and concentrated under reduced pressure to obtain 3-amino-6,6-dimethylbicyclo[3.1.0]hexane-3-carboxylic acid **A1d** (hydrochloride, 4.4 g), with a yield of 98%.

LC/MS: $[M+H]^+$ = 170.1.

Step 4

3-Amino-6,6-dimethylbicyclo[3.1.0]hexane-3-carboxylic acid **A1d** (hydrochloride, 4.4 g, 21.39 mmol) was dissolved in a sodium bicarbonate solution (100 mL), and a solution of 9-fluorenylmethyl-N-succinimidyl carbonate (10.79 g, 32.09 mmol) in dioxane (80 mL) was added and reacted for 1 hour at room temperature. The reaction liquid was extracted twice with ethyl acetate (100 mL), and the organic phases were combined and then washed with water. The aqueous phase was adjusted to pH 2 with 2N dilute hydrochloric acid and extracted again twice with ethyl acetate (50 mL). The organic phases were combined, dried over anhydrous sodium sulfate, and filtered. After the filtrate was concentrated, the residue was subjected to silica gel column chromatography (gradient elution with an MeOH/DCM system) to obtain **intermediate A1** 3-(((9*H*-fluoren-9-yl)methoxy)carbonyl)amino)-6,6-dimethylbicyclo[3.1.0]hexane-3-carboxylic acid (5.6 g), with a yield of 66.9%.

LC/MS: $[M-H]^-$ = 390.2.

## Intermediate A2

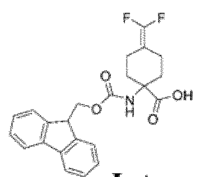

Intermediate A2

By reference to the synthesis method for **intermediate A1, intermediate A2** 1-((((9H-fluoren-9-yl)methoxy) carbonyl)amino)-4-(difluoromethylene) cyclohexane-1-carboxylic acid was obtained via 4-(difluoromethylene)cyclohexane (CAS: 137780-61-1) and by means of subsequent similar experimental operations.

LC/MS: $[M-H]^-$ = 412.1.

## Intermediate A3

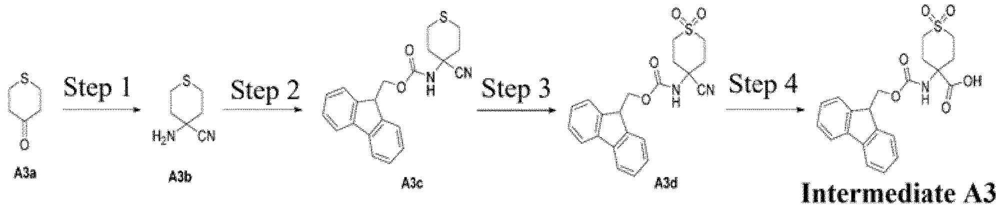


A3a A3b A3c A3d **Intermediate A3**

Step 1

At 0°C, an ammonia methanol solution (7M, 300 mL) was added to a solution of tetrahydropyran-4-one **A3a** (50 g, 431.0 mmol) in methanol (30 mL), and the mixture was maintained at 0°C and stirred for 3 h. Trimethylcyanosilane (44.9 g, 452.6 mmol) was then dropwise added, and after the addition was complete, the mixture was gradually heated to room temperature and stirred for 1.5 h. The reaction liquid was concentrated to obtain a light brown oil, which was slurried with petroleum ether (300 mL) at 0°C. A solid was precipitated. After filtration and then drying, 4-aminotetrahydropyran-4-carbonitrile **A3b** (61.5 g) was obtained, which was directly brought to the next reaction without further purification.
LC/MS: $[M+H]^+$ = 143.1.

Step 2

**A3b** (61.5 g, 433.0 mmol) was dissolved in a mixed solution of dioxane (300 mL) and water (300 mL), and sodium carbonate (55.1 g, 519.7 mmol) was added. A solution of 9-fluorenylmethyl chloroformate (117.6 g, 454.7 mmol) in dioxane (100 mL) was dropwise added to the reaction liquid at 0°C, and the mixture was transferred to room temperature and stirred for 1 h. The reaction liquid was slurried by adding water (400 mL) and filtered to obtain a white solid, and the obtained solid was slurried with petroleum ether (300 mL), filtered, and dried to obtain (9H-fluoren-9-yl)methyl(4-cyanotetrahydro-2H-thiopyran-4-yl)carbamate **A3c** (124 g), with a yield of 79%.
LC/MS: $[M+H]^+$ = 365.2.

Step 3

(9*H*-fluoren-9-yl)methyl(4-cyanotetrahydro-2*H*-thiopyran-4-yl)carbamate **A3c** (50.0 g, 137.4 mmol) was dissolved in dichloromethane (150 mL), 85% m-chloroperoxybenzoic acid (83.7 g, 412.1 mmol) was added in portions at 0°C, and the mixture was gradually heated to room temperature and stirred overnight. The reaction liquid was filtered, and the filter cake was dissolved clear with dichloromethane/methanol = 10:1 (800 mL) and then washed with a saturated sodium bicarbonate solution (300 mL * 4). The organic phase was dried and then concentrated to obtain (9*H*-fluoren-9-yl)methyl(4-cyano-1,1-dioxotetrahydro-2*H*-thiopyran-4-yl)carbamate **A3d** (38.1 g), with a yield of 28.2%.
LC/MS: $[M+H]^+$ = 396.2.

Step 4

**A3d** (38.1 g, 96.2 mmol) was dissolved in a solution of 4N hydrochloric acid in dioxane (300 mL), concentrated hydrochloric acid (400 mL) was then added, and the mixture was stirred for 30 h at 90°C 下. The reaction liquid was concentrated to 200 mL and slurried by adding water (400 mL), whereupon a solid was precipitated. After filtration, the resulting solid was slurried with ethyl acetate (200 mL), and after filtration, the resulting solid was dried to obtain **intermediate A3** (12.77 g, purity: 97.80%).

LC/MS: $[M+H]^+$ = 416.2;
$^1$H NMR (400 MHz, DMSO-$d_6$) δ 7.91 (d, 3H),7.72 (d, 2H), 7.43 (t, 2H), 7.35 (td, 2H), 4.35 (d, 2H), 4.25 (t, 1H), 3.09 (s, 4H), 2.46 (s, 2H), 2.29 (s, 2H).

## Intermediate B

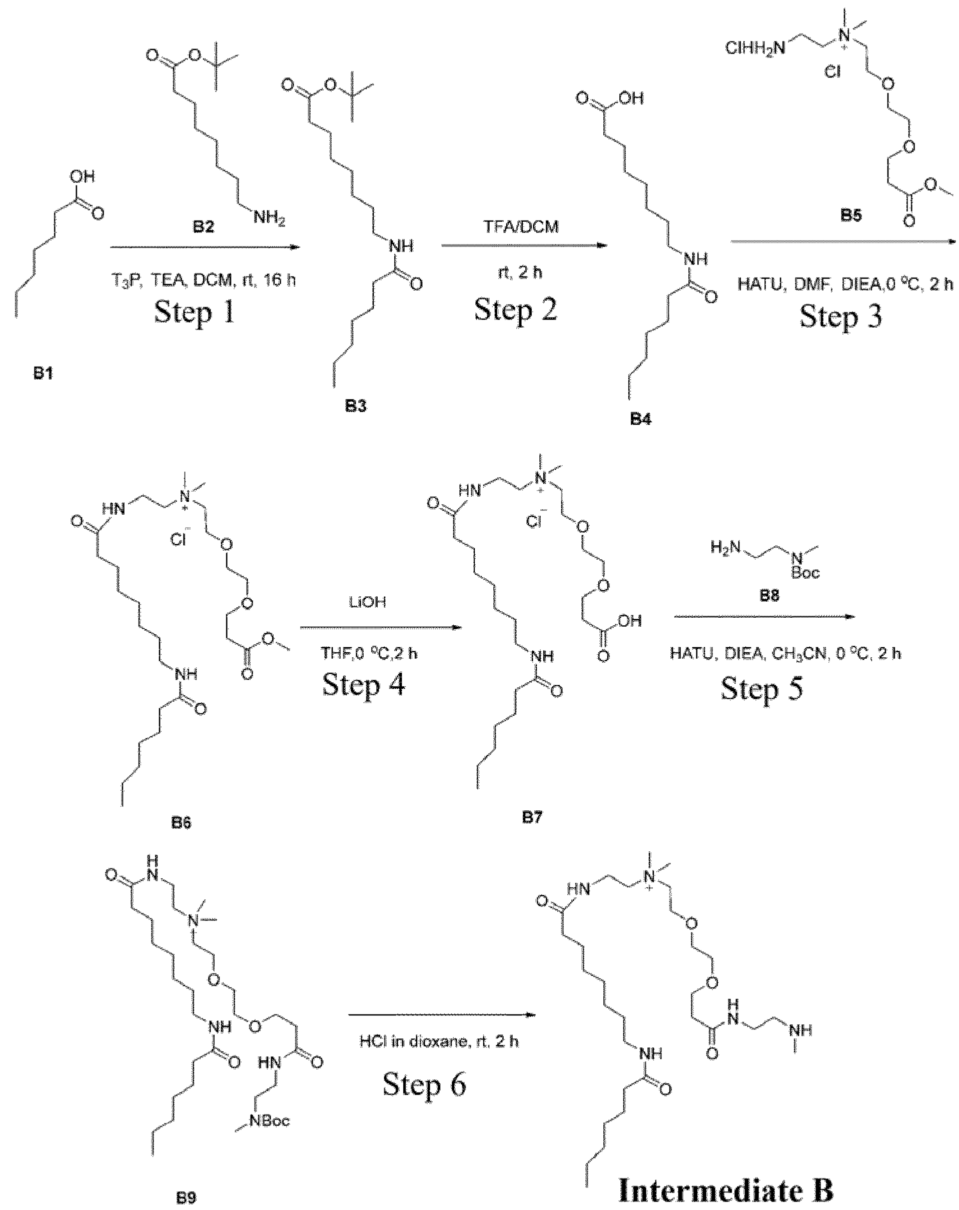


B1 B3 B4 B6 B7 B9 Intermediate B

Step 1

At room temperature, compound **B2** (3.5 g, 16.25 mmol) and TEA (4.93 g, 48.72 mmol) were added to a solution of compound **B1** (2.1 g, 16.13 mmol) in DCM (21 mL). $T_3P$ (50%, 20.69 g, 32.51 mmol) was further added in an ice bath. The mixture was stirred for 16 hours at room temperature and then concentrated, ethyl acetate and water were added, and the aqueous phase was further extracted twice with ethyl acetate. The combined organic phases were washed with 1M HCl and then with a saturated $NaHCO_3$ solution, dried over $Na_2SO_4$, and then filtered, and the filtrate was concentrated to obtain compound **B3** (5 g), with a yield of 94.6%. The crude product could be directly used in the next reaction.
LC/MS: $[M+H]^+$-56 = 272.2.

Step 2

Compound **B3** (5 g, 15.27 mmol) was dissolved in a solution of TFA/DCM (v:v = 1:1, 50 mL) and stirred for 2 hours at room temperature. The reaction liquid was concentrated, then dissolved with an appropriate amount of DCM, and then concentrated. After the operation was repeated 3 times, the system was dried and DCM was concentrated to obtain compound **B4** (2.7 g), with a yield of 65.2%. The crude product could be directly used in the next reaction.
LC/MS: $[M+H]^+$ = 272.2.

Step 3

At room temperature, compound **B5** (4.47 g, 14.92 mmol) was added to a solution of compound **B4** (2.7 g, 9.95 mmol) in DMF (30 mL). After cooling in an ice bath, HATU (4.54 g, 11.94 mmol) and DIEA (7.71 g, 59.69 mmol) were added to the above solution. The mixture was stirred for 2 hours at 0°C and then concentrated, and the residue was dissolved by adding an appropriate amount of $CH_3CN$ and purified by reverse column chromatography (5-40%, 40-70%, $CH_3CN$/0.03% TFA/water) to obtain compound **B6** (1.5 g), with a yield of 29.4%.

LC/MS: $[M]^+$ = 516.4.

Step 4

In an ice bath, LiOH (244 mg, 5.8 mmol) was added to a solution of compound **B6** (1.5 g, 2.9 mmol) in THF(15 mL) and stirred for 2 hours at 0°C. The reaction liquid was adjusted to neutral pH with 1 M HCl and then concentrated, and the residue was dissolved with an appropriate amount of $CH_3CN$ and purified by reverse column chromatography (5-40%, 40-70%, $CH_3CN$/0.03% TFA/water) to obtain compound **B7** (1.3 g), with a yield of 89.6%.

LC/MS: $[M]^+$ = 502.4.

Step 5

At room temperature, compound **B8** (763 mg, 4.4 mmol) was added to a solution of compound **B7** (1.1 g, 2.2 mmol) in $CH_3CN$ (10 mL). After cooling in an ice bath, HATU (1.0 g, 2.6 mmol) and DIEA (1.41 g, 10.9 mmol) were added. The mixture was stirred for 2 hours at 0°C and then concentrated, and the residue was dissolved by adding an appropriate amount of $CH_3CN$ and purified by reverse column chromatography (5-40%, 40-70%, $CH_3CN$/0.03% TFA/water) to obtain compound **B9** (1.3 g), with a yield of 89.8%.

LC/MS: $[M]^+$ = 658.6.

Step 6

Compound **B9** (1.3 g, 1.97 mmol) was dissolved in HCl/dioxane (4N, 100 mL) and stirred for 2 hour at room temperature. The reaction liquid was concentrated to obtain the compound **intermediate B** (as hydrochloride, 1.08 g), with a yield of 98.2%.

LC/MS: $[M]^+$ = 558.6;

$^1H$ NMR (400 MHz, MeOD) δ 3.94 (s, 2H), 3.76 (t, *J* = 6.2 Hz, 2H), 3.66 (t, *J* = 4.5 Hz, 8H), 3.52 (dd, *J* = 12.9, 6.7 Hz, 4H), 3.27 - 3.07 (m, 10H), 2.73 (s, 3H), 2.52 (t, *J* = 6.1 Hz, 2H), 2.23 (td, *J* = 7.6, 3.8 Hz, 4H), 1.61 (s, 4H), 1.50 (d, *J* = 6.6 Hz, 2H), 1.33 (d, *J* = 9.9 Hz, 12H), 0.91 (t, *J* = 6.7 Hz, 3H).

## Intermediate C

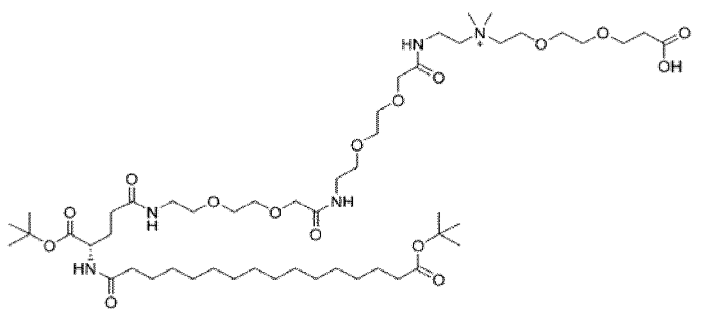

Intermediate C

By reference to the preparation method of WO 2021/127460, **intermediate C** was synthesized.

## Intermediate D

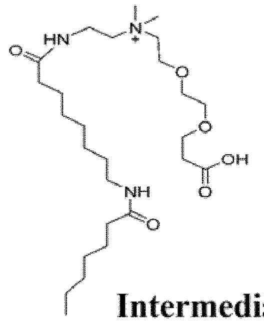

Intermediate D

By reference to the preparation method of WO 2021/041770, **intermediate D** was synthesized.

## Intermediate L1

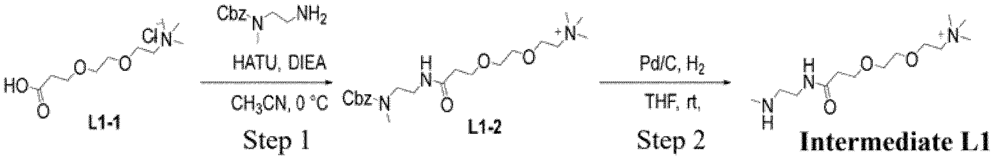


Step 1

At 0°C, DIEA (2.73 g, 21.1 mmol), HATU (1.61 g, 4.22 mmol), and *N*-Cbz-*N*-methylethanediamine (hydrochloride, 1.29 g, 5.28 mmol) were successively added to a solution of compound **L1-1** (0.9 g, 3.52 mmol) in $CH_3CN$ (25 mL), and the solution was then stirred for 1 hour at 0°C. The mixture was concentrated under reduced pressure, and the residue was purified by reverse column chromatography ($CH_3CN$/0.03% TFA/water, 0-100% gradient elution) to obtain compound **L1-2** (as a TFA salt, 1.35 g), with a yield of 73%.

LC/MS: $[M]^+$ = 410.3.

Step 2

At room temperature, 10% Pd/C (120 mg) was added to a solution of compound **L1-2** (1.2 g, 2.29 mmol) in THF (50 mL), and the reaction was stirred for 16 hours in a hydrogen atmosphere (1.5 atmospheres) at room temperature. The reaction liquid was filtered through diatomite, the filtrate was concentrated under reduced pressure, and the residue was purified by reverse column chromatography ($CH_3CN$/0.03% TFA/water, 0-100% gradient elution) to obtain the compound **intermediate L1** (as a TFA salt, 760 mg), with a yield of 85%.

LC/MS: $[M]^+$ = 276.2.

$^1H$ NMR (400 MHz, MeOD) δ 3.99 - 3.91 (m, 2H), 3.77 (t, $J$ = 6.2 Hz, 2H), 3.70 - 3.62 (m, 4H), 3.62 - 3.52 (m, 4H), 3.23 (s, 9H), 3.10 (t, $J$ = 5.6 Hz, 2H), 2.70 (s, 3H), 2.57 (t, $J$ = 6.2 Hz, 2H).

## Intermediate L2

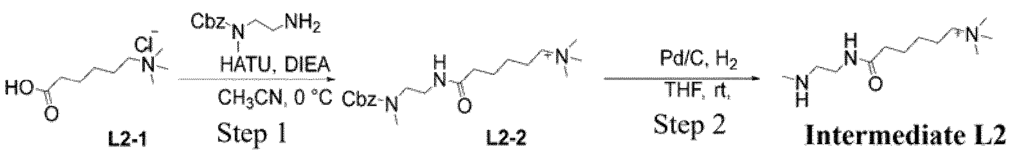


Step 1

At 0°C, DIEA (2.22 g, 17.2 mmol), HATU (1.31 g, 3.43 mmol), and *N*-Cbz-*N*-methylethanediamine (hydrochloride, 1.05 g, 4.29 mmol) were successively added to a solution of compound **L2-1** (0.6 g, 2.86 mmol) in $CH_3CN$ (20 mL), and the solution was then stirred for 1 hour at 0°C. The mixture was concentrated under reduced pressure, and the residue was purified by reverse column chromatography ($CH_3CN$/0.03% TFA/water, 0-100% gradient elution) to obtain

compound **L2-2** (as a TFA salt, 1.17 g), with a yield of 86%.

LC/MS: $[M]^+$ = 364.3.

Step 2

At room temperature, 10% Pd/C (100 mg) was added to a solution of compound **L2-2** (1.0 g, 2.29 mmol) in THF (50 mL), and the reaction was stirred for 16 hours in a hydrogen atmosphere (1.5 atmospheres) at room temperature. The reaction liquid was filtered through diatomite, the filtrate was concentrated under reduced pressure, and the residue was purified by reverse column chromatography ($CH_3CN$/0.03% TFA/water, 0-100% gradient elution) to obtain the compound **intermediate L2** (as a TFA salt, 0.66 mg), with a yield of 92%.

LC/MS: $[M]^+$ = 230.

$^1$H NMR (400 MHz, DMSO-$d_6$) δ 8.47-8.37 (m, 2H), 3.35 - 3.21 (m, 4H), 3.05 (s, 9H), 2.79 (t, $J$ = 6.0 Hz, 2H), 2.53 - 2.45 (m, 3H), 2.16-2.09 (m, 2H), 1.72 - 1.62 (m, 2H), 1.60-1.51 (m, 2H), 1.29-1.21 (m, 2H).

## Intermediate L3

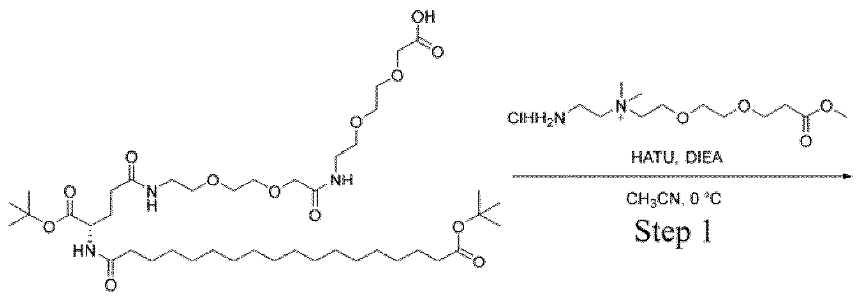


L3-1

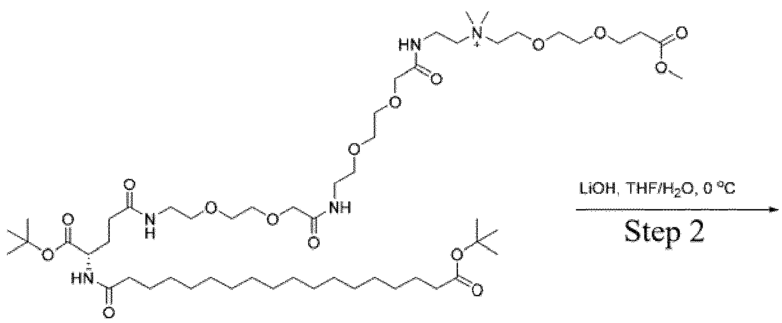


L3-2

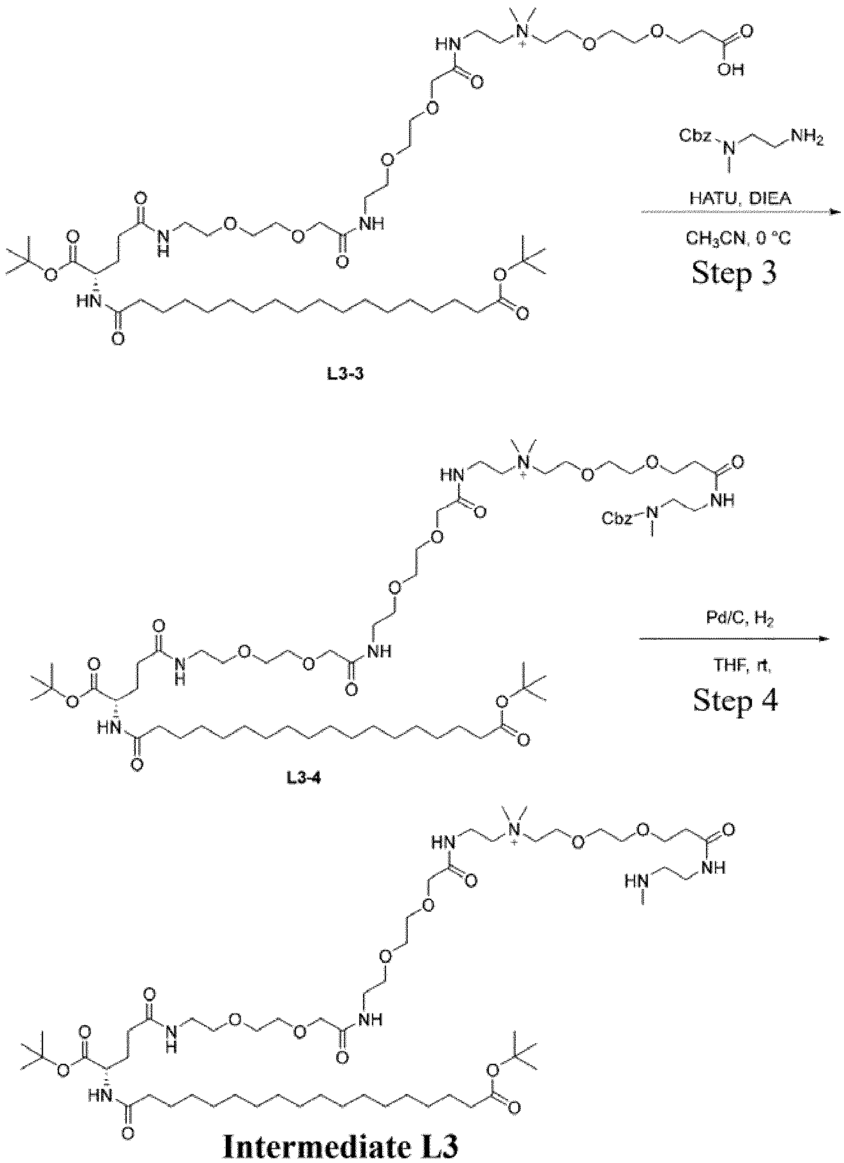


L3-3

L3-4

Intermediate L3

Step 1

At 0°C, DIEA (3.67 g, 28.37 mmol), HATU (2.16 g, 5.67 mmol), and 2-amino-N-(2-(2-(3-methoxy-3-oxopropoxy) ethoxy)ethyl)-*N,N*-dimethylethane-1-ammonium hydrochloride (2.13 g, 7.09 mmol) were successively added to a solution of compound L3-1 (CAS: 1118767-16-0, 4.0 g, 4.73 mmol) in $CH_3CN$ (45 mL), and the solution was then stirred for 1 hour at 0°C. The mixture was concentrated under reduced pressure, and the residue was purified by reverse column chromatography (5-40%, 40-70%, $CH_3CN$/0.03% TFA/water) to obtain compound **L3-2** (as a TFA salt, 3.05 g), with a yield of 53.6%.
LC/MS: $[M]^+$ = 1090.8.

Step 2

At 0°C, an aqueous LiOH solution (143 mg, 5.97 mmol, 12 mL) was added to a solution of compound **L3-2** (2.4 g, 1.99 mmol) in THF (24 mL) and reacted for 1 hour at this temperature. The reaction liquid was concentrated to remove THF, the residue was adjusted to pH = 7 by dropwise adding 1N HCl, and the mixture was purified by reverse column chromatography (5-40%, 40-70%, $CH_3CN$/0.03% TFA/water) to obtain compound **L3-3** (as a TFA salt, 1.1 g), with a yield of 46.4%.
LC/MS: $[M]^+$ = 1076.7.

Step 3

At 0°C, DIEA (521 mg, 4.03 mmol), HATU (306 mg, 0.81 mmol), and*N*-Cbz-N-methylethanediamine (hydrochloride, 245 mg, 1.01 mmol) were successively added to a solution of compound **L3-3** (0.8 g, 0.67 mmol) in $CH_3CN$ (12 mL), and the solution was then stirred for 1 hour at 0°C. The mixture was concentrated under reduced pressure, and the residue was purified by reverse column chromatography (5-40%, 40-70%, $CH_3CN$/0.03% TFA/water) to obtain compound **L3-4** (as a TFA salt, 870 mg), with a yield of 93.8%.
LC/MS: $[(M+H)/2]^+ = 632.9$.

Step 4

At room temperature, 10% Pd/C (65 mg) was added to a solution of compound **L3-4** (650 mg, 0.56 mmol) in THF (20 mL), and the reaction was stirred for 16 hours in a hydrogen atmosphere (1.5 atmospheres) at room temperature. The reaction liquid was filtered through diatomite, the filtrate was concentrated under reduced pressure, and the residue was purified by reverse column chromatography (5-40%, 40-70%, $CH_3CN$/0.03% TFA/water) to obtain the compound **intermediate L3** (as a TFA salt, 570 mg), with a yield of 97.1%.
LC/MS: $[M]^+ = 1132.8$.

## Intermediate L4

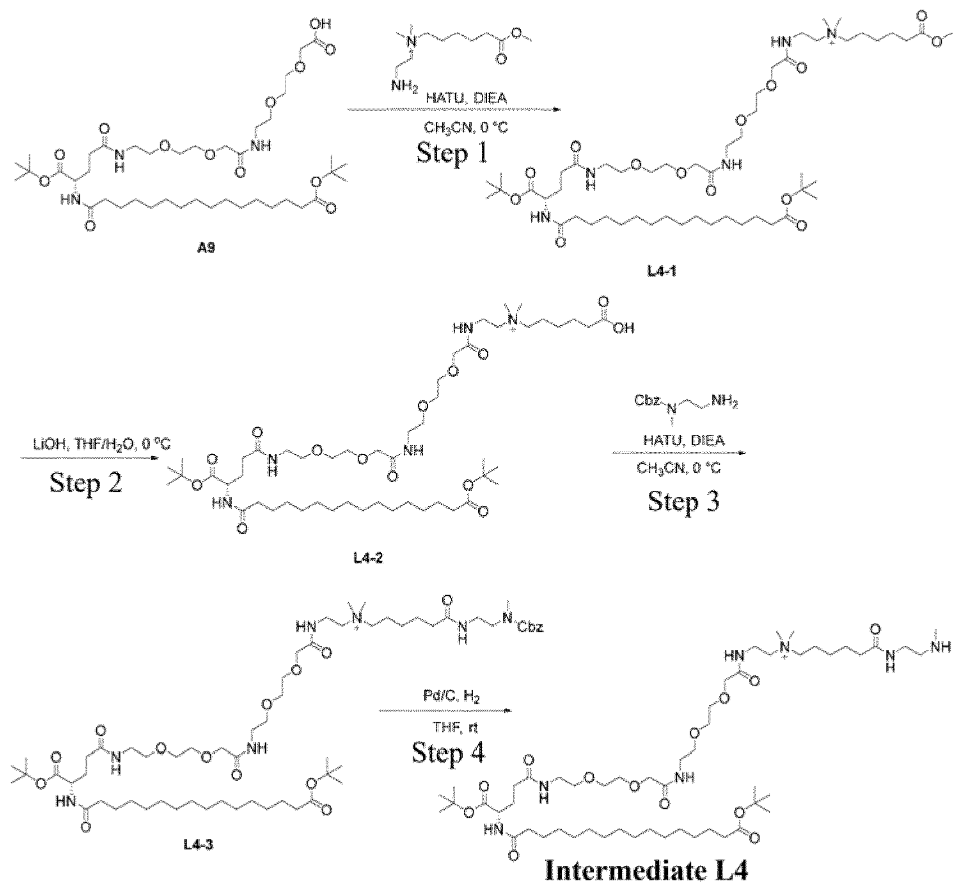


A9 L4-1 L4-2 L4-3 Intermediate L4

Step 1

At 0°C, DIEA (2.84 g, 22.0 mmol), HATU (1.67 g, 4.40 mmol), and N-(2-aminoethyl)-6-methoxy-*N,N*-dimethyl-6-oxohexane-1-ammonium hydrochloride (1.4 g, 5.50 mmol) were successively added to a solution of compound **A9** (3.0 g, 3.67 mmol) in $CH_3CN$ (40 mL), and the solution was then stirred for 1 hour at 0°C. The mixture was concentrated under reduced pressure, and the residue was purified by reverse column chromatography (5-40%, 40-70%, $CH_3CN$/0.03% TFA/water) to obtain compound **L4-1** (as a TFA salt, 1.75 g), with a yield of 42.2%.
LC/MS: $[M]^+ = 1016.7$.

Step 2

At 0°C, an aqueous LiOH solution (89 mg, 3.71 mmol, 5 mL) was added to a solution of compound **L4-1** (1.4 g, 1.24 mmol) in THF (15 mL) and reacted for 1 hour at this temperature. The reaction liquid was concentrated to remove THF, the residue was adjusted to pH = 7 by dropwise adding 1N HCl, and the mixture was purified by reverse column chromatography (5-40%, 40-70%, $CH_3CN$/0.03% TFA/water) to obtain compound **L4-2** (as a TFA salt, 740 mg), with a yield of 53.6%.

LC/MS: $[M]^+$ = 1002.7.

Step 3

At 0°C, DIEA (486 mg, 3.76 mmol), HATU (286 mg, 0.75 mmol), and*N*-Cbz-N-methylethanediamine (hydrochloride, 229 mg, 0.94 mmol) were successively added to a solution of compound **L3-3** (0.7 g, 0.63 mmol) in $CH_3CN$ (10 mL), and the solution was then stirred for 1 hour at 0°C. The mixture was concentrated under reduced pressure, and the residue was purified by reverse column chromatography (5-40%, 40-70%, $CH_3CN$/0.03% TFA/water) to obtain compound **L4-3** (as a TFA salt, 770 mg), with a yield of 94.0%.

LC/MS: $[(M+H)/2]^+$ = 596.9.

Step 4

At room temperature, 10% Pd/C (70 mg) was added to a solution of compound **L4-3** (0.7 g, 0.56 mmol) in THF (20 mL), and the reaction was stirred for 16 hours in a hydrogen atmosphere (1.5 atmospheres) at room temperature. The reaction liquid was filtered through diatomite, the filtrate was concentrated under reduced pressure, and the residue was purified by reverse column chromatography (5-40%, 40-70%, $CH_3CN$/0.03% TFA/water) to obtain the compound **intermediate L4** (as a TFA salt, 590 mg), with a yield of 93.9%.

LC/MS: $[M]^+$ = 1044.8.

$^1$H NMR (400 MHz, MeOD) δ 4.31-4.23 (m, 1H), 4.04 (d, *J* = 10.8 Hz, 4H), 3.77-3.67 (m, 10H), 3.64-3.56 (m, 4H), 3.53-3.45 (m, 6H), 3.43-3.36 (m, 4H), 3.20-3.12 (m, 8H), 2.75 (s, 3H), 2.37-2.20 (m, 8H), 2.18-2.09 (m, 1H), 1.98-1.81 (m, 4H), 1.79-1.70 (m, 2H), 1.69-1.53 (m, 5H), 1.47 (d, *J* = 8.0 Hz, 18H), 1.31 (s, 20H).

## Intermediate L5

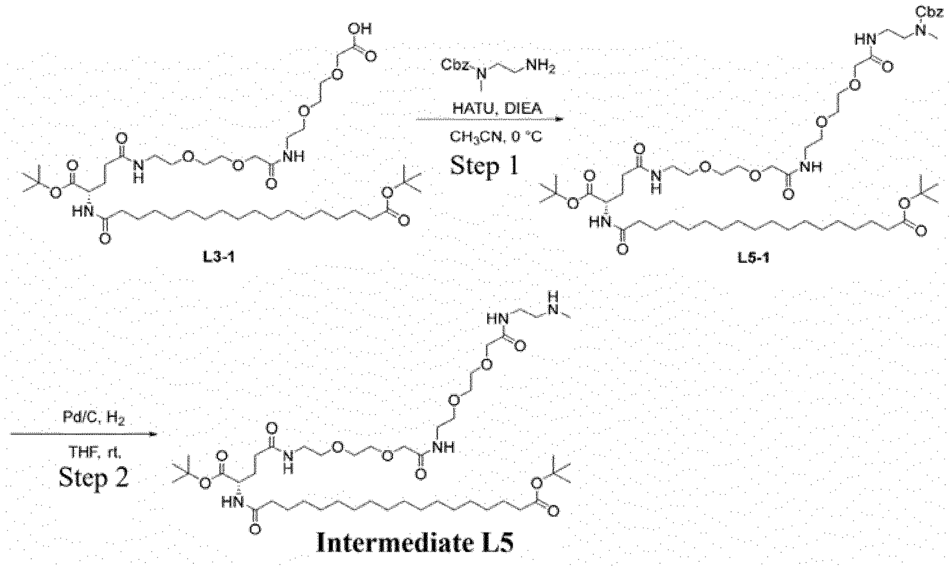


L3-1

L5-1

Intermediate L5

Step 1

At 0°C, DIEA (1.83 g, 14.2 mmol), HATU (1.08 g, 2.84 mmol), and*N*-Cbz-*N*-methylethanediamine (hydrochloride, 864 mg, 3.55 mmol) were successively added to a solution of compound **L3-1** (2 g, 2.36 mmol) in $CH_3CN$ (20 mL), and the solution was then stirred for 1 hour at 0°C. The mixture was concentrated under reduced pressure, and the residue was purified by reverse column chromatography ($CH_3CN$/0.03% TFA/water, 0-100% gradient elution) to obtain compound

**L5-1** (1.9 g), with a yield of 77.6%.
LC/MS: [M+H] $^{+}$ = 1036.7.

Step 2

At room temperature, 10% Pd/C (120 mg) was added to a solution of compound **L1-2** (1.2 g, 1.16 mmol) in THF (30 mL), and the reaction was stirred for 16 hours in a hydrogen atmosphere (1.5 atmospheres) at room temperature. The reaction liquid was filtered through diatomite, the filtrate was concentrated under reduced pressure, and the residue was purified by reverse column chromatography ($CH_3CN$/0.03% TFA/water, 0-100% gradient elution) to the obtain compound **intermediate L5** (940 mg), with a yield of 90%.
LC/MS: [M+H] $^{+}$ = 902.6.
$^{1}$H NMR (400 MHz, MeOD) δ 4.27 (dd, *J* = 9.1, 5.2 Hz, 1H), 4.04 (d, *J* = 5.9 Hz, 4H), 3.70 (s, 6H), 3.68 (d, *J* = 5.6 Hz, 3H), 3.62 (dd, *J* = 12.1, 6.8 Hz, 3H), 3.56 (dd, *J* = 9.5, 5.7 Hz, 4H), 3.51 - 3.44 (m, 3H), 3.41 (d, *J* = 5.9 Hz, 2H), 3.08 (t, *J* = 5.8 Hz, 2H), 2.67 (s, 3H), 2.31 (t, *J* = 7.6 Hz, 2H), 2.28 - 2.19 (m, 5H), 2.13 (dd, *J* = 13.7, 5.9 Hz, 1H), 2.05 (d, *J* = 5.5 Hz, 1H), 1.91 (dd, *J* = 13.9, 9.1 Hz, 1H), 1.61 (dd, *J* = 16.0, 8.1 Hz, 4H), 1.48 (d, *J* = 9.0 Hz, 18H), 1.31 (s, 24H).

**Example 1**

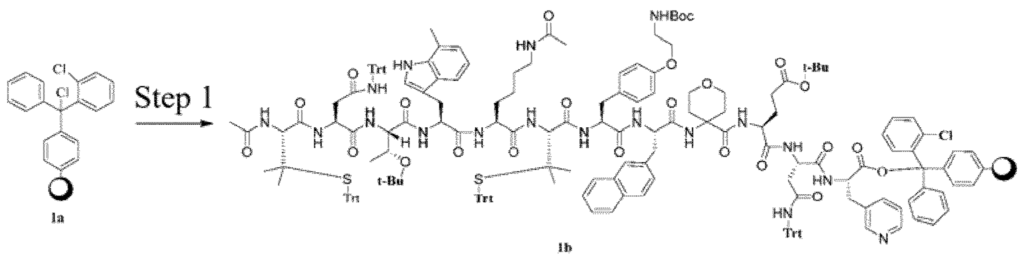


1a

1b

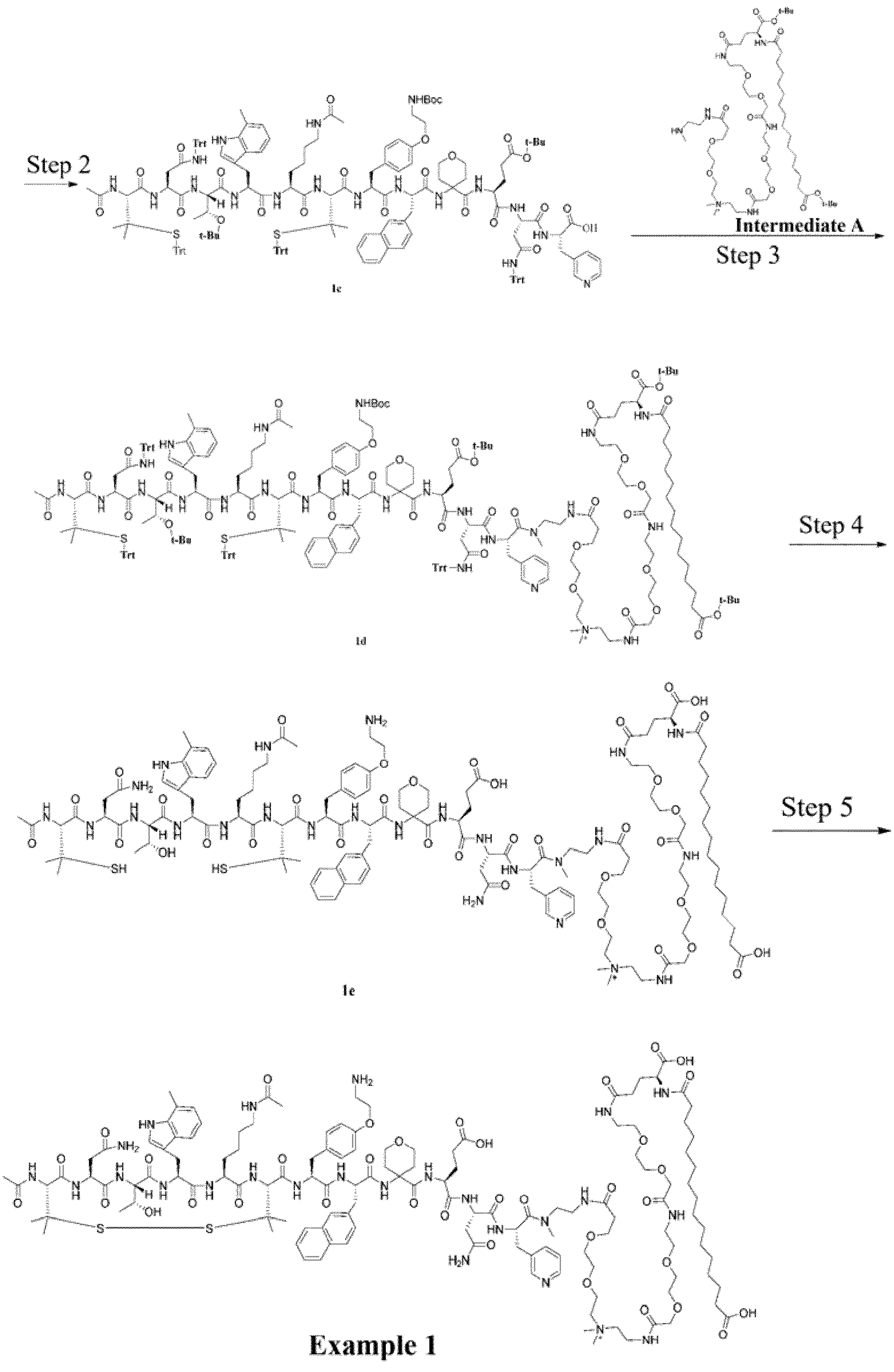


1c

1d

1e

**Example 1**

Step 1: Synthesis of fully protected linear peptide

1.1 Resin attachment

1.1.1 0.6 g of 2-CTC Resin (degree of substitution S = 1.08 mmol/g) and 78 mg of N-FMOC-L-3-pyridylalanine (0.2 mmol, degree of substitution 0.33) were weighed and added to a reaction column, followed by the addition of DCM (10 mL). Subsequently, 0.6 mL of DIEA was added to the reaction column, followed by sparging with nitrogen for 2 hrs. 0.6 mL of MeOH was then added to the reaction column, followed by continued sparging with nitrogen for 30 min. Waste was

discharged until no liquid flowed out. After washing 5 times by adding DMF (30 mL), with 1 min each time, waste was discharged until no liquid flowed out.

1.1.2 20% piperidine/DMF (15 mL) was added to the reaction column, followed by sparging with nitrogen for 20 min, and waste was discharged until no liquid flowed out. After washing 5 times by adding DMF (30 mL), with 1 min each time, waste was discharged until no liquid flowed out. After detection with ninhydrin, the resin was blue.

1.2. Coupling of amino acids

1.2.1 Coupling of Fmoc-Asn(Trt)-OH

| Raw material | Feeding amount |
|---|---|
| **Fmoc-Asn(Trt)-OH** | 3.0 eq |
| **HBTU** | 2.85 eq |
| **DIEA** | 6.00 eq |

1. Fmoc-Asn(Trt)-OH (3.0 eq) was weighed and added to the above resin, followed by the addition of DIEA (6.00 eq) was added. 5 mL of DMF was additionally added to the reaction column, followed by sparging with nitrogen. After amino acids were dissolved, HBTU (2.85 eq) was added. Nitrogen was properly adjusted to make the resin swell evenly.
2. The reaction was carried out for 0.5 h in a 25°C environment, and after detection with ninhydrin, the resin was colorless and transparent.
3. The reaction liquid was drained, and after washing 5 times with DMF (20 mL each time) for 1 min each time, waste was discharged until no liquid flowed out.

1.2.2 Coupling of Fmoc-Glu(OtBu)-OH

| Raw material | Feeding amount |
|---|---|
| **Fmoc-Glu(OtBu)-OH** | 3.0 eq |
| **HBTU** | 2.85 eq |
| **DIEA** | 6.00 eq |

1. 20% piperidine/DMF(20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-Glu(OtBu)-OH (3.0 eq) was weighed and added to the above resin, followed by the addition of DIEA (6.00 eq). 5 mL of DMF was additionally added to the reaction column, followed by sparging with nitrogen. After amino acids were dissolved, HBTU (2.85 eq) was added. Nitrogen was properly adjusted to make the resin swell evenly.
3. The reaction was carried out for 0.5 h in a 25°C environment, and after detection with ninhydrin, the resin was colorless and transparent.
4. The reaction liquid was drained, and after washing 5 times with DMF (20 mL each time) for 1 min each time, waste was discharged until no liquid flowed out.

1.2.3 Coupling of Fmoc-Thp(Gly)-OH

| Raw material | Feeding amount |
|---|---|
| **Fmoc-Thp(Gly)-OH** | 3.0 eq |
| **HATU** | 2.85 eq |

(continued)

| Raw material | Feeding amount |
|---|---|
| **DIEA** | 6.00 eq |

1. 20% piperidine/DMF(20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-Thp(Gly)-OH (3.0 eq) was weighed and added to the above resin, followed by the addition of DIEA (6.00 eq). 5 mL of DMF was additionally added to the reaction column, followed by sparging with nitrogen. After amino acids were dissolved, HATU (2.85 eq) was added. Nitrogen was properly adjusted to make the resin swell evenly.
3. The reaction was carried out for 0.5 h in a 25°C environment, and after detection with ninhydrin, the resin was colorless and transparent.
4. The reaction liquid was drained, and after washing 5 times with DMF for 1 min each time, waste was discharged until no liquid flowed out.

1.2.4 Coupling of Fmoc-2Nal-OH

| Raw material | Feeding amount |
|---|---|
| **Fmoc-2Nal-OH** | 3.0 eq |
| **HATU** | 2.85 eq |
| **DIEA** | 6.00 eq |

1. 20% piperidine/DMF(20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with tetrachlorobenzoquinone, the resin was green.
2. Fmoc-2-Nal-OH (3.0 eq) was weighed and added to the above resin, followed by the addition of DIEA (6.00 eq). 5 mL of DMF was additionally added to the reaction column, followed by sparging with nitrogen. After amino acids were dissolved, HATU (2.85 eq) was added. Nitrogen was properly adjusted to make the resin swell evenly.
3. The reaction was carried out for 0.5 h in a 25°C environment, and after detection with tetrachlorobenzoquinone, the resin was colorless and transparent.
4. The reaction liquid was drained, and after washing 5 times with DMF (20 mL each time) for 1 min each time, waste was discharged until no liquid flowed out.

1.2.5 Coupling of Fmoc-4-[2-(Boc-amino)ethoxy]-L-Phenylalanine

| Raw material | Feeding amount |
|---|---|
| **Fmoc-4-[2-(Boc-amino)ethoxy]-L-Phenylalanine** | 3.0 eq |
| **HBTU** | 2.85 eq |
| **DIEA** | 6.00 eq |

1. 20% piperidine/DMF(20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-4-[2-(Boc-amino)ethoxy]-L-Phenylalanine (3.0 eq) was weighed and added to the above resin, followed by the addition of DIEA (6.00 eq). 5 mL of DMF was additionally added to the reaction column, followed by sparging with nitrogen. After amino acids were dissolved, HBTU (2.85 eq) was added. Nitrogen was properly adjusted to make the resin swell evenly.
3. The reaction was carried out for 0.5 h in a 25°C environment, and after detection with ninhydrin, the resin was

colorless and transparent.
4. The reaction liquid was drained, and after washing 5 times with DMF (20 mL each time) for 1 min each time, waste was discharged until no liquid flowed out.

1.2.6 Coupling of Fmoc-Pen(Trt)-OH

| Raw material | Feeding amount |
|---|---|
| **Fmoc-Pen(Trt)-OH** | 3.0 eq |
| **HBTU** | 2.85 eq |
| **DIEA** | 6.00 eq |

1. 20% piperidine/DMF(20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-Pen(Trt)-OH (3.0 eq) was weighed and added to the above resin, followed by the addition of DIEA (6.00 eq) was added. 5 mL of DMF was additionally added to the reaction column, followed by sparging with nitrogen. After amino acids were dissolved, HBTU (2.85 eq) was added. Nitrogen was properly adjusted to make the resin swell evenly.
3. The reaction was carried out for 0.5 h in a 25°C environment, and after detection with ninhydrin, the resin was colorless and transparent.
4. The reaction liquid was drained, and after washing 5 times with DMF (20 mL each time) for 1 min each time, waste was discharged until no liquid flowed out.

1.2.7 Coupling of Fmoc-Lys(Ac)-OH

| Raw material | Feeding amount |
|---|---|
| **Fmoc-Lys(Ac)-OH** | 3.0 eq |
| **HBTU** | 2.85 eq |
| **DIEA** | 6.00 eq |

1. 20% piperidine/DMF(20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-Lys(Ac)-OH (3.0 eq) was weighed and added to the above resin, followed by the addition of DIEA (6.00 eq) was added. 5 mL of DMF was additionally added to the reaction column, followed by sparging with nitrogen. After amino acids were dissolved, HBTU (2.85 eq) was added. Nitrogen was properly adjusted to make the resin swell evenly.
3. The reaction was carried out for 0.5 h in a 25°C environment, and after detection with ninhydrin, the resin was colorless and transparent.
4. The reaction liquid was drained, and after washing 5 times with DMF (20 mL each time) for 1 min each time, waste was discharged until no liquid flowed out.

1.2.8 Coupling of Fmoc-(7Me)Trp-OH

| Raw material | Feeding amount |
|---|---|
| **Fmoc-(7Me)Trp-OH** | 3.0 eq |
| **HATU** | 2.85 eq |

(continued)

| Raw material | Feeding amount |
|---|---|
| **DIEA** | 6.00 eq |

1. 20% piperidine/DMF(20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-(7Me)Trp-OH (3.0 eq) was weighed and added to the above resin, followed by the addition of DIEA (6.00 eq). 5 mL of DMF was additionally added to the reaction column, followed by sparging with nitrogen. After amino acids were dissolved, HBTU (2.85 eq) was added. Nitrogen was properly adjusted to make the resin swell evenly.
3. The reaction was carried out for 0.5 h in a 25°C environment, and after detection with ninhydrin, the resin was colorless and transparent.
4. The reaction liquid was drained, and after washing 5 times with DMF (20 mL each time) for 1 min each time, waste was discharged until no liquid flowed out.

1.2.9 Coupling of Fmoc-Thr(Tbu)-OH

| Raw material | Feeding amount |
|---|---|
| **Fmoc-Thr(Tbu)-OH** | 3.0 eq |
| **HBTU** | 2.85 eq |
| **DIEA** | 6.00 eq |

1. 20% piperidine/DMF(20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-Thr(Tbu)-OH (3.0 eq) was weighed and added to the above resin, followed by the addition of DIEA (6.00 eq) was added. 5 mL of DMF was additionally added to the reaction column, followed by sparging with nitrogen. After amino acids were dissolved, HBTU (2.85 eq) was added. Nitrogen was properly adjusted to make the resin swell evenly.
3. The reaction was carried out for 0.5 h in a 25°C environment, and after detection with ninhydrin, the resin was colorless and transparent.
4. The reaction liquid was drained, and after washing 5 times with DMF (20 mL each time) for 1 min each time, waste was discharged until no liquid flowed out.

1.2.10 Coupling of Fmoc-Asn(Trt)-OH

| Raw material | Feeding amount |
|---|---|
| **Fmoc-Asn(Trt)-OH** | 3.0 eq |
| **HBTU** | 2.85 eq |
| **DIEA** | 6.00 eq |

1. 20% piperidine/DMF(20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-Asn (Trt)-OH (3.0 eq) was weighed and added to the above resin, followed by the addition of DIEA (6.00 eq) was added. 5 mL of DMF was additionally added to the reaction column, followed by sparging with nitrogen. After amino acids were dissolved, HBTU (2.85 eq) was added. Nitrogen was properly adjusted to make the resin swell evenly.
3. The reaction was carried out for 0.5 h in a 25°C environment, and after detection with ninhydrin, the resin was

colorless and transparent.

4. The reaction liquid was drained, and after washing 5 times with DMF (20 mL each time) for 1 min each time, waste was discharged until no liquid flowed out.

1.2.11 Coupling of Fmoc-Pen(Trt)-OH

| Raw material | Feeding amount |
|---|---|
| **Fmoc-Pen(Trt)-OH** | 3.0 eq |
| **HBTU** | 2.85 eq |
| **DIEA** | 6.00 eq |

1. 20% piperidine/DMF(20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.

2. Fmoc-Pen(Trt)-OH (3.0 eq) was weighed and added to the above resin, followed by the addition of DIEA (6.00 eq) was added. 5 mL of DMF was additionally added to the reaction column, followed by sparging with nitrogen. After amino acids were dissolved, HBTU (2.85 eq) was added. Nitrogen was properly adjusted to make the resin swell evenly.

3. The reaction was carried out for 0.5 h in a 25°C environment, and after detection with ninhydrin, the resin was colorless and transparent.

4. The reaction liquid was drained, and after washing 5 times with DMF (20 mL each time) for 1 min each time, waste was discharged until no liquid flowed out.

1.2.12 Capping with $Ac_2O$

| Raw material | Feeding amount |
|---|---|
| **$Ac_2O$** | 5.0 eq |
| **DIEA** | 6.00 eq |

1. 20% piperidine/DMF(20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.

2. DMF (10 mL) was added, and DIEA (6.00 eq) and $Ac_2O$ (5.0 eq) were added to the above resin, followed by sparging with nitrogen, such that nitrogen was properly adjusted to make the resin swell evenly.

3. The reaction was carried out for 10 min in a 25°C environment, and after detection with ninhydrin, the resin was colorless and transparent.

4. The reaction liquid was drained, and after washing 5 times with DMF (20 mL each time) for 1 min each time, waste was discharged until no liquid flowed out.

Step 2: Cleavage of fully protected linear peptide

2.1 After washing 5 times with DMF (20 mL each time) for 1 min each time, waste was discharged until no liquid flowed out.

2.2 The resin was shrunk with MeOH (20 mL) for 3 min each time, and waste was discharged until no liquid flowed out. The resin was then washed three times with 20 mL of methyl tert-butyl ether, and the resin was dried by blowing with nitrogen, for later use.

2.3 50 mL of a prepared 20% HFIP/DCM solution was poured into a reactor, and after sparging with nitrogen, reaction once for 30 min, and cleavage three times, the filtrate was all collected and concentrated under reduced pressure to dryness. 50 mL of DCM was then added for continued concentration under reduced pressure.

2.4 100 mL of acetonitrile and 100 mL of water were added, and the mixture was ultrasonicated until uniformly dispersed and then freeze-dried by a freeze-dryer to obtain 500 mg of intermediate **1c** with a purity of 75% and a yield of

62%.

LC/MS: $[M+H]^+$ = 3012.7; $[M+H-243]^+$ = 2769.7.

Step 3: Condensation of intermediate **1c** and **intermediate A**

200 mg of the above intermediate **1c** (0.06 mmol) was taken, 1 mL of DMF and DIEA (3 eq, 0.18 mmol) were added, followed by **intermediate A** (77 mg, 0.06 mmol, 1 eq), and HATU (23 mg, 0.06 mmol, 1 eq) dissolved in DMF (0.2 mL) was then slowly dropwise added. As detected by Tof-LCMS, the raw materials were completely consumed. The product had a very small polarity, and the gradient was 80-100% over 2 min. LC/MS: $[M]^+$ = 4099.20, $[M-243]^+$ = 3856.2.

After the reaction was complete, DMF was spin-dried by an oil pump for later use.

Step 4: Cleavage of linear peptide

4.1. Preparation of cleavage liquid according to the following volume

| Reagent | Proportion |
|---|---|
| **TFA** | 90 |
| **Tis** | 2.5 |
| **$H_2O$** | 2.5 |
| **Mpr** | 5.0 |

The crude peptide obtained in step 3 was added to the prepared cleavage liquid, shaken in a shaking table for 2.5 h, and filtered, and the filtrate was added to 10 volumes of ice-cold isopropyl ether, centrifuged, and then washed 3 times with isopropyl ether. After vacuum drying for 2 h, a crude peptide was obtained. LC/MS: $[(M+H)/2]^+$ = 1402.8; $[(M+2H)/3]^+$ = 936.1.

Step 5: Oxidation of disulfide bond

5.1 160 mg of the crude peptide obtained in step 4 was added to 100 mL of water and 100 mL of acetonitrile and dissolved clear, and 0.1M $I_2$/MeOH was then slowly dropwise added until the solution became bright yellow. After continued stirring for 5 min, the yellow color did not disappear. 0.1 M sodium thiosulfate was then dropwise added until the yellow color disappeared. When no change was found after stirring for two min, the sample was freeze-dried and purified to obtain 43 mg of a product of **Example 1,** with a purity of 97.41% and a yield of 18.7%.

LC/MS: $[(M+H)/2]^+$ = 1401.8; $[(M+2H)/3]^+$ = 935.1.

Purification conditions:

| Separation condition | |
|---|---|
| Dissolution condition | Dissolve in 30% $CH_3CN-H_2O$ |
| Instrument | Gilson GX-281 |
| Mobile phase | A: $H_2O$ (0.075% TFA in $H_2O$) |
| | B: $CH_3CN$ |
| Gradient | 30-60%-60 min. Retention time: 21 min |
| Column type | Gemini, 5 μm, c18, 110A+luna, c18, 10 μm, 100 A |
| Flow rate | 20 mL/Min |
| Detection wavelength | 214/254 nm |
| Column temperature | 30°C |

## Example 2

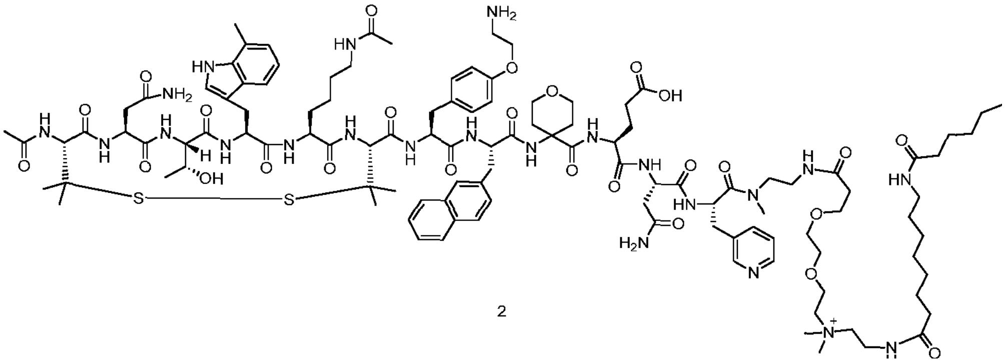

2

By reference to the synthesis method of **Example 1,** 26.4 mg of a product of **Example 2** was obtained by the reaction of **intermediate B** with polypeptide intermediate **1c** and the subsequent similar experimental operations, with a purity of 92.94% and a yield of 13.6%.

LC/MS: $[(M+H)/2]^+ = 1184.8$; $[(M+2H)/3]^+ = 790.2$.

## Example 3

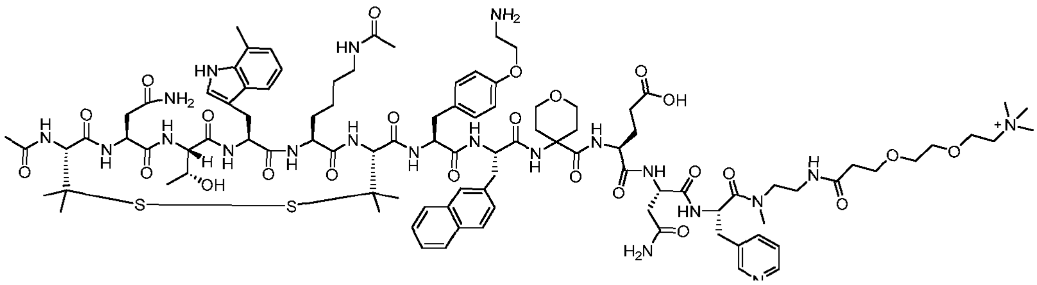

By reference to the synthesis method of **Example 1,** 42.0 mg of a product of **Example 3** was obtained by the reaction of **intermediate L1** with polypeptide intermediate **1c** and the subsequent similar experimental operations, with a purity of 96.4% and a yield of 20.2%.

LC/MS: $[(M+H)/2]^+ = 1043.5$.

## Example 4

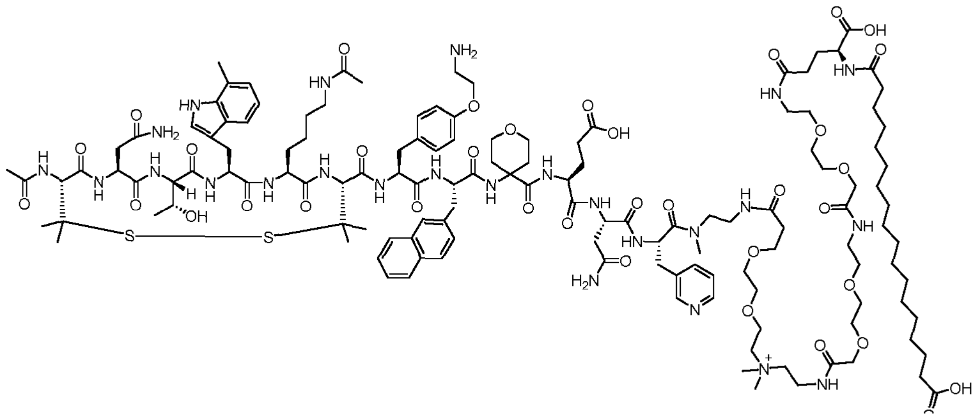

By reference to the synthesis method of **Example 1,** 28.1mg of a product of **Example 4** was obtained by the reaction of **intermediate L3** with polypeptide intermediate **1c** and the subsequent similar experimental operations, with a purity of 96.2% and a yield of 14.9%.

LC/MS: $[(M+H)/2]^+ = 1415.7$; $[(M+2H)/3]^+ = 944.2$.

**Example 6**

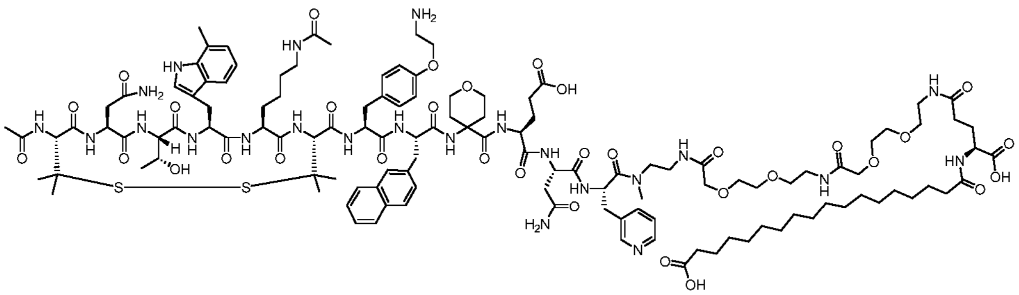

By reference to the synthesis method of **Example 1,** 31.2 mg of a product of **Example 6** was obtained by the reaction of **intermediate L5** with polypeptide intermediate **1c** and the subsequent similar experimental operations, with a purity of 97.8% and a yield of 18.1%.

LC/MS: $[(M+H)/2]^+ = 1300.1$; $[(M+2H)/3]^+ = 867.1$.

**Example 12**

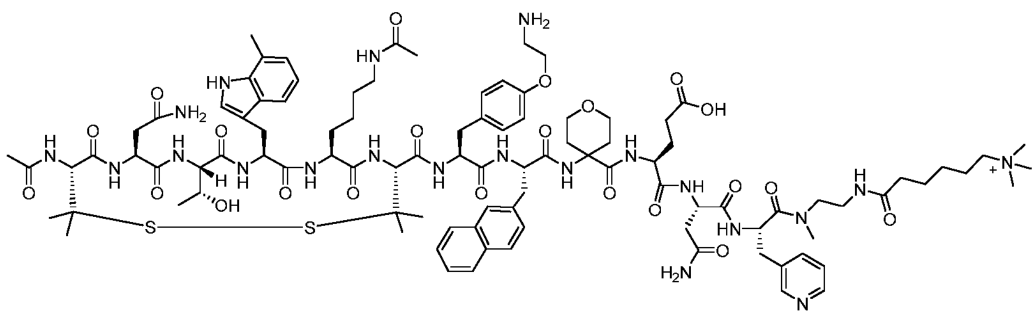

By reference to the synthesis method of **Example 1,** 23.8 mg of a product of **Example 12** was obtained by the reaction of **intermediate L2** with polypeptide intermediate **1c** and the subsequent similar experimental operations, with a purity of 95.7% and a yield of 17.5%.

LC/MS: [(M+H)/2] $^{+}$ = 1020.5.

**Example 12** could also be prepared by the following method:

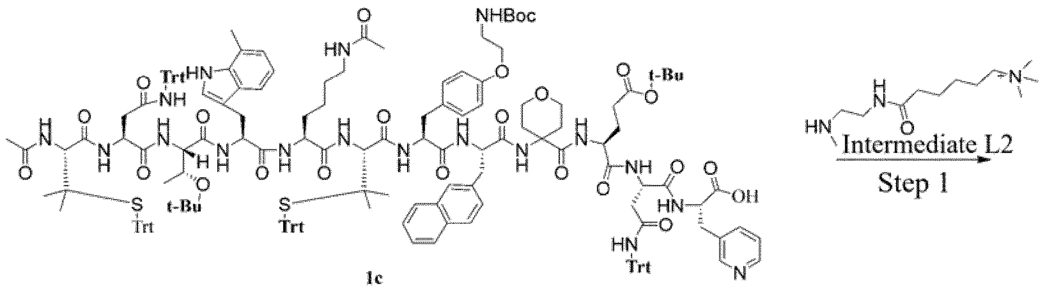


1c

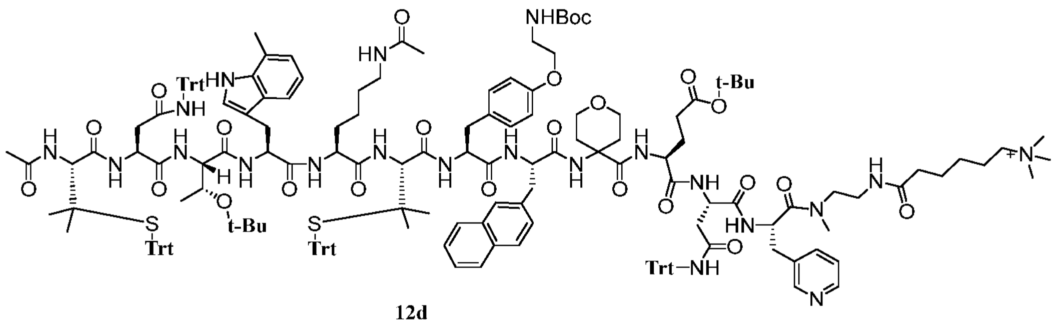

12d

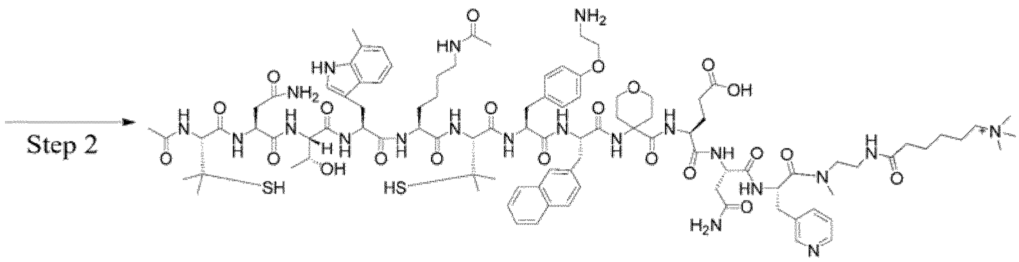


12e

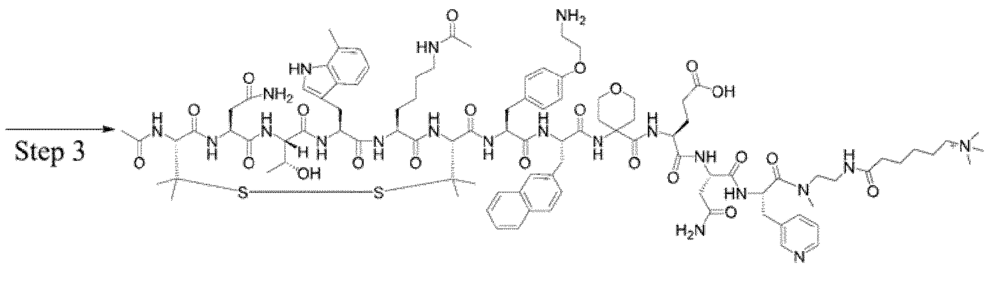


**Example 12**

Step 1: Condensation of intermediate **1c** and intermediate **L2**

The above intermediate **1c** (200 mg, 0.06 mmol) was taken, DMF (1 mL) and DIEA (10 eq, 0.6 mmol) were added, followed by **intermediate L2** (28 mg, 0.12 mmol, 2 eq), and HATU (69 mg, 0.18 mmol, 3 eq) dissolved in DMF (0.2 mL) was then slowly dropwise added. As detected by Tof-LCMS, the raw materials were completely consumed. After the reaction was complete, DMF was spin-dried to obtain intermediate **12d** for later use.

Step 2: Removal of protective group

Preparation of cleavage liquid according to the following volume

| Reagent | Proportion |
|---|---|
| **TFA** | 92.5 |
| **Tis** | 2.5 |
| **$H_2O$** | 2.5 |
| **DTT** | 2.5 |

The intermediate **12d** obtained in the previous step was added to the prepared cleavage liquid, shaken in a shaking table for 2.5 hours, and filtered, and the filtrate was added to 10 volumes of ice-cold isopropyl ether, centrifuged, then washed 3 times with isopropyl ether, and dried in vacuo for 2 hours to obtain intermediate **12e.**

Step 3: Oxidation and purification of disulfide bond and salt conversion

The intermediate **12e** obtained above in the previous step (190 mg) was added to water (160 mL) and acetonitrile (40 mL) and dissolved clear, and 0.1M $I_2$/MeOH was then slowly dropwise added until the solution became bright yellow. After continued stirring for 5 min, the yellow color did not disappear. 0.1 M sodium thiosulfate was then dropwise added until the yellow color disappeared. When no change was found after stirring for two min, a reaction liquid of **Example 12** was obtained.

**3.1 Purification**

The reaction liquid was purified under the following purification conditions.

**Purification conditions:**

| Separation condition | |
|---|---|
| Dissolution condition | Dissolve in 30% ACN-$H_2O$ |
| Instrument | Hanbon NEWSTYLE preparative chromatograph |
| Mobile phase | A: $H_2O$ (0.1% TFA in $H_2O$) |
| | B: $CH_3CN$ |
| Gradient | 25-60%-60 min. Retention time: 30 min |
| Column type | Waters Xselect CSH C18 (20 $\times$ 250 mm, 10 $\mu$m) |
| Flow rate | 15 mL/Min |
| Detection wavelength | 214/254 nm |
| Column temperature | Room temperature |

After the purified liquid was detected by RP-HPLC, the qualified portions were combined and diluted with one fold of purified water, for later use.

**3.2 Salt conversion and freeze-drying**

**Salt conversion conditions:**

| Separation condition | |
|---|---|
| Dissolution condition | From the previous step |
| Instrument | Hanbon NEWSTYLE preparative chromatograph |
| Mobile phase | A: 0.5% AcOH/water |
| | B: 0.5% AcOH/ACN |
| | C: 0.15M (NH4OAc/water) |
| Gradient | 5% MPB/MPC (20 min)-5% MPB/MPA (20 min)-60% MPB/MPA (10 min) |
| Column type | Waters Xselect CSH C18 (20 × 250 mm, 10 μm) |
| Flow rate | 15 mL/Min |
| Detection wavelength | 214/254 nm |
| Column temperature | Room temperature |

Equilibration was carried out at 15 ml/min for 15 min by the same preparative RP-HPLC column using MPA containing 5% MPB. (MPA = 0.5% AcOH/water, MPB = 0.5% AcOH/ACN, and MPC = 0.15M $NH_4OAc$/water). The diluted purified liquid was loaded onto the chromatographic column at 15 ml/min. After washing with 5% MPB/MPC for 20 min, then with 5% MPB/MPA for 20 min, and finally with 60% MPB/MPA, the product was eluted off. After concentration under reduced pressure to remove the organic solvent, followed by freeze-drying, 35 mg of the final product of **Example 12** was obtained, with a purity of 96.1% and a yield of 18.4%.

LC/MS: $[(M+H)/2]^+$ = 1020.5.

**Example 20**

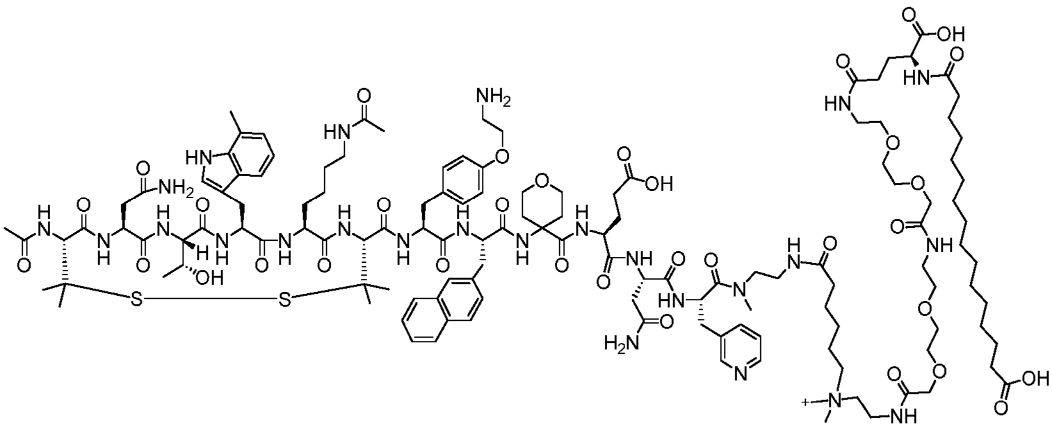

By reference to the synthesis method of **Example 1,** 35.1 mg of a product of **Example 20** was obtained by the reaction of **intermediate L4** with polypeptide intermediate **1c** and the subsequent similar experimental operations, with a purity of 97.1% and a yield of 19.1%.

LC/MS: $[(M+H)/2]^+$ = 1378.7; $[(M+2H)/3]^+$ = 919.5.

Other examples were prepared by reference to the above preparation method:

| Serial No. | Structure | MS m/z (ESI) |
|---|---|---|
| 3 | 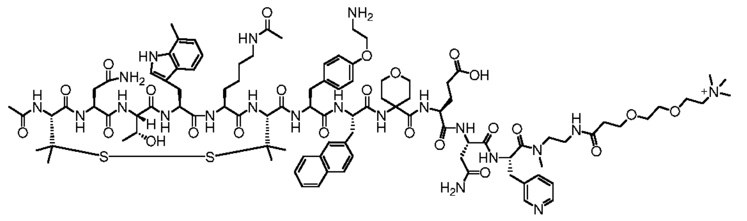 | 1043.5 [(M+H)/2]+ |
| 4 | 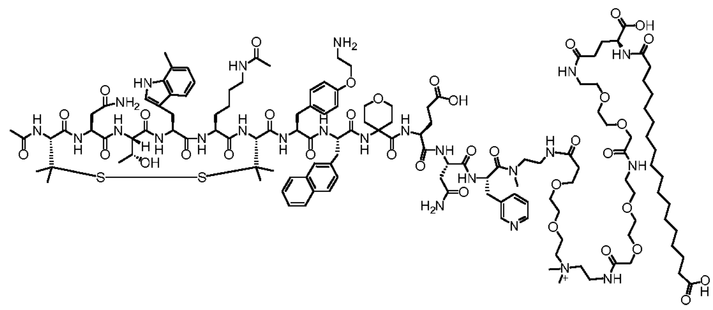 | 1415.7 [(M+H)/2]+ |
| 5 | 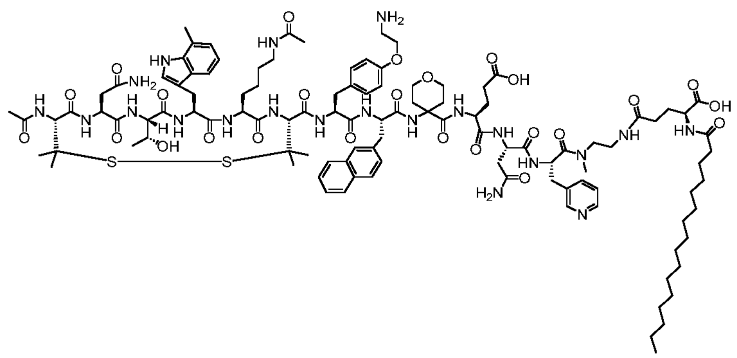 | 1126.2 [(M+H)/2]+ |
| 6 | 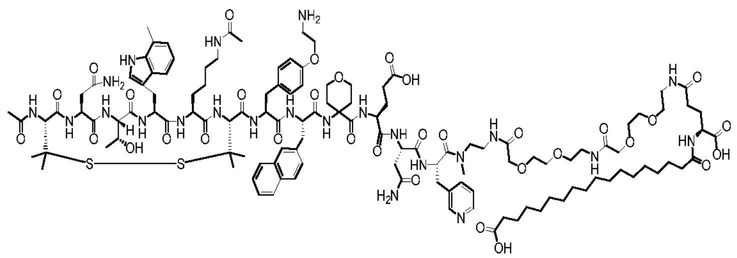 | 1300.1 [(M+H)/2]+ |
| 7 | 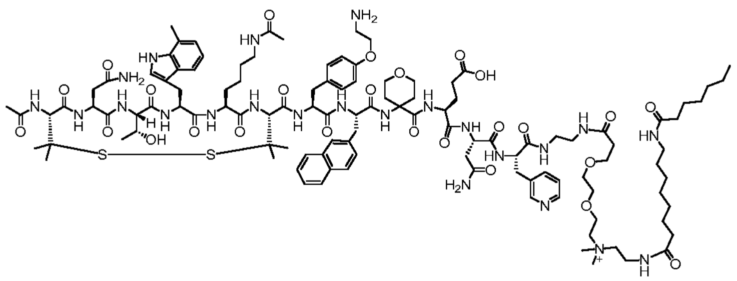 | 1177.9 [(M+H)/2]+ |

(continued)

| Serial No. | Structure | MS m/z (ESI) |
|---|---|---|
| 8 | 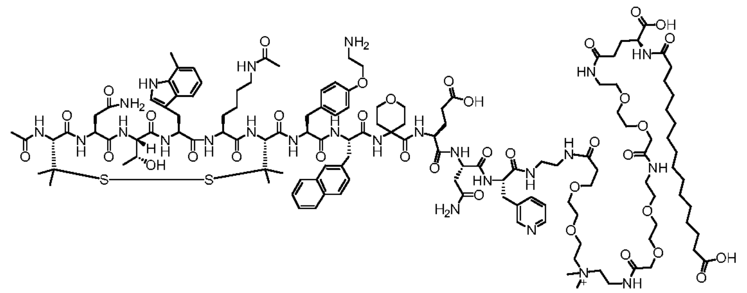 | 1394.7 [(M+H)/2]+ |
| 9 | 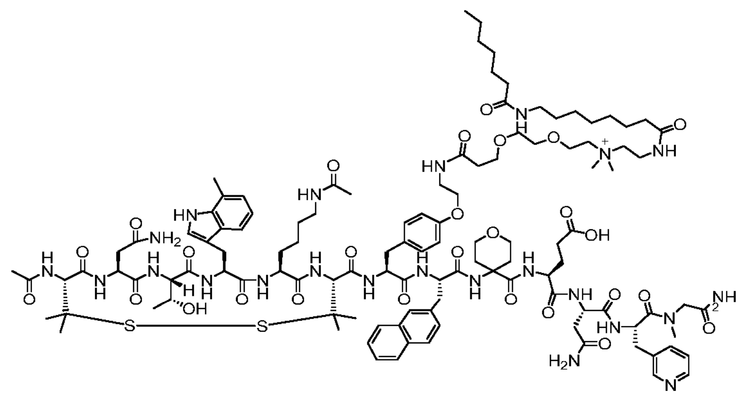 | 1191.6 [(M+H)/2]+ |
| 10 | 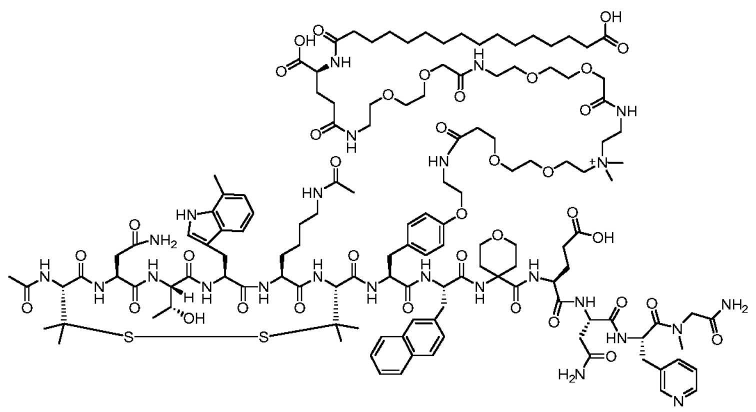 | 1408.7 [(M+H)/2]+ |
| 11 | 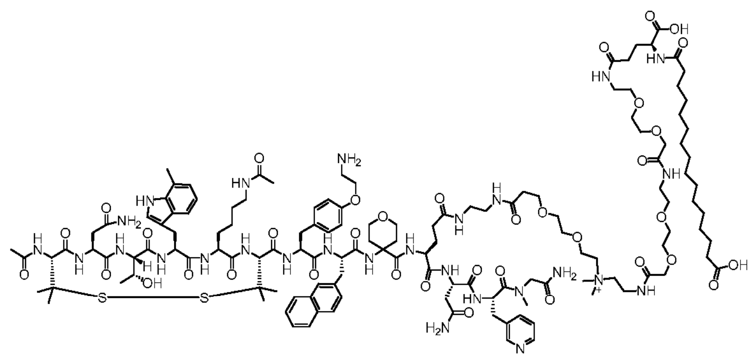 | 1429.6 [(M+H)/2]+ |

(continued)

| Serial No. | Structure | MS m/z (ESI) |
|---|---|---|
| 12 | 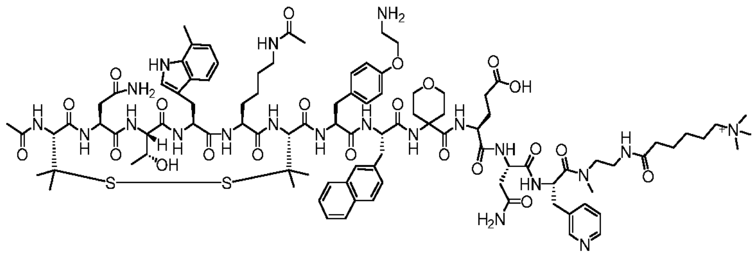 | 1020.5 [(M+H)/2]+ |
| 13 | 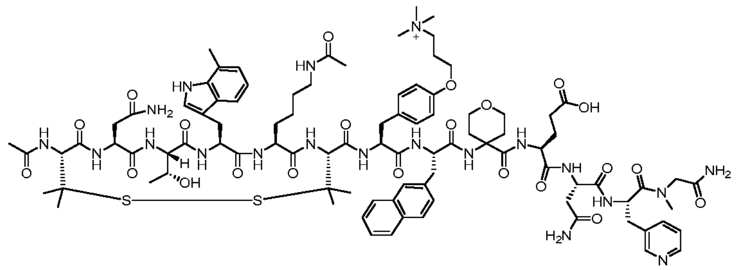 | 978.0 [(M+H)/2]+ |
| 14 | 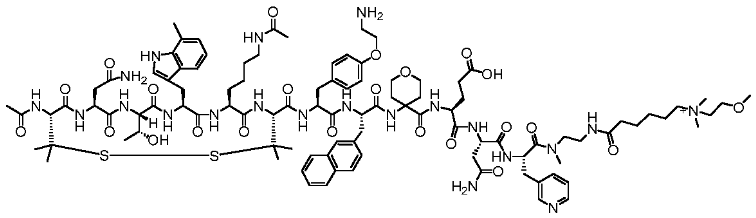 | 1042.5 [(M+H)/2]+ |
| 15 | 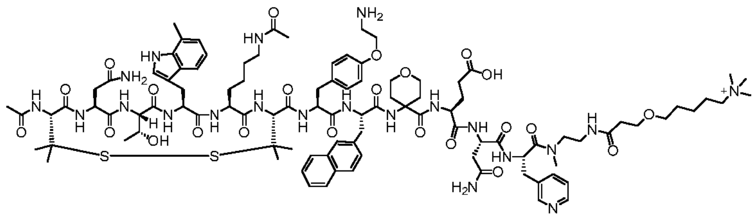 | 1042.5 [(M+H)/2]+ |
| 16 | 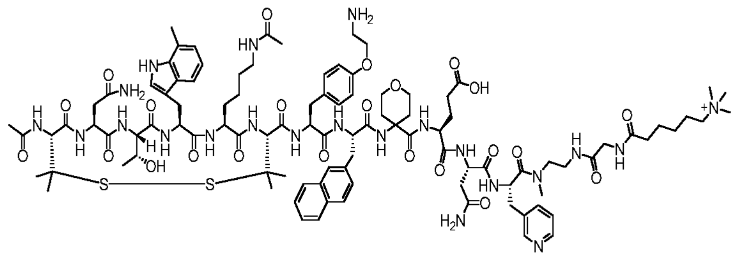 | 1049.0 [(M+H)/2]+ |
| 17 | 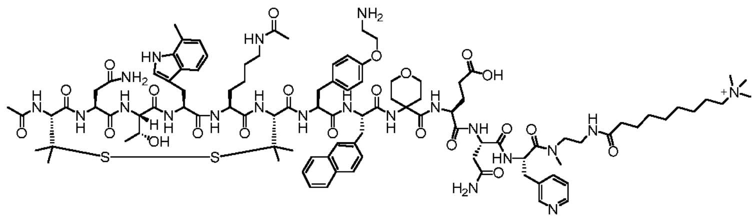 | 1041.5 [(M+H)/2]+ |

(continued)

| Serial No. | Structure | MS m/z (ESI) |
|---|---|---|
| 18 | 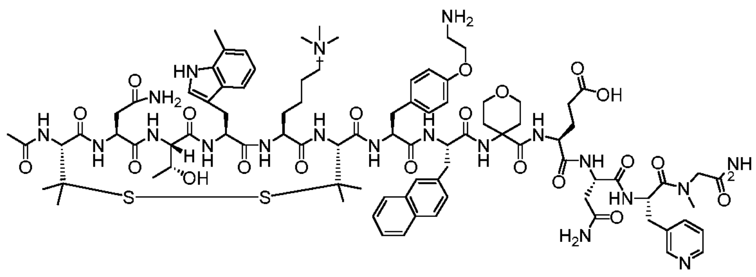 | 949.4 [(M+H)/2]$^+$ |
| 19 | 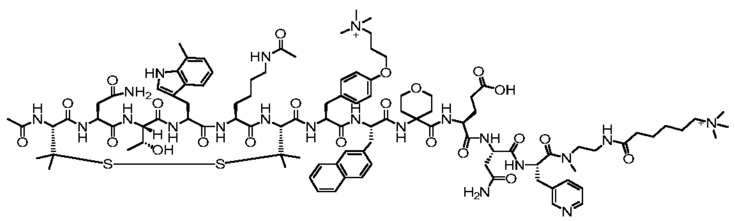 | 1048.5 [M/2]$^+$ |
| 20 | 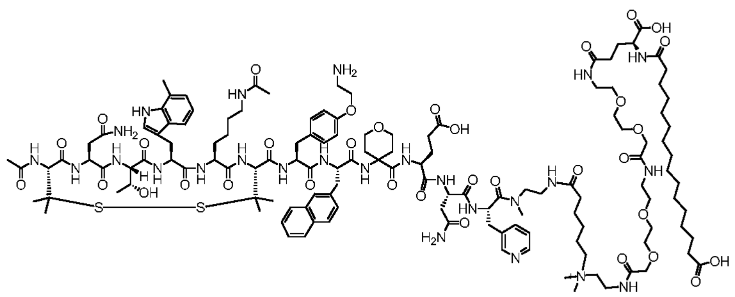 | 1378.7 [(M+H)/2]$^+$ |
| 21 | 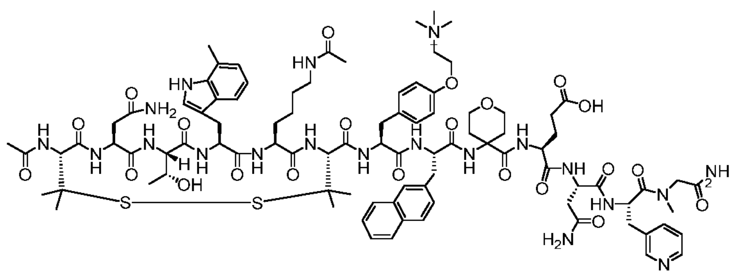 | 970.9 [(M+H)/2]$^+$ |
| 22 | 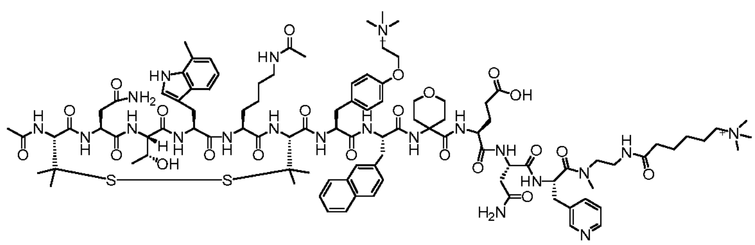 | 1041.5 [M/2]$^+$ |

## Example 1-14

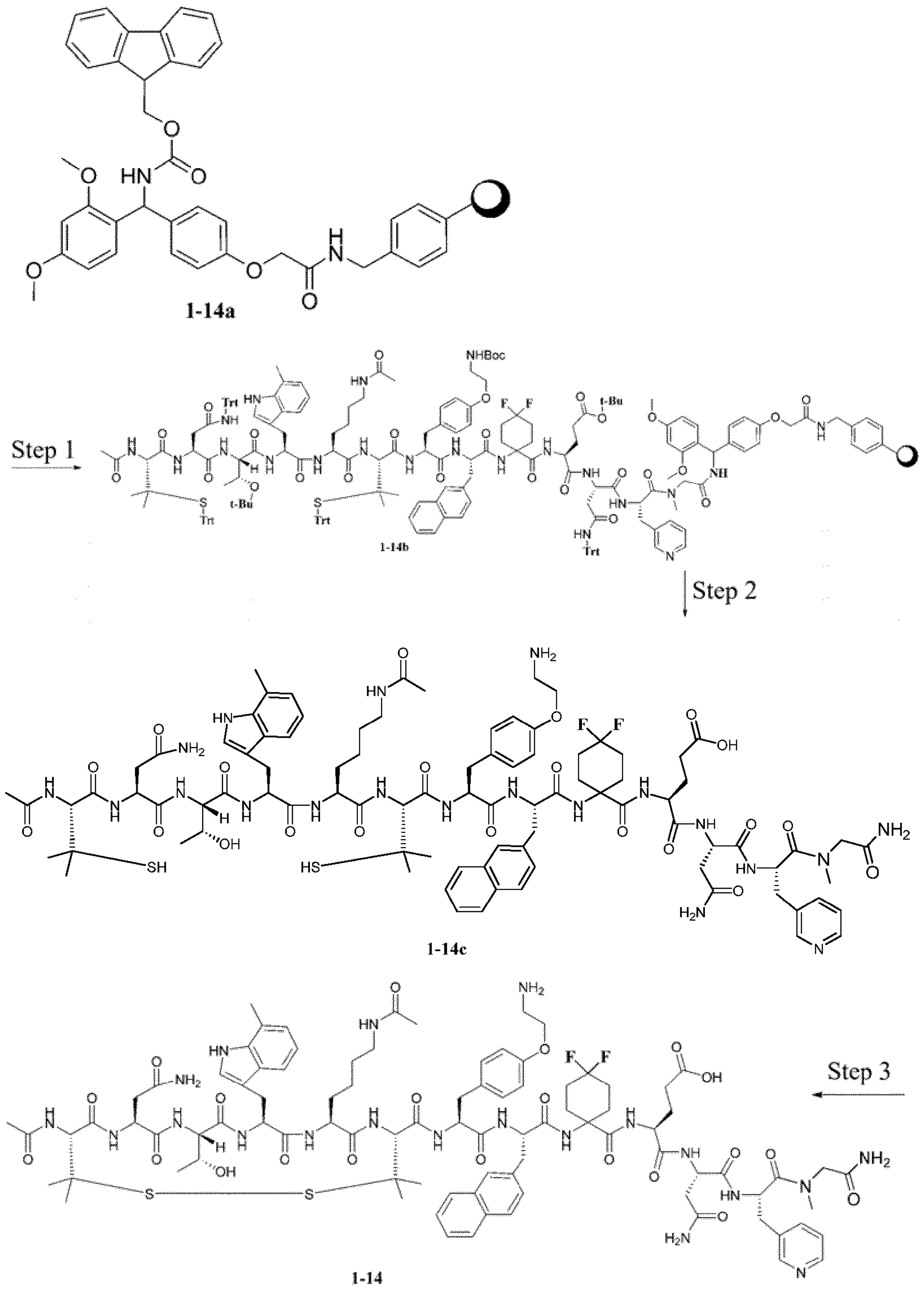


1-14a

1-14b

1-14c

1-14

Step 1: Solid-phase synthesis of fully protected linear peptide

1.1 Resin attachment

1.1.1 Swelling

Rink Amide-AM Resin **(1-14a,** 0.4 g, degree of substitution 0.5-0.7 mmol/g) was weighed and added to the reaction column, DMF (10 mL) was added, and after sparging with nitrogen for 20 min, waste was discharged until no liquid flowed out.

1.1.2 Resin attachment

1) 20% piperidine/DMF(10 mL) was added, followed by sparging with nitrogen for 30 min, and waste was discharged until no liquid flowed out. After washing 5 times by adding DMF (10 mL), with 1 min each time, waste was discharged until no liquid flowed out.
After sampling and detection with ninhydrin, the resin was blue.
2) Fluorenomethoxycarbonyl sarcosine (186.6 mg, 0.6 mmol), *N,N'*-diisopropylcarbodiimide (76 mg), and 1-hydroxybenzotriazole (81 mg) were weighed and added to the reaction column.
3) DMF (10 mL) was added for dissolution, followed by sparging with nitrogen for 4 hours.
4) The solvent was drained. The resin was washed successively with DMF (10 mL), isopropanol (10 mL), DMF (10 mL), DMF (10 mL), and DMF (10 mL). For each instance of washing, sparging with nitrogen was maintained, and stirring was carried out for 2-3 min before the solvent was drained.

After sampling and detection with ninhydrin, the resin was colorless.

1.2. Coupling of amino acids

1.2.1 Coupling of Fmoc-3-(4-pyridinyl)-L-alanine

| Raw material | Feeding amount |
|---|---|
| **Fmoc-3-(4-pyridinyl)-*L*-alanine** | 3.0 eq |
| ***N,N'*-diisopropylcarbodiimide (DIC)** | 3.0 eq |
| **1-Hydroxybenzotriazole (HOBt)** | 3.0 eq |

1) 20% piperidine/DMF(10 mL) was added to the reaction column, followed by sparging with nitrogen for 20 min, and waste was discharged until no liquid flowed out. After washing 5 times by adding DMF (10 mL), with 1 min each time, waste was discharged until no liquid flowed out.
After sampling and detection with tetrachlorobenzoquinone, the resin was blue.
2) Fmoc-3-(4-pyridinyl)-L-alanine (234 mg), *N,N'*-diisopropylcarbodiimide (76 mg), and 1-hydroxybenzotriazole (81 mg) were weighed and added to the reaction column.
3) After dissolution by adding DMF (10 mL), followed by sparging with nitrogen, a reaction was carried out for 3 hours at room temperature.
After sampling and detection with tetrachlorobenzoquinone, the resin was colorless and transparent.
4) The solvent was drained, and the resin was washed successively with 10 mL of DMF (10 mL), 10 mL of isopropanol (10 mL), 10 mL of DMF (10 mL), 10 mL of DMF (10 mL), and 10 of mL DMF (10 mL). For each instance of washing, sparging with nitrogen was maintained, and stirring was carried out for 2-3 min before the solvent was drained.

1.2.2 Coupling of Fmoc-Asn(Trt)-OH

| Raw material | Feeding amount |
|---|---|
| **Fmoc-Asn(Trt)-OH** | 3.0 eq |
| ***N,N'*-diisopropylcarbodiimide (DIC)** | 3.0 eq |
| **1-Hydroxybenzotriazole (HOBt)** | 3.0 eq |

1) 20% piperidine/DMF(10 mL) was added to the reaction column, followed by sparging with nitrogen for 20 min, and waste was discharged until no liquid flowed out. After washing 5 times by adding DMF (10 mL), with 1 min each time, waste was discharged until no liquid flowed out.
After sampling and detection with ninhydrin, the resin was blue.
2) Fmoc-Asn(Trt)-OH (358 mg), N,N'-diisopropylcarbodiimide (76 mg), and 1-hydroxybenzotriazole (81 mg) were

weighed and added to the reaction column.
3) After dissolution by adding DMF (10 mL), followed by sparging with nitrogen, a reaction was carried out for 3 hours at room temperature.
After sampling and detection with ninhydrin, the resin was colorless and transparent.
4) The solvent was drained, and the resin was washed successively with DMF (10 mL), isopropanol (10 mL), DMF (10 mL), DMF (10 mL), and DMF (10 mL). For each instance of washing, sparging with nitrogen was maintained, and stirring was carried out for 2-3 min before the solvent was drained.

1.2.3 Coupling of Fmoc-Glu(OtBu)-OH

| Raw material | Feeding amount |
|---|---|
| **Fmoc-Glu(OtBu)-OH** | 3.0 eq |
| ***N,N'*-diisopropylcarbodiimide (DIC)** | 3.0 eq |
| **1-Hydroxybenzotriazole (HOBt)** | 3.0 eq |

1) 20% piperidine/DMF(10 mL) was added to the reaction column, followed by sparging with nitrogen for 20 min, and waste was discharged until no liquid flowed out. After washing 5 times by adding DMF (10 mL), with 1 min each time, waste was discharged until no liquid flowed out.
After sampling and detection with ninhydrin, the resin was blue.
2) Fmoc-Glu(OtBu)-OH (255 mg), *N*,*N*'-diisopropylcarbodiimide (76 mg), and 1-hydroxybenzotriazole (81 mg) were weighed and added to the reaction column.
3) After dissolution by adding DMF (10 mL), followed by sparging with nitrogen, a reaction was carried out for 3 hours at room temperature.
After sampling and detection with ninhydrin, the resin was colorless and transparent.
4) The solvent was drained, and the resin was washed successively with DMF (10 mL), isopropanol (10 mL), DMF (10 mL), DMF (10 mL), and DMF (10 mL). For each instance of washing, sparging with nitrogen was maintained, and stirring was carried out for 2-3 min before the solvent was drained.

1.2.4 Coupling of 1-(9*H*-fluoren-9-ylmethoxycarbonylamino)-4,4-difluoro-cyclohexanecarboxylic acid

| Raw material | Feeding amount |
|---|---|
| **1-(9*H*-fluoren-9-ylmethoxycarbonylamino)-4,4-difluoro-cyclohexanecarboxylic acid** | 3.0 eq |
| ***N,N'*-diisopropylcarbodiimide (DIC)** | 3.0 eq |
| **1-Hydroxybenzotriazole (HOBt)** | 3.0 eq |

1) 20% piperidine/DMF(10 mL) was added to the reaction column, followed by sparging with nitrogen for 20 min, and waste was discharged until no liquid flowed out. After washing 5 times by adding DMF (10 mL), with 1 min each time, waste was discharged until no liquid flowed out.
After sampling and detection with ninhydrin, the resin was blue.
2) 1-(9*H*-fluoren-9-ylmethoxycarbonylamino)-4,4-difluoro-cyclohexanecarboxylic acid (241 mg), N,N'-diisopropylcarbodiimide (76 mg), and 1-hydroxybenzotriazole (81 mg) were weighed and added to the reaction column.
3) After dissolution by adding DMF (10 mL), followed by sparging with nitrogen, a reaction was carried out for 3 hours at room temperature.
After sampling and detection with ninhydrin, the resin was colorless and transparent.
4) The solvent was drained, and the resin was washed successively with DMF (10 mL), isopropanol (10 mL), DMF (10 mL), DMF (10 mL), and DMF (10 mL). For each instance of washing, sparging with nitrogen was maintained, and stirring was carried out for 2-3 min before the solvent was drained.

1.2.5 Coupling of Fmoc-2Nal-OH

| Raw material | Feeding amount |
|---|---|
| **Fmoc-2Nal-OH** | 3.0 eq |
| ***N,N'*-diisopropylcarbodiimide (DIC)** | 3.0 eq |
| **1-Hydroxybenzotriazole (HOBt)** | 3.0 eq |

1) 20% piperidine/DMF(10 mL) was added to the reaction column, followed by sparging with nitrogen for 20 min, and waste was discharged until no liquid flowed out. After washing 5 times by adding DMF (10 mL), with 1 min each time, waste was discharged until no liquid flowed out.
After sampling and detection with tetrachlorobenzoquinone, the resin was redish brown.
2) Fmoc-2Nal-OH (262 mg), N,N'-diisopropylcarbodiimide (76 mg), and 1-hydroxybenzotriazole (81 mg) were weighed and added to the reaction column.
3) After dissolution by adding DMF (10 mL), followed by sparging with nitrogen, a reaction was carried out for 3 hours at room temperature.
After sampling and detection with tetrachlorobenzoquinone, the resin was colorless and transparent.
4) The solvent was drained, and the resin was washed successively with DMF (10 mL), isopropanol (10 mL), DMF (10 mL), DMF (10 mL), and DMF (10 mL). For each instance of washing, sparging with nitrogen was maintained, and stirring was carried out for 2-3 min before the solvent was drained.

1.2.6 Coupling of Fmoc-4-[2-(Boc-amino)ethoxy]-*L*-Phenylalanine

| Raw material | Feeding amount |
|---|---|
| **Fmoc-4-[2-(Boc-amino)ethoxy]-*L*-Phenylalanine** | 3.0 eq |
| ***N,N'*-diisopropylcarbodiimide (DIC)** | 3.0 eq |
| **1-Hydroxybenzotriazole (HOBt)** | 3.0 eq |

1) 20% piperidine/DMF(10 mL) was added to the reaction column, followed by sparging with nitrogen for 20 min, and waste was discharged until no liquid flowed out. After washing 5 times by adding DMF (10 mL), with 1 min each time, waste was discharged until no liquid flowed out.
After sampling and detection with ninhydrin, the resin was blue.
2) Fmoc-4-[2-(Boc-amino)ethoxy]-L-Phenylalanine (328 mg), *N,N'*-diisopropylcarbodiimide (76 mg), and 1-hydroxybenzotriazole (81 mg) were weighed and added to the reaction column.
3) After dissolution by adding DMF (10 mL), followed by sparging with nitrogen, a reaction was carried out for 3 hours at room temperature.
After sampling and detection with ninhydrin, the resin was colorless and transparent.
4) The solvent was drained, and the resin was washed successively with DMF (10 mL), isopropanol (10 mL), DMF (10 mL), DMF (10 mL), and DMF (10 mL). For each instance of washing, sparging with nitrogen was maintained, and stirring was carried out for 2-3 min before the solvent was drained.

1.2.7 Coupling of Fmoc-Pen(Trt)-OH

| Raw material | Feeding amount |
|---|---|
| **Fmoc-Pen(Trt)-OH** | 3.0 eq |
| ***N,N'*-diisopropylcarbodiimide (DIC)** | 3.0 eq |
| **1-Hydroxybenzotriazole (HOBt)** | 3.0 eq |

1) 20% piperidine/DMF(10 mL) was added to the reaction column, followed by sparging with nitrogen for 20 min, and waste was discharged until no liquid flowed out. After washing 5 times by adding DMF (10 mL), with 1 min each time, waste was discharged until no liquid flowed out.
After sampling and detection with ninhydrin, the resin was blue.

2) Fmoc-Pen(Trt)-OH (368 mg), *N,N*'-diisopropylcarbodiimide (76 mg), and 1-hydroxybenzotriazole (81 mg) were weighed and added to the reaction column.
3) After dissolution by adding DMF (10 mL), followed by sparging with nitrogen, a reaction was carried out for 3 hours at room temperature.
After sampling and detection with ninhydrin, the resin was colorless and transparent.
4) The solvent was drained, and the resin was washed successively with DMF (10 mL), isopropanol (10 mL), DMF (10 mL), DMF (10 mL), and DMF (10 mL). For each instance of washing, sparging with nitrogen was maintained, and stirring was carried out for 2-3 min before the solvent was drained.

1.2.8 Coupling of Fmoc-Lys(Ac)-OH

| Raw material | Feeding amount |
|---|---|
| **Fmoc-Lys(Ac)-OH** | 3.0 eq |
| ***N,N*'-diisopropylcarbodiimide (DIC)** | 3.0 eq |
| **1-Hydroxybenzotriazole (HOBt)** | 3.0 eq |

1) 20% piperidine/DMF(10 mL) was added to the reaction column, followed by sparging with nitrogen for 20 min, and waste was discharged until no liquid flowed out. After washing 5 times by adding DMF (10 mL), with 1 min each time, waste was discharged until no liquid flowed out.
After sampling and detection with ninhydrin, the resin was blue.
2) Fmoc-Lys(Ac)-OH (246 mg), N,N'-diisopropylcarbodiimide (76 mg), and 1-hydroxybenzotriazole (81 mg) were weighed and added to the reaction column.
3) After dissolution by adding DMF (10 mL), followed by sparging with nitrogen, a reaction was carried out for 3 hours at room temperature.
After sampling and detection with ninhydrin, the resin was colorless and transparent.
4) The solvent was drained, and the resin was washed successively with DMF (10 mL), isopropanol (10 mL), DMF (10 mL), DMF (10 mL), and DMF (10 mL). For each instance of washing, sparging with nitrogen was maintained, and stirring was carried out for 2-3 min before the solvent was drained.

1.2.9 Coupling of Fmoc-(7Me)Trp-OH

| Raw material | Feeding amount |
|---|---|
| **Fmoc-(7Me)Trp-OH** | 3.0 eq |
| ***N,N*'-diisopropylcarbodiimide (DIC)** | 3.0 eq |
| **1-Hydroxybenzotriazole (HOBt)** | 3.0 eq |

1) 20% piperidine/DMF(10 mL) was added to the reaction column, followed by sparging with nitrogen for 20 min, and waste was discharged until no liquid flowed out. After washing 5 times by adding DMF (10 mL), with 1 min each time, waste was discharged until no liquid flowed out.
After sampling and detection with ninhydrin, the resin was blue.
2) Fmoc-(7Me)Trp-OH (264 mg), N,N'-diisopropylcarbodiimide (76 mg), and 1-hydroxybenzotriazole (81 mg) were weighed and added to the reaction column.
3) After dissolution by adding DMF (10 mL), followed by sparging with nitrogen, a reaction was carried out for 3 hours at room temperature.
After sampling and detection with ninhydrin, the resin was colorless and transparent.
4) The solvent was drained, and the resin was washed successively with DMF (10 mL), isopropanol (10 mL), DMF (10 mL), DMF (10 mL), and DMF (10 mL). For each instance of washing, sparging with nitrogen was maintained, and stirring was carried out for 2-3 min before the solvent was drained.

1.2.10 Coupling of Fmoc-Thr(Tbu)-OH

| Raw material | Feeding amount |
|---|---|
| **Fmoc-Thr(Tbu)-OH** | 3.0 eq |
| ***N,N'*-diisopropylcarbodiimide (DIC)** | 3.0 eq |
| **1-Hydroxybenzotriazole (HOBt)** | 3.0 eq |

1) 20% piperidine/DMF(10 mL) was added to the reaction column, followed by sparging with nitrogen for 20 min, and waste was discharged until no liquid flowed out. After washing 5 times by adding DMF (10 mL), with 1 min each time, waste was discharged until no liquid flowed out.
After sampling and detection with ninhydrin, the resin was blue.
2) Fmoc-Thr(Tbu)-OH (238 mg), N,N'-diisopropylcarbodiimide (76 mg), and 1-hydroxybenzotriazole (81 mg) were weighed and added to the reaction column.
3) After dissolution by adding DMF (10 mL), followed by sparging with nitrogen, a reaction was carried out for 3 hours at room temperature.
After sampling and detection with ninhydrin, the resin was colorless and transparent.
4) The solvent was drained, and the resin was washed successively with DMF (10 mL), isopropanol (10 mL), DMF (10 mL), DMF (10 mL), and DMF (10 mL). For each instance of washing, sparging with nitrogen was maintained, and stirring was carried out for 2-3 min before the solvent was drained.

1.2.11 Coupling of Fmoc-Asn(Trt)-OH

| Raw material | Feeding amount |
|---|---|
| **Fmoc-Asn(Trt)-OH** | 3.0 eq |
| ***N,N'*-diisopropylcarbodiimide (DIC)** | 3.0 eq |
| **1-Hydroxybenzotriazole (HOBt)** | 3.0 eq |

1) 20% piperidine/DMF(10 mL) was added to the reaction column, followed by sparging with nitrogen for 20 min, and waste was discharged until no liquid flowed out. After washing 5 times by adding DMF (10 mL), with 1 min each time, waste was discharged until no liquid flowed out.
After sampling and detection with ninhydrin, the resin was blue.
2) Fmoc-Asn(Trt)-OH (358 mg), N,N'-diisopropylcarbodiimide (76 mg), and 1-hydroxybenzotriazole (81 mg) were weighed and added to the reaction column.
3) After dissolution by adding DMF (10 mL), followed by sparging with nitrogen, a reaction was carried out for 3 hours at room temperature.
After sampling and detection with ninhydrin, the resin was colorless and transparent.
4) The solvent was drained, and the resin was washed successively with DMF (10 mL), isopropanol (10 mL), DMF (10 mL), DMF (10 mL), and DMF (10 mL). For each instance of washing, sparging with nitrogen was maintained, and stirring was carried out for 2-3 min before the solvent was drained.

1.2.12 Coupling of Fmoc-Pen(Trt)-OH

| Raw material | Feeding amount |
|---|---|
| **Fmoc-Pen(Trt)-OH** | 3.0 eq |
| ***N,N'*-diisopropylcarbodiimide (DIC)** | 3.0 eq |
| **1-Hydroxybenzotriazole (HOBt)** | 3.0 eq |

1) 20% piperidine/DMF(10 mL) was added to the reaction column, followed by sparging with nitrogen for 20 min, and waste was discharged until no liquid flowed out. After washing 5 times by adding DMF (10 mL), with 1 min each time, waste was discharged until no liquid flowed out.
After sampling and detection with ninhydrin, the resin was blue.
2) Fmoc-Pen(Trt)-OH (368 mg), N,N'-diisopropylcarbodiimide (76 mg), and 1-hydroxybenzotriazole (81 mg) were weighed and added to the reaction column.
3) After dissolution by adding DMF (10 mL), followed by sparging with nitrogen, a reaction was carried out for 3 hours at room temperature.
After sampling and detection with ninhydrin, the resin was colorless and transparent.
4) The solvent was drained, and the resin was washed successively with DMF (10 mL), isopropanol (10 mL), DMF (10 mL), DMF (10 mL), and DMF (10 mL). For each instance of washing, sparging with nitrogen was maintained, and stirring was carried out for 2-3 min before the solvent was drained.

1.2.13 Capping with $Ac_2O$

| Raw material | Feeding amount |
|---|---|
| **Acetic anhydride ($Ac_2O$)** | 5.0 eq |
| ***N,N*-diisopropylethylamine (DIEA)** | 6.00 eq |

1) 20% piperidine/DMF(10 mL) was added to the reaction column, followed by sparging with nitrogen for 20 min, and waste was discharged until no liquid flowed out. After washing 5 times by adding DMF (10 mL), with 1 min each time, waste was discharged until no liquid flowed out.
After sampling and detection with ninhydrin, the resin was blue.
2) DCM (10 mL) was added, and DIEA (6.0 eq) and $Ac_2O$ (5.0 eq) were added to the above resin, followed by sparging with nitrogen, such that nitrogen was properly adjusted to make the resin swell evenly.
3) A reaction was carried out for 30 min at room temperature.
After sampling and detection with ninhydrin, the resin was colorless and transparent.
4) The resin was washed successively with DMF (10 mL), isopropanol (10 mL), DMF (10 mL), DMF (10 mL), and DMF (10 mL). For each instance of washing, sparging with nitrogen was maintained, and stirring was carried out for 2-3 min before the solvent was drained.
5) Sample collection and washing: The resin was washed with isopropyl ether (10 mL), sparged with nitrogen, and stirred and washed for 2-3 min, followed by draining. This operation was further repeated three times to obtain resin peptide 1-**14b.**

Step 2: Cleavage and deprotection of linear peptide

Preparation of cleavage liquid according to the following volume

| Reagent | Proportion % |
|---|---|
| **Trifluoroacetic acid (TFA)** | 92.5 |
| **Triisopropylsilane (Tis)** | 2.5 |
| **Dithiothreitol (DTT)** | 2.5 |
| **$H_2O$** | 2.5 |

The obtained resin peptide **1-14b** was added to 10 mL of the prepared cleavage liquid, shaken in a 25°C water bath in a shaking table for 2.5 hours, and filtered. The filtrate was added to 10 volumes of ice-cold isopropyl ether and centrifuged, and the supernatant was discarded. After further washing 3 times with isopropyl ether, vacuum drying was carried out for 2 hours to obtain crude peptide **1-14c.**

Step 3: Oxidation of disulfide bond

The obtained crude peptide **1-14c** obtained above (190 mg) was added to water (100 mL) and acetonitrile (100

mL) and dissolved clear, and 0.1M $I_2$/MeOH was then slowly dropwise added thereto until the solution became bright yellow. After continued stirring for 5 min, the yellow color did not disappear. 0.1 M sodium thiosulfate was then dropwise added until the yellow color disappeared. When no change was found after stirring for two min, the sample was purified under the following purification conditions.

Purification conditions:

| Separation condition | |
|---|---|
| Dissolution condition | Dissolve in 30% ACN-$H_2O$ |
| Instrument | Hanbon NEWSTYLE preparative chromatograph |
| Mobile phase | A: $H_2O$ (0.1% TFA in $H_2O$) |
| | B: $CH_3CN$ |
| Gradient | 30-60%-60 min. Retention time: 30 min |
| Column type | Waters Xselect CSH C18 (50 × 250 mm, 10 μm) |
| Flow rate | 7 mL/Min |
| Detection wavelength | 214/254 nm |
| Column temperature | Room temperature |

The purified liquid was concentrated under reduced pressure to remove the organic solvent and freeze-dried to obtain 45 mg of the final product of **Example 1-14,** with a purity of 97.5 % and a yield of 11.7%.
MS: m/z = 1931.8, $[M+H]^+$; m/z = 966.4, $[M+2H]^{2+}$.

**Example 1-16-1**

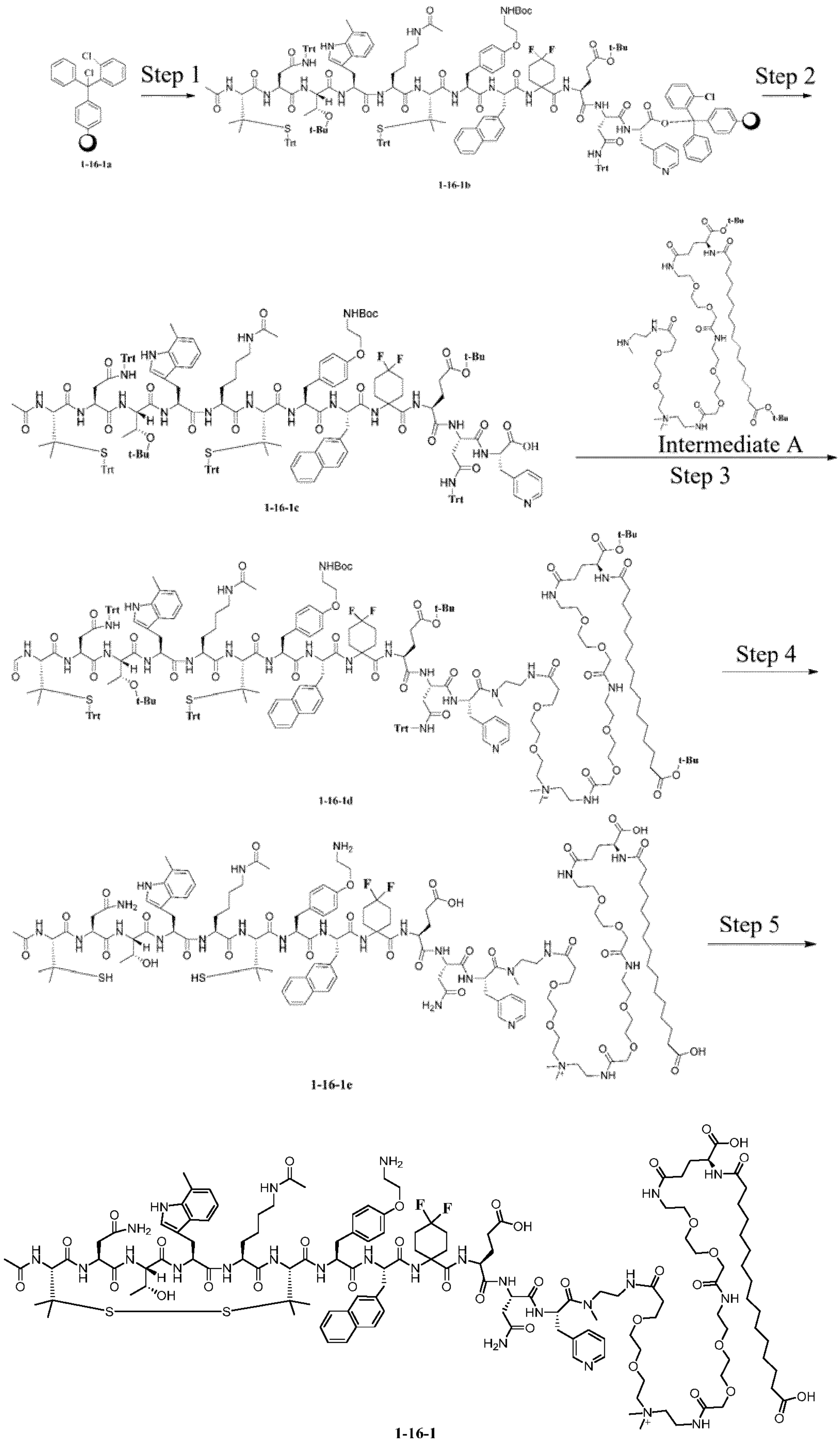


1-16-1a

1-16-1b

1-16-1c

1-16-1d

1-16-1e

1-16-1

Step 1: Synthesis of fully protected linear peptide

1.1 Resin attachment

1.1.1 0.6 g of 2-CTC Resin (**1-16-1a,** degree of substitution S = 1.08 mmol/g) and 78 mg of *N*-Fmoc-*L*-3-pyridinylalanine (0.2 mmol, degree of substitution 0.33) were weighed and added to a reaction column, followed by DCM (10 mL). Subsequently, 0.6 mL of DIEA was added to the reaction column, followed by sparging with nitrogen for 2 hours. 0.6 mL of MeOH was then added to the reaction column, followed by continued sparging with nitrogen for 30 min. Waste was discharged until no liquid flowed out. After washing 5 times by adding DMF (30 mL), with 1 min each time, waste was discharged until no liquid flowed out.

1.1.2 20% piperidine/DMF (15 mL) was added to the reaction column, followed by sparging with nitrogen for 20 min, and waste was discharged until no liquid flowed out. After washing 5 times by adding DMF (30 mL), with 1 min each time, waste was discharged until no liquid flowed out. After detection with ninhydrin, the resin was blue.

1.2. Coupling of amino acids

1.2.1 Coupling of Fmoc-Asn(Trt)-OH

| Raw material | Feeding amount |
|---|---|
| **Fmoc-Asn(Trt)-OH** | 358 mg, 3.0 eq |
| **HBTU** | 216 mg, 2.85 eq |
| **DIEA** | 155 mg, 6.00 eq |

1. Fmoc-Asn(Trt)-OH (358 mg, 3.0 eq) was weighed and added to the above resin, DIEA (155 mg, 6.00 eq) was added, and 5 mL of DMF was additionally added to the reaction column, followed by sparging with nitrogen. After amino acids were dissolved, HBTU (216 mg, 2.85 eq) was added. Nitrogen was properly adjusted to make the resin swell evenly.
2. The reaction was carried out for 0.5 hours in a 25°C environment, and after detection with ninhydrin, the resin was colorless and transparent.
3. The reaction liquid was drained, and after washing 5 times with DMF (20 mL each time) for 1 min each time, waste was discharged until no liquid flowed out.

1.2.2 Coupling of Fmoc-Glu(OtBu)-OH

| Raw material | Feeding amount |
|---|---|
| **Fmoc-Glu(OtBu)-OH** | 255 mg, 3.0 eq |
| **HBTU** | 216 mg, 2.85 eq |
| **DIEA** | 155 mg, 6.00 eq |

1. 20% piperidine/DMF (20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-Glu(OtBu)-OH (255 mg, 3.0 eq) was weighed and added to the above resin, DIEA (155 mg, 6.00 eq) was added, and 5 mL of DMF was additionally added to the reaction column, followed by sparging with nitrogen. After amino acids were dissolved, HBTU (216 mg, 2.85 eq) was added. Nitrogen was properly adjusted to make the resin swell evenly.
3. The reaction was carried out for 0.5 hours in a 25°C environment, and after detection with ninhydrin, the resin was colorless and transparent.
4. The reaction liquid was drained, and after washing 5 times with DMF (20 mL each time) for 1 min each time, waste was discharged until no liquid flowed out.

1.2.3 Coupling of 1-(9*H*-fluoren-9-ylmethoxycarbonylamino)-4,4-difluoro-cyclohexanecarboxylic acid

| Raw material | Feeding amount |
|---|---|
| **1-(9*H*-fluoren-9-ylmethoxycarbonylamino)-4,4-difluoro-cyclohexanecarboxylic acid** | 241 mg, 3.0 eq |
| **HATU** | 217 mg, 2.85 eq |
| **DIEA** | 155 mg, 6.00 eq |

1. 20% piperidine/DMF (20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. 1-(9*H*-fluoren-9-ylmethoxycarbonylamino)-4,4-difluoro-cyclohexanecarboxylic acid (CAS: 1986905-26-3, 241 mg, 3.0 eq) was weighed and added to the above resin, DIEA (155 mg, 6.00 eq) was added, and 5 mL of DMF was additionally added to the reaction column, followed by sparging with nitrogen. After amino acids were dissolved, HATU (217 mg, 2.85 eq) was added. Nitrogen was properly adjusted to make the resin swell evenly.
3. The reaction was carried out for 0.5 hours in a 25°C environment, and after detection with ninhydrin, the resin was colorless and transparent.
4. The reaction liquid was drained, and after washing 5 times with DMF for 1 min each time, waste was discharged until no liquid flowed out.

1.2.4 Coupling of Fmoc-2Nal-OH

| Raw material | Feeding amount |
|---|---|
| **Fmoc-2Nal-OH** | 262 mg, 3.0 eq |
| **HATU** | 217 mg, 2.85 eq |
| **DIEA** | 155 mg, 6.00 eq |

1. 20% piperidine/DMF (20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with tetrachlorobenzoquinone, the resin was green.
2. Fmoc-2-Nal-OH (262 mg, 3.0 eq) was weighed and added to the above resin, DIEA (155 mg, 6.00 eq) was added, and 5 mL of DMF was additionally added to the reaction column, followed by sparging with nitrogen. After amino acids were dissolved, HATU (217 mg, 2.85 eq) was added. Nitrogen was properly adjusted to make the resin swell evenly.
3. The reaction was carried out for 0.5 hours in a 25°C environment, and after detection with tetrachlorobenzoquinone, the resin was colorless and transparent.
4. The reaction liquid was drained, and after washing 5 times with DMF (20 mL each time) for 1 min each time, waste was discharged until no liquid flowed out.

1.2.5 Coupling of Fmoc-4-[2-(Boc-amino)ethoxy]-L-Phenylalanine

| Raw material | Feeding amount |
|---|---|
| **Fmoc-4-[2-(Boc-amino)ethoxy]-*L*-Phenylalanine** | 328 mg, 3.0 eq |
| **HBTU** | 216 mg, 2.85 eq |
| **DIEA** | 155 mg, 6.00 eq |

1. 20% piperidine/DMF(20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.

2. Fmoc-4-[2-(Boc-amino)ethoxy]-*L*-Phenylalanine (328 mg, 3.0 eq) was weighed and added to the above resin, DIEA (155 mg, 6.00 eq) was added, and 5 mL of DMF was additionally added to the reaction column, followed by sparging with nitrogen. After amino acids were dissolved, HBTU (216 mg, 2.85 eq) was added. Nitrogen was properly adjusted to make the resin swell evenly.
3. The reaction was carried out for 0.5 hours in a 25°C environment, and after detection with ninhydrin, the resin was colorless and transparent.
4. The reaction liquid was drained, and after washing 5 times with DMF (20 mL each time) for 1 min each time, waste was discharged until no liquid flowed out.

1.2.6 Coupling of Fmoc-Pen(Trt)-OH

| Raw material | Feeding amount |
|---|---|
| **Fmoc-Pen(Trt)-OH** | 368 mg, 3.0 eq |
| **HBTU** | 216 mg, 2.85 eq |
| **DIEA** | 155 mg, 6.00 eq |

1. 20% piperidine/DMF (20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-Pen(Trt)-OH (368 mg, 3.0 eq) was weighed and added to the above resin, DIEA (155 mg, 6.00 eq) was added, and 5 mL of DMF was additionally added to the reaction column, followed by sparging with nitrogen. After amino acids were dissolved, HBTU (216 mg, 2.85 eq) was added. Nitrogen was properly adjusted to make the resin swell evenly.
3. The reaction was carried out for 0.5 hours in a 25°C environment, and after detection with ninhydrin, the resin was colorless and transparent.
4. The reaction liquid was drained, and after washing 5 times with DMF (20 mL each time) for 1 min each time, waste was discharged until no liquid flowed out.

1.2.7 Coupling of Fmoc-Lys(Ac)-OH

| Raw material | Feeding amount |
|---|---|
| **Fmoc-Lys(Ac)-OH** | 246 mg, 3.0 eq |
| **HBTU** | 216 mg, 2.85 eq |
| **DIEA** | 155 mg, 6.00 eq |

1. 20% piperidine/DMF (20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-Lys(Ac)-OH (246 mg, 3.0 eq) was weighed and added to the above resin, DIEA (155 mg, 6.00 eq) was added, and 5 mL of DMF was additionally added to the reaction column, followed by sparging with nitrogen. After amino acids were dissolved, HBTU (216 mg, 2.85 eq) was added. Nitrogen was properly adjusted to make the resin swell evenly.
3. The reaction was carried out for 0.5 hours in a 25°C environment, and after detection with ninhydrin, the resin was colorless and transparent.
4. The reaction liquid was drained, and after washing 5 times with DMF (20 mL each time) for 1 min each time, waste was discharged until no liquid flowed out.

1.2.8 Coupling of Fmoc-(7Me)Trp-OH

| Raw material | Feeding amount |
|---|---|
| **Fmoc-(7Me)Trp-OH** | 264 mg, 3.0 eq |
| **HATU** | 217 mg, 2.85 eq |
| **DIEA** | 155 mg, 6.00 eq |

1. 20% piperidine/DMF (20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-(7Me)Trp-OH (264 mg, 3.0 eq) was weighed and added to the above resin, DIEA (155 mg, 6.00 eq) was added, and 5 mL of DMF was additionally added to the reaction column, followed by sparging with nitrogen. After amino acids were dissolved, HATU (217 mg, 2.85 eq) was added. Nitrogen was properly adjusted to make the resin swell evenly.
3. The reaction was carried out for 0.5 hours in a 25°C environment, and after detection with ninhydrin, the resin was colorless and transparent.
4. The reaction liquid was drained, and after washing 5 times with DMF (20 mL each time) for 1 min each time, waste was discharged until no liquid flowed out.

1.2.9 Coupling of Fmoc-Thr(Tbu)-OH

| Raw material | Feeding amount |
|---|---|
| **Fmoc-Thr(Tbu)-OH** | 238mg, 3.0 eq |
| **HBTU** | 216 mg, 2.85 eq |
| **DIEA** | 155 mg, 6.00 eq |

1. 20% piperidine/DMF (20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-Thr(Tbu)-OH (238 mg, 3.0 eq) was weighed and added to the above resin, DIEA (155 mg, 6.00 eq) was added, and 5 mL of DMF was additionally added to the reaction column, followed by sparging with nitrogen. After amino acids were dissolved, HBTU (216 mg, 2.85 eq) was added. Nitrogen was properly adjusted to make the resin swell evenly.
3. The reaction was carried out for 0.5 hours in a 25°C environment, and after detection with ninhydrin, the resin was colorless and transparent.
4. The reaction liquid was drained, and after washing 5 times with DMF (20 mL each time) for 1 min each time, waste was discharged until no liquid flowed out.

1.2.10 Coupling of Fmoc-Asn(Trt)-OH

| Raw material | Feeding amount |
|---|---|
| **Fmoc-Asn(Trt)-OH** | 358 mg, 3.0 eq |
| **HBTU** | 216 mg, 2.85 eq |
| **DIEA** | 155 mg, 6.00 eq |

1. 20% piperidine/DMF (20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-Asn(Trt)-OH (358 mg, 3.0 eq) was weighed and added to the above resin, DIEA (155 mg, 6.00 eq) was added, and 5 mL of DMF was additionally added to the reaction column, followed by sparging with nitrogen. After amino acids were dissolved, HBTU (216 mg, 2.85 eq) was added. Nitrogen was properly adjusted to make the resin swell evenly.

3. The reaction was carried out for 0.5 hours in a 25°C environment, and after detection with ninhydrin, the resin was colorless and transparent.
4. The reaction liquid was drained, and after washing 5 times with DMF (20 mL each time) for 1 min each time, waste was discharged until no liquid flowed out.

1.2.11 Coupling of Fmoc-Pen(Trt)-OH

| Raw material | Feeding amount |
|---|---|
| **Fmoc-Pen(Trt)-OH** | 368 mg, 3.0 eq |
| **HBTU** | 216 mg, 2.85 eq |
| **DIEA** | 155 mg, 6.00 eq |

1. 20% piperidine/DMF (20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. Fmoc-Pen(Trt)-OH (368 mg, 3.0 eq) was weighed and added to the above resin, DIEA (155 mg, 6.00 eq) was added, and 5 mL of DMF was additionally added to the reaction column, followed by sparging with nitrogen. After amino acids were dissolved, HBTU (216 mg, 2.85 eq) was added. Nitrogen was properly adjusted to make the resin swell evenly.
3. The reaction was carried out for 0.5 hours in a 25°C environment, and after detection with ninhydrin, the resin was colorless and transparent.
4. The reaction liquid was drained, and after washing 5 times with DMF (20 mL each time) for 1 min each time, waste was discharged until no liquid flowed out.

1.2.12 Capping with $Ac_2O$

| Raw material | Feeding amount |
|---|---|
| **$Ac_2O$** | 102 mg, 5.0 eq |
| **DIEA** | 155 mg, 6.00 eq |

1. 20% piperidine/DMF (20 mL) was added to the reaction column, followed by sparging with nitrogen for 20 min, and waste was discharged until no liquid flowed out. After washing 5 times by adding DMF (20 mL), with 1 min each time, waste was discharged until no liquid flowed out. After detection with ninhydrin, the resin was blue.
2. DMF (10 mL), DIEA (155 mg, 6.00 eq), and $Ac_2O$ (102 mg, 5.0 eq) were added to the above resin, followed by sparging with nitrogen, such that nitrogen was properly adjusted to make the resin swell evenly.
3. The reaction was carried out for 10 min in a 25°C environment, and after detection with ninhydrin, the resin was colorless and transparent.
4. The reaction liquid was drained, and after washing 5 times with DMF (20 mL each time) for 1 min each time, waste was discharged until no liquid flowed out, whereby linear peptide **1-16-1b** was obtained.

Step 2: Cleavage of fully protected linear peptide

2.1 After washing 5 times with DMF (20 mL each time) for 1 min each time, waste was discharged until no liquid flowed out.
2.2 The resin was shrunk with MeOH (20 mL) for 3 min each time, and waste was discharged until no liquid flowed out. The resin was then washed three times with 20 mL of methyl tert-butyl ether, and the resin was dried by blowing with nitrogen, for later use.
2.3 50 mL of a prepared 20% HFIP/DCM solution was poured into a reactor, and after sparging with nitrogen, reaction once for 30 min, and cleavage three times, the filtrate was all collected and concentrated under reduced pressure to dryness. 50 mL of DCM was then added for continued concentration under reduced pressure.
2.4 100 mL of acetonitrile and 100 mL of water were added, and the mixture was ultrasonicated until uniformly dispersed and then freeze-dried by a freeze-dryer to obtain 480 mg of intermediate **1-16-1c** with a purity of 76% and a yield

of 60%.

LC/MS: $[M+H]^+$ = 3044.4; $[M+H-243]^+$ = 2801.4.

Step 3: Condensation of intermediate **1-16-1c** with **intermediate A**

The above intermediate **1-16-1c** (200 mg, 0.06 mmol) was taken, 1 mL of DMF and DIEA (3 eq, 0.18 mmol) were added, followed by **intermediate A** (77 mg, 0.06 mmol, 1 eq). HATU (23 mg, 0.06 mmol, 1 eq, dissolved in 0.2 mL of DMF) was then slowly dropwise added. As detected by Tof-LCMS, the raw materials were completely consumed. DMF was spin-dried by an oil pump to obtain crude intermediate **1-16-1d,** for later use.

LC/MS: $[M]^+$ = 4130.2, $[M-243]^+$ = 3887.2.

Step 4: Cleavage of linear peptide

4.1. Preparation of cleavage liquid according to the following volume

| Reagent | Proportion |
|---|---|
| **TFA** | 90 |
| **Tis** | 2.5 |
| **$H_2O$** | 2.5 |
| **Mpr** | 5.0 |

The crude peptide obtained in step 3 was added to the prepared cleavage liquid, shaken in a shaking table for 2.5 h, and filtered, and the filtrate was added to 10 volumes of ice-cold isopropyl ether, centrifuged, and then washed 3 times with isopropyl ether. After vacuum drying for 2 h, crude peptide **1-16-1e** was obtained.

LC/MS: $[(M+H)/2]^+$ = 1419.2; $[(M+2H)/3]^+$ = 946.5.

Step 5: Oxidation of disulfide bond

5.1 160 mg of the crude peptide **1-16-1e** obtained in step 4 was added to 100 mL of water and 100 mL of acetonitrile and dissolved clear, and 0.1M $I_2$/MeOH was then slowly dropwise added until the solution became bright yellow. After continued stirring for 5 min, the yellow color did not disappear. 0.1 M sodium thiosulfate was then dropwise added until the yellow color disappeared. When no change was found after stirring for two min, the sample was freeze-dried and purified to obtain 42 mg of a product of **Example 1-16-1,** with a purity of 97.1% and a yield of 18.0%.

LC/MS: $[(M+H)/2]^+$ = 1418.2; $[(M+2H)/3]^+$ = 945.8.

Purification conditions:

| Separation condition | |
|---|---|
| Dissolution condition | Dissolve in 10% ACN-$H_2O$ |
| Instrument | Waters 2545 pump and waters 2489 detector |
| Mobile phase | A: $H_2O$ (0.1% TFA in $H_2O$) |
| | B: $CH_3CN$ |
| Gradient | B%, 10-30%-5 min, 30-40%-45 min. Retention time: 20 min |
| Column type | Welch LP-C18 (21.2h LP-C, 10 $\mu$m) |
| Flow rate | 20 mL/min |
| Detection wavelength | 220/254 nm |
| Column temperature | Room temperature |

**Example 3-16-1**

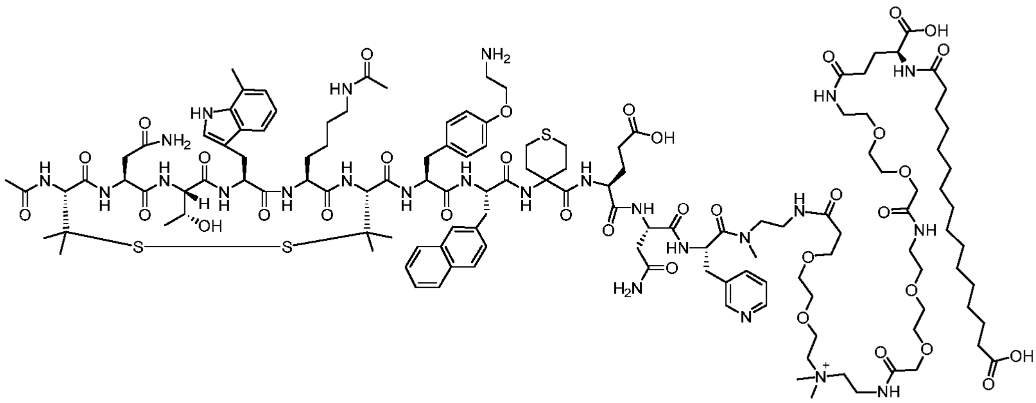

**Example 3-16-1** could also be prepared by reference to the following method:

By reference to the preparation method of **Example 1-16-1,** in which 1-(9*H*-fluoren-9-ylmethoxycarbonylamino)-4,4-difluoro-cyclohexanecarboxylic acid in step 1 of the solid-phase synthesis of the linear peptide was replaced with (9*H*-fluoren-9-yl)methyl(4-cyanotetrahydro-2*H*-thiopyran-4-yl)carbamate **A3c,** 120 mg of the product of **Example 3-16-1** was obtained, with a purity of 98.5% and a yield of 23.0%.

LC/MS: $[(M+H)/2]^+$ = 1409.6; $[(M+2H)/3]^+$ = 940.8.

**Example 4-16-1**

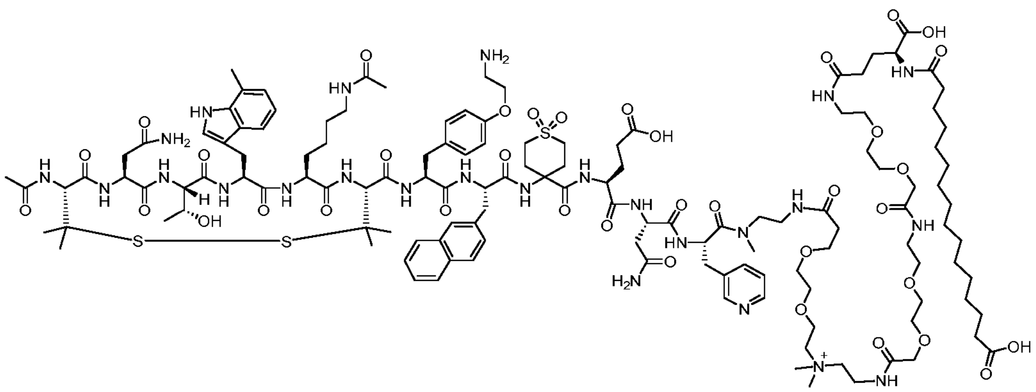

**Example 4-16-1** could also be prepared by reference to the following method:

By reference to the preparation method of **Example 1-16-1,** in which 1-(9*H*-fluoren-9-ylmethoxycarbonylamino)-4,4-difluoro-cyclohexanecarboxylic acid in step 1 of the solid-phase synthesis of the linear peptide was replaced with **intermediate A3,** 110 mg of the product of **Example 4-16-1** was obtained, with a purity of 99.4% and a yield of 21.0%.

LC/MS: $[(M+H)/2]^+$ = 1425.7; $[(M+2H)/3]^+$ = 951.0.

Purification conditions:

| Separation condition | |
|---|---|
| Dissolution condition | Dissolve in 10% ACN-$H_2O$ |

(continued)

| Separation condition | |
|---|---|
| Instrument | Waters 2545 pump and waters 2489 detector |
| Mobile phase | A: $H_2O$ (0.1% TFA in $H_2O$) |
| | B: $CH_3CN$ |
| Gradient | B%, 10-30%-5 min, 30-40%-45 min. Retention time: 20 min |
| Column type | Welch LP-C18 (21.2h LP-C, 10 μm) |
| Flow rate | 20 mL/min |
| Detection wavelength | 220/254 nm |
| Column temperature | Room temperature |

**Example 1-19**

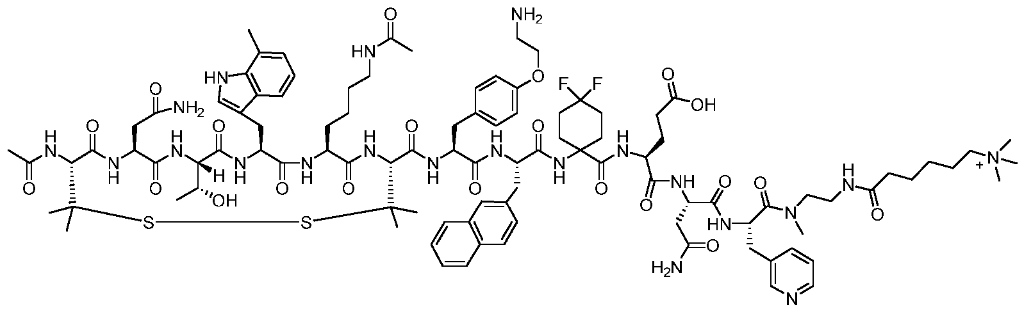

**Example 1-19** could also be prepared by reference to the following method:

By reference to the preparation method of **Example 1-16-1,** in which **intermediate A** in step 3 of coupling the linear peptide to the side chain was replaced with **intermediate L2** to obtain 110 mg of a product of **Example 1-19,** with a purity of 95.1% and a yield of 15.4%.

LC/MS: $[(M+H)/2]^+$ = 1037.5; $[(M+2H)/3]^+$ = 692.4.

**Example 3-19**

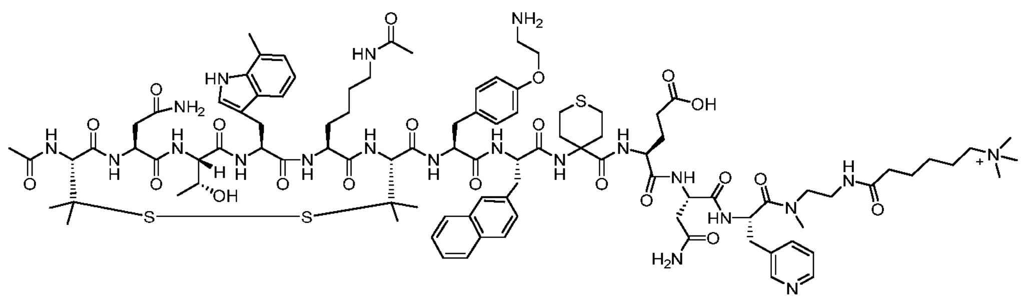

**Example 3-19** could also be prepared by reference to the following method:

By reference to the preparation method of **Example 1-16-1,** in which 1-(9*H*-fluoren-9-ylmethoxycarbonylamino)-4,4-difluoro-cyclohexanecarboxylic acid in step 1 of the solid-phase synthesis of the linear peptide was replaced with (9*H*-fluoren-9-yl)methyl(4-cyanotetrahydro-2*H*-thiopyran-4-yl)carbamate **A3c** and the side chain **intermediate A** in step 3 of coupling the linear peptide to the side chain was replaced with **intermediate L2,** 110 mg of a product of **Example 3-19**

was obtained, with a purity of 99.0% and a yield of 15.4%.

LC/MS: $[(M+H)/2]^+ = 1028.5$; $[(M+2H)/3]^+ = 686.4$.

## Example 4-19

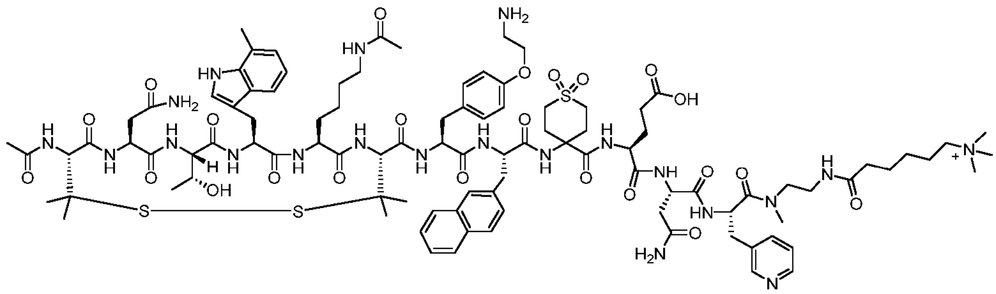

**Example 4-19** could also be prepared by reference to the following method:

By reference to the preparation method of **Example 1-16-1,** in which 1-(9*H*-fluoren-9-ylmethoxycarbonylamino)-4,4-difluoro-cyclohexanecarboxylic acid in step 1 of the solid-phase synthesis of the linear peptide was replaced with intermediate **A3** and the side chain intermediate **A** in step 3 of coupling the linear peptide to the side chain was replaced with intermediate **L2,** 102 mg of a product of Example **4-19** was obtained, with a purity of 99.8% and a yield of 14.2%.

LC/MS: $[(M+H)/2]^+ = 1044.5$; $[(M+2H)/3]^+ = 697.0$.

The following examples could be prepared by reference to the above preparation method:

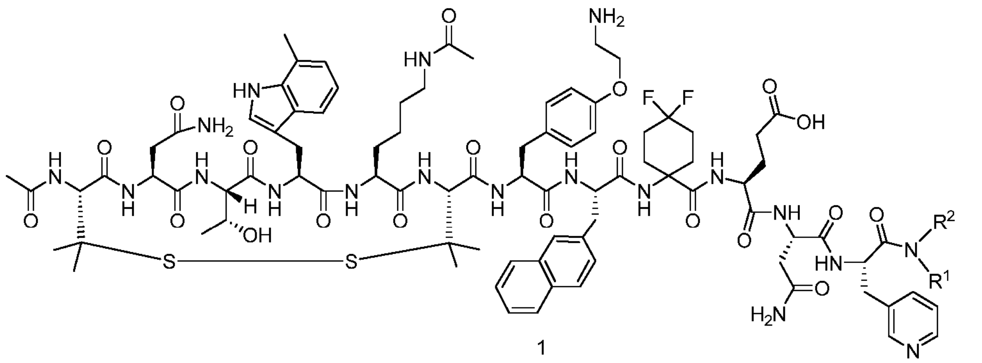

1

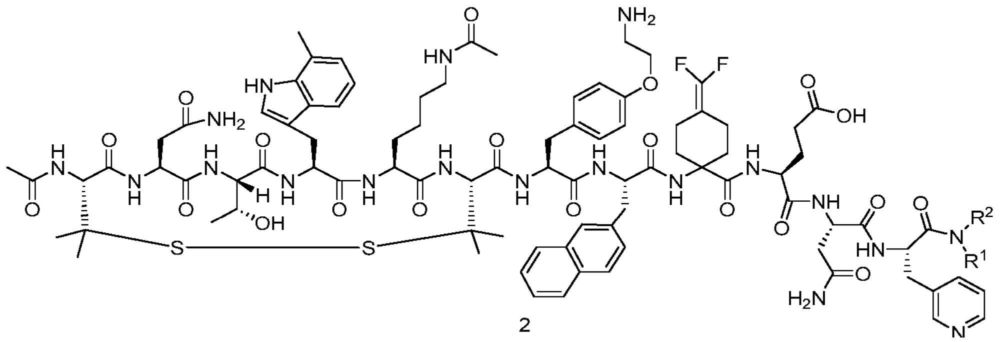

2

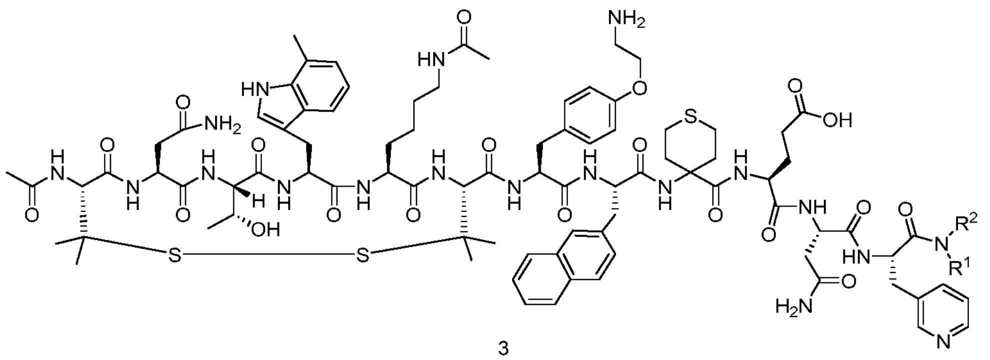

3

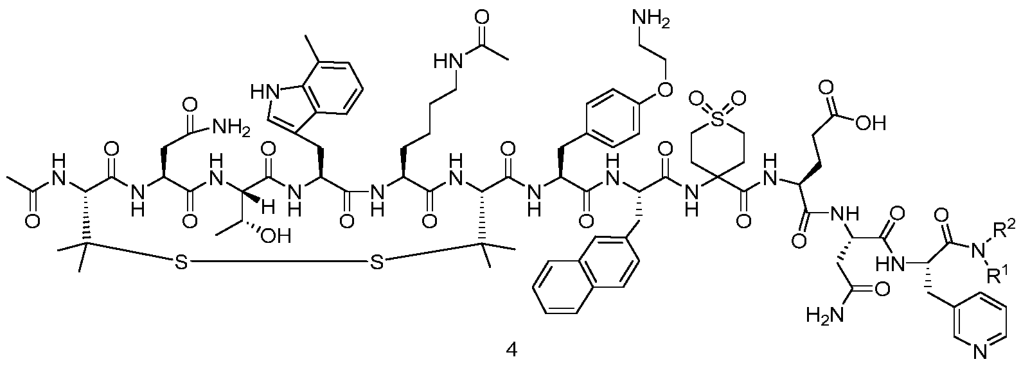

4

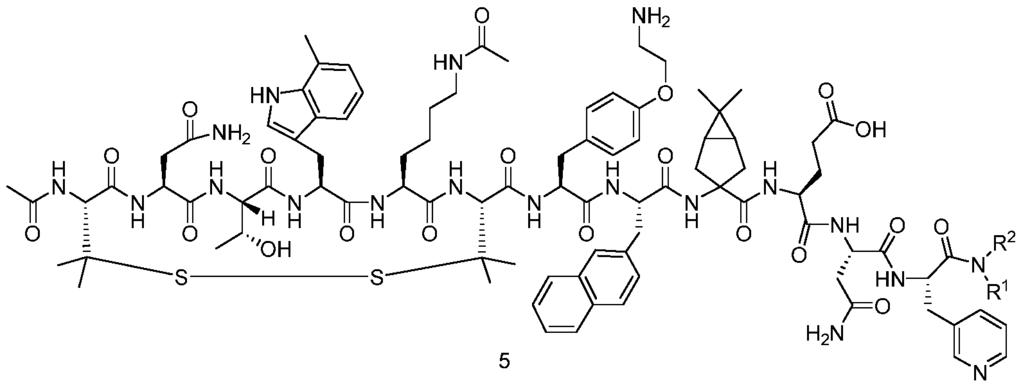

5

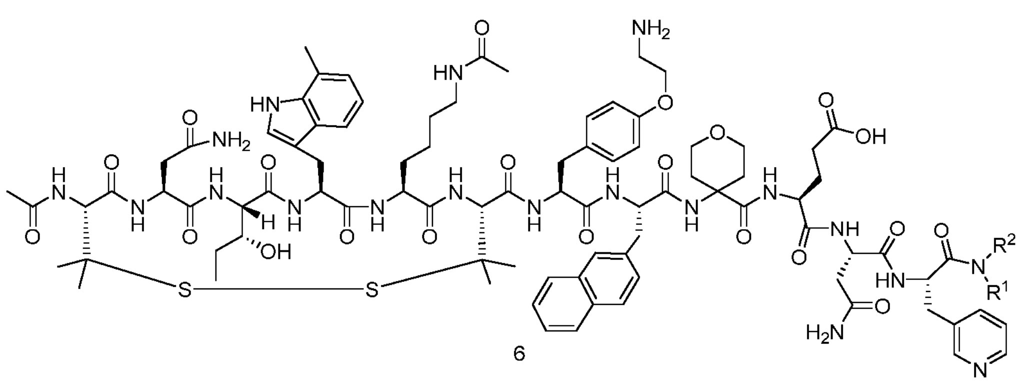

6

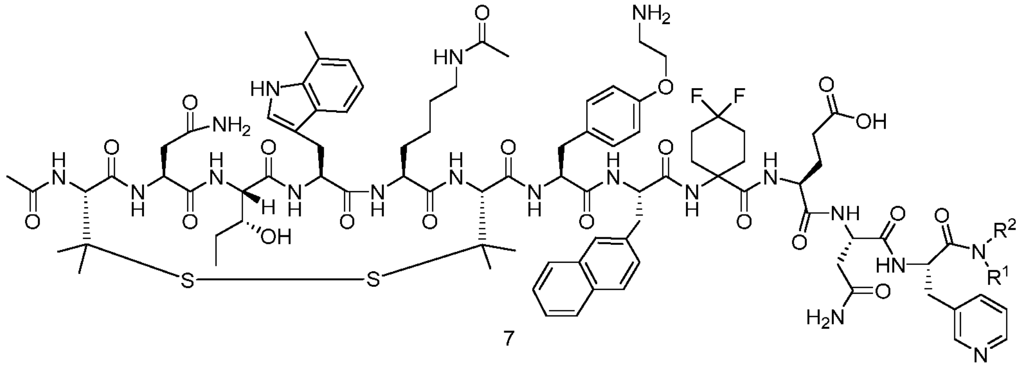

7

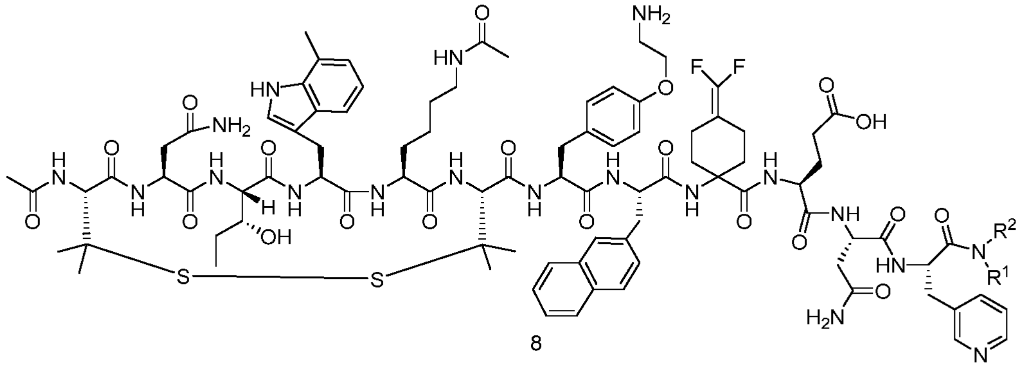

8

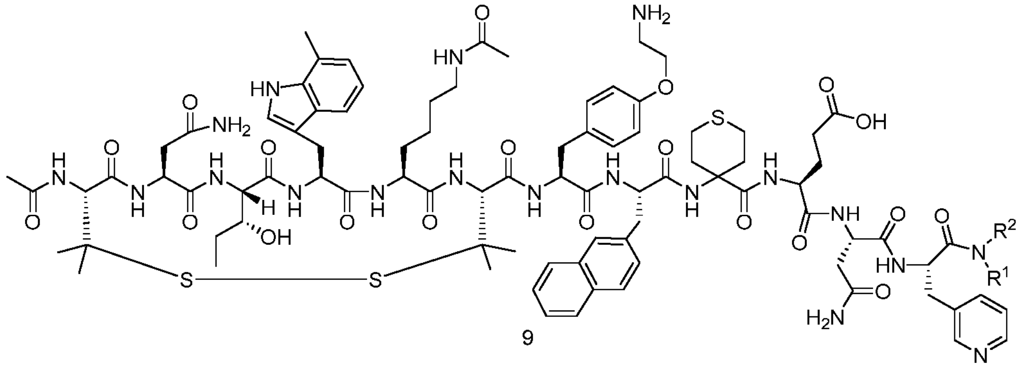

9

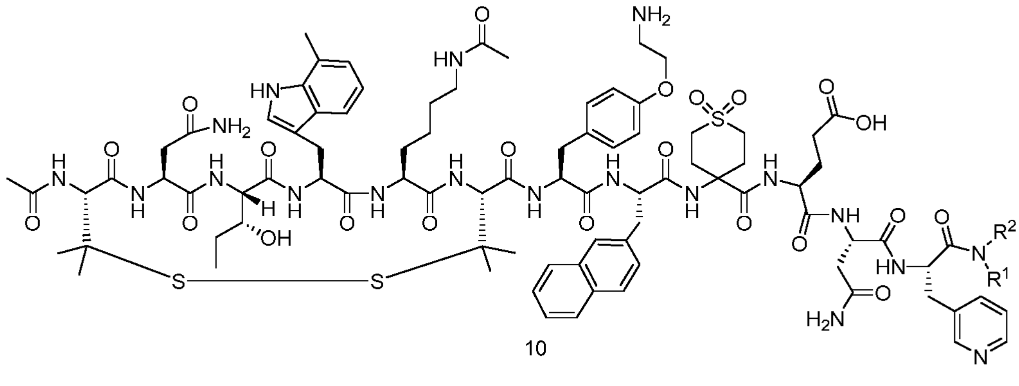

10

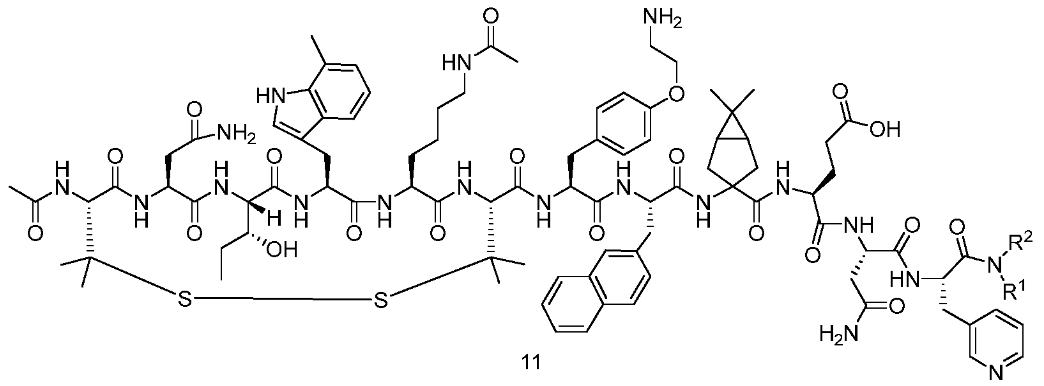

11

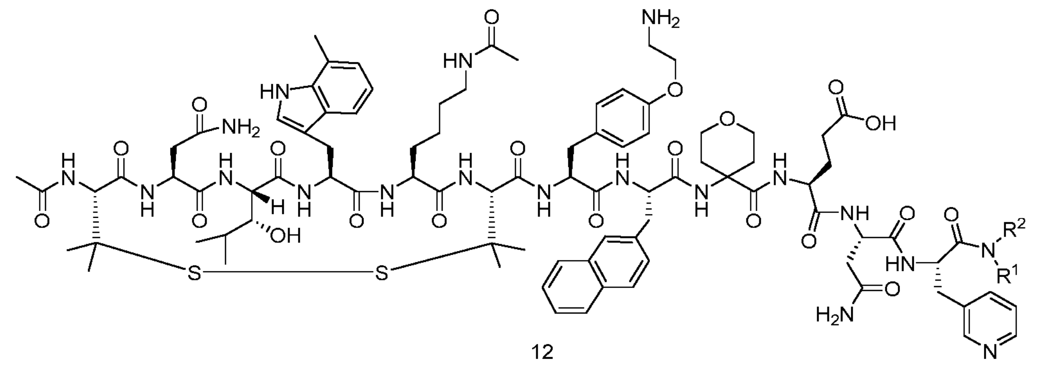

12

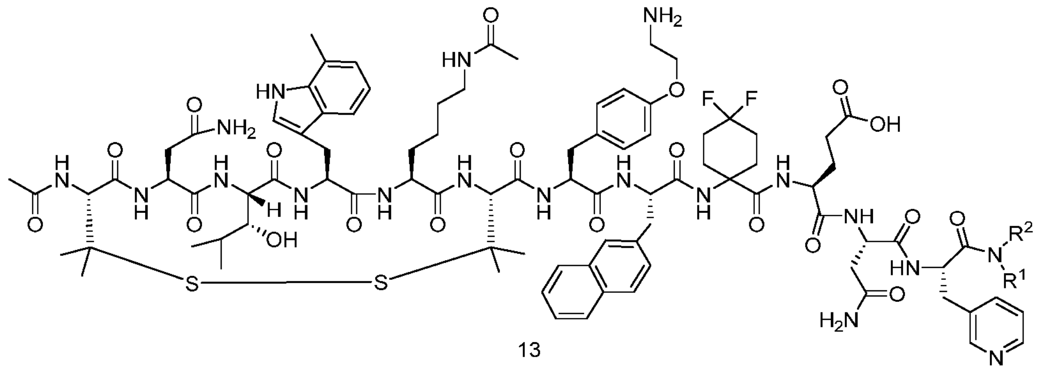

13

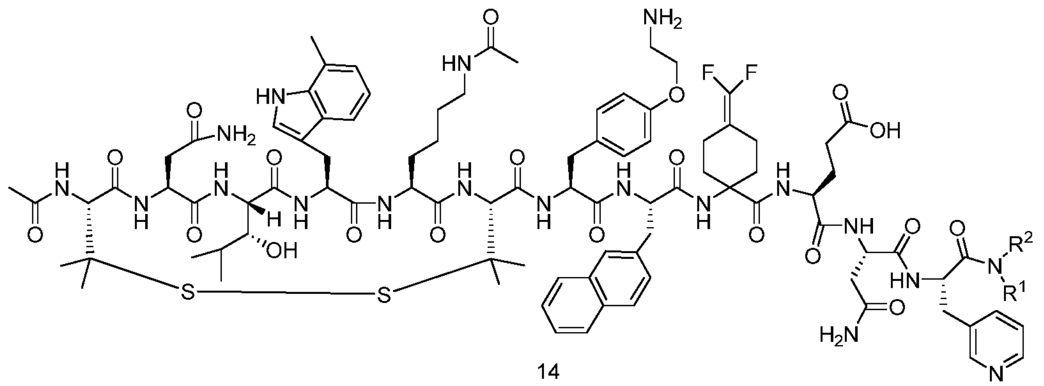

14

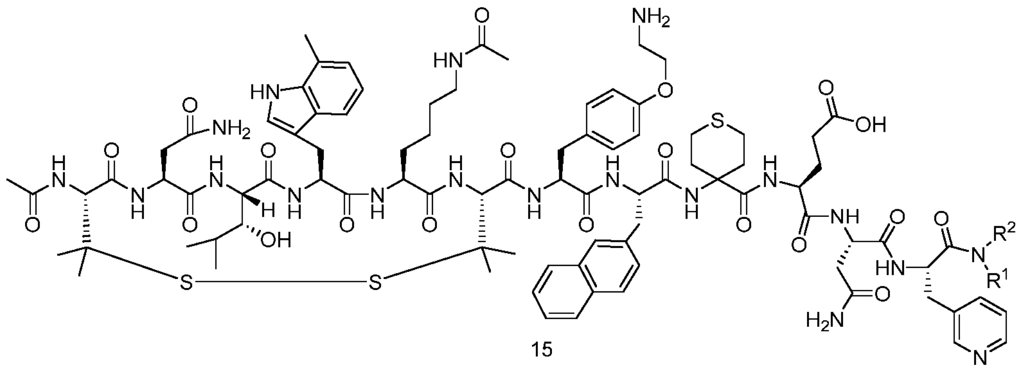

15

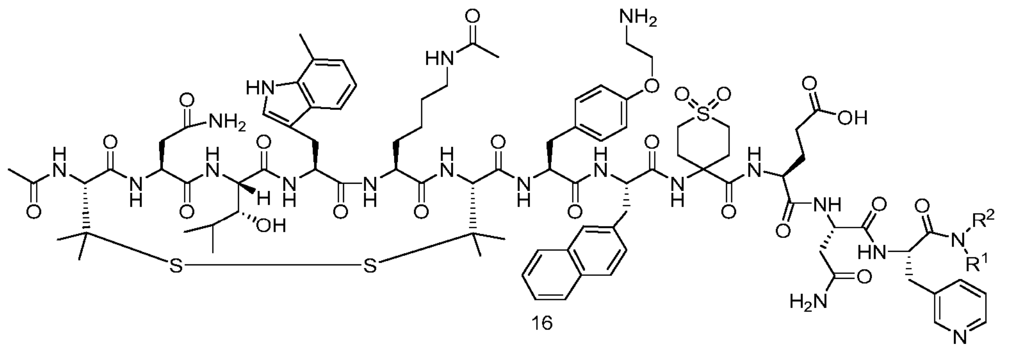

16

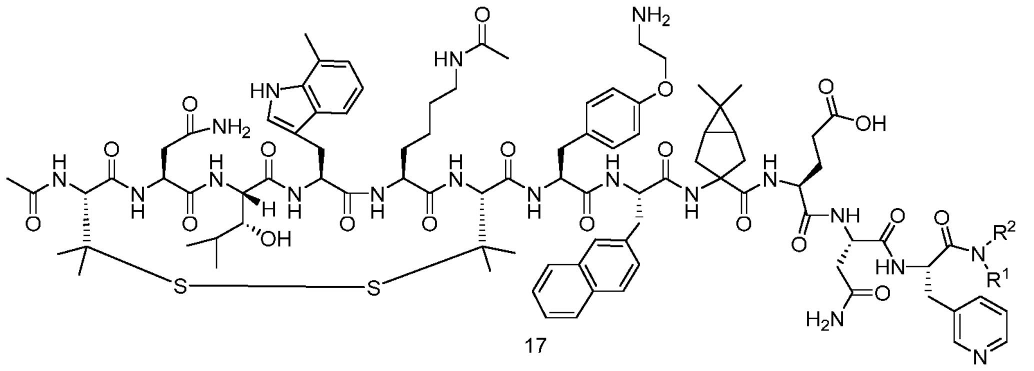

17

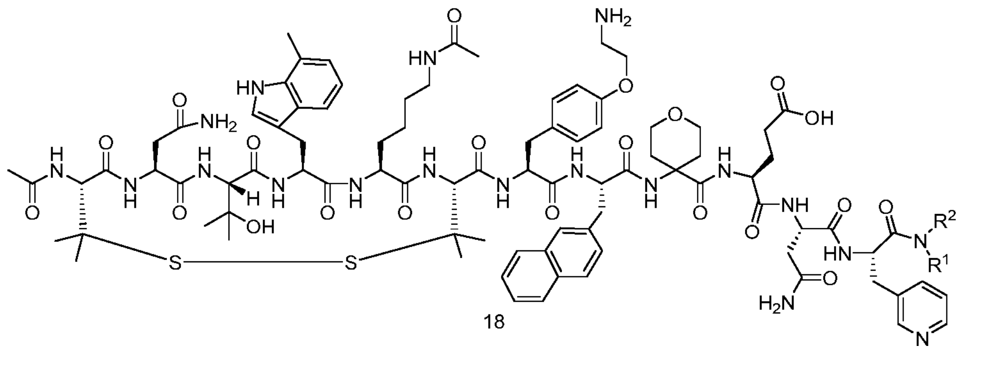

18

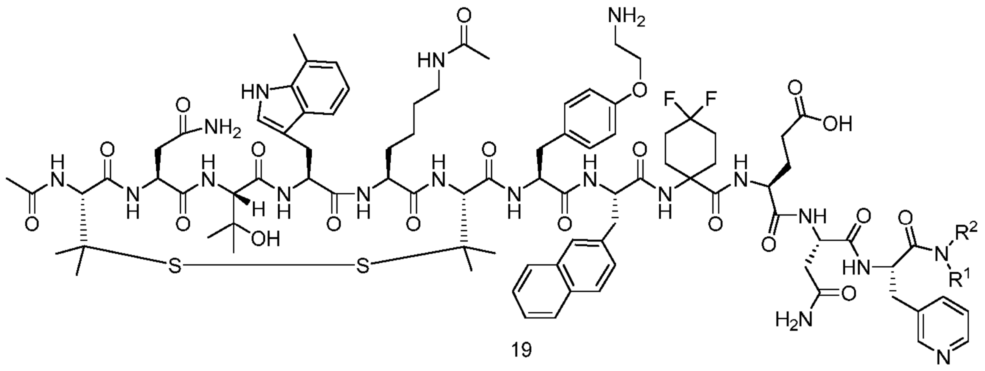

19

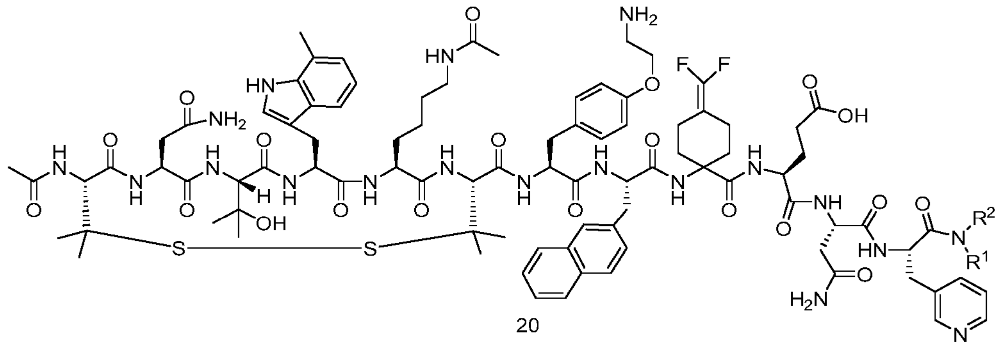

20

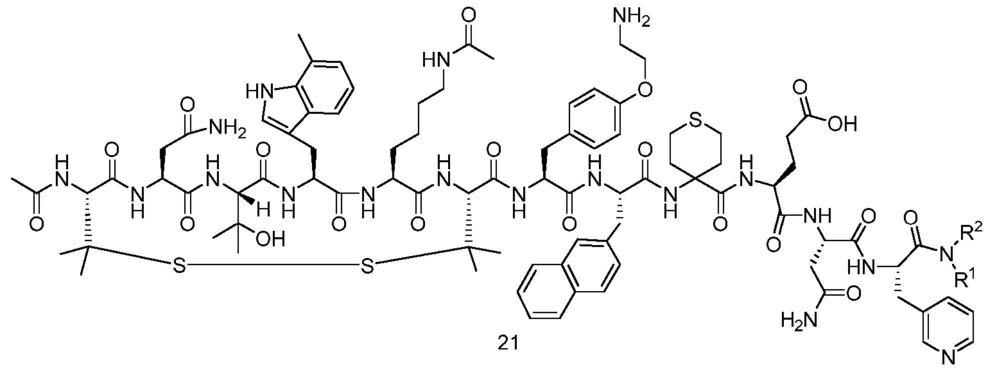

21

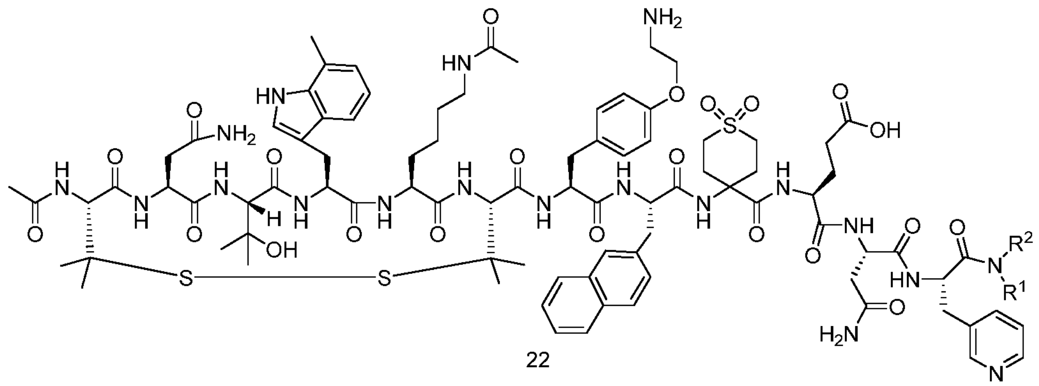

22

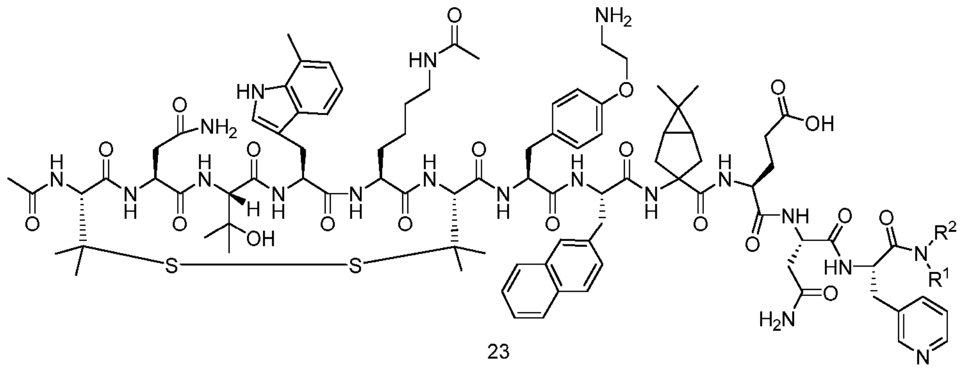

23

wherein $R^1$ and $R^2$ were selected from the following structures

Table 1

| General formula | $R^2$ | Compound | $R^1$ | Compound | $R^1$ |
|---|---|---|---|---|---|
| 1 | $-CH_2C(O)NH_2$ | 1-1 | -H | 1-14 | $-CH_3$ |
| 2 | | 2-1 | | 2-14 | |
| 3 | | 3-1 | | 3-14 | |
| 4 | | 4-1 | | 4-14 | |
| 5 | | 5-1 | | 5-14 | |
| 6 | | 6-1 | | 6-14 | |
| 7 | | 7-1 | | 7-14 | |
| 8 | | 8-1 | | 8-14 | |
| 9 | | 9-1 | | 9-14 | |
| 10 | | 10-1 | | 10-14 | |
| 11 | | 11-1 | | 11-14 | |
| 12 | | 12-1 | | 12-14 | |
| 13 | | 13-1 | | 13-14 | |
| 14 | | 14-1 | | 14-14 | |
| 15 | | 15-1 | | 15-14 | |
| 16 | | 16-1 | | 16-14 | |

(continued)

| General formula | $R^2$ | Compound | $R^1$ | Compound | $R^1$ |
|---|---|---|---|---|---|
| 17 | | 17-1 | | 17-14 | |
| 18 | | 18-1 | | 18-14 | |
| 19 | | 19-1 | | 19-14 | |
| 20 | | 20-1 | | 20- 14 | |
| 21 | | 21-1 | | 21-14 | |
| 22 | | 22-1 | | 22-14 | |
| 23 | | 23-1 | | 23-14 | |
| 1 | 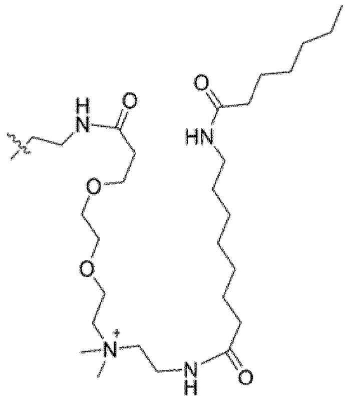 | 1-2 | | 1-15 | |
| 2 | | 2-2 | | 2-15 | |
| 3 | | 3-2 | | 3-15 | |
| 4 | | 4-2 | | 4-15 | |
| 5 | | 5-2 | | 5-15 | |
| 6 | | 6-2 | | 6-15 | |
| 7 | | 7-2 | | 7-15 | |
| 8 | | 8-2 | | 8-15 | |
| 9 | | 9-2 | | 9-15 | |
| 10 | | 10-2 | | 10-15 | |
| 11 | | 11-2 | | 11-15 | |
| 12 | | 12-2 | | 12-15 | |
| 13 | | 13-2 | | 13-15 | |
| 14 | | 14-2 | | 14-15 | |
| 15 | | 15-2 | | 15-15 | |
| 16 | | 16-2 | | 16-15 | |
| 17 | | 17-2 | | 17-15 | |
| 18 | | 18-2 | | 18-15 | |
| 19 | | 19-2 | | 19-15 | |
| 20 | | 20-2 | | 20-15 | |
| 21 | | 21-2 | | 21-15 | |
| 22 | | 22-2 | | 22-15 | |
| 23 | | 23-2 | | 23-15 | |
| 1 | | 1-3 | | 1-16 | |
| 2 | | 2-3 | | 2-16 | |
| 3 | | 3-3 | | 3-16 | |

(continued)

| General formula | $R^2$ | Compound | $R^1$ | Compound | $R^1$ |
|---|---|---|---|---|---|
| 4 | 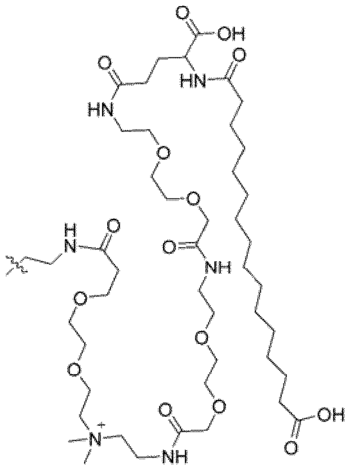 | 4-3 | | 4-16 | |
| 5 | | 5-3 | | 5-16 | |
| 6 | | 6-3 | | 6-16 | |
| 7 | | 7-3 | | 7-16 | |
| 8 | | 8-3 | | 8-16 | |
| 9 | | 9-3 | | 9-16 | |
| 10 | | 10-3 | | 10-16 | |
| 11 | | 11-3 | | 11-16 | |
| 12 | | 12-3 | | 12-16 | |
| 13 | | 13-3 | | 13-16 | |
| 14 | | 14-3 | | 14-16 | |
| 15 | | 15-3 | | 15-16 | |
| 16 | | 16-3 | | 16-16 | |
| 17 | | 17-3 | | 17-16 | |
| 18 | | 18-3 | | 18-16 | |
| 19 | | 19-3 | | 19-16 | |
| 20 | | 20-3 | | 20-16 | |
| 21 | | 21-3 | | 21-16 | |
| 22 | | 22-3 | | 22-16 | |
| 23 | | 23-3 | | 23-16 | |
| 1 | 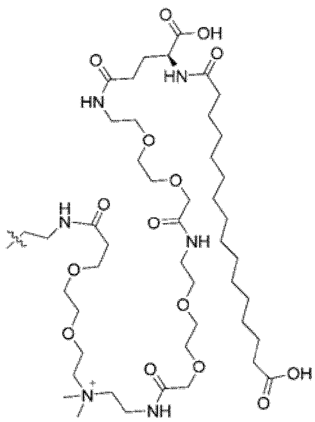 | 1-3-1 | | 1-16-1 | |
| 2 | | 2-3-1 | | 2-16-1 | |
| 3 | | 3-3-1 | | 3-16-1 | |
| 4 | | 4-3-1 | | 4-16-1 | |
| 5 | | 5-3-1 | | 5-16-1 | |
| 6 | | 6-3-1 | | 6-16-1 | |
| 7 | | 7-3-1 | | 7-16-1 | |
| 8 | | 8-3-1 | | 8-16-1 | |
| 9 | | 9-3-1 | | 9-16-1 | |
| 10 | | 10-3-1 | | 10-16-1 | |
| 11 | | 11-3-1 | | 11-16-1 | |
| 12 | | 12-3-1 | | 12-16-1 | |
| 13 | | 13-3-1 | | 13-16-1 | |

(continued)

| General formula | $R^2$ | Compound | $R^1$ | Compound | $R^1$ |
|---|---|---|---|---|---|
| 14 | | 14-3-1 | | 14-16-1 | |
| 15 | | 15-3-1 | | 15-16-1 | |
| 16 | | 16-3-1 | | 16-16-1 | |
| 17 | | 17-3-1 | | 17-16-1 | |
| 18 | | 18-3-1 | | 18-16-1 | |
| 19 | | 19-3-1 | | 19-16-1 | |
| 20 | | 20-3-1 | | 20-16-1 | |
| 21 | | 21-3-1 | | 21-16-1 | |
| 22 | | 22-3-1 | | 22-16-1 | |
| 23 | | 23-3-1 | | 23-16-1 | |
| 1 | 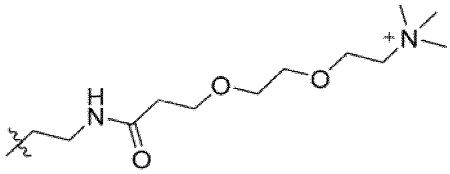 | 1-4 | | 1-17 | |
| 2 | | 2-4 | | 2-17 | |
| 3 | | 3-4 | | 3-17 | |
| 4 | | 4-4 | | 4-17 | |
| 5 | | 5-4 | | 5-17 | |
| 6 | | 6-4 | | 6-17 | |
| 7 | | 7-4 | | 7-17 | |
| 8 | | 8-4 | | 8-17 | |
| 9 | | 9-4 | | 9-17 | |
| 10 | | 10-4 | | 10-17 | |
| 11 | | 11-4 | | 11-17 | |
| 12 | | 12-4 | | 12-17 | |
| 13 | | 13-4 | | 13-17 | |
| 14 | | 14-4 | | 14-17 | |
| 15 | | 15-4 | | 15-17 | |
| 16 | | 16-4 | | 16-17 | |
| 17 | | 17-4 | | 17-17 | |
| 18 | | 18-4 | | 18-17 | |
| 19 | | 19-4 | | 19-17 | |
| 20 | | 20-4 | | 20-17 | |
| 21 | | 21-4 | | 21-17 | |
| 22 | | 22-4 | | 22-17 | |
| 23 | | 23-4 | | 23-17 | |

(continued)

| General formula | $R^2$ | Compound | $R^1$ | Compound | $R^1$ |
|---|---|---|---|---|---|
| 1 | 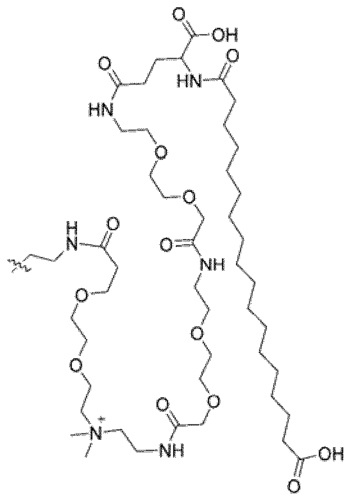 | 1-5 | | 1-18 | |
| 2 | | 2-5 | | 2-18 | |
| 3 | | 3-5 | | 3-18 | |
| 4 | | 4-5 | | 4-18 | |
| 5 | | 5-5 | | 5-18 | |
| 6 | | 6-5 | | 6-18 | |
| 7 | | 7-5 | | 7-18 | |
| 8 | | 8-5 | | 8-18 | |
| 9 | | 9-5 | | 9-18 | |
| 10 | | 10-5 | | 10-18 | |
| 11 | | 11-5 | | 11-18 | |
| 12 | | 12-5 | | 12-18 | |
| 13 | | 13-5 | | 13-18 | |
| 14 | | 14-5 | | 14-18 | |
| 15 | | 15-5 | | 15-18 | |
| 16 | | 16-5 | | 16-18 | |
| 17 | | 17-5 | | 17-18 | |
| 18 | | 18-5 | | 19-18 | |
| 19 | | 19-5 | | 19-18 | |
| 20 | | 20-5 | | 20-18 | |
| 21 | | 21-5 | | 21-18 | |
| 22 | | 22-5 | | 22-18 | |
| 23 | | 23-5 | | 23-18 | |
| 1 | 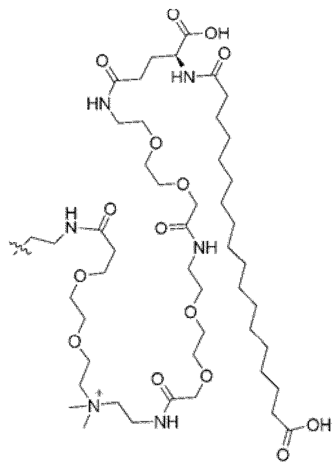 | 1-5-1 | | 1-18-1 | |
| 2 | | 2-5-1 | | 2-18-1 | |
| 3 | | 3-5-1 | | 3-18-1 | |
| 4 | | 4-5-1 | | 4-18-1 | |
| 5 | | 5-5-1 | | 5-18-1 | |
| 6 | | 6-5-1 | | 6-18-1 | |
| 7 | | 7-5-1 | | 7-18-1 | |
| 8 | | 8-5-1 | | 8-18-1 | |
| 9 | | 9-5-1 | | 9-18-1 | |
| 10 | | 10-5-1 | | 10-18-1 | |

(continued)

| General formula | $R^2$ | Compound | $R^1$ | Compound | $R^1$ |
|---|---|---|---|---|---|
| 11 | | 11-5-1 | | 11-18-1 | |
| 12 | | 12-5-1 | | 12-18-1 | |
| 13 | | 13-5-1 | | 13-18-1 | |
| 14 | | 14-5-1 | | 14-18-1 | |
| 15 | | 15-5-1 | | 15-18-1 | |
| 16 | | 16-5-1 | | 16-18-1 | |
| 17 | | 17-5-1 | | 17-18-1 | |
| 18 | | 18-5-1 | | 18-18-1 | |
| 19 | | 19-5-1 | | 19-18-1 | |
| 20 | | 20-5-1 | | 20-18- 1 | |
| 21 | | 21-5-1 | | 21-18-1 | |
| 22 | | 22-5-1 | | 22-18-1 | |
| 23 | | 23-5-1 | | 23-18-1 | |
| 1 | 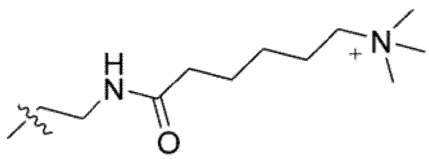 | 1-6 | | 1-19 | |
| 2 | | 2-6 | | 2-19 | |
| 3 | | 3-6 | | 3-19 | |
| 4 | | 4-6 | | 4-19 | |
| 5 | | 5-6 | | 5-19 | |
| 6 | | 6-6 | | 6-19 | |
| 7 | | 7-6 | | 7-19 | |
| 8 | | 8-6 | | 8-19 | |
| 9 | | 9-6 | | 9-19 | |
| 10 | | 10-6 | | 10-19 | |
| 11 | | 11-6 | | 11-19 | |
| 12 | | 12-6 | | 12-19 | |
| 13 | | 13-6 | | 13-19 | |
| 14 | | 14-6 | | 14-19 | |
| 15 | | 15-6 | | 15-19 | |
| 16 | | 16-6 | | 16-19 | |
| 17 | | 17-6 | | 17-19 | |
| 18 | | 18-6 | | 18-19 | |
| 19 | | 19-6 | | 19-19 | |
| 20 | | 20-6 | | 20-19 | |

(continued)

| General formula | $R^2$ | Compound | $R^1$ | Compound | $R^1$ |
|---|---|---|---|---|---|
| 21 | | 21-6 | | 21-19 | |
| 22 | | 22-6 | | 22-19 | |
| 23 | | 23-6 | | 23-19 | |
| 1 | 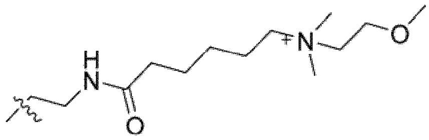 | 1-7 | | 1-20 | |
| 2 | | 2-7 | | 2-20 | |
| 3 | | 3-7 | | 3-20 | |
| 4 | | 4-7 | | 4-20 | |
| 5 | | 5-7 | | 5-20 | |
| 6 | | 6-7 | | 6-20 | |
| 7 | | 7-7 | | 7-20 | |
| 8 | | 8-7 | | 8-20 | |
| 9 | | 9-7 | | 9-20 | |
| 10 | | 10-7 | | 10-20 | |
| 11 | | 11-7 | | 11-20 | |
| 12 | | 12-7 | | 12-20 | |
| 13 | | 13-7 | | 13-20 | |
| 14 | | 14-7 | | 14-20 | |
| 15 | | 15-7 | | 15-20 | |
| 16 | | 16-7 | | 16-20 | |
| 17 | | 17-7 | | 17-20 | |
| 18 | | 18-7 | | 18-20 | |
| 19 | | 19-7 | | 19-20 | |
| 20 | | 20-7 | | 20-20 | |
| 21 | | 21-7 | | 21-20 | |
| 22 | | 22-7 | | 22-20 | |
| 23 | | 23-7 | | 23-20 | |
| 1 | 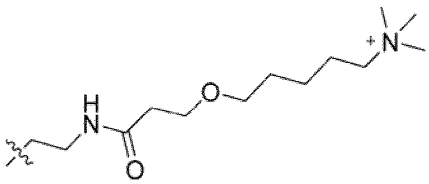 | 1-8 | | 1-21 | |
| 2 | | 2-8 | | 2-21 | |
| 3 | | 3-8 | | 3-21 | |
| 4 | | 4-8 | | 4-21 | |
| 5 | | 5-8 | | 5-21 | |
| 6 | | 6-8 | | 6-21 | |
| 7 | | 7-9 | | 7-21 | |

(continued)

| General formula | $R^2$ | Compound | $R^1$ | Compound | $R^1$ |
|---|---|---|---|---|---|
| 8 | | 8-8 | | 8-21 | |
| 9 | | 9-8 | | 9-21 | |
| 10 | | 10-8 | | 10-21 | |
| 11 | | 11-8 | | 11-21 | |
| 12 | | 12-8 | | 12-21 | |
| 13 | | 13-8 | | 13-21 | |
| 14 | | 14-8 | | 14-21 | |
| 15 | | 15-8 | | 15-21 | |
| 16 | | 16-8 | | 16-21 | |
| 17 | | 17-8 | | 17-21 | |
| 18 | | 1 8-8 | | 18-21 | |
| 19 | | 19-8 | | 19-21 | |
| 20 | | 20-8 | | 20-21 | |
| 21 | | 21-8 | | 21-21 | |
| 22 | | 22-8 | | 22-21 | |
| 23 | | 23-8 | | 23-21 | |
| 1 | 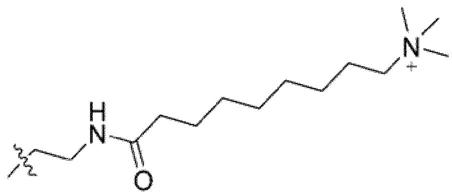 | 1-9 | | 1-22 | |
| 2 | | 2-9 | | 2-22 | |
| 3 | | 3-9 | | 3-22 | |
| 4 | | 4-9 | | 4-22 | |
| 5 | | 5-9 | | 5-22 | |
| 6 | | 6-9 | | 6-22 | |
| 7 | | 7-9 | | 7-22 | |
| 8 | | 8-9 | | 8-22 | |
| 9 | | 9-9 | | 9-22 | |
| 10 | | 10-9 | | 10-22 | |
| 11 | | 11-9 | | 11-22 | |
| 12 | | 12-9 | | 12-22 | |
| 13 | | 13-9 | | 13-22 | |
| 14 | | 14-9 | | 14-22 | |
| 15 | | 15-9 | | 15-22 | |
| 16 | | 16-9 | | 16-22 | |
| 17 | | 17-9 | | 17-22 | |

(continued)

| General formula | $R^2$ | Compound | $R^1$ | Compound | $R^1$ |
|---|---|---|---|---|---|
| 18 | | 18-9 | | 18-22 | |
| 19 | | 19-9 | | 19-22 | |
| 20 | | 20-9 | | 20-22 | |
| 21 | | 21-9 | | 21-22 | |
| 22 | | 22-9 | | 22-22 | |
| 23 | | 23-9 | | 23-22 | |
| 1 | 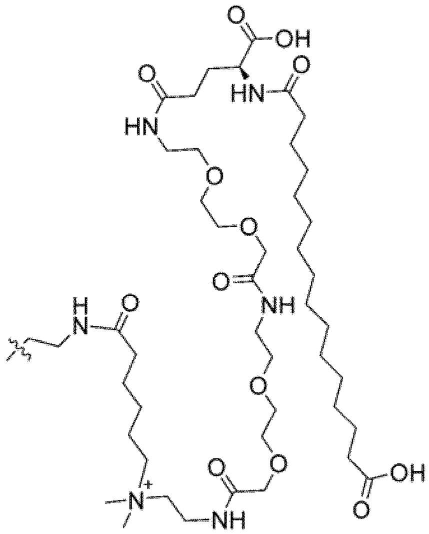 | 1-10 | | 1-23 | |
| 2 | | 2-10 | | 2-23 | |
| 3 | | 3-10 | | 3-23 | |
| 4 | | 4-10 | | 4-23 | |
| 5 | | 5-10 | | 5-23 | |
| 6 | | 6-10 | | 6-23 | |
| 7 | | 7-10 | | 7-23 | |
| 8 | | 8-10 | | 8-23 | |
| 9 | | 9-10 | | 9-23 | |
| 10 | | 10-10 | | 10-23 | |
| 11 | | 11-10 | | 11-23 | |
| 12 | | 12-10 | | 12-23 | |
| 13 | | 13-10 | | 13-23 | |
| 14 | | 14-10 | | 14-23 | |
| 15 | | 15-10 | | 15-23 | |
| 16 | | 16-10 | | 16-23 | |
| 17 | | 17-10 | | 17-23 | |
| 18 | | 18-10 | | 18-23 | |
| 19 | | 19-10 | | 19-23 | |
| 20 | | 20-10 | | 20-23 | |
| 21 | | 21-10 | | 21-23 | |
| 22 | | 22-10 | | 22-23 | |
| 23 | | 23-10 | | 23-23 | |
| 1 | | 1-11 | | 1-24 | |
| 2 | | 2-11 | | 2-24 | |
| 3 | | 3-11 | | 3-24 | |
| 4 | | 4-11 | | 4-24 | |

(continued)

| General formula | $R^2$ | Compound | $R^1$ | Compound | $R^1$ |
|---|---|---|---|---|---|
| 5 | 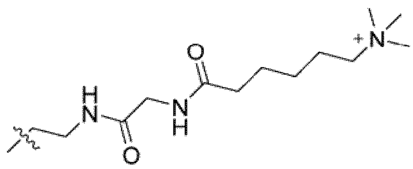 | 5-11 | | 5-24 | |
| 6 | | 6-11 | | 6-24 | |
| 7 | | 7-11 | | 7-24 | |
| 8 | | 8-11 | | 8-24 | |
| 9 | | 9-11 | | 9-24 | |
| 10 | | 10-11 | | 10-24 | |
| 11 | | 11-11 | | 11-24 | |
| 12 | | 12-11 | | 12-24 | |
| 13 | | 13-11 | | 13-24 | |
| 14 | | 14-11 | | 14-24 | |
| 15 | | 15-11 | | 15-24 | |
| 16 | | 16-11 | | 16-24 | |
| 17 | | 17-11 | | 17-24 | |
| 18 | | 18-11 | | 18-24 | |
| 19 | | 19-11 | | 19-24 | |
| 20 | | 20-11 | | 20-24 | |
| 21 | | 21-11 | | 21-24 | |
| 22 | | 22-11 | | 22-24 | |
| 23 | | 23-11 | | 23-24 | |
| 1 | 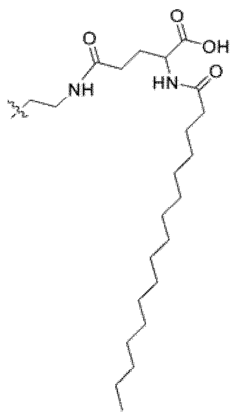 | 1-12 | | 1-25 | |
| 2 | | 2-12 | | 2-25 | |
| 3 | | 3-12 | | 3-25 | |
| 4 | | 4-12 | | 4-25 | |
| 5 | | 5-12 | | 5-25 | |
| 6 | | 6-12 | | 6-25 | |
| 7 | | 7-12 | | 7-25 | |
| 8 | | 8-12 | | 8-25 | |
| 9 | | 9-12 | | 9-25 | |
| 10 | | 10-12 | | 10-25 | |
| 11 | | 11-12 | | 11-25 | |
| 12 | | 12-12 | | 12-25 | |
| 13 | | 13-12 | | 13-25 | |
| 14 | | 14-12 | | 14-25 | |

(continued)

| General formula | $R^2$ | Compound | $R^1$ | Compound | $R^1$ |
|---|---|---|---|---|---|
| 15 | | 15-12 | | 15-25 | |
| 16 | | 16-12 | | 16-25 | |
| 17 | | 17-12 | | 17-25 | |
| 18 | | 18-12 | | 18-25 | |
| 19 | | 19-12 | | 19-25 | |
| 20 | | 20-12 | | 20-25 | |
| 21 | | 21-12 | | 21-25 | |
| 22 | | 22-12 | | 22-25 | |
| 23 | | 23-12 | | 23-25 | |
| 1 | 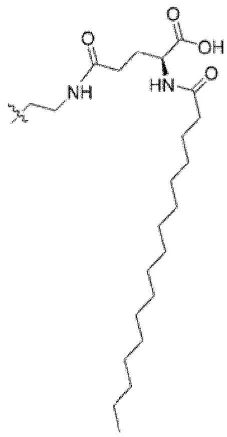 | 1-12-1 | | 1-25-1 | |
| 2 | | 2-12-1 | | 2-25-1 | |
| 3 | | 3-12-1 | | 3-25-1 | |
| 4 | | 4-12-1 | | 4-25-1 | |
| 5 | | 5-12-1 | | 5-25-1 | |
| 6 | | 6-12-1 | | 6-25-1 | |
| 7 | | 7-12-1 | | 7-25-1 | |
| 8 | | 8-12-1 | | 8-25-1 | |
| 9 | | 9-12-1 | | 9-25-1 | |
| 10 | | 10-12-1 | | 10-25-1 | |
| 11 | | 11-12-1 | | 11-25-1 | |
| 12 | | 12-12-1 | | 12-25-1 | |
| 13 | | 13-12-1 | | 13-25-1 | |
| 14 | | 14-12-1 | | 14-25-1 | |
| 15 | | 15-12-1 | | 15-25-1 | |
| 16 | | 16-12-1 | | 16-25-1 | |
| 17 | | 17-12-1 | | 17-25-1 | |
| 18 | | 18-12-1 | | 18-25-1 | |
| 19 | | 19-12-1 | | 19-25-1 | |
| 20 | | 20-12-1 | | 20-25-1 | |
| 21 | | 21-12-1 | | 21-25-1 | |
| 22 | | 22-12-1 | | 22-25-1 | |
| 23 | | 23-12-1 | | 23-25-1 | |
| 1 | | 1-13 | | 1-26 | |

(continued)

| General formula | $R^2$ | Compound | $R^1$ | Compound | $R^1$ |
|---|---|---|---|---|---|
| 2 | 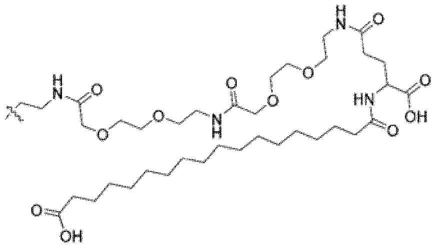 | 2-13 | | 2-26 | |
| 3 | | 3-13 | | 3-26 | |
| 4 | | 4-13 | | 4-26 | |
| 5 | | 5-13 | | 5-26 | |
| 6 | | 6-13 | | 6-26 | |
| 7 | | 7-13 | | 7-26 | |
| 8 | | 8-13 | | 8-26 | |
| 9 | | 9-13 | | 9-26 | |
| 10 | | 10-13 | | 10-26 | |
| 11 | | 11-13 | | 11-26 | |
| 12 | | 12-13 | | 12-26 | |
| 13 | | 13-13 | | 13-26 | |
| 14 | | 14-13 | | 14-26 | |
| 15 | | 15-13 | | 15-26 | |
| 16 | | 16-13 | | 16-26 | |
| 17 | | 17-13 | | 17-26 | |
| 18 | | 18-13 | | 18-26 | |
| 19 | | 19-13 | | 19-26 | |
| 20 | | 20-13 | | 20-26 | |
| 21 | | 21-13 | | 21-26 | |
| 22 | | 22-13 | | 22-26 | |
| 23 | | 23-13 | | 23-26 | |
| 1 | 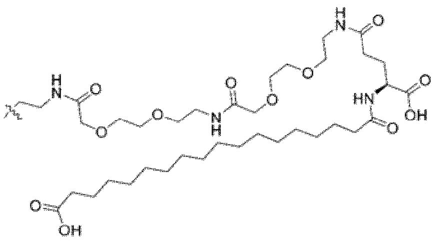 | 1-13-1 | | 1-26-1 | |
| 2 | | 2-13-1 | | 2-26-1 | |
| 3 | | 3-13-1 | | 3-26-1 | |
| 4 | | 4-13-1 | | 4-26-1 | |
| 5 | | 5-13-1 | | 5-26-1 | |
| 6 | | 6-13-1 | | 6-26-1 | |
| 7 | | 7-13-1 | | 7-26-1 | |
| 8 | | 8-13-1 | | 8-26-1 | |
| 9 | | 9-13-1 | | 9-26-1 | |
| 10 | | 10-13-1 | | 10-26-1 | |
| 11 | | 11-13-1 | | 11-26-1 | |

(continued)

| General formula | $R^2$ | Compound | $R^1$ | Compound | $R^1$ |
|---|---|---|---|---|---|
| 12 | | 12-13-1 | | 12-26-1 | |
| 13 | | 13-13-1 | | 13-26-1 | |
| 14 | | 14-13-1 | | 14-26-1 | |
| 15 | | 15-13-1 | | 15-26-1 | |
| 16 | | 16-13-1 | | 16-26-1 | |
| 17 | | 17-13-1 | | 17-26-1 | |
| 18 | | 18-13-1 | | 18-26-1 | |
| 19 | | 19-13-1 | | 19-26-1 | |
| 20 | | 20-13-1 | | 20-26-1 | |
| 21 | | 21-13-1 | | 21-26-1 | |
| 22 | | 22-13-1 | | 22-26-1 | |
| 23 | | 23-13-1 | | 23-26-1 | |

## Biological test and evaluation

The present invention will be further described and explained below in conjunction with test examples, but these examples are not meant to limit the scope of the present invention.

**Test example 1:** Experiment on the compounds of the present invention competing with IL23 for binding to IL23R

1.1 Experimental objective: To determine the efficiency of the compounds of the present invention in competing with IL23 for binding to IL23R
1.2 Experimental instruments and reagents:
1.2.1 Instruments

| Name of instrument | Manufacturer | Specification and model |
|---|---|---|
| EnVision | PerkinElmer | ENVISION 2015 |

1.2.2 Reagents

| Reagents and materials | Manufacturer | Item No. |
|---|---|---|
| IL23/IL23R BINDING ASSAY KITS | PerkinElmer | 64BDPIL23PEH |
| White 384-shallow well Microplate | PerkinElmer | 6008280 |

1.3 Experimental method:
The compounds of the present invention were screened in IL23/IL23R BINDING ASSAY KITS (PerkinElmer Cat#64BDPIL23PEH). The screening of the compounds of the present invention was carried out by using a multi-well plate (suitable for HTRF determination). Generally, 2 μl of several compounds of the present invention and STANDARD SOLUTIONS in KIT were first respectively added to the wells of the multi-well plate. 4 μl of Tag1-IL23, 4 μl of Tag2-IL23R, and 10 μL of pre-mixed Anti-Tag1 Eu Cryptate Antibody and Anti-Tag2 d2 reagent were necessarily added to all the wells. The multi-well plate was protected from light by an opaque cover membrane, and the opaque cover membrane was then removed after 2 hours of incubation at room temperature. The signal value was read by an HTRF-compatible detection instrument to detect $IC_{50}$ value.
1.4 Experimental data processing method
The inhibition rate was calculated as follows:

% inhibition = (Signal cmpd - Signal Ave_PC) / (Signal Ave_VC - Signal Ave_PC) × 100. H = Ave (DMSO); and L = Ave (Guselkumab)

The $IC_{50}$ value of the compound was calculated as follows:
By using log(inhibitor) vs. response -- Variable slope (four parameters) in Graphpad, the data of HTRF experiment results were subjected to non-linear regression fitting to fit a curve and obtain the $IC_{50}$ value.

$$Y = Bottom + (Top - Bottom) / (1 + 10^{((LogIC_{50}-X)*HillSlope)})$$

X: log of inhibitor concentration; and Y: % Inhibition

1.5 Experimental results:

| Example | Competing with IL23 for binding to IL23R $IC_{50}$ (nM) |
|---|---|
| 1 | 1.20 |
| 2 | 0.23 |
| 3 | 1.23 |
| 6 | 1.25 |
| 12 | 1.14 |
| 1-14 | 1.14 |
| 3-14 | 1.09 |
| 4-14 | 1.11 |
| 1-16-1 | 2.73 |
| 3-16-1 | 1.24 |
| 4-16-1 | 1.23 |
| 1-19 | 1.35 |
| 3-19 | 1.13 |
| 4-19 | 1.18 |

1.6 Experimental conclusion: The compounds of the preferred examples of the present invention showed excellent biological activity in the inhibition test of competing with IL23 for binding to IL23R.

**Test example 2:** Experiment on the compounds of the present invention competing with IL12 for binding to IL12Rβ1

2.1 Experimental objective: To determine the efficiency of the compounds of the present invention in competing with IL12 for binding to IL12Rβ1
2.2 Experimental instruments and reagents
2.2.1 Instruments

| Name of instrument | Manufacturer | Specification and model |
|---|---|---|
| EnVision | PerkinElmer | ENVISION 2015 |

2.2.2 Reagents

| Reagents and materials | Manufacturer | Item No. |
|---|---|---|
| IL 12/IL 12RB 1 BINDING ASSAY KITS | PerkinElmer | 64BDIL12PEG |
| White 384-shallow well Microplate | PerkinElmer | 6008280 |

2.3 Experimental method:
The compounds of the present invention were screened in IL12/IL12RB1 BINDING ASSAY KITS (PerkinElmer Cat# 64BDIL12PEG). The screening of the compounds of the present invention was carried out by using a multi-well plate (suitable for HTRF determination). Generally, 2 μl of several compounds of the present invention and STANDARD SOLUTIONS in KIT were first respectively added to the wells of the multi-well plate. 4 μl of Tag1-IL12, 4 μl of Tag2-IL12Rb1, and 10 μL of pre-mixed Anti-Tag1 Eu Cryptate Antibody and Anti-Tag2 XL665 reagent were necessarily added to all the wells. The multi-well plate was protected from light by an opaque cover membrane, and the opaque cover membrane was then removed after 2 hours of incubation at room temperature. The signal value was read by an HTRF-compatible detection instrument to detect $IC_{50}$ value.
2.4 Experimental data processing method

The inhibition rate was calculated as follows:

% inhibition = (Signal cmpd - Signal Ave_PC) / (Signal Ave_VC - Signal Ave_PC) × 100. H = Ave (DMSO); and L = Ave (Guselkumab)

The $IC_{50}$ value of the compound was calculated as follows:
By using log(inhibitor) vs. response -- Variable slope (four parameters) in Graphpad, the data of HTRF experiment results were subjected to non-linear regression fitting to fit a curve and obtain the $IC_{50}$ value.

$$Y = Bottom + (Top - Bottom) / (1 + 10^\wedge((LogIC_{50}-X)*HillSlope))$$

X: log of inhibitor concentration; and Y: % Inhibition

2.5 Experimental conclusion:

By the above scheme, it was concluded that the compounds shown in the present invention showed a biological activity of more than 1000 nM ($IC_{50}$) in the inhibition test of competing with IL12 for binding to IL12Rβ1.

**Test example 3:** Experiment on the compounds of the present invention blocking the activation of STAT3 phosphorylation by hIL23 in DB cells

3.1 Experimental objective: To determine the inhibitory activity of the compounds of the present invention on the activation of STAT3 phosphorylation by hIL23 in DB cells
3.2 Experimental instruments and reagents
3.2.1 Instruments

| Name of instrument | Manufacturer | Specification and model |
|---|---|---|
| EnVision | PerkinElmer | ENVISION 2015 |

3.2.2 Reagents

| Reagents and materials | Manufacturer | Item No. |
|---|---|---|
| DB cell | ATCC | CRL-2289 |
| Fetal bovine serum (FBS) | GIBCO | 10099-141 |
| RPMI Medium 1640 | GIBCO | A10491-01 |
| Guselkumab | Wuhan Chemstan Biotechnology Co., Ltd. | CSD00323 |

(continued)

| Reagents and materials | Manufacturer | Item No. |
|---|---|---|
| Recombinant Human IL-23 Protein | R&D | 1290-IL-010/CF |
| PathScan Phospho-Stat3 (Tyr705) Sandwich ELISA Kit | CST | 7300C |

3.3 Experimental method: The compounds produced above were added to DB (ATCC Cat#CRL-2289) cells to inhibit the signal transduction of rhIL-23 cytokine. The screening of compounds was carried out by a multi-well plate (suitable for ELISA assay). Generally, in the presence of some of the above compounds of the present invention, $6.25 \times 10^6$ cells/ml of DB cells were stimulated by rhIL-23 (0.5 nM, R&D SYSTEMS) in RPMI medium (Invitrogen) containing 10% of FBS, and after half an hour, lysis was carried out on ice using 50 μL of 2×lysis buffer (CST). The content of p-STAT3 in the lysate was determined by PathScan Phospho-Stat3 (Tyr705) Sandwich ELISA Kit (CST Cat#7300C), so as to calculate the activity of the compounds of the present invention in blocking rhIL23 signal transduction in DB cells.

3.4 Experimental data processing method

The inhibition rate was calculated as follows:

$$\text{Inhibition \%} = (\text{Ave_H-Sample}) / (\text{Ave_H-Ave_L}) \times 100$$

H = Ave (DMSO); and L = Ave (Guselkumab)

The $IC_{50}$ value of the compound was calculated as follows:

By using log(inhibitor) vs. response -- Variable slope (four parameters) in Graphpad, the data of ELISA experiment results were subjected to non-linear regression fitting to fit a curve and obtain the $IC_{50}$ value.

$$Y = \text{Bottom} + (\text{Top} - \text{Bottom}) / (1 + 10\text{^}((\text{LogIC}_{50}\text{-X})*\text{HillSlope}))$$

X: log of inhibitor concentration; and Y: % Inhibition

3.5 Experimental results:

| Example | pStat3 $IC_{50}$ (pM) |
|---|---|
| 1 | 25.0 |
| 2 | 10.0 |
| 3 | 1.4 |
| 12 | 6.0 |
| 1-14 | 1.4 |
| 3-14 | 1.4 |
| 4-14 | 1.6 |
| 1-16-1 | 4.8 |
| 3-16-1 | 8.9 |
| 4-16-1 | 3.6 |
| 1-19 | 0.5 |
| 3-19 | 0.9 |
| 4-19 | 0.1 |

3.6 Experimental conclusion: The compounds of the preferred examples of the present invention showed excellent inhibitory activity in the inhibition test of the activation of STAT3 phosphorylation by hIL23.

**Test example 4:** Pharmacokinetic determination in rats

4.1 Research objective: SD rats were used as test animals to study the pharmacokinetic behavior of the compounds of the examples in the rats (plasma) after oral or IV administration.

4.2. Test scheme

4.2.1 Test drugs:

The compounds of the examples of the present invention, made in house.

4.2.2 Test animals:

3 SD rats/group, male. From Shanghai Jiesijie Laboratory Animal Co., Ltd., animal production license number (SCXK (Shanghai) 2013-0006 NO.311620400001794).

4.2.3 Drug preparation:

Preparation of oral drug: 20% Labrasol
20% Labrasol: 40 ml of Labrasol were weighed into a 500 ml glass flask, PBS was added to reach 200 ml, and after magnetic stirring, the Labrasol was completely dissolved to prepare 20% Labrasol.
The compounds of the examples were weighed and dissolved in this solution, uniformly shaken, and ultrasonicated for 5 min to obtain colorless clear solutions with a concentration of 0.5 mg/mL.

Preparation of intravenous drug: PBS

The compounds of the examples were weighed and dissolved in this solution, vortexed, ultrasonicated for 5 min, and filtered through a 0.22 μm filter membrane to obtain colorless clear solutions with a concentration of 0.2 mg/mL.

4.2.4 Administration:

3 SD rats/group, male were fasted overnight and then respectively administered PO at a dose of 5 mg/kg and a volume of 10 mL/kg.
3 SD rats/group, male were fasted overnight and then respectively administered IV at a dose of 1 mg/kg and a volume of 5 mL/kg.

4.2.5 Sample collection:

Before administration and at 0.083 h, 0.25 h, 0.5 h, 1.0 h, 2.0 h, 4.0 h, 6.0 h, 8.0 h, and 24.0 h after administration (IV), 0.2 mL of blood was collected from the jugular vein, placed in an EDTA-2K test tube, and centrifuged at 6000 rpm for 6 min at 4°C to separate plasma, which was stored at -20°C; and the rats were fed 4 h after administration.

4.3 Sample treatment:

1) Acetonitrile (200 μL) was added to the plasma sample (50 μL) for precipitation, and the mixture was mixed and centrifuged at 4500 rpm for 15 min.
2) The treated supernatant solution was taken and subjected to LC/MS/MS to analyze the concentrations of the test compounds. LC/MS/MS analysis instrument: AB Sciex Triple Quad 6500+.

4.4 Liquid phase analysis:

• Liquid phase conditions: Shimadzu LC-30AD pump

• Chromatographic column: HALO 90A PFP 2.7 μm 2.1 × 30 mm, mobile phase: liquid A: 5% acetonitrile aqueous solution (0.1% formic acid), and liquid B: an aqueous solution of 95% acetonitrile (0.1% formic acid)
• Flow rate: 0.6 mL/min
• Elution time: 0-2.0 min, the eluent was as follows:

| Time/min | Liquid A | Liquid B |
|---|---|---|
| 0.20 | 95% | 5% |
| 1.50 | 5% | 95% |
| 1.70 | 5% | 95% |
| 1.71 | 95% | 5% |
| 2.00 | 95% | 5% |

4.5. Experimental results and analysis

The main pharmacokinetic parameters were calculated using WinNonlin 8.1.

| Example | Pharmacokinetic experiments (IV administration, 1 mg/kg) | | | | | |
|---|---|---|---|---|---|---|
| | Plasma concentrat ion | Area under the curve | | Half-life | Mean residence time | Clearance rate |
| | $C_0$ (ng/mL) | $AUC_{0-t}$ (ng/mL × h) | $AUC_{0-\infty}$ (ng/mL × h) | $t_{1/2}$ (h) | MRT (h) | CL (mL/min/k g) |
| PN-235 | 4292 | 2617 | 2671 | 0.7 | 0.9 | 6.2 |
| 1 | 10268 | 45367 | 46708 | 4.9 | 6.1 | 0.35 |
| 1-16-1 | 12648 | 46149 | 47609 | 5.0 | 6.2 | 0.35 |
| 3-16-1 | 15062 | 41550 | 42565 | 4.7 | 5.4 | 0.39 |
| 4-16-1 | 17405 | 51530 | 53332 | 5.1 | 6.3 | 0.31 |

4.6 Experimental conclusion:

The data showed that in the experiment of pharmacokinetic evaluation in rats, the compounds of the preferred examples of the present invention had stronger plasma concentration and higher exposure as compared with PN-235.

**Test example 5:** Pharmacokinetic determination in beagle dogs

5.1 Research objective: Beagle dogs were used as test animals to study the pharmacokinetic behavior of the compounds of the examples in the rats (plasma) after IV administration.

5.2. Test scheme

5.2.1 Test drugs:

The compounds of the examples of the present invention, made in house.

5.2.2 Test animals:

3 beagle dogs/group, male, from Yizheng Anlimao Biotechnology Co., Ltd.

5.2.3 Drug preparation:

Preparation of intravenous drug: PBS

The compounds of the examples were weighed and dissolved in this solution, vortexed, ultrasonicated for 5 min, and filtered through a 0.22 μm filter membrane to obtain colorless clear solutions with a concentration of 0.1 mg/mL.

5.2.4 Administration:

3 beagle dogs/group, male were fasted overnight and then respectively administered IV at a dose of 0.2 mg/kg and a volume of 2 mL/kg.

5.2.5 Sample collection:

Before administration and at 0.083 h, 0.25 h, 0.5 h, 1 h, 2 h, 4 h, 6 h, 8 h, 24 h, 48 h, 72 h, 96 h, 120 h, 144 h, and 168 h after administration (IV), 0.5 mL of blood was collected from the vein of an anterior limb, placed in an EDTA-2K test tube, and centrifuged at 6000 rpm for 6 min at 4°C to separate plasma, which was stored at -20°C; and the beagle dogs were fed 4 h after administration.

5.3 Sample treatment:

1) Acetonitrile (200 μL) was added to the plasma sample (50 μL) for precipitation, and the mixture was mixed and centrifuged at 4500 rpm for 15 min.
2) The treated supernatant solution was taken and subjected to LC/MS/MS to analyze the concentrations of the test compounds. LC/MS/MS analysis instrument: AB Sciex Triple Quad 6500+.

5.4 Liquid phase analysis:

• Liquid phase conditions: ExionLC
• Chromatographic column: ACQUITYTM Premier Peptide CSH C18 130A 1.7 μm 2.1 * 100 mm Column, mobile phase: liquid A: 5% acetonitrile aqueous solution (0.1% formic acid), and liquid B: an aqueous solution of 95% acetonitrile (0.1% formic acid)
• Flow rate: 0.6 mL/min
• Elution time: 0-2.0 min, the eluent was as follows:

| Time (min) | Flow rate (mL/min) | A (%) | B (%) |
|---|---|---|---|
| 0.00 | 0.6 | 95.0 | 5.0 |
| 0.20 | 0.6 | 95.0 | 5.0 |
| 2.00 | 0.6 | 5.0 | 95.0 |
| 2.50 | 0.6 | 5.0 | 95.0 |
| 2.60 | 0.6 | 80.0 | 20.0 |
| 2.80 | 0.6 | 5.0 | 95.0 |
| 2.90 | 0.6 | 95.0 | 5.0 |
| 3.50 | 0.6 | 95.0 | 5.0 |

5.5. Experimental results and analysis

The main pharmacokinetic parameters were calculated using WinNonlin 8.1.

| Example | Pharmacokinetic experiments (IV administration, 0.2 mg/kg) | | | | | |
|---|---|---|---|---|---|---|
| | Plasma concentrat ion | Area under the curve | | Half-life | Mean residence time | Clearance rate |
| | $C_0$ (ng/mL) | $AUC_{0-t}$ (ng/mL × h) | $AUC_{0-\infty}$ (ng/mL × h) | $t_{1/2}$ (h) | MRT (h) | CL (mL/min/kg) |
| PN-235 | 1251 | 2016 | 2031 | 4.1 | 3.1 | 1.68 |
| 1-16-1 | 2749 | 68798 | 76403 | 53.8 | 66.9 | 0.04 |
| 4-16-1 | 3190 | 96145 | 112150 | 91.7 | 80.6 | 0.03 |

5.6 Experimental conclusion:

The data showed that in the experiment of pharmacokinetic evaluation in dogs, the compounds of the preferred examples of the present invention had stronger plasma concentration and higher exposure as compared with PN-235.

**Test example 6:** Determination of the solubility of the compounds of the present invention

6.1 Research objective: To study the solubility of the compounds in PBS.
6.2 Experimental instruments and reagents:

| Instruments and reagents | Model | Source |
|---|---|---|
| pH 7.4 isotonic solution (PBS) | / | Laboratory preparation |
| Electronic balance | 2.1g/XPR2 | METTLER TOLEDO |
| Pipette | 2-20 μL | Eppendorf |
| Vortex mixer | LAB DANCER | IKA |
| Ultrasonic cleaner | SL2200LHC | Shanghai Kudos Ultrasonic Instrument Co., Ltd. |

6.3 Experimental method:

About 2 mg of the compound was weighed, an appropriate amount of a PBS solution was added, and the mixture was dissolved clear by ultrasonication for 10 seconds. No precipitation was found after standing at room temperature overnight.

6.4 Experimental results and conclusion:

At room temperature, the compounds of the preferred examples of the present invention showed excellent solubility advantages, for example, the solubility of Example 1-16-1 was 39.58-59.38 mg/mL; and the solubility of Example 4-16-1 was greater than 124.75 mg/mL.

**Test example 7:** Stability of the compounds of the present invention in simulated gastrointestinal fluid

7.1 Research objective: To study the stability of the compounds of the examples in simulated gastrointestinal fluid.

7.2 Experimental instruments and reagents:

7.2.1 Reagents

Acetonitrile (F22M4L201, Fisher), trifluoroacetic acid (17110655, TEDIA), sodium hydroxide (20170616, Sinopharm), pancreatic enzyme (20220811, Hushi), pepsin (20220909, Hushi), potassium dihydrogen phosphate (20210528, Vokai), sodium chloride (20200105, Hushi), hydrochloric acid (20181203, Sinopharm), anhydrous sodium dihydrogen phosphate (20200602, Hushi), 3F Powder (FFF-0723-B, Biorelevant), and glacial acetic acid (20181112, Sinopharm).

7.2.2 Instruments

High-performance liquid chromatograph (1260, Agilent), electronic balance (MCE-C, Sartorius), pH meter (Five Easy Plus, METTLER TOLEDO), pipettes (500-5000 μL, 100-1000 μL, and 10-100 μL, Eppendorf), and ultrasonic cleaner (SK5200LHC, Shanghai Kudos Ultrasonic Instrument Co., Ltd.).

7.3 Experimental method:

7.3.1 Preparation of mobile phase and simulated gastrointestinal fluid

0.05% trifluoroacetic acid aqueous solution: obtained by measuring 2 L of purified water and adding 1 mL of trifluoroacetic acid, followed by uniform mixing and then ultrasonication.

0.05% trifluoroacetic acid acetonitrile solution: obtained by measuring 2 L of acetonitrile and adding 1 mL of trifluoroacetic acid, followed by uniform mixing and then ultrasonication.

SIF (E): 69.48 mg of potassium dihydrogen phosphate and 108.49 mg of pancreatic enzyme were weighed, 10 mL of purified water was added, and the mixture was uniformly mixed and then adjusted to pH 6.76 with a 1N sodium hydroxide aqueous solution (about 210 μL).

SGF (E): 21.64 mg of sodium chloride and 33.70 mg of pepsin were weighed, 10 mL of purified water was added, followed by 20 μL of hydrochloric acid, and the mixture was adjusted to pH 1.98 with a 1N sodium hydroxide aqueous solution (about 120 μL).

FaSSIF: 0.42 g of sodium hydroxide, 3.43 g of anhydrous sodium dihydrogen phosphate, and 6.19 g of sodium chloride were weighed, 1 L of purified water was added, and the mixture was dissolved by ultrasonication and then adjusted to pH 6.50 with a 1N sodium hydroxide or 1N hydrochloric acid aqueous solution. 2.24 g of 3F Powder was then added, and the mixture was stirred until dissolved.

FeSSIF: 4.04 g of sodium hydroxide, 8.65 g of glacial acetic acid, and 11.87 g of sodium chloride were weighed, 1 L of purified water was added, and the mixture was dissolved by ultrasonication and then adjusted to pH 5.0 with a 1N sodium hydroxide or 1N hydrochloric acid aqueous solution. 11.2 g of 3F Powder was added, and the mixture was stirred until dissolved.

7.3.2 Determination of stability

About 2 mg of the compound was weighed, an appropriate amount of purified water was added, and the mixture was dissolved clear by ultrasonication and used as a Stock solution of the compound. 50 μL of the Stock solution was taken, 950 μL of SGF, FaSSIF, FeSSIF, SGF (E), and SIF (E) were respectively added, and after uniform mixing, the stability of the sample was investigated at different time points.

7.4 Liquid phase analysis:

The liquid chromatography conditions were as follows:

| | |
|---|---|
| Mobile phase | A: 0.05% trifluoroacetic acid aqueous solution; B: 0.05% trifluoroacetic acid acetonitrile solution |
| Chromatographic column | Waters XBridge C18 (4.6 * 150 mm, 3.5 μm) |
| Column temperature | 40°C |
| Sample tray temperature | 37°C |
| Flow rate | 1.0 mL/min |
| Wavelength | 210 nm |
| Injection volume | 20 μL |

(continued)

| | Time (min) | A% | B% |
|---|---|---|---|
| Gradient method | 0.00 | 90.0 | 10.0 |
| | 9.00 | 10.0 | 90.0 |
| | 11.00 | 10.0 | 90.0 |
| | 11.50 | 90.0 | 10.0 |
| | 15.00 | 90.0 | 10.0 |

7.5. Test results and analysis

The compounds of the preferred examples of the present invention had excellent stability in the simulated gastrointestinal fluid.

**Test example 8:** Investigation of in vitro metabolic stability of the compounds of the present invention in liver microsomes

8.1 Research objective: To study the phase I and partial phase II metabolic stability of the compounds of the examples in liver microsomes of mice, rats, dogs and humans.

8.2 Experimental reagents

8.2.1 Reagents

The compounds of the present invention (made in house), liver microsomes (H0610/M1000/D1000, Xenotech), phosphate buffer (Lot#SLBS7904 and Lot#SLBR3106V, pH 7.4, Gibco), NADPH (reduced nicotinamide adenine dinucleotide phosphate, Shanghai Bide Pharmatech Ltd.), UDPGA (Sigma), Alamethicin (Lot#GR3226732-1, J&K Scientific), methanol (Merck), acetonitrile (Merck), DMSO (Sigma), and 7-Hydroxycoumarin (J&K Scientific).

8.2.2 Drug preparation

The test compound was prepared into a 10 mM stock solution with DMSO and stored in a refrigerator at -20°C, for later use.

8.3 Experimental steps

1) Preparation of buffer solution

4.01 mL of 1M $K_2HPO_4 \cdot PO_2O$ (AR grade) and 0.99 mL of 1M $KH_2PO_4$ (AR grade) were taken, dissolved with ultrapure water, and made up to 50 mL to prepare a phosphate buffer with a final concentration of 100 mM.

2) Preparation of working solution of compound

Preparation of working solution of compound: 1 μL of the compound stock solution was added to 999 μL of phosphate buffer, such that the final concentration was 10 μM. According to the nature of the compound, the preparation proportion could be appropriately adjusted to adjust the final concentration.

3) Preparation of working solution of liver microsomes

156.3 μL of 20 mg/mL microsomes was diluted with 100 mM phosphate buffer to 5 mL and uniformly mixed, such that the final concentration was 0.625 mg/mL.

4) Preparation of NADPH and UDPGA

33.3 mg of NADPH and 25.8 mg of UDPGA were weighed, and 2 mL of 100 mM phosphate buffer was added, such that both had a final concentration of 20 mM.

5) Preparation of pore-forming agent (Alamethicin)

1 mg of Alamethicin was weighed, and 200 μL of methanol was added to prepare a 5 mg/mL solution. 10 μL was further taken from this solution, and 990 μL of phosphate buffer (pH 7.4) was added, such that the final concentration was 50 μg/mL.

6) Preparation of reaction stop solution

The internal standard was diluted with acetonitrile as a stop solution and stored in a 2-8°C refrigerator.

7) Incubation process

400 μL of the preapred liver microsomes, 25 μL of the working solution of the compound (10 μM), and 25 μL of Alamethicin (50 μg/mL) were successively added to a 96-well plate and pre-incubated at 37°C for 10 min. 50 μL of formulated NADPH/UDPGA was then added to start the reaction and the mixture was incubated at 37°C. The total volume of the reaction system was 500 μL. The final content of each component was as follows: the compounds of the examples (0.5 μM), liver microsomes (0.5 mg/mL), NADPH (1 mM), UDPGA (1 mM), and Alamethicin (2.5 μg/mL).

50 μL was taken at the time points of 0, 5, 15, 30, 60, and 120 min, respectively, and 200 μL of a cold stop solution containing an internal standard was added to stop the reaction of the sample. The reaction solution was centrifuged at 3500 rpm for 10 min, and the supernatant was taken and analyzed by LC-MS/MS.

8.4 Biological analysis

1) Chromatographic conditions:

Instrument: Shimadzu LC-20 AD
Chromatographic column: Phenomenex Gemiu® C18 (50 * 4.6 mm, 5 μm particle size)
Mobile phase: A: acetonitrile, B: 0.1% formic acid solution
Elution gradient: 0.2-1.6 min 5% A to 95% A, 3.0-3.1 min 95% A to 5% A
Flow rate: 1.0 ml/min
Run time: 4.0 min
Injection volume: 5 μL.

2) Mass spectrometry conditions:

Instrument: API4000 liquid chromatography mass spectrometer, AB Sciex
Ion source: electrospray ionization source (ESI)
Drying gas: N2, temperature 500°C
Electrospray voltage: 5000 V
Detection mode: positive ion detection
Scan mode: multi reaction monitoring (MRM) mode
Scan time: 0.8401s

8.5 Data processing

The raw data were subjected to calculation according to the following formula:

Residual ratio % = peak area ratio of compound to internal standard at any time point / peak area ratio of compound to internal standard at 0 min × 100%.

$T_{1/2} = 0.693/K_e$, in which $K_e$ represented elimination rate constant.

The intrinsic clearance rate of liver microsome in vitro ($CL_{int}$) and liver intrinsic clearance rate ($CL_{int,liver}$) were calculated by $K_e$.

$CL_{int}$ = 0.693 / $T_{1/2}$ / microsome protein content (microsome concentration mg/mL during incubation)

$CL_{int,liver} = CL_{int}$ nt microsome protein amount in liver (mg/g) × × liver to body weight ratio

According to a well-stirred model, the in vivo liver clearance rate (CL) was estimated.

$$CL = (CL_{int,liver}\ nt,liver\ ratio) / (CL_{int,liver}\ nt,liver\ ratio),$$

in which fu represented the free fraction in blood, and the default value was 1.

The parameters in the formula are as shown in the following table.

| Species | Body weight (kg) | Hepatic blood flow (mL/min/kg) | Microsome protein amount (mg/g liver weight) | Liver to body weight ratio (g/kg body weight) |
|---|---|---|---|---|
| Mice | 0.025 | 90 | 45 | 60 |
| Rat | 0.25 | 72 | 61 | 40 |
| Monkey | 5 | 44 | 45 | 32 |
| Dog | 12 | 55 | 55 | 32 |
| Human | 70 | 20 | 40 | 24 |

8.6 Experimental conclusion:

The results showed that the compounds of the preferred examples of the present invention had stable metabolic effects in liver microsomes of various species.

**Test example 9:** Metabolic stability of the compounds of the present invention in liver/kidney tissue homogenate.

9.1 Experimental objective: To test the metabolic stability of the compounds of the examples in liver/kidney tissues of rats, dogs and monkeys.

9.2 Experimental reagents

Rat liver tissue homogenate (WuXi AppTec), monkey liver tissue homogenate (WuXi AppTec), dog liver tissue homogenate (WuXi AppTec), human liver tissue homogenate (BioreclamationIVT, S06585), rat kidney tissue homogenate (WuXi AppTec), monkey kidney tissue homogenate (WuXi AppTec), dog kidney tissue homogenate (WuXi AppTec), and human kidney tissue homogenate (BioreclamationIVT, S06585).

9.3 Experimental steps

1) Solution preparation

Working solution of test compound: 5 μL of a compound stock solution (10 mM dimethyl sulfoxide solution) was diluted with 995 μL of dimethyl sulfoxide (concentration of the working solution: 50 μM, 100% DMSO);

working solution of Deslorelin as a control: 5 μL of a Deslorelin stock solution (10 mM in dimethyl sulfoxide (DMSO)) was diluted with 495 μL of dimethyl sulfoxide (concentration of the working solution: 100 μM, 100% DMSO); and working solution of Semagulide as a control: 50 μL of a stock solution of Semagulide (1 mM dimethyl sulfoxide solution) was diluted with 450 μL of dimethyl sulfoxide (concentration of the working solution: 100 μM, 100% DMSO).

2) Incubation process

Before the experiment, the combined frozen liver/kidney homogenate was thawed in a 37°C water bath.

98 μL/well of a blank liver/kidney homogenate aliquot was added to all 96-well reaction plates. (Blank, T0, T10, T30, T60, and T120)

2 μL/well of an aliquot of the working solution (50 or 100 μM) was added to all the reaction plates except the blank. (T0, T10, T30, T60, and T120)

All the reaction plates containing the compound and liver/kidney homogenate mixture were incubated in a 37°C water bath.

The reaction plate was incubated at 37°C and a timer was started.
At the end of incubation, 100 μL of 4% $H_3PO_4$ was mixed with 100 μL of the sample, and 800 μL of the stop solution (methanol with tolbutamide and labetalol as internal standards) was then added for protein precipitation. Full mixing was carried out.
Each plate was sealed and shaken for 20 min.
After shaking, each plate was centrifuged at 4000 rpm and 4°C for 20 min.
After centrifugation, 150 μL of the supernatant was transferred from each reaction plate to the corresponding biological acylation plate thereof.
Before LC-MS/MS analysis, each bioanalysis plate was sealed and shaken for 10 min.

9.4 Data processing

The residual percentage of the test compound in the liver/kidney homogenate after incubation was calculated using the following equation:
residual % = 100 x (PAR at an incubation time point / PAR at time T0), in which PAR was the peak area ratio of analyte to internal standard (is), and the incubation time points were T0 (0 min) and Tn (n = 0, 10, 30, 60, and 120 min).

9.5 Experimental conclusion

The results showed that the compounds of the preferred examples of the present invention had excellent stability in the liver tissue and kidney tissue homogenates.

**Test example 10:** Investigation on the stability of the compounds of the present invention in plasma

10.1 Research objective: To study the stability of the compounds of the examples in the plasmas of mice, rats, dogs and humans.

10.2 Experimental instruments

Centrifuge (Eppendorf 5804R/5424R), vortex mixer (IKA VORTEX GENIUS 3), pipettors (Eppendorf 10-100 μL, Eppendorf 100-1000 μL, and RAININ 0.5-10 μL), and water bath (Shanghai Yiheng Scientific Instrument Co., Ltd.).

10.3 Experimental steps

1) Solution preparation
Preparation of plasma: Animal or human whole blood was collected, then put into a test tube containing an anticoagulant, and centrifuged at 3500 rpm for 10 min, and the upper pale yellow plasma was collected;

working solution of 10 μM test compound: a stock solution was prepared with DMSO, and the working solution was prepared with 100 mM phosphate buffer;
working solution of 10 μM positive control:

procaine: 2.36 mg of procaine was weighed and diluted into a 10 mM stock solution with 1 mL of DMSO. 10 μL of the 10 mM stock solution was pipetted into 1 mL of 100 mM phosphate buffer, such that the final concentration was 100 μM; and
enalapril: 4.93 mg of enalapril was weighed and diluted into a 10 mM stock solution with 1 mL of DMSO; and 10 μL of the 10 mM stock solution was pipetted into 1 mL of 100 mM phosphate buffer, such that the final concentration was 100 μM.

2) Incubation process

In a 96-well plate, 285 μL of plasma and 15 μL of 10 μM compound (test compound, and positive control) were successively added and incubated at 37°C.
40 μL was taken at 0, 15, 30, 60, 90, and 120 min (sampling points could be fine-tuned), respectively, and 160 μL of an acetonitrile stop solution containing an internal standard was added.
After centrifugation (3500 rpm, 10 min), 50 μL of the supernatant was taken, diluted by adding 50 μL of $DDH_2O$,

and then injected for LC-MS/MS.

10.4 Biological analysis

1) Chromatographic conditions

Instrument: Shimadzu LC-20 AD
Chromatographic column: Phenomenex Gemiu ® $C_{18}$ (50 * 4.6 mm, 5 μm particle size)
Mobile phase: A: acetonitrile, B: 0.1% formic acid solution
Elution gradient: 0-8 min: 5% A → 95% A, 2.0-2.1 min: 90% A → 5% A
Flow rate: 0.8 mL/min
Run time: 5.0 min
Injection volume: 5 μL

2) Mass spectrometry conditions:

Instrument: API4000 liquid chromatography mass spectrometer, AB, US
Ion source: electrospray ionization source (ESI)
Drying gas: N2, temperature 500°C
Electrospray voltage: 5500V
Detection mode: positive ion detection
Scan mode: multi reaction monitoring (MRM) mode
Scan time: 0.1 s

10.5 Experimental results and data processing

All calculations were carried out using Microsoft Excel. The peak area ratio was determined by an extracted ion chromatogram.

The residual percentage of the compound at each time point was calculated by the following equation:

Residual percentage at t hours (%) = peak area ratio at t hours / peak area ratio at 0 hour × 100%,

in which the peak area ratio at t hr was the peak area ratio of the control compound and the test compound at t min; and the slope value k was determined by linear regression on the natural logarithm of the curve of the residual percentage of the parent drug versus the incubation time.

In vitro half-life (in vitro $t_{1/2}$) was determined by the slope value:

In vitro $t_{1/2}$ = -(0.693/k)

| Example | Residual ratio % at 4 h | | | | $t_{1/2}$ (h) | | | |
|---|---|---|---|---|---|---|---|---|
| | Human | Rat | Mice | Dog | Human | Rat | Mice | Dog |
| 1 | 94.29 | 98.65 | 93.76 | 99.66 | 64.63 | 75.72 | 31.96 | 148.36 |
| 1-16-1 | 94.81 | 103.76 | 103.59 | 90.47 | 41.28 | 603.43 | 2005.26 | 75.50 |
| 4-16-1 | 96.56 | 98.72 | 113.80 | 101.32 | 94.50 | 74.87 | ∞ | ∞ |

10.6 Experimental conclusion:

The compounds of the preferred examples of the present invention had excellent stability in all the plasmas of mice, rats, dogs and humans.

**Test example 11:** Study on the in vivo pharmacodynamics of the compounds of the present invention in IL23-induced rat otitis model

11.1 Experimental objective: To evaluate the in vivo efficacy of the compounds in an IL23-induced rat otitis model.

11.2. Experimental instruments and reagents

11.2.1 Experimental instruments

Refrigerator (BCD-268TN, Haier), biosafety cabinet (BSC-1300II A2, Shanghai Boxun Industry & Commerce Co., Ltd. Medical Equipment Factory), ultraclean bench (CJ-2F, Suzhou Fengshi Laboratory Equipment Co., Ltd.), motorized pipette controller (Easypet 3, Eppendorf), thermostat water bath (HWS-12, Shanghai Yiheng Scientific Instrument Co., Ltd.), ultrasonic cleaner (115F0032, Shanghai Kudos Ultrasonic Instrument Co., Ltd.), water purifier (Pacific TII, Thermo), magnetic stirrer (08-2G, Chijiu), electronic balance (CPA2202S, Sartorius), electronic balance (BSA2202S-CW, Sartorius), ultrasonic cell crusher (JY92-IIN, Ningbo Scientz), and micrometer caliper (MDC-25PX, Mitutoyo).

11.2.2 Experimental reagents

IL-23 Protein, Rat, Recombinant (CT045-R08H, Sino Biological), PBS (10010-049, gibco), penetration enhancer, isoflurane (R510-22-10, RWD), and 4% tissue cell fixative (AR-0211, Dingguo).

11.3 Experimental steps

11.3.1 Animal procurement

SD rats, 6-8 weeks old, male, purchased from Jiangsu GemPharmatech Co., Ltd.

11.3.2 Establishment of otitis model

a. After animals were acclimatized to the environment for one week, tail numbers were marked with a marker, and after weighing, the animals were randomly divided into groups according to the body weights thereof;
b. the day before modeling (i.e., D-1), the animals were administered by gavage according to the experimental design and grouping;
c. rrIL-23 was diluted into a concentration of 50 μg/ml with PBS, split into 1 ml centrifuge tubes, and stored at -80°C for later use;
d. on the first day of modeling (i.e., D0), the animals were administered by gavage according to the experimental design and grouping; after half an hour, the rats were anesthetized with isoflurane; and after the initial thickness of the right ear was measured, IL-23 (1 μg, 20 μL) or 20 μL of PBS was intracutaneously injected into the right ear;
e. on the second day of modeling (i.e., D1), the animals were administered by gavage according to the experimental design and grouping; after half an hour, the rats were anesthetized with isoflurane; and after the initial thickness of the right ear was measured, IL-23 (1 μg, 20 μL) or 20 μL of PBS was intracutaneously injected into the right ear;
f. on the third day of modeling (i.e., D2), the animals were administered by gavage according to the experimental design and grouping; after half an hour, the rats were anesthetized with isoflurane; and after the initial thickness of the right ear was measured, IL-23 (1 μg, 20 μL) or 20 μL of PBS was intracutaneously injected into the right ear;
g. on the fourth day of modeling (i.e., D3), the animals were administered by gavage according to the experimental design and grouping; after half an hour, the rats were anesthetized with isoflurane; and after the initial thickness of the right ear was measured, IL-23 (1 μg, 20 μL) or 20 μL of PBS was intracutaneously injected into the right ear; and
h. on the fifth day of the experiment (i.e., D4), the final thickness of the right ear was measured. The animals were administered by gavage according to the experimental design and grouping; and after 1 h, the animals were euthanized, and blood and ear tissue samples were taken. The ear was divided into two portions, one of which was frozen quickly and used for the detection of inflammatory factor mRNA, and the other one of which was fixed with 4% tissue cell fixative for histological treatment and analysis (HE staining); and the plasma was used for PK detection.

11.4 Data processing:

a. The initial ear thickness was subtracted from the daily ear thickness of the animals in each group (ear thickening) and comparison was made with the Model group (Vehicle).

b. The result of the ear thickness on the last day of the experiment (Day 4) - that on Day 0 was subjected to diagraph analysis by Graphpad prism 9. Ear thickness variation analysis between groups was carried out by One-way ANOVA Dunnett's test, and $p < 0.05$ was considered to be significantly different. Ear thickness inhibition rate (%) = [1 - (Average value in administration group / average value in model group)] × 100%.

11.5 Experimental results:

| Example | Inhibition rate on Day 4, 10 mg/kg |
|---|---|
| PN-235 (BID) | 55.35% |
| 1 (QD) | 51.22% |
| 1-16-1 (QD) | 70.77% |
| 4-16-1 (QD) | 56.60% |

11.6 Experimental conclusion: In the IL23-induced rat otitis model, the compounds of the preferred examples of the present invention could effectively inhibit ear thickening and were more effective in inhibiting ear thickening when administered in a once-a-day mode than PN-235 administered twice a day, thus achieving the purpose of prolonging the administration cycle.

## Claims

**1.** A compound represented by general formula (I) or a stereoisomer or pharmaceutically acceptable salt thereof:

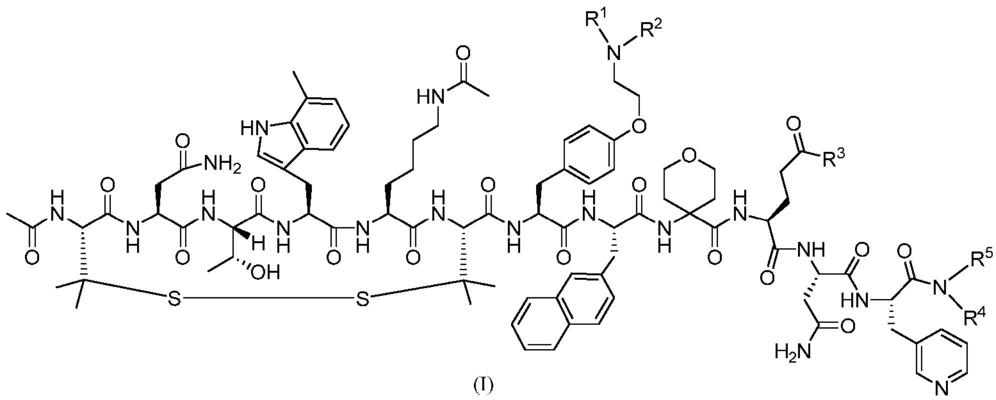

(I)

**characterized in that**:

$R^1$ and $R^2$ are each independently selected from H, alkyl,

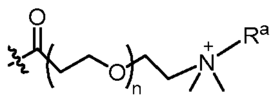

or an amino acid;

$R^a$ is selected from H, alkyl,

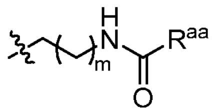

or an amino acid;
$R^{aa}$ is selected from alkyl,

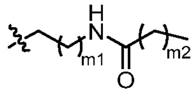
,

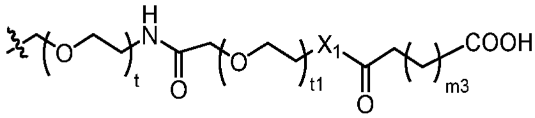

or an amino acid;
$X_1$ is an amino acid;
preferably, $R^{aa}$ is selected from alkyl,

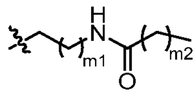
,

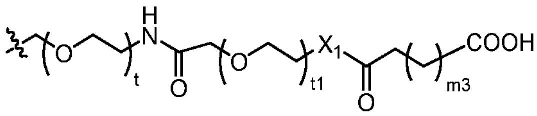
,

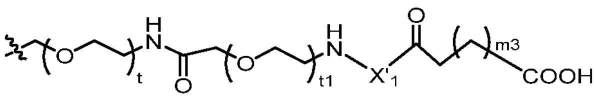

or an amino acid;
$X_1$ is an amino acid;
$X'_1$ is an amino acid;
$R^3$ is selected from hydroxyl, alkyl, $-NR^6R^7$ or an amino acid;
$R^6$ and $R^7$ are each independently selected from H, alkyl, $-CH_2C(O)NH_2$,

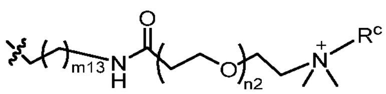
,
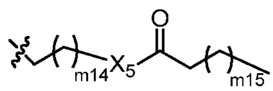
,

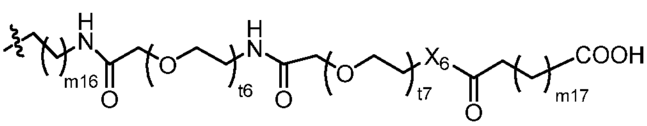

or an amino acid;
$R^c$ is selected from H, alkyl,

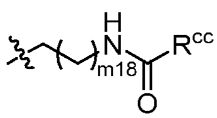

or an amino acid;
$R^{cc}$ is selected from alkyl,

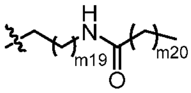

,

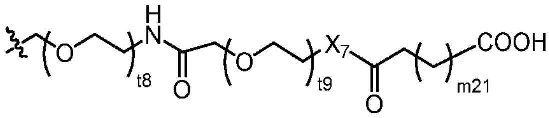

or an amino acid;
$X_5$ is an amino acid;
$X_6$ is an amino acid;
$X_7$ is an amino acid;
preferably, $R^3$ is selected from hydroxyl, alkyl, $-NR^6R^7$ or an amino acid;
$R^6$ is selected from H, alkyl, $-CH_2C(O)NH_2$,

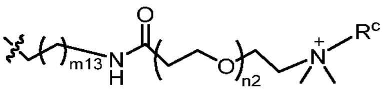

,

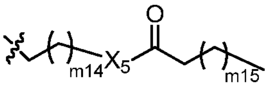

,

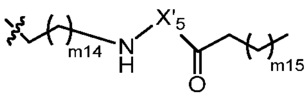

,

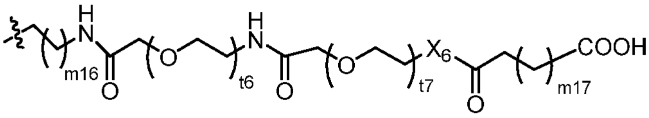

,

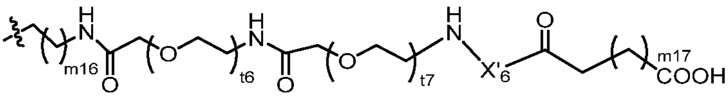

or an amino acid;
$R^7$ is selected from H, alkyl, $-CH_2C(O)NH_2$,

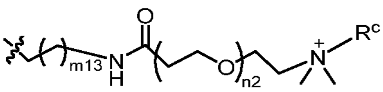

,

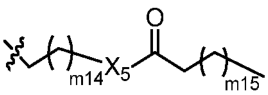

,

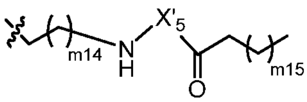

,

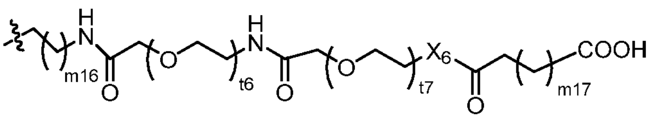

,

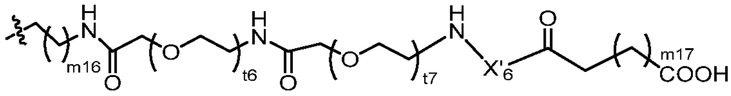

or an amino acid;

$R^c$ is selected from H, alkyl,

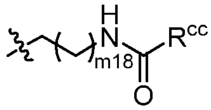

or an amino acid;

$R^{cc}$ is selected from alkyl,

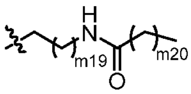

,

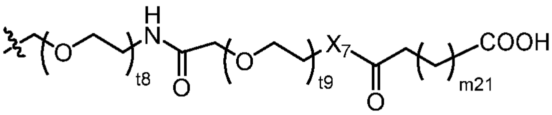

,

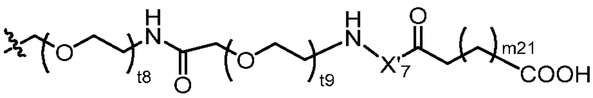

or an amino acid;

$X_5$ is an amino acid;

$X'_5$ is an amino acid;

$X_6$ is an amino acid;

$X'_6$ is an amino acid;

$X_7$ is an amino acid;

$X'_7$ is an amino acid;

$R^4$ and $R^5$ are each independently selected from H, alkyl, $-CH_2C(O)NH_2$,

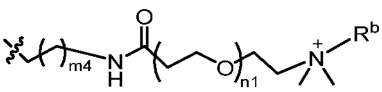

,

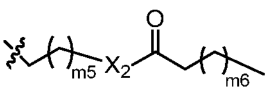

,

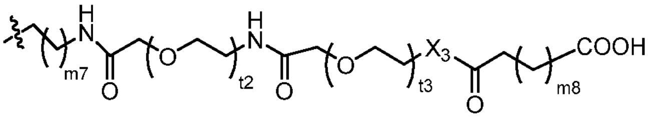

or an amino acid;

$R^b$ is selected from H, alkyl,

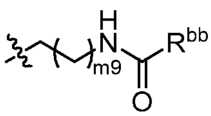

or an amino acid;

$R^{bb}$ is selected from alkyl,

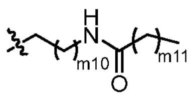

,

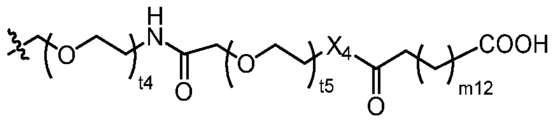

or an amino acid;

$X_2$ is an amino acid;

$X_3$ is an amino acid;

$X_4$ is an amino acid;

preferably, $R^4$ is selected from H, alkyl, $-CH_2C(O)NH_2$,

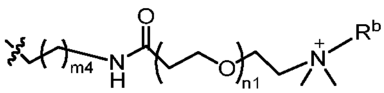

,

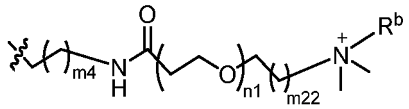

,

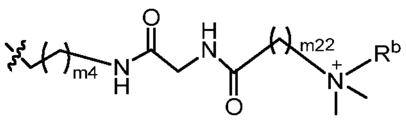

,

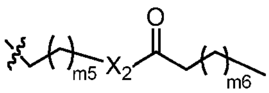

,

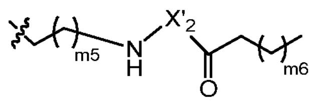

,

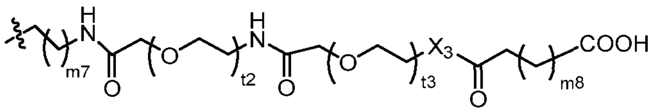

,

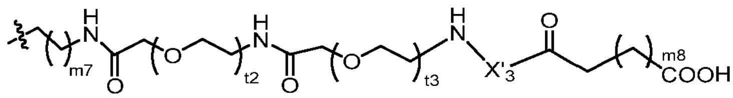

or an amino acid;

$R^5$ is selected from H, alkyl, $-CH_2C(O)NH_2$,

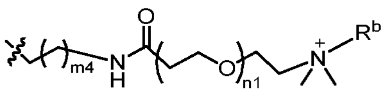

,

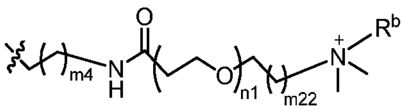

,

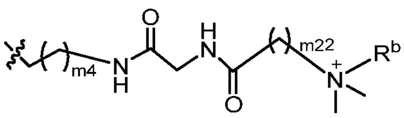

,

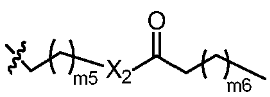

,

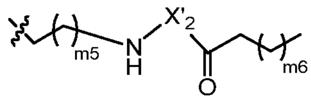

,

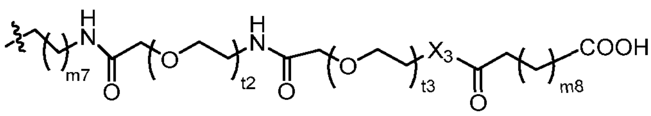

,

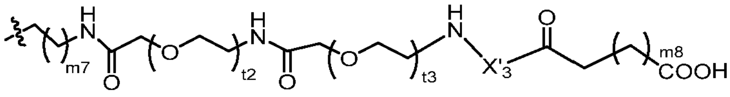

or an amino acid; preferably from

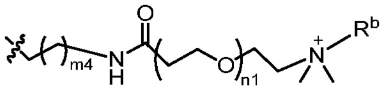, 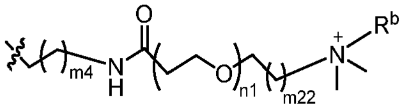,

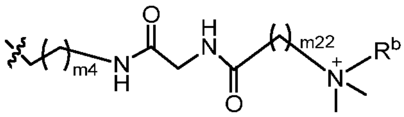, 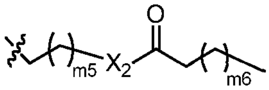, 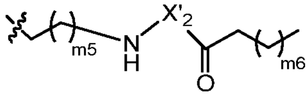,

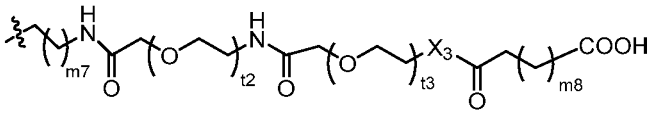

or

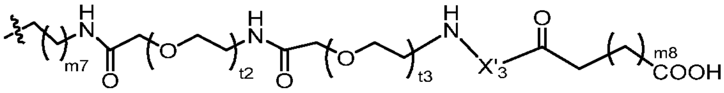;

$R^b$ is selected from H, alkyl,

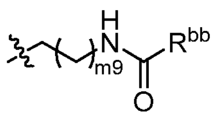

or an amino acid, wherein the alkyl can be further substituted with hydroxyl or alkoxy;
$R^{bb}$ is selected from alkyl,

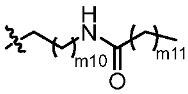

,

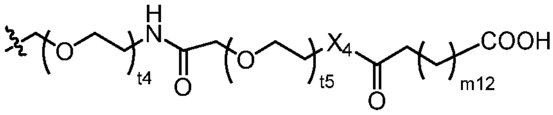

,

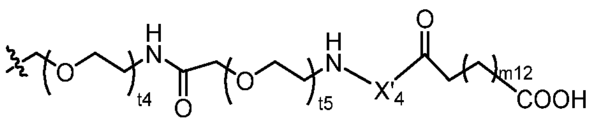

or an amino acid;
$X_2$ is an amino acid;
$X'_2$ is an amino acid;
$X_3$ is an amino acid;
$X'_3$ is an amino acid;
$X_4$ is an amino acid;
$X'_4$ is an amino acid;

n-n2 are each independently selected from integers of 0-12;
t-t9 are each independently selected from integers of 0-12;
m-m21 are each independently selected from integers of 0-24;
m22 is selected from integers of 0-24;
the compound is not

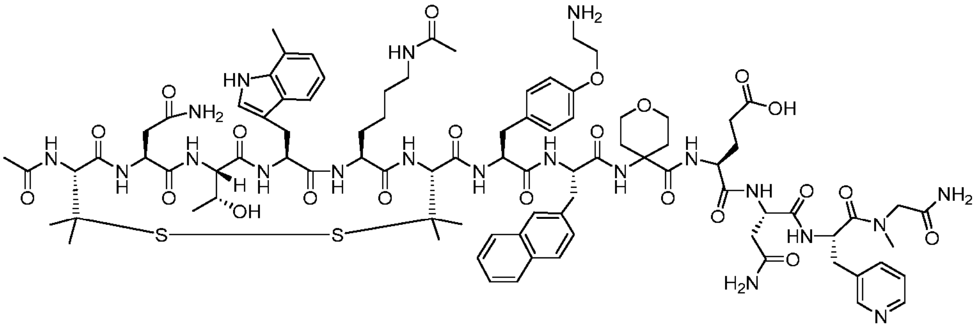

;

preferably,
$R^1$ and $R^2$ are each independently selected from H, alkyl, or an amino acid;

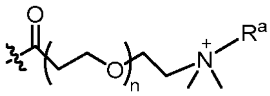

$R^a$ is selected from H, alkyl,

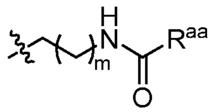

or an amino acid;
$R^{aa}$ is selected from alkyl,

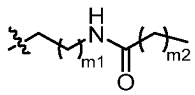

,

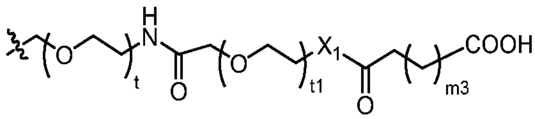

or an amino acid;
$X_1$ is an amino acid;
preferably, $R^{aa}$ is selected from alkyl,

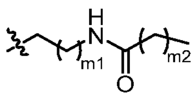

,

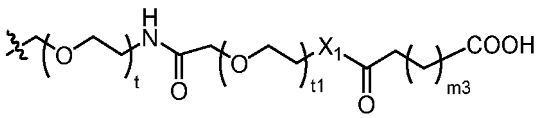

,

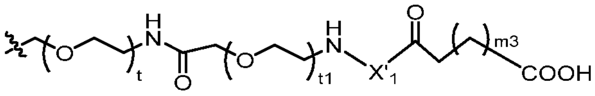

or an amino acid;

$X_1$ is an amino acid;

$X'_1$ is an amino acid;

$R^3$ is hydroxyl;

$R^4$ and $R^5$ are each independently selected from H, alkyl and $-CH_2C(O)NH_2$;

n is selected from integers of 0-12;

t-t1 are each independently selected from integers of 0-12;

m-m3 are each independently selected from integers of 0-24;

or

$R^1$ and $R^2$ are H;

$R^3$ is selected from hydroxyl, alkyl, $-NR^6R^7$ or an amino acid;

$R^6$ and $R^7$ are each independently selected from H, alkyl, $-CH_2C(O)NH_2$,

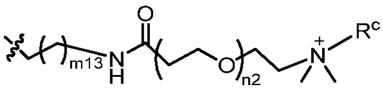

,

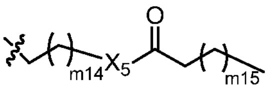

,

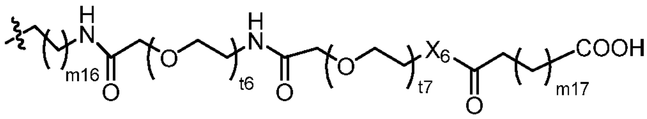

or an amino acid;

$R^c$ is selected from H, alkyl,

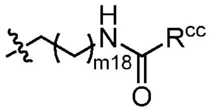

or an amino acid;

$R^{cc}$ is selected from alkyl,

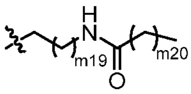

,

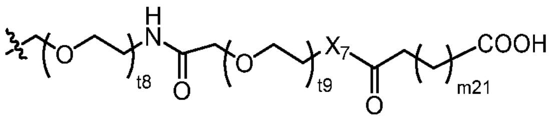

or an amino acid;

$X_5$ is an amino acid;

$X_6$ is an amino acid;

$X_7$ is an amino acid;

preferably, $R^3$ is selected from hydroxyl, alkyl, $-NR^6R^7$ or an amino acid;

$R^6$ is selected from H, alkyl, $-CH_2C(O)NH_2$,

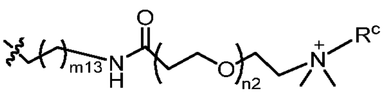

,

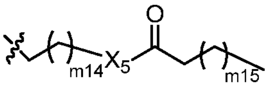 , 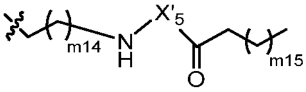 ,

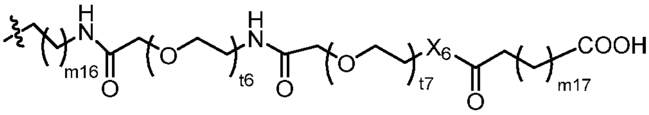 ,

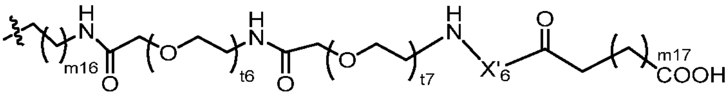

or an amino acid;

$R^7$ is selected from H, alkyl, $-CH_2C(O)NH_2$,

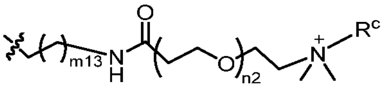 ,

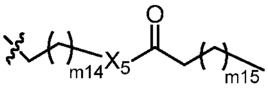 , 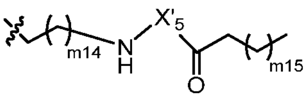 ,

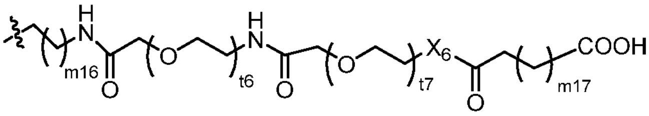 ,

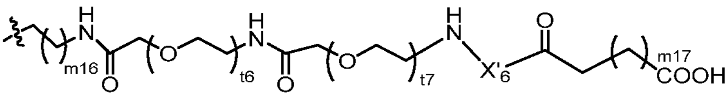

or an amino acid;

$R^c$ is selected from H, alkyl,

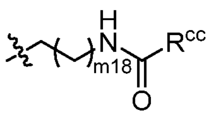

or an amino acid;

$R^{cc}$ is selected from alkyl,

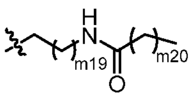 ,

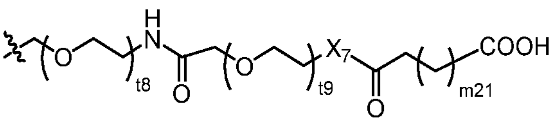 ,

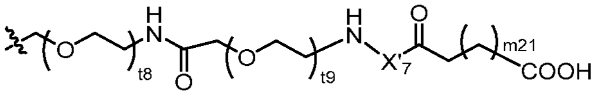

or an amino acid;
$X_5$ is an amino acid;
$X'_5$ is an amino acid;
$X_6$ is an amino acid;
$X'_6$ is an amino acid;
$X_7$ is an amino acid;
$X'_7$ is an amino acid;
$R^4$ and $R^5$ are each independently selected from H, alkyl and $-CH_2C(O)NH_2$;
n2 is selected from integers of 0-12;
t6-t9 are each independently selected from integers of 0-12;
m13-m21 are each independently selected from integers of 0-24;
or
$R^1$ and $R^2$ are H;
$R^3$ is hydroxyl;
$R^4$ and $R^5$ are each independently selected from H, alkyl, $-CH_2C(O)NH_2$,

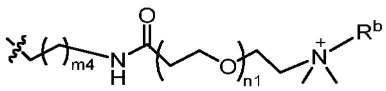
,
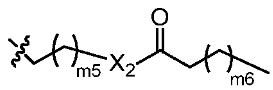
,
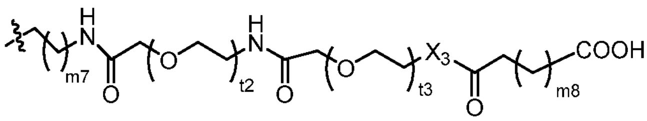

or an amino acid;
$R^b$ is selected from H, alkyl,

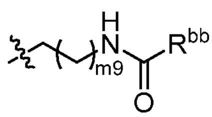

or an amino acid;
$R^{bb}$ is selected from alkyl,

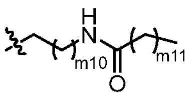
,
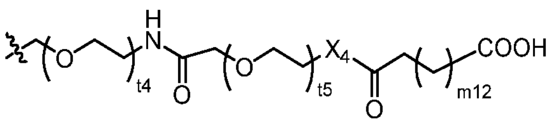

or an amino acid;
$X_2$ is an amino acid;
$X_3$ is an amino acid;
$X_4$ is an amino acid;
preferably, $R^4$ is selected from H, alkyl, $-CH_2C(O)NH_2$,

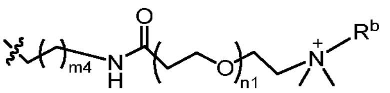 , 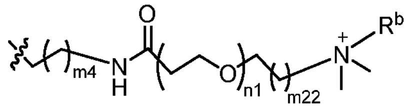 ,

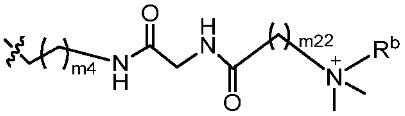 , 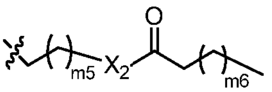 , 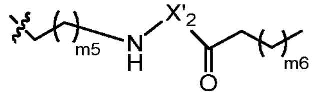 ,

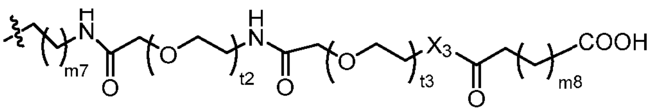 ,

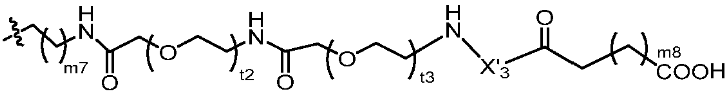

or an amino acid;
$R^5$ is selected from H, alkyl, $-CH_2C(O)NH_2$,

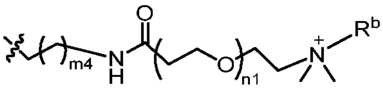 ,

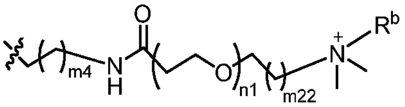 , 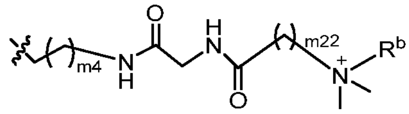 ,

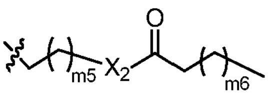 , 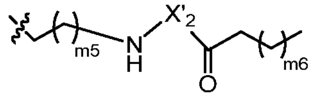 ,

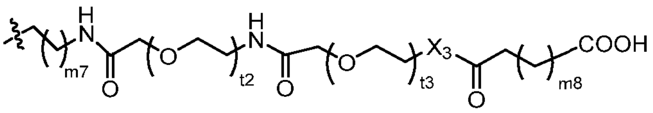 ,

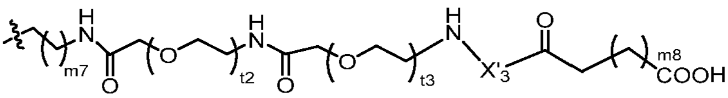

or an amino acid; preferably from

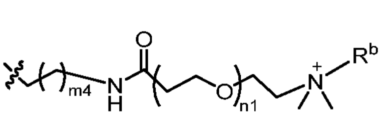 , 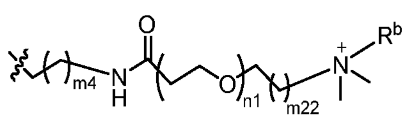 ,

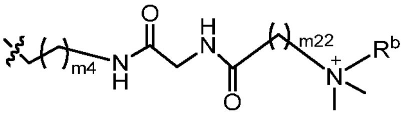 , 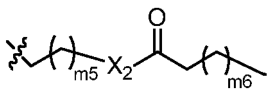 , 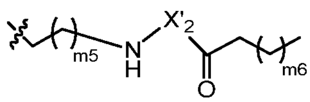 ,

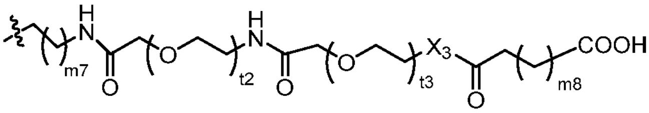

or

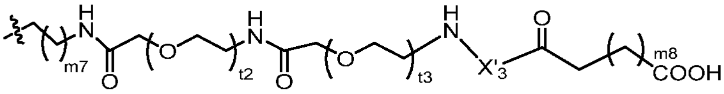 ,

$R^b$ is selected from H, alkyl,

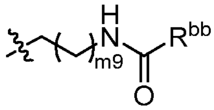

or an amino acid, wherein the alkyl can be further substituted with hydroxyl or alkoxy;
$R^{bb}$ is selected from alkyl,

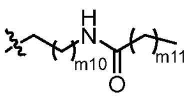

,

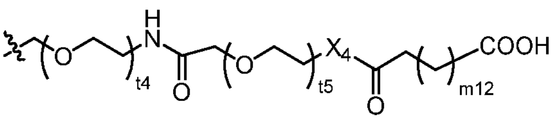

,

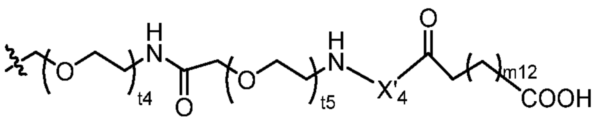

or an amino acid;
$X_2$ is an amino acid;
$X'_2$ is an amino acid;
$X_3$ is an amino acid;
$X'_3$ is an amino acid;
$X_4$ is an amino acid;
$X'_4$ is an amino acid;
n1 is selected from integers of 0-12;
t2-t5 are each independently selected from integers of 0-12;
m4-m12 are each independently selected from integers of 0-24; and
m22 is selected from integers of 0-24.

**2.** A compound represented by general formula (I-V) or a stereoisomer or pharmaceutically acceptable salt thereof:

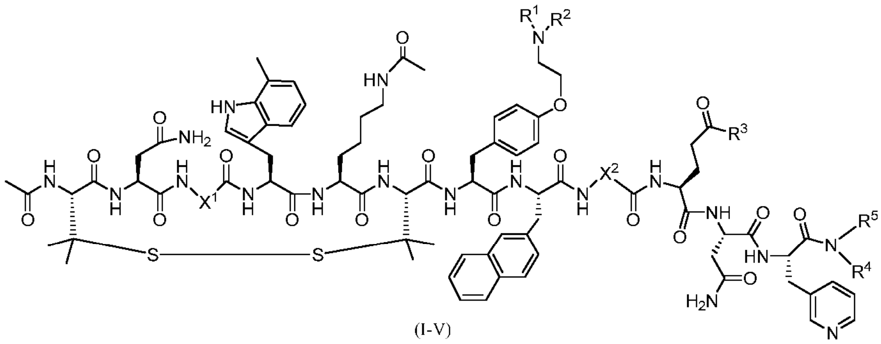

(I-V)

**characterized in that**:

$X^1$ is an amino acid;
$X^2$ is an amino acid;
$R^1$ and $R^2$ are each independently selected from H, alkyl,

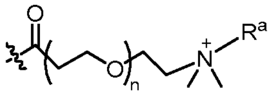

or an amino acid;
$R^a$ is selected from H, alkyl,

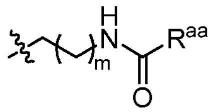

or an amino acid;
$R^{aa}$ is selected from alkyl,

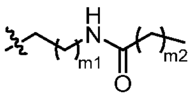

,

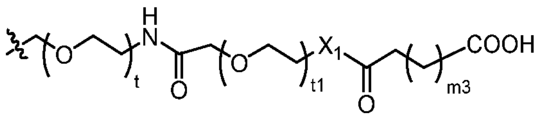

,

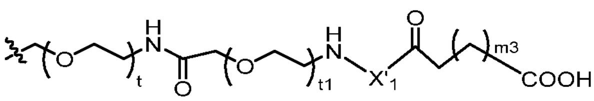

or an amino acid;
$X_1$ is an amino acid;
$X'_1$ is an amino acid;
$R^3$ is selected from hydroxyl, alkyl, $-NR^6R^7$ or an amino acid;
$R^6$ is selected from H, alkyl, $-CH_2C(O)NH_2$,

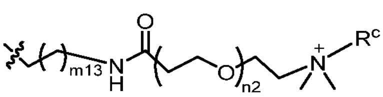

,

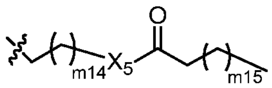,
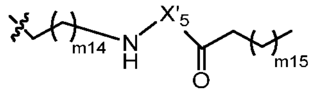,

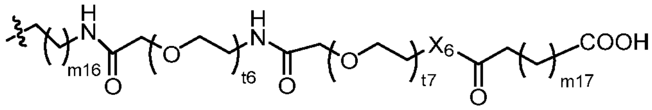,

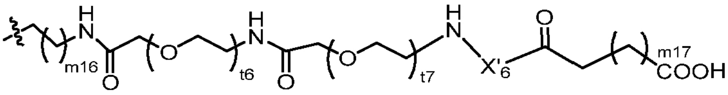

or an amino acid;

$R^7$ is selected from H, alkyl, $-CH_2C(O)NH_2$,

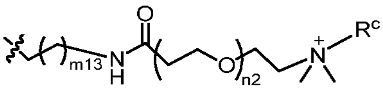,

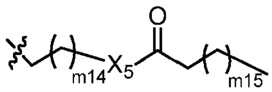,
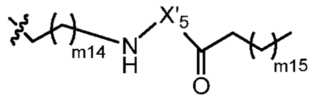,

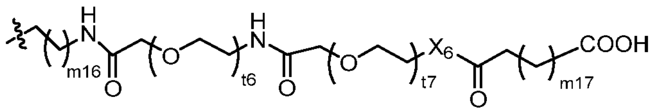,

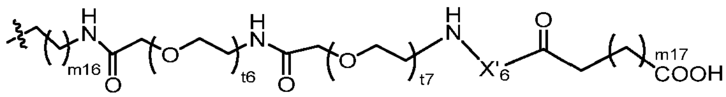

or an amino acid;

$R^c$ is selected from H, alkyl,

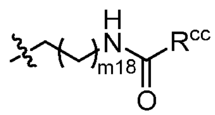

or an amino acid;

$R^{cc}$ is selected from alkyl,

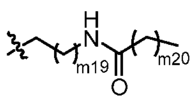,

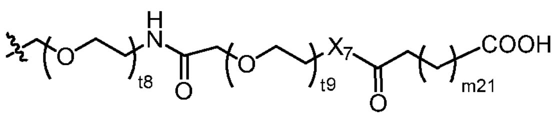,

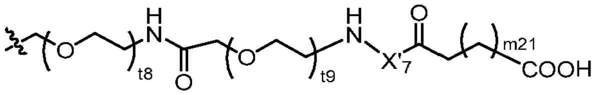

or an amino acid;

$X_5$ is an amino acid;

$X'_5$ is an amino acid;

$X_6$ is an amino acid;

$X'_6$ is an amino acid;

$X_7$ is an amino acid;

$X'_7$ is an amino acid;

$R^4$ is selected from H, alkyl, $-CH_2C(O)NH_2$,

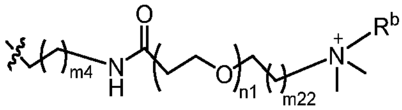

,

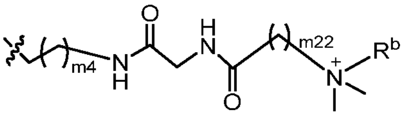

,

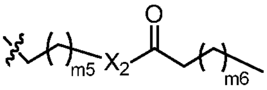

,

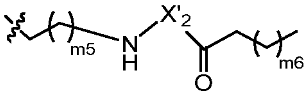

,

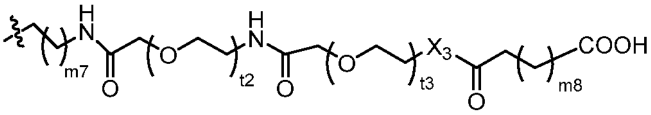

,

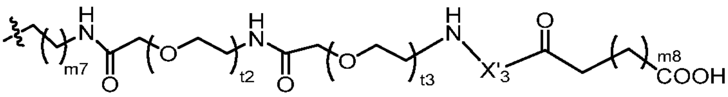

or an amino acid;

$R^5$ is selected from H, alkyl, $-CH_2C(O)NH_2$,

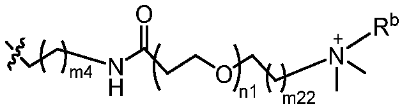

,

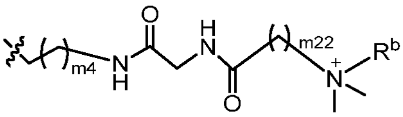

,

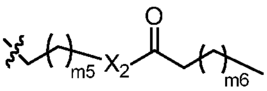

,

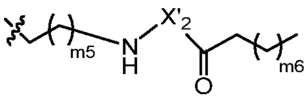

,

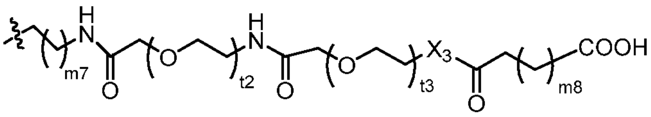

,

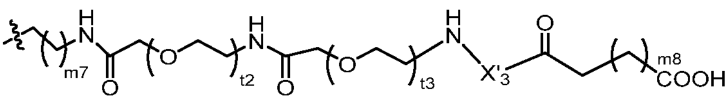

or an amino acid;

$R^b$ is selected from H, alkyl,

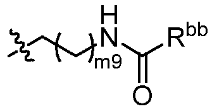

or an amino acid, wherein the alkyl can be further substituted with hydroxyl or alkoxy;
$R^{bb}$ is selected from alkyl,

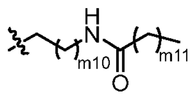

,

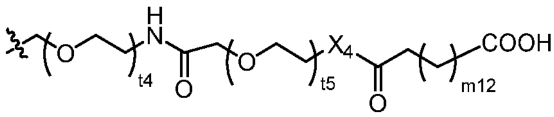

,

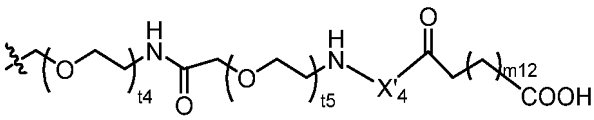

or an amino acid;
$X_2$ is an amino acid;
$X'_2$ is an amino acid;
$X_3$ is an amino acid;
$X'_3$ is an amino acid;
$X_4$ is an amino acid;
$X'_4$ is an amino acid;
n-n2 are each independently selected from integers of 0-12;
t-t9 are each independently selected from integers of 0-12;
m-m21 are each independently selected from integers of 0-24;
m22 is selected from integers of 0-24; and
the compound is not

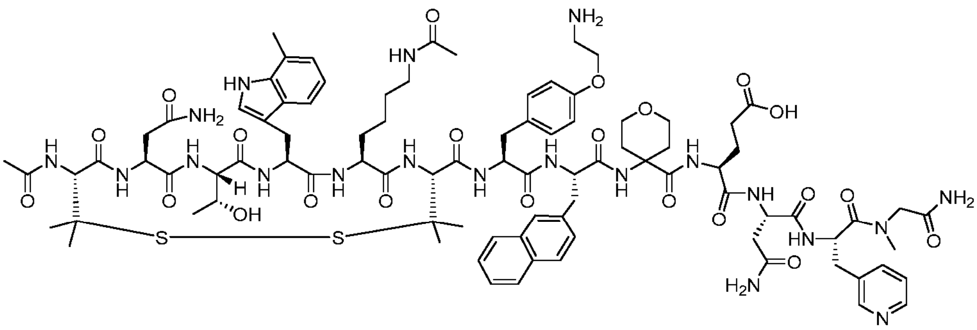

.

**3.** The compound or the stereoisomer or pharmaceutically acceptable salt thereof according to either claim 1 or 2, **characterized in that** the compound is further as represented by general formula (II) or (III):

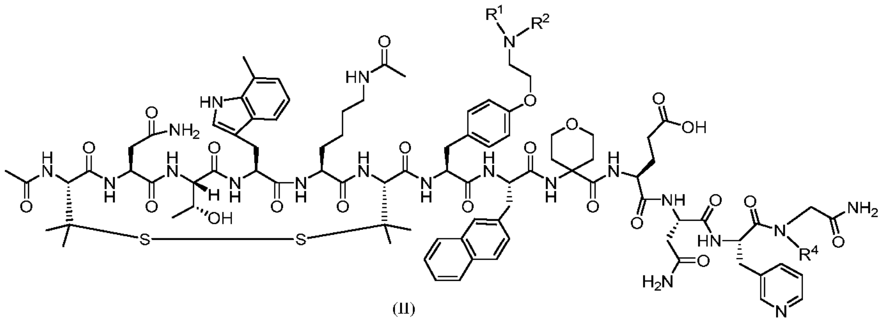

(II)

or

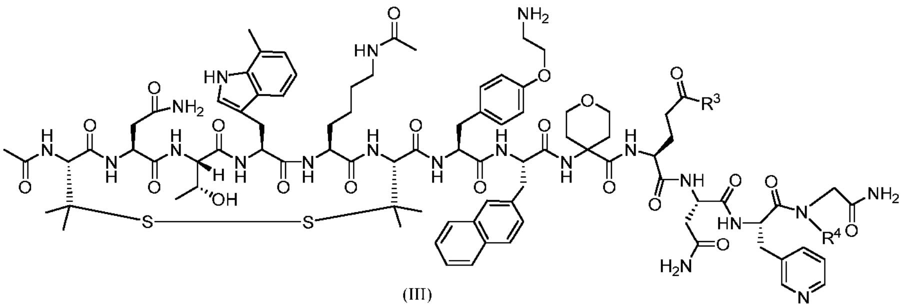

(III)

wherein:

$R^1$ and $R^2$ are each independently selected from H, alkyl,

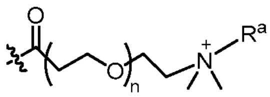

or an amino acid;

$R^a$ is selected from H, alkyl,

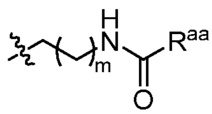

or an amino acid;

$R^{aa}$ is selected from alkyl,

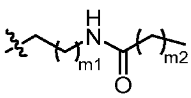

,

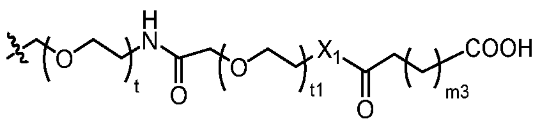

or an amino acid;

$X^1$ is an amino acid;

preferably, $R^{aa}$ is selected from alkyl,

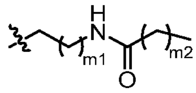

,

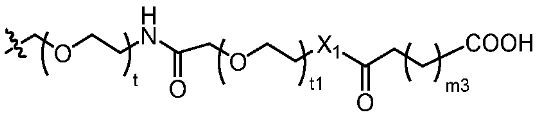

,

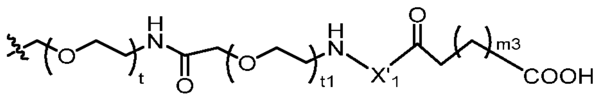

or an amino acid;

$X_1$ is an amino acid;

$X'_1$ is an amino acid;

$R^3$ is selected from hydroxyl, alkyl, $-NR^6R^7$ or an amino acid;

$R^6$ and $R^7$ are each independently selected from H, alkyl, $-CH_2C(O)NH_2$,

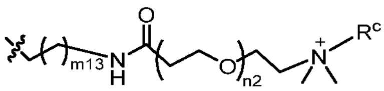

,

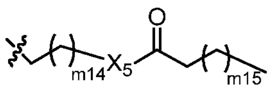

,

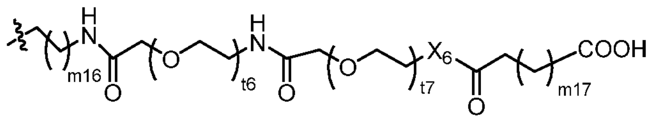

or an amino acid;

$R^c$ is selected from H, alkyl,

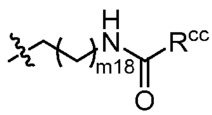

or an amino acid;

$R^{cc}$ is selected from alkyl,

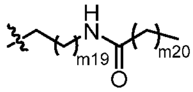

,

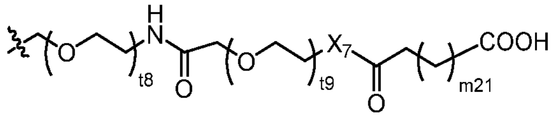

or an amino acid;

$X_5$ is an amino acid;

$X_6$ is an amino acid;

$X_7$ is an amino acid;

preferably, $R^3$ is selected from hydroxyl, alkyl, $-NR^6R^7$ or an amino acid;

$R^6$ is selected from H, alkyl, $-CH_2C(O)NH_2$,

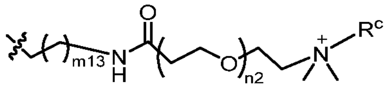

,

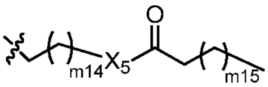

,

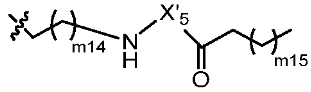

,

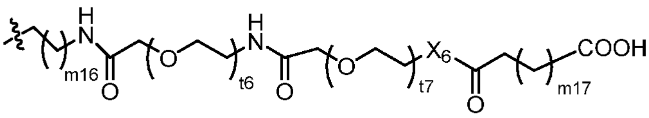

,

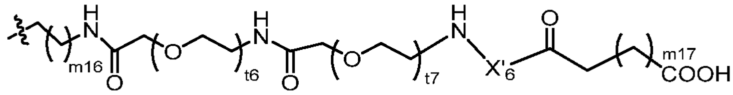

or an amino acid;

$R^7$ is selected from H, alkyl, $-CH_2C(O)NH_2$,

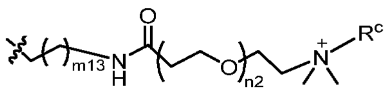

,

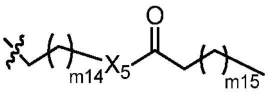

,

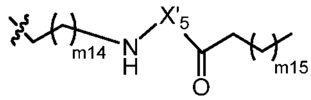

,

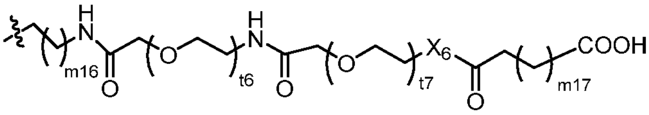

,

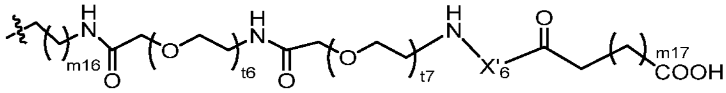

or an amino acid;

$R^c$ is selected from H, alkyl,

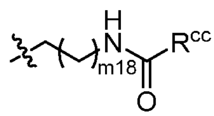

or an amino acid;

$R^{cc}$ is selected from alkyl,

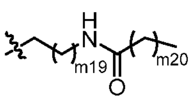

,

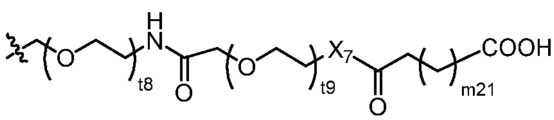

,

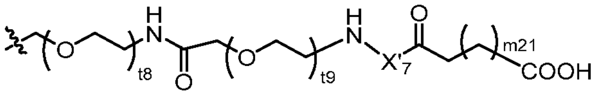

or an amino acid;
$X_5$ is an amino acid;
$X'_5$ is an amino acid;
$X_6$ is an amino acid;
$X'_6$ is an amino acid;
$X_7$ is an amino acid;
$X'_7$ is an amino acid;
$R^4$ is selected from H or alkyl;
n is selected from 0, 1, 2, 3, 4, 5, 6, 7, 8, 9, 10, 11, or 12;
t is selected from 0, 1, 2, 3, 4, 5, 6, 7, 8, 9, 10, 11, or 12;
t1 is selected from 0, 1, 2, 3, 4, 5, 6, 7, 8, 9, 10, 11, or 12;
m is selected from 0, 1, 2, 3, 4, 5, 6, 7, 8, 9, 10, 11, 12, 13, 14, 15, 16, 17, 18, 19, 20, 21, 22, 23, or 24;
m1 is selected from 0, 1, 2, 3, 4, 5, 6, 7, 8, 9, 10, 11, 12, 13, 14, 15, 16, 17, 18, 19, 20, 21, 22, 23, or 24;
m2 is selected from 0, 1, 2, 3, 4, 5, 6, 7, 8, 9, 10, 11, 12, 13, 14, 15, 16, 17, 18, 19, 20, 21, 22, 23, or 24;
m3 is selected from 0, 1, 2, 3, 4, 5, 6, 7, 8, 9, 10, 11, 12, 13, 14, 15, 16, 17, 18, 19, 20, 21, 22, 23, or 24;
n2 is selected from 0, 1, 2, 3, 4, 5, 6, 7, 8, 9, 10, 11, or 12;
t6 is selected from 0, 1, 2, 3, 4, 5, 6, 7, 8, 9, 10, 11, or 12;
t7 is selected from 0, 1, 2, 3, 4, 5, 6, 7, 8, 9, 10, 11, or 12;
t8 is selected from 0, 1, 2, 3, 4, 5, 6, 7, 8, 9, 10, 11, or 12;
t9 is selected from 0, 1, 2, 3, 4, 5, 6, 7, 8, 9, 10, 11, or 12;
m13 is selected from 0, 1, 2, 3, 4, 5, 6, 7, 8, 9, 10, 11, 12, 13, 14, 15, 16, 17, 18, 19, 20, 21, 22, 23, or 24;
m14 is selected from 0, 1, 2, 3, 4, 5, 6, 7, 8, 9, 10, 11, 12, 13, 14, 15, 16, 17, 18, 19, 20, 21, 22, 23, or 24;
m15 is selected from 0, 1, 2, 3, 4, 5, 6, 7, 8, 9, 10, 11, 12, 13, 14, 15, 16, 17, 18, 19, 20, 21, 22, 23, or 24;
m16 is selected from 0, 1, 2, 3, 4, 5, 6, 7, 8, 9, 10, 11, 12, 13, 14, 15, 16, 17, 18, 19, 20, 21, 22, 23, or 24;
m17 is selected from 0, 1, 2, 3, 4, 5, 6, 7, 8, 9, 10, 11, 12, 13, 14, 15, 16, 17, 18, 19, 20, 21, 22, 23, or 24;
m18 is selected from 0, 1, 2, 3, 4, 5, 6, 7, 8, 9, 10, 11, 12, 13, 14, 15, 16, 17, 18, 19, 20, 21, 22, 23, or 24;
m19 is selected from 0, 1, 2, 3, 4, 5, 6, 7, 8, 9, 10, 11, 12, 13, 14, 15, 16, 17, 18, 19, 20, 21, 22, 23, or 24;
m20 is selected from 0, 1, 2, 3, 4, 5, 6, 7, 8, 9, 10, 11, 12, 13, 14, 15, 16, 17, 18, 19, 20, 21, 22, 23, or 24; and
m21 is selected from 0, 1, 2, 3, 4, 5, 6, 7, 8, 9, 10, 11, 12, 13, 14, 15, 16, 17, 18, 19, 20, 21, 22, 23, or 24.

4. The compound or the stereoisomer or pharmaceutically acceptable salt thereof according to any one of claims 1-3, **characterized in that** $R^1$ is selected from H, $C_{1-6}$ alkyl,

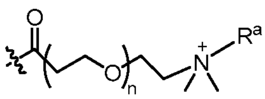

or an amino acid;

$R^a$ is selected from H, $C_{1-6}$ alkyl,

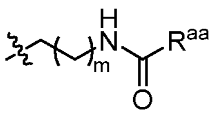

or an amino acid;
$R^{aa}$ is selected from $C_{1-6}$ alkyl,

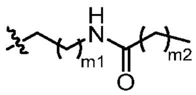

,

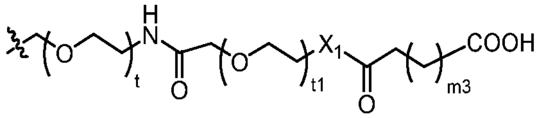

or an amino acid;

$X^1$ is an amino acid;

preferably, $R^{aa}$ is selected from $C_{1-6}$ alkyl,

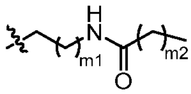

,

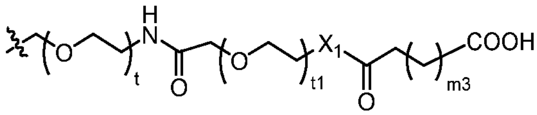

,

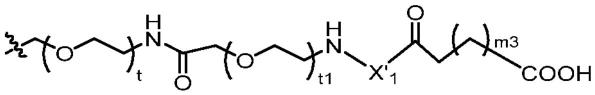

or an amino acid;

$X_1$ is an amino acid;

$X'_1$ is an amino acid;

n is selected from integers of 0-12;

t-t1 are each independently selected from integers of 0-12;

m-m3 are each independently selected from integers of 0-24;

and/or

$R^2$ is selected from H, $C_{1-6}$ alkyl,

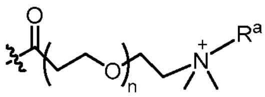

or an amino acid;

$R^a$ is selected from H, $C_{1-6}$ alkyl,

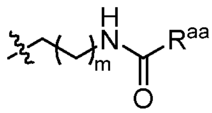

or an amino acid;

$R^{aa}$ is selected from $C_{1-6}$ alkyl,

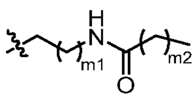

,

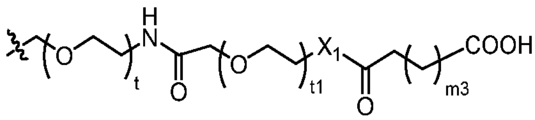

or an amino acid;
$X_1$ is an amino acid;
preferably, $R^{aa}$ is selected from $C_{1-6}$ alkyl,

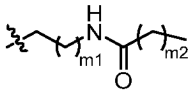
,

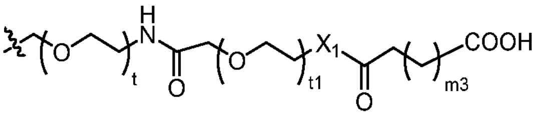
,

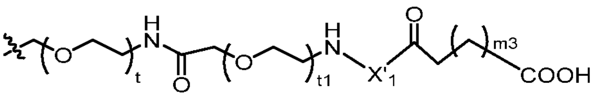

or an amino acid;
$X_1$ is an amino acid;
$X'_1$ is an amino acid;
n is selected from integers of 0-12;
t-t1 are each independently selected from integers of 0-12;
m-m3 are each independently selected from integers of 0-24;
and/or
$R^3$ is selected from hydroxyl, $C_{1-6}$ alkyl, $-NR^6R^7$ or an amino acid;
$R^6$ and $R^7$ are each independently selected from H, alkyl, $-CH_2C(O)NH_2$,

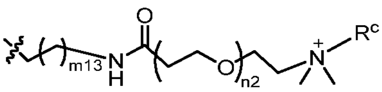
,
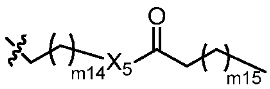
,

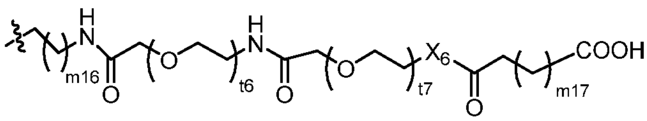

or an amino acid;
$R^c$ is selected from H, alkyl,

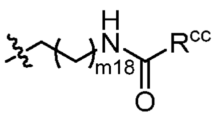

or an amino acid;
$R^{cc}$ is selected from alkyl,

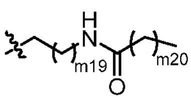
,

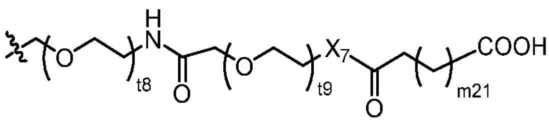

or an amino acid;

$X_5$ is an amino acid;

$X_6$ is an amino acid;

$X_7$ is an amino acid;

preferably, $R^3$ is selected from hydroxyl, $C_{1-6}$ alkyl, $-NR^6R^7$ or an amino acid;

$R^6$ is selected from H, $C_{1-6}$ alkyl, $-CH_2C(O)NH_2$,

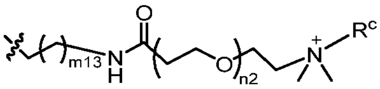

,

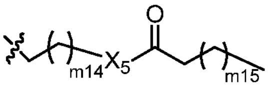

,

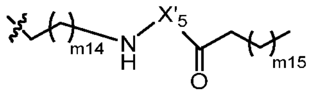

,

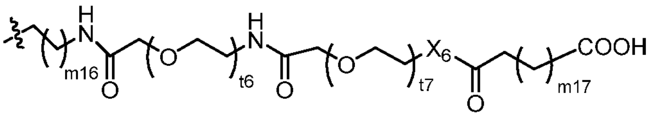

,

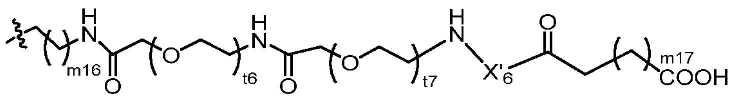

or an amino acid;

$R^7$ is selected from H, $C_{1-6}$ alkyl, $-CH_2C(O)NH_2$,

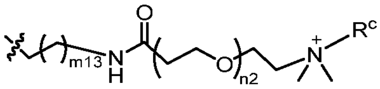

,

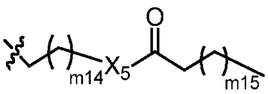

,

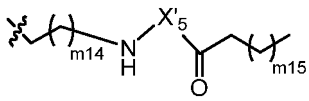

,

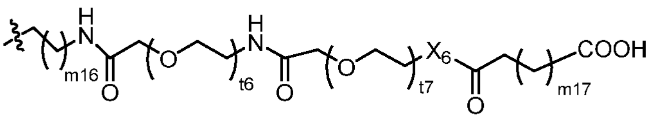

,

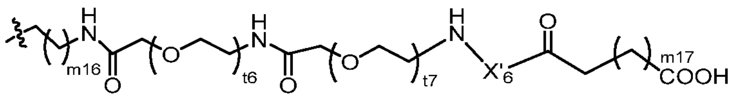

or an amino acid;

$R^c$ is selected from H, $C_{1-6}$ alkyl,

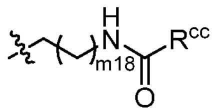

or an amino acid;
$R^{cc}$ is selected from $C_{1-6}$ alkyl,

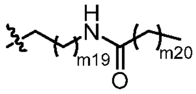

,

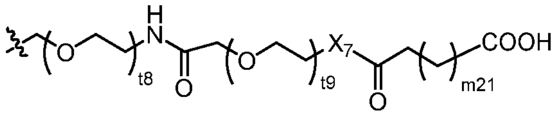

,

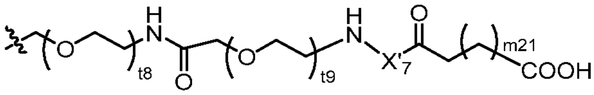

or an amino acid;
$X_5$ is an amino acid;
$X'_5$ is an amino acid;
$X_6$ is an amino acid;
$X'_6$ is an amino acid;
$X_7$ is an amino acid;
$X'_7$ is an amino acid;
n2 is selected from integers of 0-12;
t6-t9 are each independently selected from integers of 0-12; and
m13-m21 are each independently selected from integers of 0-24.

**5.** The compound or the stereoisomer or pharmaceutically acceptable salt thereof according to claim 2, **characterized in that** the compound is further as represented by general formula (V):

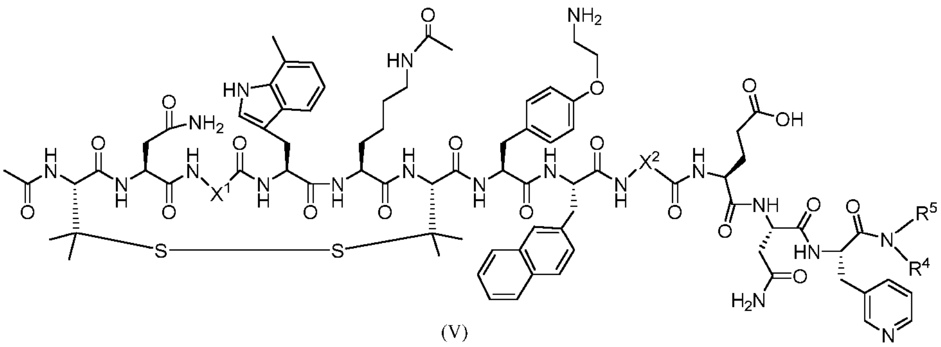

(V)

wherein:

$X^1$ is an amino acid;
$X^2$ is an amino acid;
$R^4$ is selected from H, alkyl, $-CH_2C(O)NH_2$,

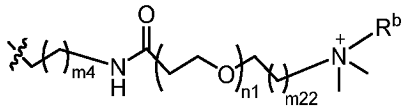

,

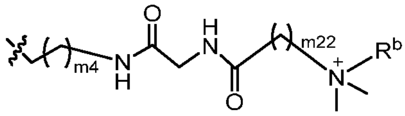

,

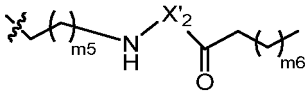

,

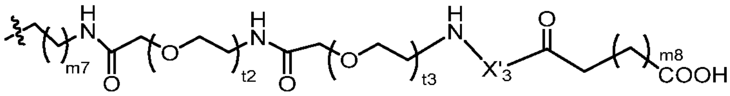

or an amino acid;
$R^5$ is selected from H, alkyl, $-CH_2C(O)NH_2$,

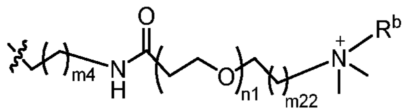

,

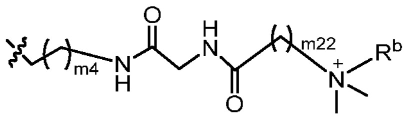

,

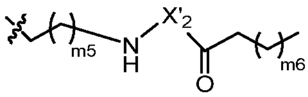

,

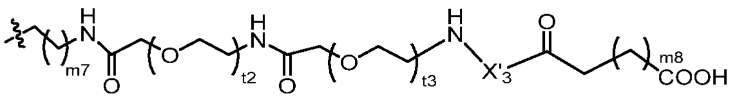

or an amino acid;
$R^b$ is selected from H, alkyl,

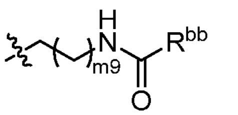

or an amino acid, wherein the alkyl can be further substituted with hydroxyl or alkoxy;
$R^{bb}$ is selected from alkyl,

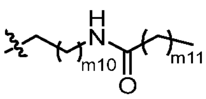

,

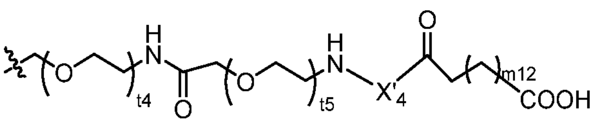

or an amino acid;
$X'_2$ is an amino acid;
$X'_3$ is an amino acid;
$X_4$ is an amino acid;
n1 is selected from integers of 0-12;

t2-t5 are each independently selected from integers of 0-12;
m4-m12 are each independently selected from integers of 0-24;
m22 is selected from integers of 0-12; and
the compound is not

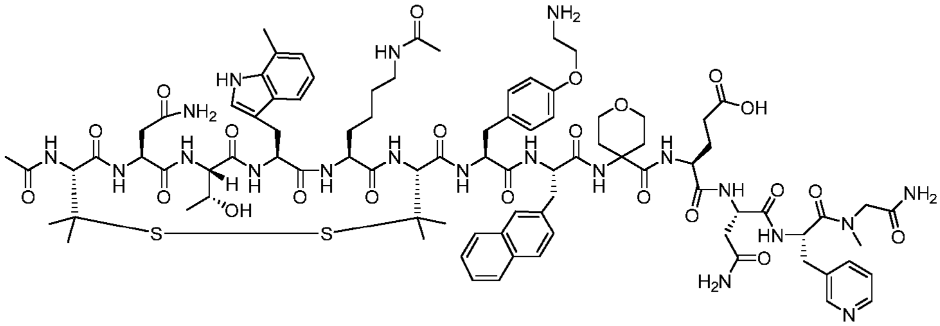
.

6. The compound or the stereoisomer or pharmaceutically acceptable salt thereof according to either claim 2 or 5, **characterized in that** $X^1$ is selected from Thr,

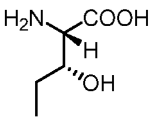
,
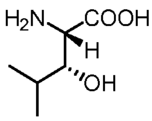
or
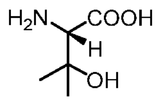
;

preferably

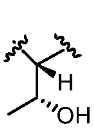
,
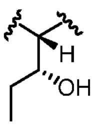
,
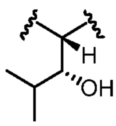

or

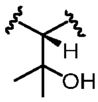
;

and/or
$X^2$ is selected from

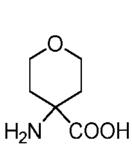
,
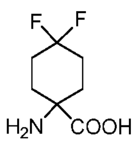
,
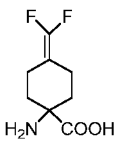
,
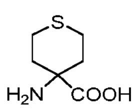
,
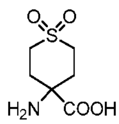

or

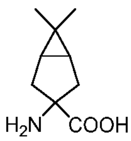
;

and preferably

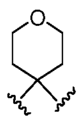, 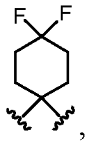, 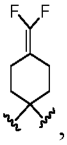, 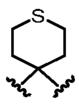, 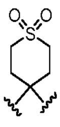 or 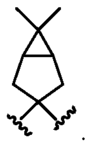.

**7.** The compound or the stereoisomer or pharmaceutically acceptable salt thereof according to any one of claims 1 and 2 or 5, **characterized in that** the compound is further as represented by general formula (IV):

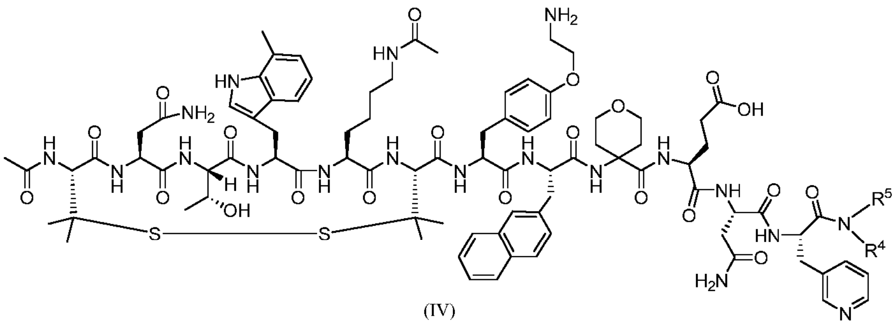

(IV)

wherein:

$R^4$ is selected from H or alkyl;
$R^5$ is selected from alkyl, $-CH_2C(O)NH_2$,

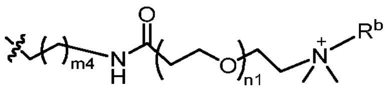,

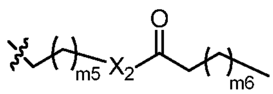, 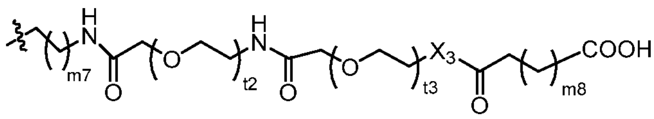

or an amino acid;
$R^b$ is selected from H, alkyl,

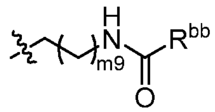

or an amino acid;
$R^{bb}$ is selected from alkyl,

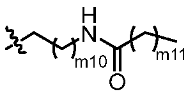,

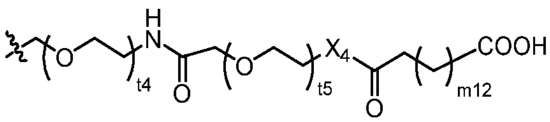

or an amino acid;
$X_2$ is an amino acid;

$X_3$ is an amino acid;
$X_4$ is an amino acid;
preferably, $R^4$ is selected from H, alkyl, $-CH_2C(O)NH_2$,

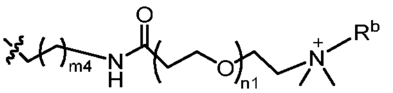 , 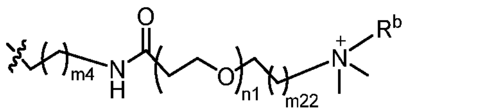 ,

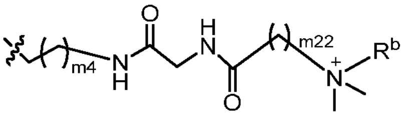 , 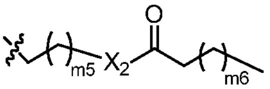 , 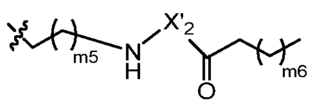 ,

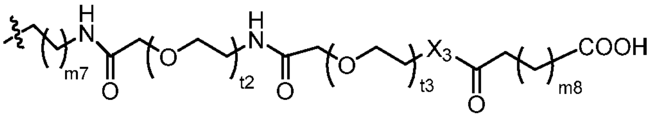 ,

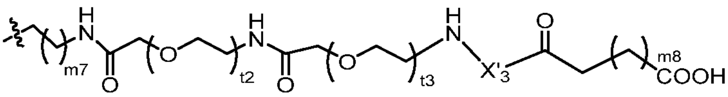

or an amino acid;
$R^5$ is selected from H, alkyl, $-CH_2C(O)NH_2$,

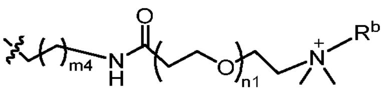 ,

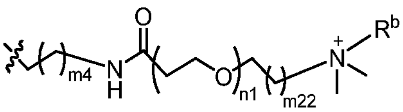 , 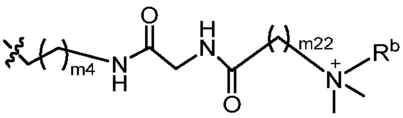 ,

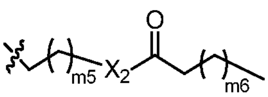 , 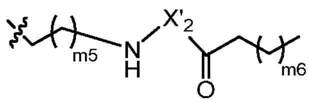 ,

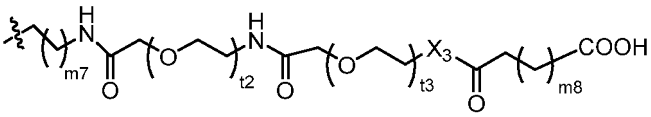 ,

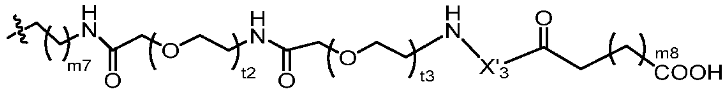 or an amino acid;

preferably from

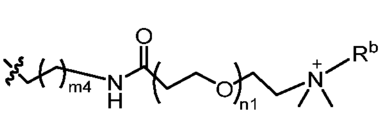 , 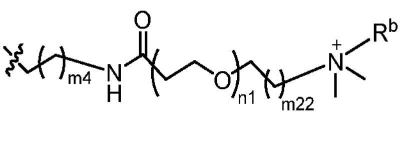 ,

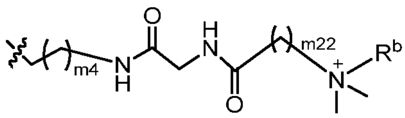, 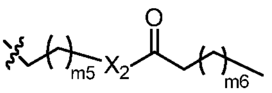, 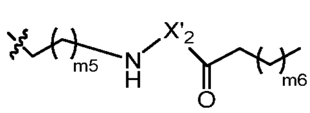,

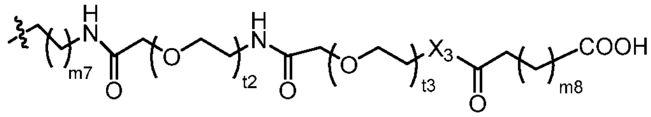

or

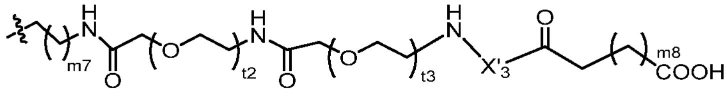;

$R^b$ is selected from H, alkyl,

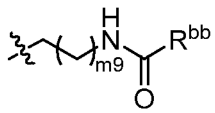

or an amino acid, wherein the alkyl can be further substituted with hydroxyl or alkoxy;
$R^{bb}$ is selected from alkyl,

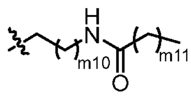,

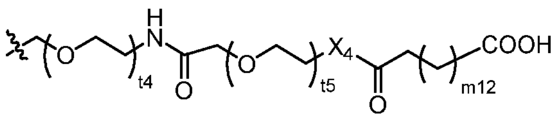,

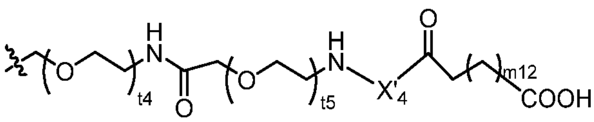

or an amino acid;
$X_2$ is an amino acid;
$X'_2$ is an amino acid;
$X_3$ is an amino acid;
$X'_3$ is an amino acid;
$X_4$ is an amino acid;
$X'_4$ is an amino acid;
n1 is selected from 0, 1, 2, 3, 4, 5, 6, 7, 8, 9, 10, 11, or 12;
t2 is selected from 0, 1, 2, 3, 4, 5, 6, 7, 8, 9, 10, 11, or 12;
t3 is selected from 0, 1, 2, 3, 4, 5, 6, 7, 8, 9, 10, 11, or 12;
t4 is selected from 0, 1, 2, 3, 4, 5, 6, 7, 8, 9, 10, 11, or 12;
t5 is selected from 0, 1, 2, 3, 4, 5, 6, 7, 8, 9, 10, 11, or 12;
m4 is selected from 0, 1, 2, 3, 4, 5, 6, 7, 8, 9, 10, 11, 12, 13, 14, 15, 16, 17, 18, 19, 20, 21, 22, 23, or 24;
m5 is selected from 0, 1, 2, 3, 4, 5, 6, 7, 8, 9, 10, 11, 12, 13, 14, 15, 16, 17, 18, 19, 20, 21, 22, 23, or 24;
m6 is selected from 0, 1, 2, 3, 4, 5, 6, 7, 8, 9, 10, 11, 12, 13, 14, 15, 16, 17, 18, 19, 20, 21, 22, 23, or 24;
m7 is selected from 0, 1, 2, 3, 4, 5, 6, 7, 8, 9, 10, 11, 12, 13, 14, 15, 16, 17, 18, 19, 20, 21, 22, 23, or 24;
m8 is selected from 0, 1, 2, 3, 4, 5, 6, 7, 8, 9, 10, 11, 12, 13, 14, 15, 16, 17, 18, 19, 20, 21, 22, 23, or 24;

m9 is selected from 0, 1, 2, 3, 4, 5, 6, 7, 8, 9, 10, 11, 12, 13, 14, 15, 16, 17, 18, 19, 20, 21, 22, 23, or 24;
m10 is selected from 0, 1, 2, 3, 4, 5, 6, 7, 8, 9, 10, 11, 12, 13, 14, 15, 16, 17, 18, 19, 20, 21, 22, 23, or 24;
m11 is selected from 0, 1, 2, 3, 4, 5, 6, 7, 8, 9, 10, 11, 12, 13, 14, 15, 16, 17, 18, 19, 20, 21, 22, 23, or 24;
m12 is selected from 0, 1, 2, 3, 4, 5, 6, 7, 8, 9, 10, 11, 12, 13, 14, 15, 16, 17, 18, 19, 20, 21, 22, 23, or 24; and
m22 is selected from 0, 1, 2, 3, 4, 5, 6, 7, 8, 9, 10, 11, 12, 13, 14, 15, 16, 17, 18, 19, 20, 21, 22, 23, or 24.

**8.** The compound or the stereoisomer or pharmaceutically acceptable salt thereof according to any one of claims 1 and 2 or 4-7, **characterized in that** $R^4$ is selected from H, $C_{1-6}$ alkyl, $-CH_2C(O)NH_2$,

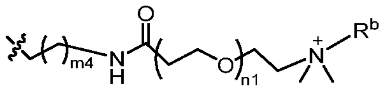
,

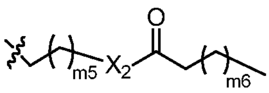
,
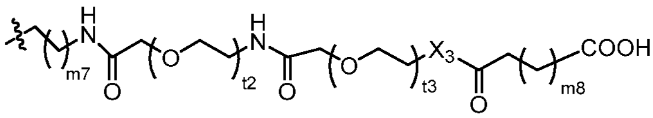

or an amino acid;

$R^b$ is selected from H, $C_{1-6}$ alkyl,

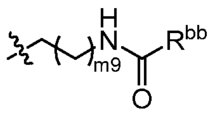

or an amino acid;
$R^{bb}$ is selected from $C_{1-6}$ alkyl,

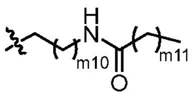
,

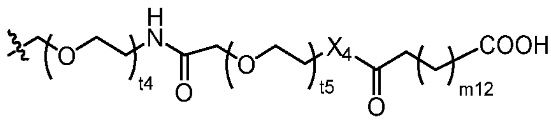

or an amino acid;
$X_2$ is an amino acid;
$X_3$ is an amino acid;
$X_4$ is an amino acid;
preferably, $R^4$ is selected from H, $C_{1-6}$ alkyl, $-CH_2C(O)NH_2$,

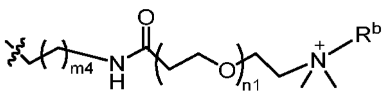
,
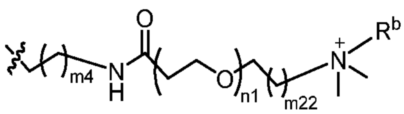
,

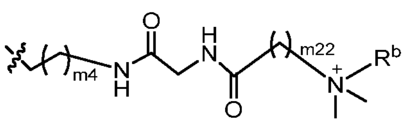
,
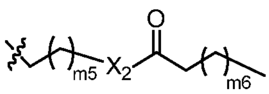
,
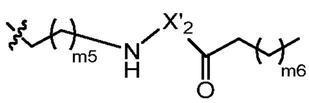
,

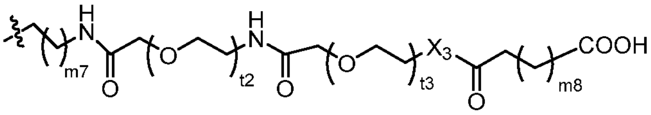

,

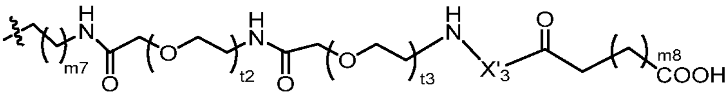

or an amino acid;

$R^b$ is selected from H, $C_{1-6}$ alkyl,

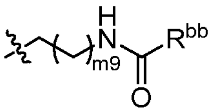

or an amino acid, wherein the alkyl can be further substituted with hydroxyl or alkoxy;

$R^{bb}$ is selected from $C_{1-6}$ alkyl,

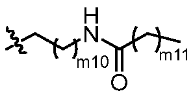

,

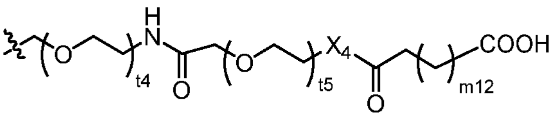

,

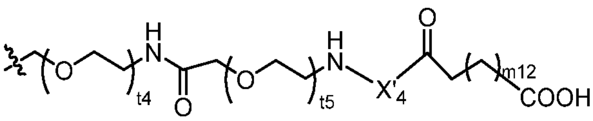

or an amino acid;

$X_2$ is an amino acid;

$X'_2$ is an amino acid;

$X_3$ is an amino acid;

$X'_3$ is an amino acid;

$X_4$ is an amino acid;

$X'_4$ is an amino acid;

n1 is selected from integers of 0-12;

t2-t5 are each independently selected from integers of 0-12;

m4-m12 are each independently selected from integers of 0-24;

m22 is selected from integers of 0-24;

and/or

$R^5$ is selected from H, $C_{1-6}$ alkyl, $-CH_2C(O)NH_2$,

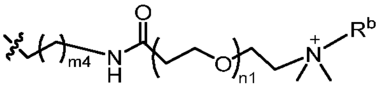

,

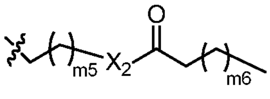

,

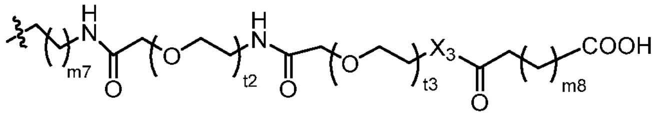

or an amino acid;
$R^b$ is selected from H, $C_{1-6}$ alkyl,

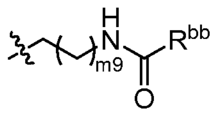

or an amino acid;
$R^{bb}$ is selected from $C_{1-6}$ alkyl,

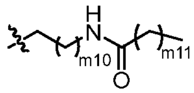

,

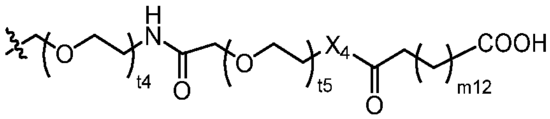

or an amino acid;
$X_2$ is an amino acid;
$X_3$ is an amino acid;
$X_4$ is an amino acid;
preferably, $R^5$ is selected from H, $C_{1-6}$ alkyl, $-CH_2C(O)NH_2$,

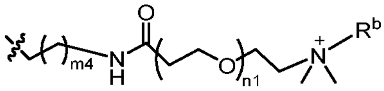 , 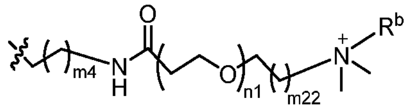 ,

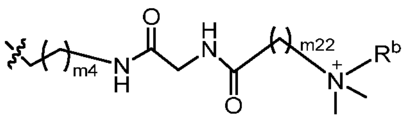 , 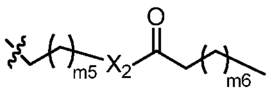 , 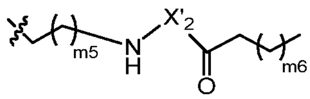 ,

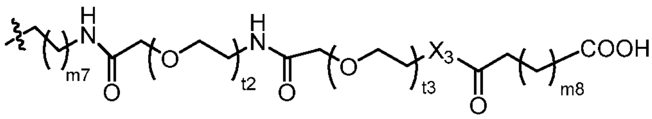

,

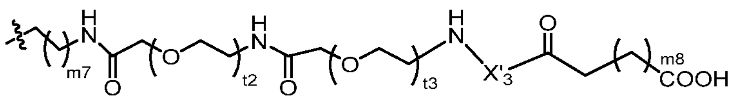

or an amino acid, further preferably from

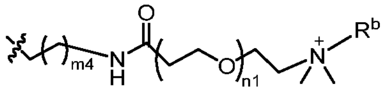 , 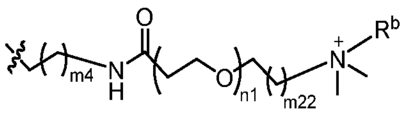 ,

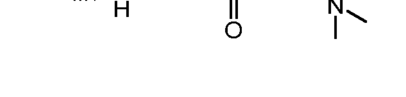 ,  , 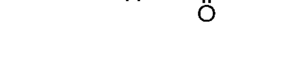 ,

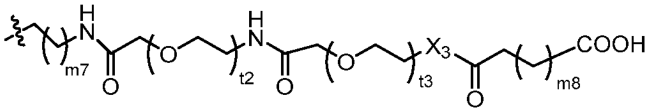

or

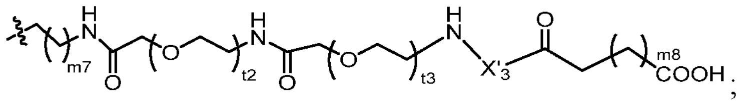

$R^b$ is selected from H, $C_{1-6}$ alkyl,

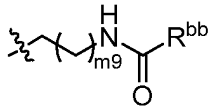

or an amino acid, wherein the alkyl can be further substituted with hydroxyl or alkoxy;
$R^{bb}$ is selected from $C_{1-6}$ alkyl,

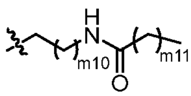

,

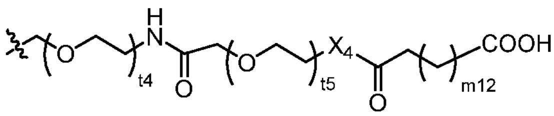

,

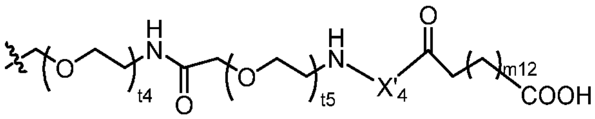

or an amino acid;
$X_2$ is an amino acid;
$X'_2$ is an amino acid;
$X_3$ is an amino acid;
$X'_3$ is an amino acid;
$X_4$ is an amino acid;
$X'_4$ is an amino acid;
n1 is selected from integers of 0-12;
t2-t5 are each independently selected from integers of 0-12;
m4-m12 are each independently selected from integers of 0-24;
m22 is selected from integers of 0-24; preferably,

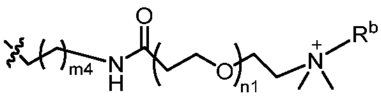

is selected from

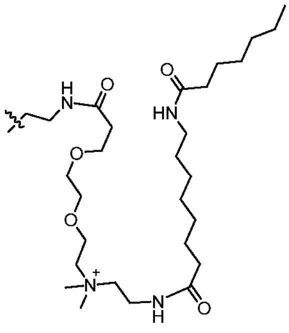
,
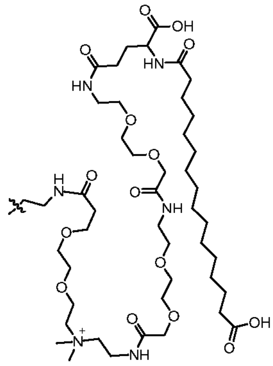
,
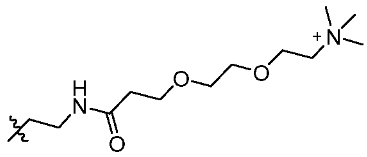

or

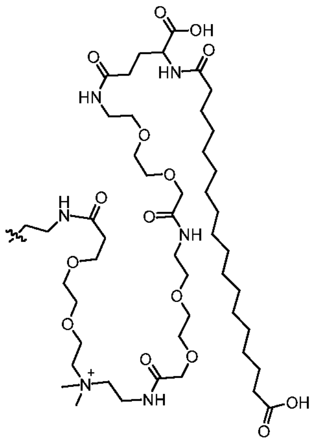
;

preferably

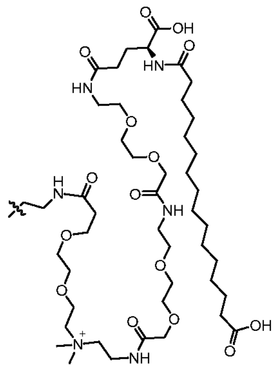
;

and

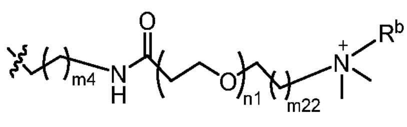

is selected from

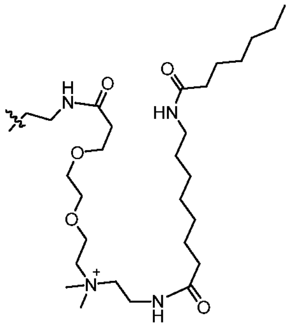,

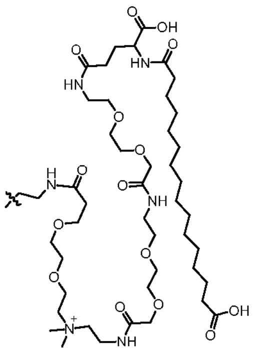, 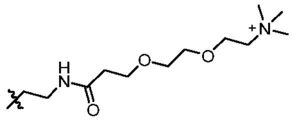, 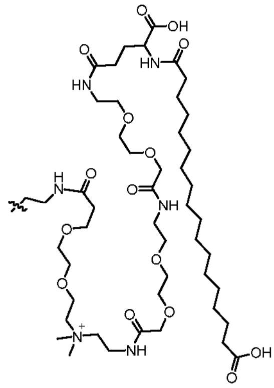,

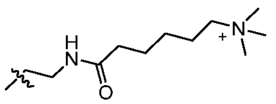, 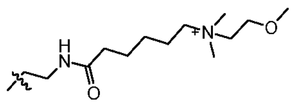, 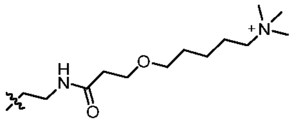,

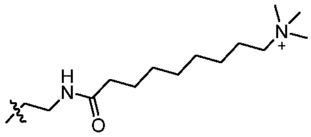 or 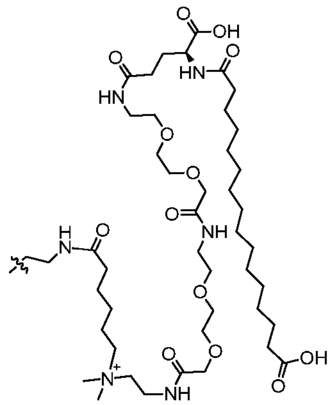;

preferably

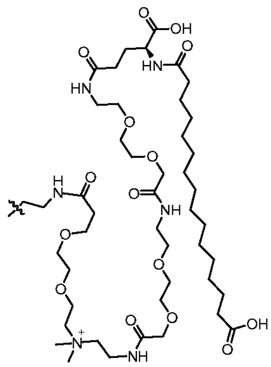.

**9.** A compound or a stereoisomer or pharmaceutically acceptable salt thereof, **characterized in that** the compound is selected from the following compounds:

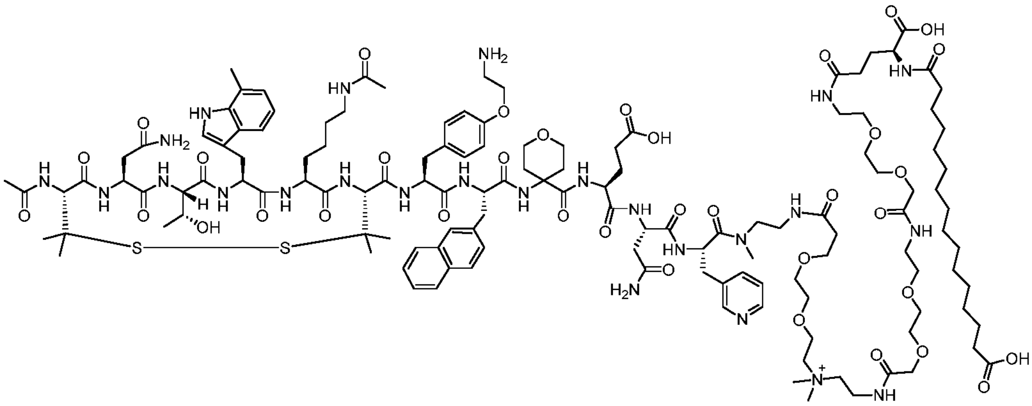

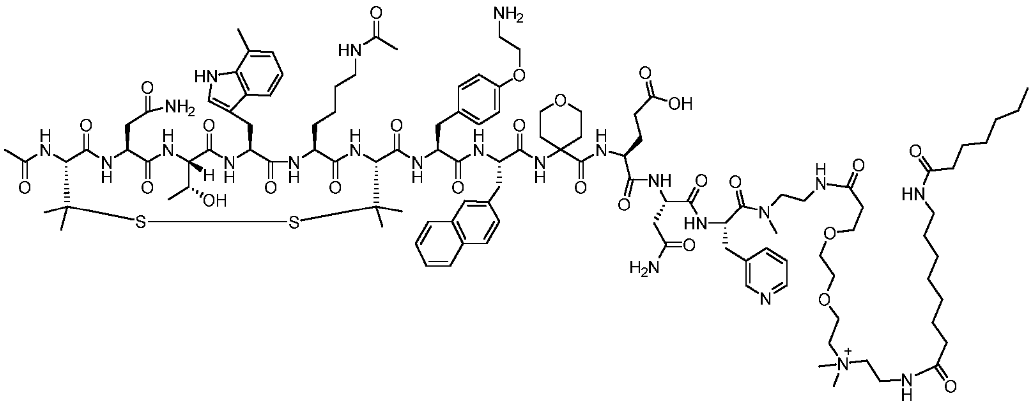

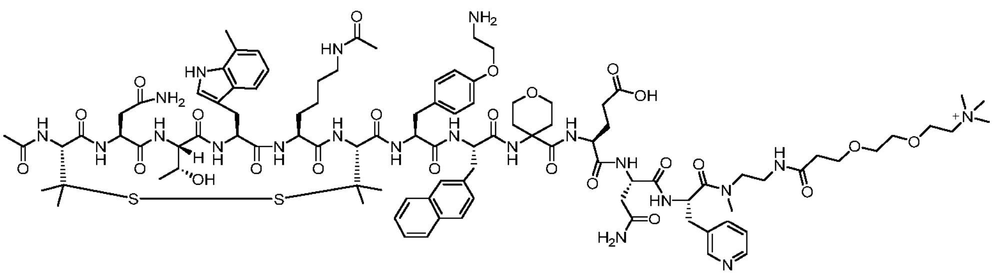

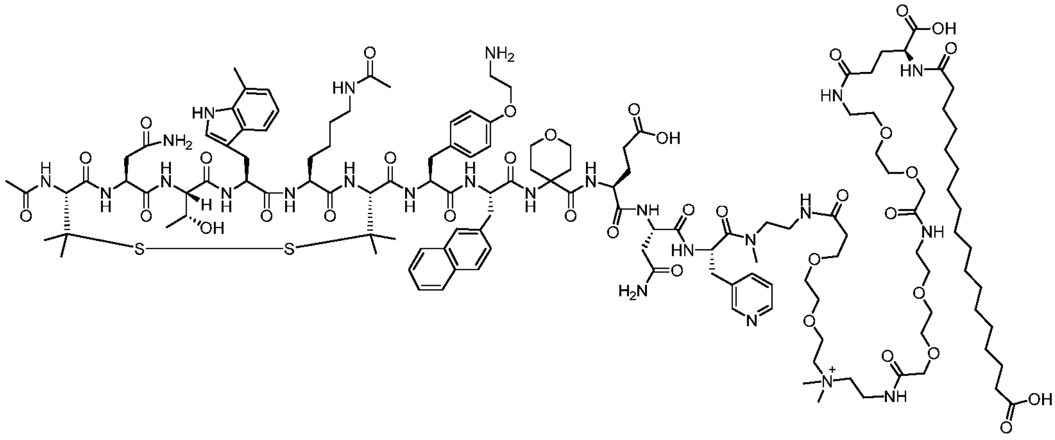

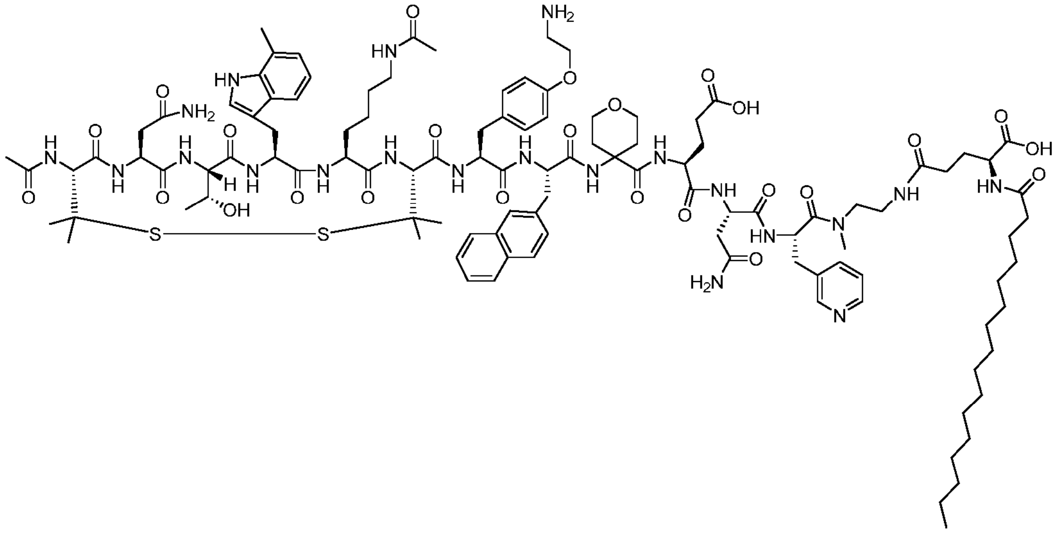

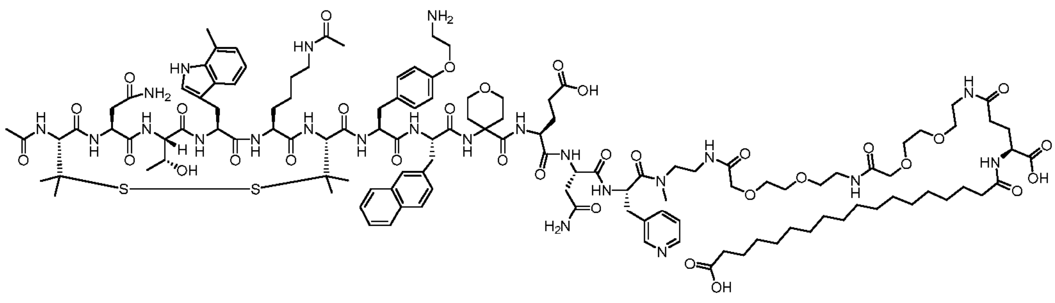

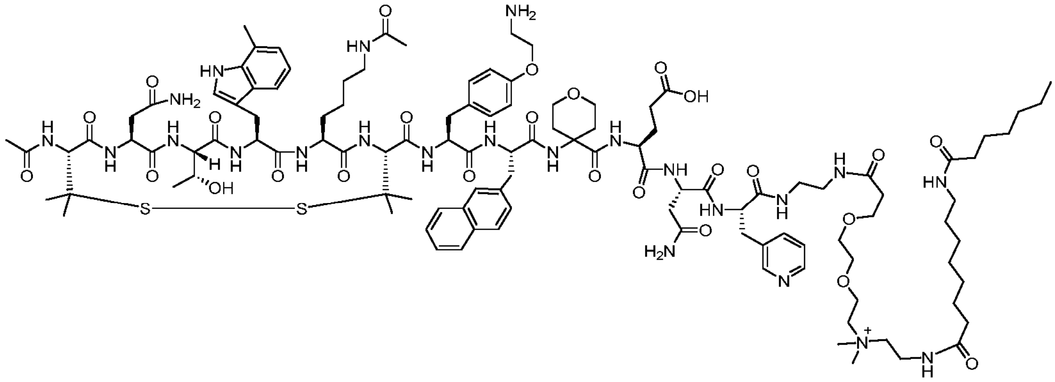

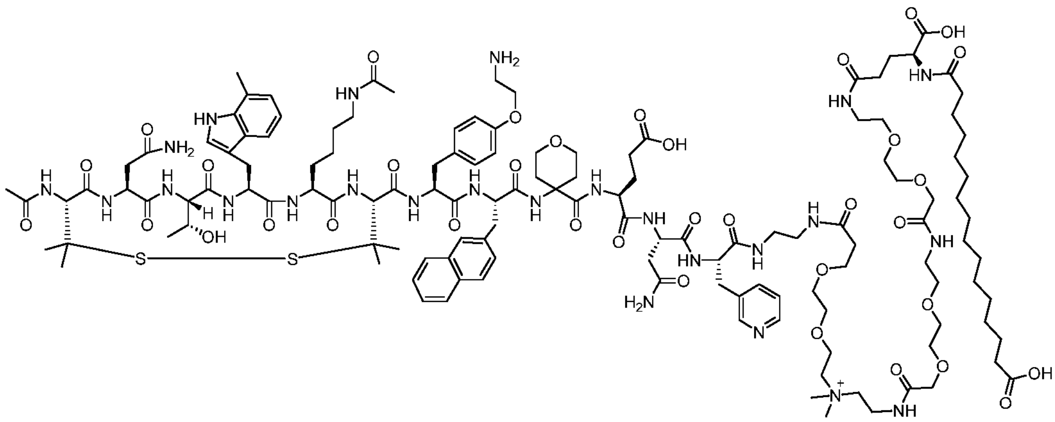

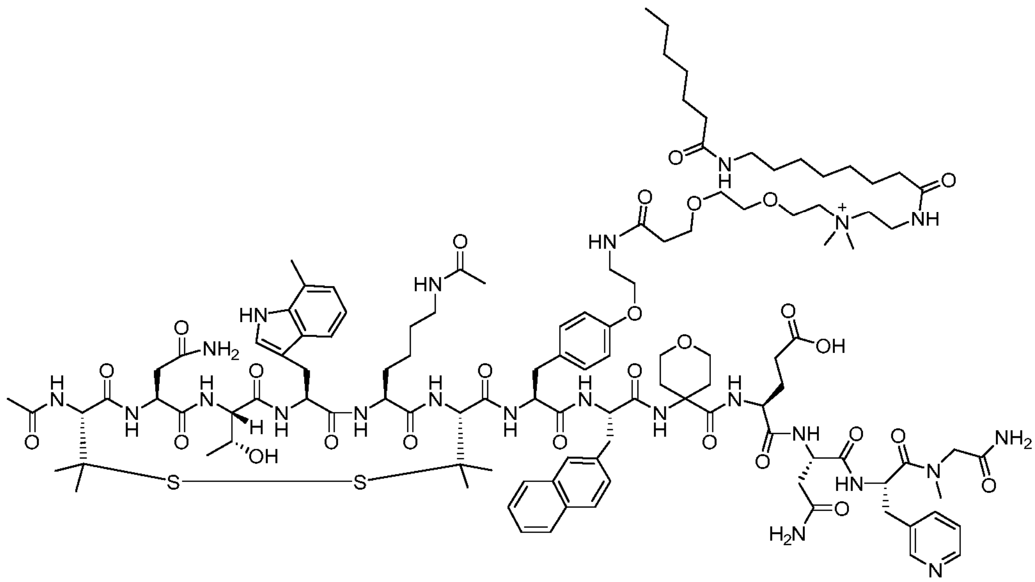

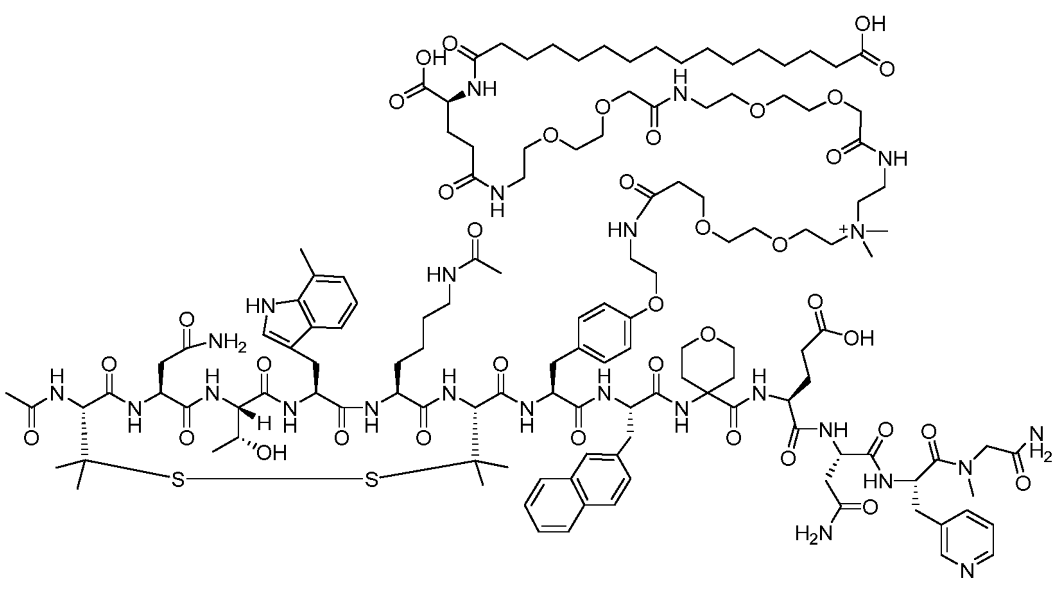

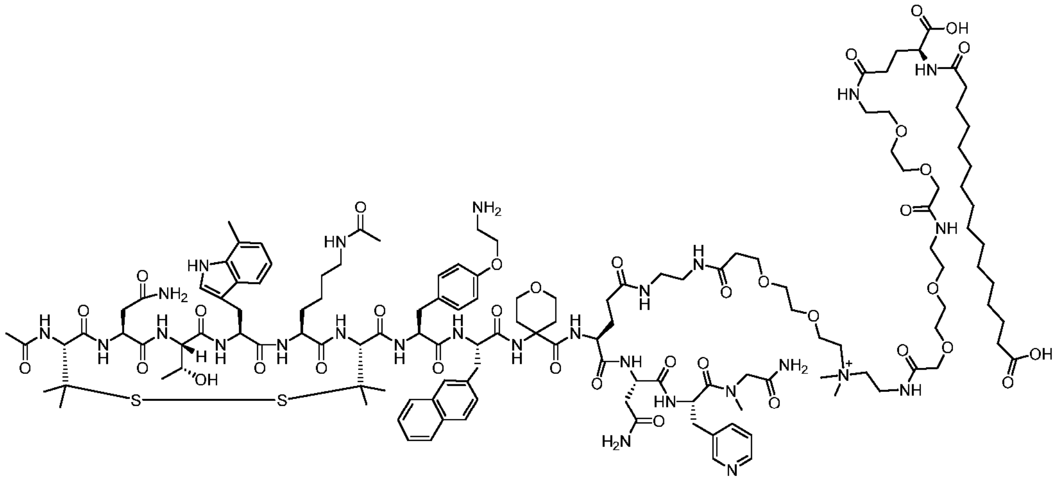

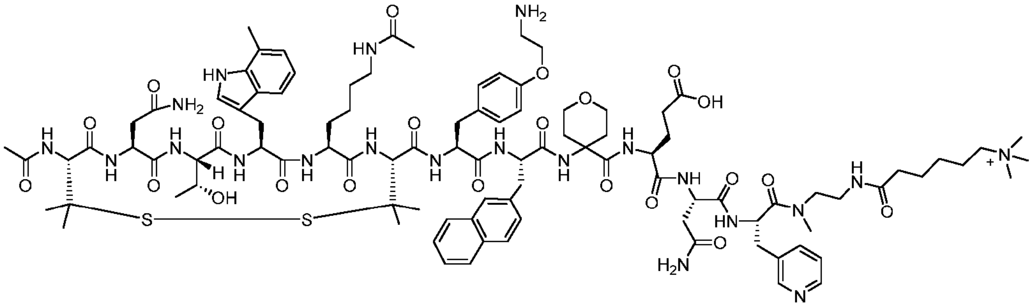

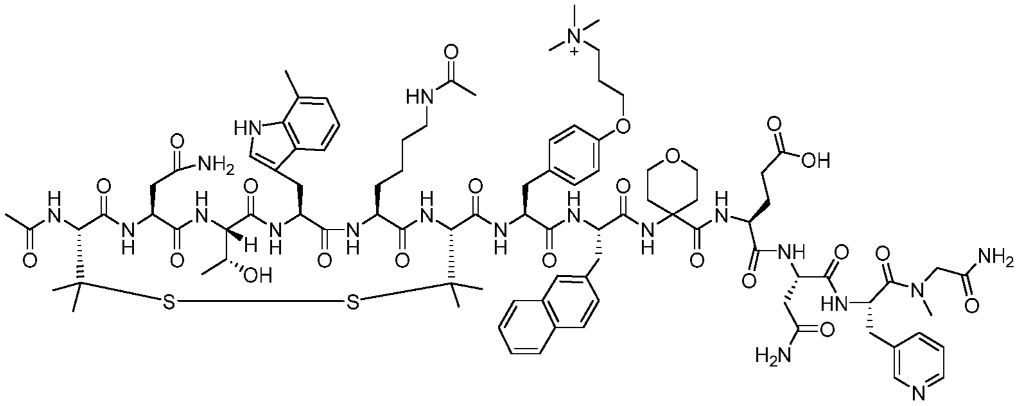

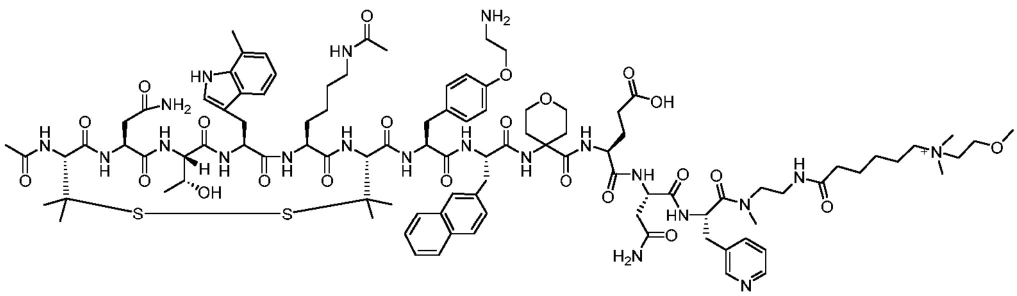

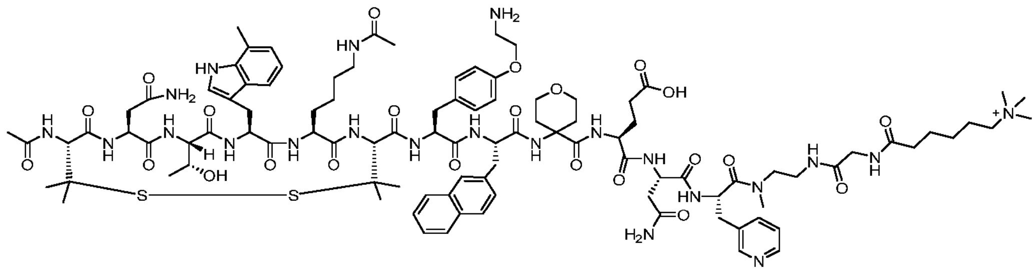

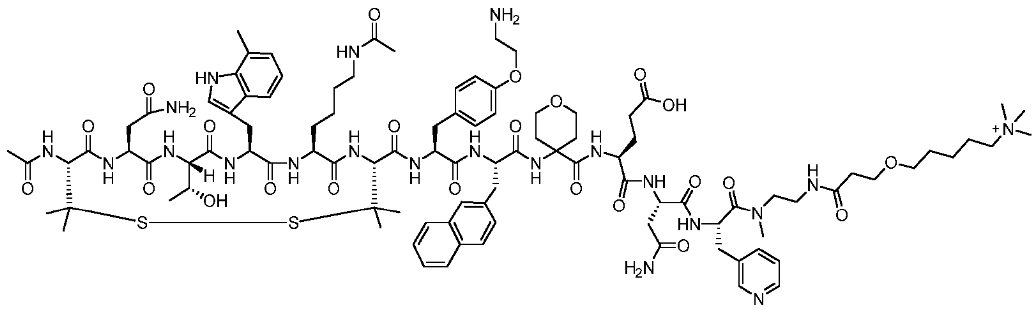

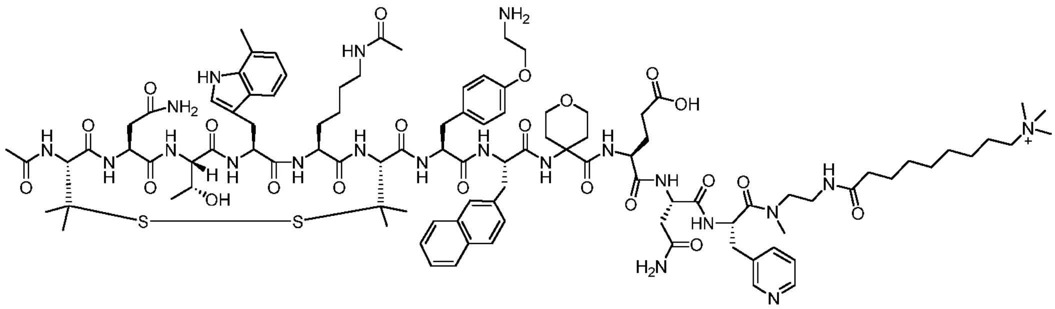

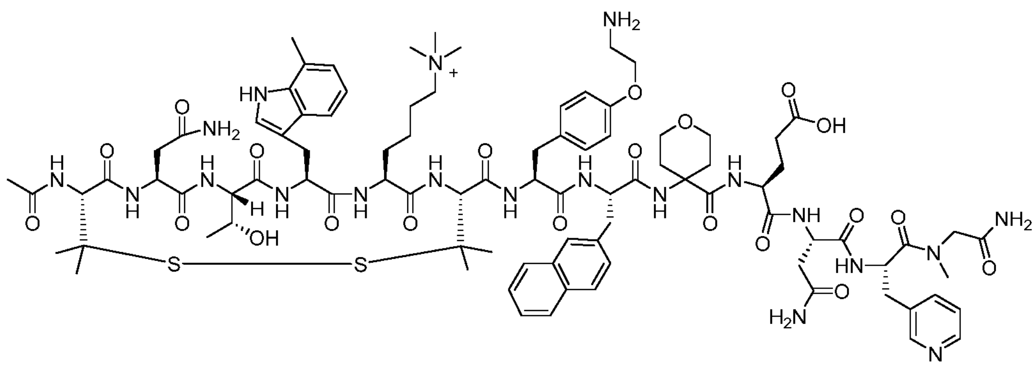

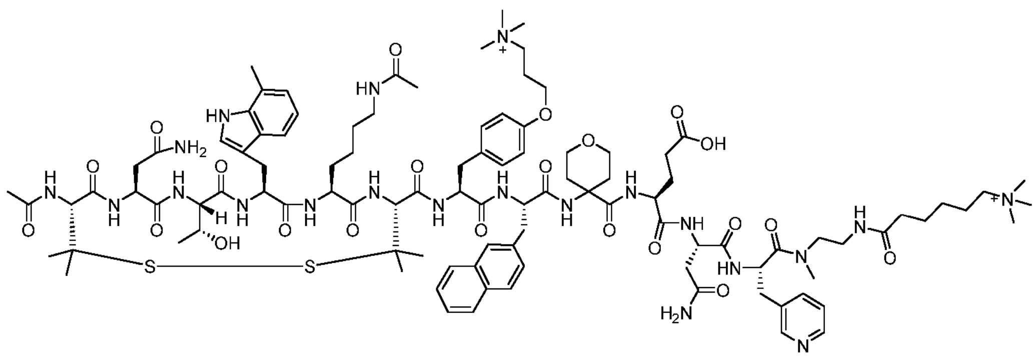

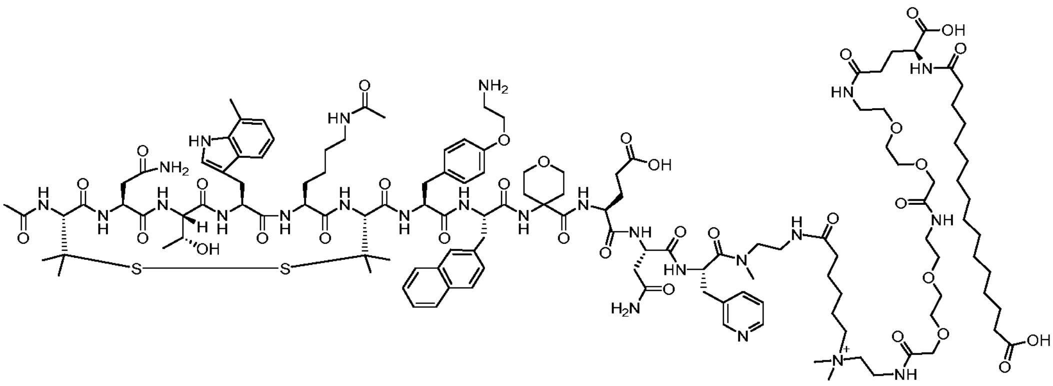

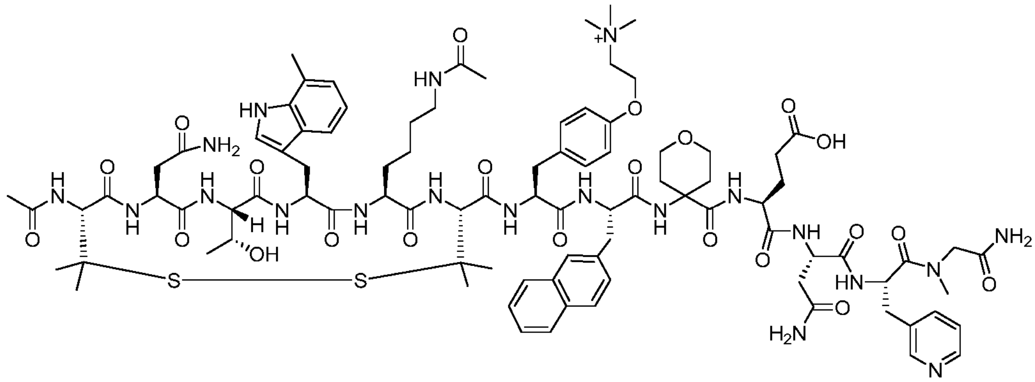

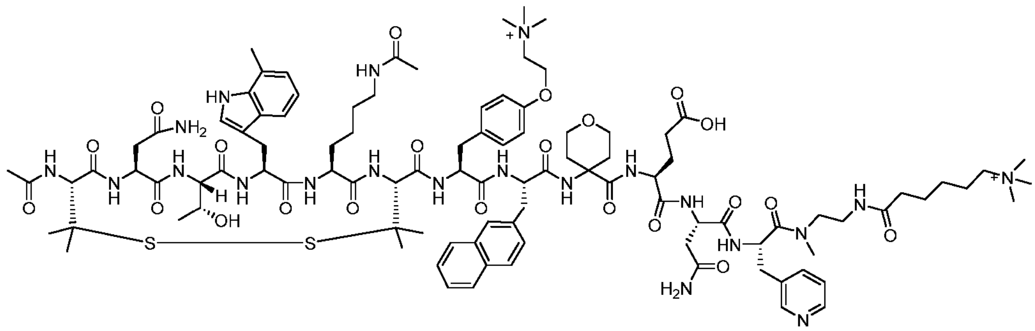

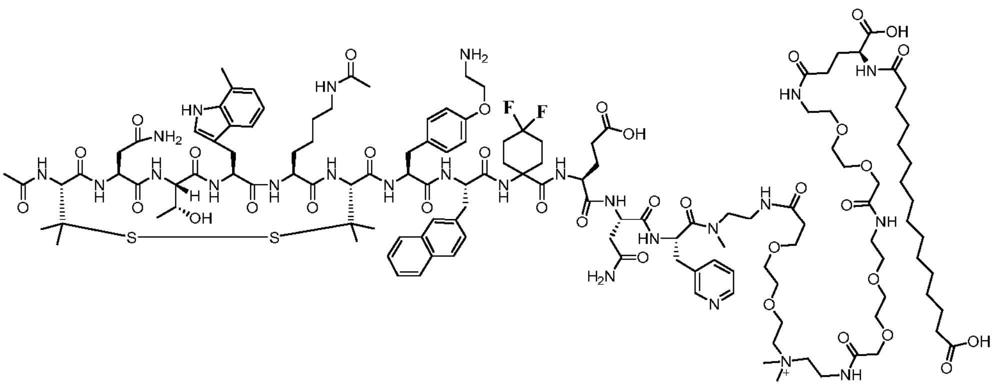

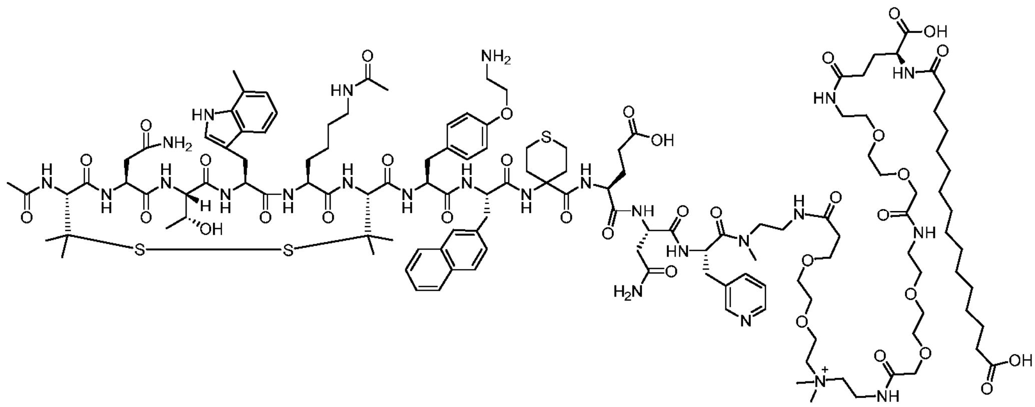

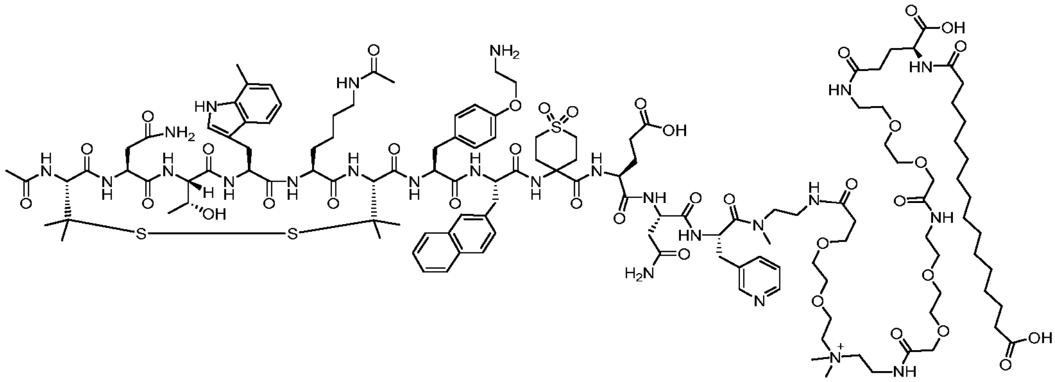

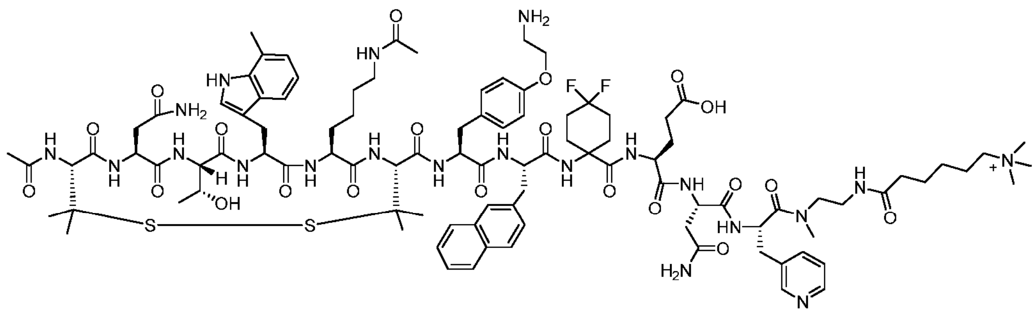

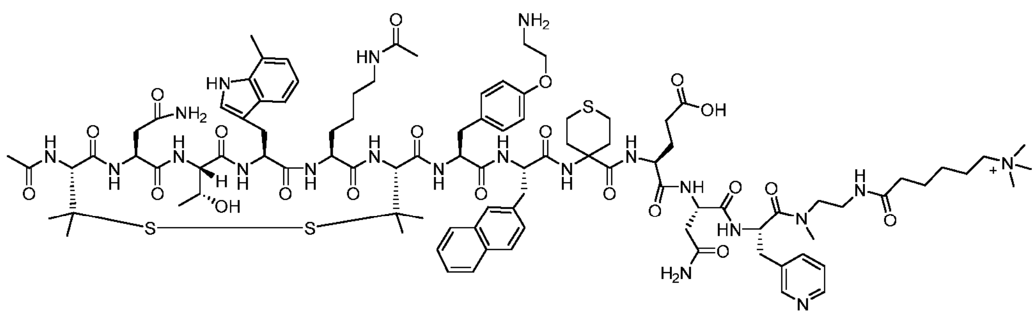

or

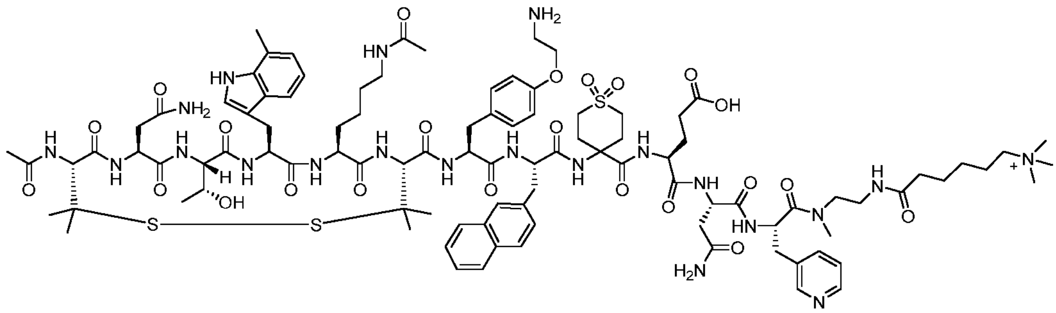

.

10. A method for preparing the compound or the stereoisomer or pharmaceutically acceptable salt thereof according to any one of claims 1-9, **characterized in that** the method is based on solid-phase or liquid-phase synthesis;

preferably, the synthesis method comprises the following steps:

1) synthesizing a resin peptide based on a solid-phase synthesis method;
2) cleaving the resin peptide obtained in step 1) to obtain a polypeptide intermediate;
3) subjecting the polypeptide intermediate to a condensation reaction with a side chain; and
4) removing a protective group from a peptide fragment obtained in step 3) and then performing cyclization to obtain the final product;

further preferably, the synthesis method comprises the following steps:

1) based on a solid-phase synthesis method of an Fmoc method, synthesizing a resin peptide and capping the resin peptide with acetic anhydride;
2) cleaving the resin peptide obtained in step 1) to obtain a polypeptide intermediate;
3) subjecting the polypeptide intermediate to a condensation reaction with a side chain by using a coupling agent; and
4) removing a protective group from a peptide fragment obtained in step 3) and then performing cyclization by oxidizing a disulfide bond to obtain the final product.

11. A pharmaceutical composition, **characterized by** comprising a therapeutically effective dose of the compound or the stereoisomer or pharmaceutically acceptable salt thereof according to any one of claims 1-9, and one or more pharmaceutically acceptable carriers, diluents, or excipients.

12. Use of the compound or the stereoisomer or pharmaceutically acceptable salt thereof according to any one of claims 1-9 or the pharmaceutical composition according to claim 11 in the preparation of an IL-23R inhibitor drug.

13. Use of the compound or the stereoisomer or pharmaceutically acceptable salt thereof according to any one of claims 1-9 or the pharmaceutical composition according to claim 11 in the preparation of a drug for treating inflammatory and autoimmune diseases and cancers, e.g., inflammatory bowel disease (IBD), ulcerative colitis, Crohn's disease, celiac disease (nontropical sprue), enteropathy associated with seronegative arthropathy, microscopic colitis, collagenous colitis, eosinophilic gastroenteritis, colitis associated with radiotherapy or chemotherapy, colitis associated with congenital immune conditions such as leukocyte adhesion deficiency-1, chronic granulomatosis, hepatic glycogen storage disease type 1b, Hermansky-Pudlak syndrome, Chediak-Higashi syndrome and Wiskott-Aldrich syndrome, pouchitis following proctocolectomy and ileoanal anastomosis, gastrointestinal cancer, pancreatitis, insulin-dependent diabetes mellitus, mastitis, cholecystitis, cholangitis, pericholangitis, chronic bronchitis, chronic sinusitis, asthma, psoriasis, psoriatic arthritis, irritable bowel syndrome (IBS), multiple sclerosis (MS), psoriasis, psoriatic arthritis, rheumatoid arthritis, pemphigus vulgaris, organ transplant rejection, Crohn's disease, systemic lupus erythematosus (SLE), or diabetic disease; preferably inflammatory bowel disease (IBD), rheumatoid arthritis, or psoriasis.

**INTERNATIONAL SEARCH REPORT**

International application No.
**PCT/CN2023/135637**

**A. CLASSIFICATION OF SUBJECT MATTER**

C07K7/64(2006.01)i; C07K1/00(2006.01)i; A61P1/00(2006.01)i; A61P11/00(2006.01)i; A61P17/06(2006.01)i; A61P19/02(2006.01)i; A61P35/00(2006.01)i; A61P37/00(2006.01)i; A61K38/00(2006.01)i; A61K38/12(2006.01)i

According to International Patent Classification (IPC) or to both national classification and IPC

**B. FIELDS SEARCHED**

Minimum documentation searched (classification system followed by classification symbols)

IPC:C07K,A61P,A61K

Documentation searched other than minimum documentation to the extent that such documents are included in the fields searched

Electronic data base consulted during the international search (name of data base and, where practicable, search terms used)

DWPI, VEN, CNABS, CNTXT, ENTXT, PUBMED, ISI Web of Science, CNKI, 万方数据知识服务平台, Wanfang Data Knowledge Service Platform, 百度学术, BAIDU SCHOLAR, STN: 通式(I), 白介素23, 抑制剂, 拮抗, 拮抗物, IL23, IL23R, IL-23, +NTWK+, inhibitor?, 环肽, cyclic-peptide, pen-asn-thr-trp-lys, 2nal, 3-萘基-L-丙氨酸, thp等.

**C. DOCUMENTS CONSIDERED TO BE RELEVANT**

| Category* | Citation of document, with indication, where appropriate, of the relevant passages | Relevant to claim No. |
|---|---|---|
| X | CN 115298196 A (CENTOCOR ORTHO BIOTECH, INC.; PROTAGONIST THERAPEUTICS INC.) 04 November 2022 (2022-11-04) abstract, claims 176 and 179, and description, paragraphs [1003], [1029], [1131]-[1143], [1623], and [1640], SEQ ID NO:104, 121, and tables 1, 2, 4, and 8 | 1-13 |
| X | WO 2022109328 A1 (JANSSEN PHARMACEUTICA NV; PROTAGONIST THERAPEUTICS INC.) 27 May 2022 (2022-05-27) abstract, claims 1, 2, and 70, and description, paragraph [0436], and SEQ ID NO:1 | 1, 3, 4, 7, 8, 10-13 |
| A | CN 107206254 A (PROTAGONIST THERAPEUTICS INC.) 26 September 2017 (2017-09-26) abstract, and claims 11, 16, and 41 | 1-13 |
| A | CN 113563423 A (PROTAGONIST THERAPEUTICS INC.) 29 October 2021 (2021-10-29) abstract, and claims 16 and 41 | 1-13 |
| A | CN 115279782 A (CENTOCOR ORTHO BIOTECH, INC.; PROTAGONIST THERAPEUTICS INC.) 01 November 2022 (2022-11-01) abstract, and claims 101-118 | 1-13 |

☐ Further documents are listed in the continuation of Box C.    ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 March 2024** | **16 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/135637**

| Patent document cited in search report | Publication date (day/month/year) | Patent family member(s) | Publication date (day/month/year) |
|---|---|---|---|
| CN 115298196 A | 04 November 2022 | BR 112022013957 A2 | 11 October 2022 |
| | | AR 121070 A1 | 13 April 2022 |
| | | KR 20220141807 A | 20 October 2022 |
| | | PE 20230370 A1 | 06 March 2023 |
| | | AU 2021207653 A1 | 04 August 2022 |
| | | JP 2023139158 A | 03 October 2023 |
| | | MX 2022008741 A | 03 October 2022 |
| | | TW 202140517 A | 01 November 2021 |
| | | CL 2022001893 A1 | 03 March 2023 |
| | | WO 2021146441 A1 | 22 July 2021 |
| | | CO 2022009931 A2 | 21 October 2022 |
| | | JP 2023511552 A | 20 March 2023 |
| | | DOP 2022000147 A | 15 January 2023 |
| | | CA 3167751 A1 | 22 July 2021 |
| | | EP 4090670 A1 | 23 November 2022 |
| | | IL 294680 A | 01 September 2022 |
| | | JOP 20220169 A1 | 30 January 2023 |
| | | US 2021261622 A1 | 26 August 2021 |
| | | US 11845808 B2 | 19 December 2023 |
| | | CR 20220332 A | 28 November 2022 |
| | | ECSP 22063238 A | 30 September 2022 |
| WO 2022109328 A1 | 27 May 2022 | CO 2023007172 A2 | 20 June 2023 |
| | | CA 3202226 A1 | 27 May 2022 |
| | | TW 202237167 A | 01 October 2022 |
| | | CL 2023001417 A1 | 15 December 2023 |
| | | AU 2021383828 A1 | 06 July 2023 |
| | | EP 4247403 A1 | 27 September 2023 |
| | | IL 302996 A | 01 July 2023 |
| | | UY 39527 A | 30 June 2022 |
| | | US 2022177532 A1 | 09 June 2022 |
| | | DOP 2023000091 A | 29 December 2023 |
| | | KR 20230110570 A | 24 July 2023 |
| | | JP 2023545212 A | 26 October 2023 |
| | | JP 7397239 B2 | 12 December 2023 |
| CN 107206254 A | 26 September 2017 | US 2021363185 A1 | 25 November 2021 |
| | | US 11884748 B2 | 30 January 2024 |
| | | JP 2017530090 A | 12 October 2017 |
| | | SG 10201810154 WA | 28 December 2018 |
| | | US 2018079783 A1 | 22 March 2018 |
| | | US 10023614 B2 | 17 July 2018 |
| | | CA 2955460 A1 | 21 January 2016 |
| | | BR 112017001010 A2 | 14 November 2017 |
| | | US 2018022778 A1 | 25 January 2018 |
| | | US 10196424 B2 | 05 February 2019 |
| | | US 2018079782 A1 | 22 March 2018 |
| | | US 10035824 B2 | 31 July 2018 |
| | | SG 11201700327 WA | 27 February 2017 |
| | | KR 20170033372 A | 24 March 2017 |
| | | KR 102482790 B1 | 29 December 2022 |
| | | US 2016145306 A1 | 26 May 2016 |

Form PCT/ISA/210 (patent family annex) (July 2022)

**INTERNATIONAL SEARCH REPORT**
**Information on patent family members**

International application No.
**PCT/CN2023/135637**

| Patent document cited in search report | Publication date (day/month/year) | Patent family member(s) | Publication date (day/month/year) |
|---|---|---|---|
| | | US 9624268 B2 | 18 April 2017 |
| | | WO 2016011208 A1 | 21 January 2016 |
| | | JP 2020128443 A | 27 August 2020 |
| | | JP 6995933 B2 | 21 February 2022 |
| | | US 2019337983 A1 | 07 November 2019 |
| | | US 10941183 B2 | 09 March 2021 |
| | | IL 285614 A | 30 September 2021 |
| | | AU 2015289642 A1 | 02 February 2017 |
| | | AU 2015289642 B2 | 25 February 2021 |
| | | RU 2017105115 A | 17 August 2018 |
| | | RU 2017105115 A3 | 21 January 2019 |
| | | RU 2736637 C2 | 19 November 2020 |
| | | RU 2736637 C9 | 08 February 2021 |
| | | EP 3169403 A1 | 24 May 2017 |
| | | EP 3169403 A4 | 24 January 2018 |
| | | EP 3169403 B1 | 14 February 2024 |
| | | IL 250111 A0 | 30 March 2017 |
| | | IL 250111 B | 31 August 2021 |
| CN 113563423 A | 29 October 2021 | None | |
| CN 115279782 A | 01 November 2022 | EP 4090669 A1 | 23 November 2022 |
| | | JP 2023511551 A | 20 March 2023 |
| | | WO 2021146454 A1 | 22 July 2021 |
| | | US 2022402983 A1 | 22 December 2022 |
| | | KR 20220141808 A | 20 October 2022 |
| | | BR 112022014011 A2 | 20 December 2022 |
| | | AU 2021209086 A1 | 04 August 2022 |
| | | CA 3168135 A1 | 22 July 2021 |
| | | MX 2022008740 A | 23 September 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2022115304381
- CN 2023102062995
- CN 2023102618188
- CN 202310349519X
- CN 2023111444895
- WO 2021127460 A
- WO 2021041770 A

### Non-patent literature cited in the description

- *CHEMICAL ABSTRACTS*, 13855-29-3
- *CHEMICAL ABSTRACTS*, 137780-61-1
- *CHEMICAL ABSTRACTS*, 1986905-26-3